(19) Europäisches Patentamt
European Patent Office
Office européen des brevets

(11) EP 2 074 219 B1

(12) **EUROPEAN PATENT SPECIFICATION**

(45) Date of publication and mention of the grant of the patent:
**20.11.2013 Bulletin 2013/47**

(21) Application number: **08716886.0**

(22) Date of filing: **15.02.2008**

(51) Int Cl.:
***C12N 15/82*** *(2006.01)* ***A01H 5/00*** *(2006.01)*

(86) International application number:
**PCT/EP2008/051882**

(87) International publication number:
**WO 2008/099013 (21.08.2008 Gazette 2008/34)**

(54) **NUCLEIC ACID SEQUENCES FOR REGULATION OF EMBRYO-SPECIFIC EXPRESSION IN MONOCOTYLEDONOUS PLANTS**

NUKLEINSÄURESEQUENZEN ZUR REGULIERUNG EMBRYOSPEZIFISCHER EXPRESSION IN EINKEIMBLÄTTRIGEN PFLANZEN

SÉQUENCES D’ACIDES NUCLÉIQUES POUR LA RÉGULATION DE L’EXPRESSION SPÉCIFIQUE DE L’EMBRYON DANS DES PLANTES MONOCOTYLES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **16.02.2007 US 890217 P**

(43) Date of publication of application:
**01.07.2009 Bulletin 2009/27**

(73) Proprietor: **BASF Plant Science GmbH**
**67056 Ludwigshafen (DE)**

(72) Inventors:
- **SONG, Hee-Sook**
  **Raleigh, NC 27606 (US)**
- **DAMMANN, Christian**
  **Durham, NC 27705 (US)**

(74) Representative: **Popp, Andreas**
**BASF SE**
**Global Intellectual Property**
**GVX - C6**
**67056 Ludwigshafen (DE)**

(56) References cited:
**WO-A-2005/103075 WO-A-2006/034479**
**WO-A-2006/094976**

- **AL-ABED ET AL: "Expression of CBF3 under the stress inducible promoter Rd29A using split-seed explant to enhance drought and cold tolerance in maize" 20050606, 6 June 2005 (2005-06-06), page 4, XP002477739**

EP 2 074 219 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> BASF Plant Science GmbH song, Hee-sook Dammann, Christian

<120> Nucleic acid sequences for regulation of embryo-specific expression in monocotyledonous plants

<130> PF58489

<160> 144

<170> PatentIn version 3.3

<210> 1
<211> 833
<212> DNA
<213> Arabidopsis thaliana

<220>
<221> promoter
<222> (1)..(833)
<223> Arabidopsis cor78 promoter regionTATA box: nt 790-804

<400> 1

```
gcaagaatct caaacacgga gatctcaaag tttgaaagaa aatttatttc ttcgactcaa      60
aacaaactta cgaaatttag gtagaactta tatacattat attgtaattt tttgtaacaa     120
aatgttttta ttattattat agaattttac tggttaaatt aaaaatgaat agaaaaggtg     180
aattaagagg agagaggagg taaacatttt cttctatttt ttcatatttt caggataaat     240
tattgtaaaa gtttacaaga tttccatttg actagtgtaa atgaggaata ttctctagta     300
agatcattat ttcatctact tcttttatct tctaccagta gaggaataaa caatatttag     360
ctcctttgta aatacaaatt aattttcgtt cttgacatca ttcaatttta attttacgta     420
taaaataaaa gatcatacct attagaacga ttaaggagaa atacaattcg aatgagaagg     480
atgtgccgct tgttataata aacagccaca cgacgtaaac gtaaaatgac cacatgatgg     540
gccaatagac atggaccgac tactaataat agtaagttac attttaggat ggaataaata     600
tcataccgac atcagtttga aagaaaaggg aaaaaaagaa aaaataaata aaagatatac     660
taccgacatg agttccaaaa agcaaaaaaa aagatcaagc cgacacagac acgcgtagag     720
agcaaaatga ctttgacgtc acaccacgaa aacagacgct tcatacgtgt ccctttatct     780
ctctcagtct ctctataaac ttagtgagac cctcctctgt tttactcaca aat            833
```

<210> 2
<211> 15553
<212> DNA
<213> unknown

<220>
<223> this is the construct

<220>
<221> misc_feature
<222> (197)..(221)
<223> left T-DNA border repeat

<220>
<221> terminator
<222> (5811)..(6063)
<223> NOS terminator

<220>
<221> misc_feature
<222> (6134)..(8134)
<223> GUS coding sequence

<220>
<221> Intron
<222> (8199)..(8781)
<223> BPS1.1 intron

<220>
<221> promoter
<222> (8813)..(9656)
<223> Cor78 promoter

<220>
<221> misc_feature
<222> (9703)..(9726)
<223> Right T-DNA border repeat

<400> 2

atgttgattg taacgatgac agagcgttgc tgcctgtgat caaatatcat ctccctcgca 60
gagatccgaa ttatcagcct tcttattcat ttctcgctta accgtgacag gctgtcgatc 120
ttgagaacta tgccgacata ataggaaatc gctggataaa gccgctgagg aagctgagtg 180
gcgctatttc tttagaagtg aacgttgacg atcgtcgacc gtaccccgat gaattaattc 240
ggacgtacgt tctgaacaca gctggatact tacttgggcg attgtcatac atgacatcaa 300
caatgtaccc gtttgtgtaa ccgtctcttg gaggttcgta tgacactagt ggttcccctc 360
agcttgcgac tagatgttga ggcctaacat tttattagag agcaggctag ttgcttagat 420
acatgatctt caggccgtta tctgtcaggg caagcgaaaa ttggccattt atgacgacca 480
atgccccgca gaagctccca tctttgccgc catagacgcc gcgccccct tttggggtgt 540
agaacatcct tttgccagat gtggaaaaga agttcgttgt cccattgttg gcaatgacgt 600
agtagccggc gaaagtgcga gacccatttg cgctatatat aagcctacga tttccgttgc 660
gactattgtc gtaattggat gaactattat cgtagttgct ctcagagttg tcgtaatttg 720
atggactatt gtcgtaattg cttatggagt tgtcgtagtt gcttggagaa atgtcgtagt 780
tggatgggga gtagtcatag ggaagacgag cttcatccac taaaacaatt ggcaggtcag 840
caagtgcctg ccccgatgcc atcgcaagta cgaggcttag aaccaccttc aacagatcgc 900
gcatagtctt ccccagctct ctaacgcttg agttaagccg cgccgcgaag cggcgtcggc 960
ttgaacgaat tgttagacat tatttgccga ctaccttggt gatctcgcct ttcacgtagt 1020
gaacaaattc ttccaactga tctgcgcgcg aggccaagcg atcttcttgt ccaagataag 1080
cctgcctagc ttcaagtatg acgggctgat actgggccgg caggcgctcc attgcccagt 1140
cggcagcgac atccttcggc gcgattttgc cggttactgc gctgtaccaa atgcgggaca 1200
acgtaagcac tacatttcgc tcatcgccag cccagtcggg cggcgagttc catagcgtta 1260

aggtttcatt tagcgcctca aatagatcct gttcaggaac cggatcaaag agttcctccg 1320
ccgctggacc taccaaggca acgctatgtt ctcttgcttt tgtcagcaag atagccagat 1380
caatgtcgat cgtggctggc tcgaagatac ctgcaagaat gtcattgcgc tgccattctc 1440
caaattgcag ttcgcgctta gctggataac gccacggaat gatgtcgtcg tgcacaacaa 1500
tggtgacttc tacagcgcgg agaatctcgc tctctccagg ggaagccgaa gtttccaaaa 1560
ggtcgttgat caaagctcgc cgcgttgttt catcaagcct tacggtcacc gtaaccagca 1620
aatcaatatc actgtgtggc ttcaggccgc catccactgc ggagccgtac aaatgtacgg 1680
ccagcaacgt cggttcgaga tggcgctcga tgacgccaac tacctctgat agttgagtcg 1740
atacttcggc gatcaccgct tccctcatga tgtttaactc ctgaattaag ccgcgccgcg 1800
aagcggtgtc ggcttgaatg aattgttagg cgtcatcctg tgctcccgag aaccagtacc 1860
agtacatcgc tgtttcgttc gagacttgag gtctagtttt atacgtgaac aggtcaatgc 1920
cgccgagagt aaagccacat tttgcgtaca aattgcaggc aggtacattg ttcgtttgtg 1980
tctctaatcg tatgccaagg agctgtctgc ttagtgccca ctttttcgca aattcgatga 2040
gactgtgcgc gactcctttg cctcggtgcg tgtgcgacac aacaatgtgt tcgatagagg 2100
ctagatcgtt ccatgttgag ttgagttcaa tcttcccgac aagctcttgg tcgatgaatg 2160
cgccatagca agcagagtct tcatcagagt catcatccga gatgtaatcc ttccggtagg 2220
ggctcacact tctggtagat agttcaaagc cttggtcgga taggtgcaca tcgaacactt 2280
cacgaacaat gaaatggttc tcagcatcca atgtttccgc cacctgctca gggatcaccg 2340
aaatcttcat atgacgccta acgcctggca cagcggatcg caaacctggc gcggcttttg 2400
gcacaaaagg cgtgacaggt ttgcgaatcc gttgctgcca cttgttaacc cttttgccag 2460
atttggtaac tataatttat gttagaggcg aagtcttggg taaaaactgg cctaaaattg 2520
ctggggattt caggaaagta aacatcacct tccggctcga tgtctattgt agatatatgt 2580
agtgtatcta cttgatcggg ggatctgctg cctcgcgcgt ttcggtgatg acggtgaaaa 2640
cctctgacac atgcagctcc cggagacggt cacagcttgt ctgtaagcgg atgccgggag 2700
cagacaagcc cgtcagggcg cgtcagcggg tgttggcggg tgtcggggcg cagccatgac 2760
ccagtcacgt agcgatagcg gagtgtatac tggcttaact atgcggcatc agagcagatt 2820
gtactgagag tgcaccatat gcggtgtgaa ataccgcaca gatgcgtaag gagaaaatac 2880
cgcatcaggc gctcttccgc ttcctcgctc actgactcgc tgcgctcggt cgttcggctg 2940
cggcgagcgg tatcagctca ctcaaaggcg gtaatacggt tatccacaga atcaggggat 3000
aacgcaggaa agaacatgtg agcaaaaggc cagcaaaagg ccaggaaccg taaaaaggcc 3060
gcgttgctgg cgtttttcca taggctccgc ccccctgacg agcatcacaa aaatcgacgc 3120
tcaagtcaga ggtggcgaaa cccgacagga ctataaagat accaggcgtt tccccctgga 3180
agctccctcg tgcgctctcc tgttccgacc ctgccgctta ccggatacct gtccgccttt 3240
ctcccttcgg gaagcgtggc gctttctcat agctcacgct gtaggtatct cagttcggtg 3300
taggtcgttc gctccaagct gggctgtgtg cacgaacccc ccgttcagcc cgaccgctgc 3360

gccttatccg gtaactatcg tcttgagtcc aacccggtaa gacacgactt atcgccactg 3420
gcagcagcca ctggtaacag gattagcaga gcgaggtatg taggcggtgc tacagagttc 3480
ttgaagtggt ggcctaacta cggctacact agaaggacag tatttggtat ctgcgctctg 3540
ctgaagccag ttaccttcgg aaaaagagtt ggtagctctt gatccggcaa acaaaccacc 3600
gctggtagcg gtggtttttt tgtttgcaag cagcagatta cgcgcagaaa aaaaggatct 3660
caagaagatc ctttgatctt ttctacgggg tctgacgctc agtggaacga aaactcacgt 3720
taagggattt tggtcatgag attatcaaaa aggatcttca cctagatcct tttaaattaa 3780
aaatgaagtt ttaaatcaat ctaaagtata tatgagtaaa cttggtctga cagttaccaa 3840
tgcttaatca gtgaggcacc tatctcagcg atctgtctat ttcgttcatc catagttgcc 3900
tgactccccg tcgtgtagat aactacgata cgggagggct taccatctgg ccccagtgct 3960
gcaatgatac cgcgagaccc acgctcaccg gctccagatt tatcagcaat aaaccagcca 4020
gccggaaggg ccgagcgcag aagtggtcct gcaactttat ccgcctccat ccagtctatt 4080
aattgttgcc gggaagctag agtaagtagt tcgccagtta atagtttgcg caacgttgtt 4140
gccattgctg cagggggggg gggggggggg gacttccatt gttcattcca cggacaaaaa 4200
cagagaaagg aaacgacaga ggccaaaaag cctcgctttc agcacctgtc gtttcctttc 4260
ttttcagagg gtattttaaa taaaaacatt aagttatgac gaagaagaac ggaaacgcct 4320
taaaccggaa aattttcata aatagcgaaa acccgcgagg tcgccgcccc gtaacctgtc 4380
ggatcaccgg aaaggacccg taaagtgata atgattatca tctacatatc acaacgtgcg 4440
tggaggccat caaaccacgt caaataatca attatgacgc aggtatcgta ttaattgatc 4500
tgcatcaact taacgtaaaa acaacttcag acaatacaaa tcagcgacac tgaatacggg 4560
gcaacctcat gtcccccccc cccccccccc tgcaggcatc gtggtgtcac gctcgtcgtt 4620
tggtatggct tcattcagct ccggttccca acgatcaagg cgagttacat gatcccccat 4680
gttgtgcaaa aaagcggtta gctccttcgg tcctccgatc gttgtcagaa gtaagttggc 4740
cgcagtgtta tcactcatgg ttatggcagc actgcataat tctcttactg tcatgccatc 4800
cgtaagatgc ttttctgtga ctggtgagta ctcaaccaag tcattctgag aatagtgtat 4860
gcggcgaccg agttgctctt gcccggcgtc aacacgggat aataccgcgc cacatagcag 4920
aactttaaaa gtgctcatca ttggaaaacg ttcttcgggg cgaaaactct caaggatctt 4980
accgctgttg agatccagtt cgatgtaacc cactcgtgca cccaactgat cttcagcatc 5040
ttttactttc accagcgttt ctgggtgagc aaaaacagga aggcaaaatg ccgcaaaaaa 5100
gggaataagg gcgacacgga aatgttgaat actcatactc ttcctttttc aatattattg 5160
aagcatttat cagggttatt gtctcatgag cggatacata tttgaatgta tttagaaaaa 5220
taaacaaata ggggttccgc gcacatttcc ccgaaaagtg ccacctgacg tctaagaaac 5280
cattattatc atgacattaa cctataaaaa taggcgtatc acgaggccct ttcgtcttca 5340
agaattggtc gacgatcttg ctgcgttcgg atattttcgt ggagttcccg ccacagaccc 5400

ggattgaagg cgagatccag caactcgcgc cagatcatcc tgtgacggaa ctttggcgcg 5460
tgatgactgg ccaggacgtc ggccgaaaga gcgacaagca gatcacgctt ttcgacagcg 5520
tcggatttgc gatcgaggat ttttcggcgc tgcgctacgt ccgcgaccgc gttgagggat 5580
caagccacag cagcccactc gaccttctag ccgacccaga cgagccaagg gatcttttg 5640
gaatgctgct ccgtcgtcag gctttccgac gtttgggtgg ttgaacagaa gtcattatcg 5700
cacggaatgc caagcactcc cgaggggaac cctgtggttg gcatgcacat acaaatggac 5760
gaacggataa accttttcac gcccttttaa atatccgatt attctaataa acgctctttt 5820
ctcttaggtt tacccgccaa tatatcctgt caaacactga tagtttatca ccactttgta 5880
caagaaagct gggtcggcgc gccgcaagaa tctcaaacac ggagatctca aagtttgaaa 5940
gaaaatttat ttcttcgact caaaacaaac ttacgaaatt taggtagaac ttatatacat 6000
tatattgtaa ttttttgtaa caaaatgttt ttattattat tatagaattt tactggttaa 6060
attaaaaatg aatagaaaag gtgaattaag aggagagagg aggtaaacat tttcttctat 6120
tttttcatat tttcaggata aattattgta aaagtttaca agatttccat ttgactagtg 6180
taaatgagga atattctcta gtaagatcat tatttcatct acttctttta tcttctacca 6240
gtagaggaat aaacaatatt tagctccttt gtaaatacaa attaattttc gttcttgaca 6300
tcattcaatt ttaattttac gtataaaata aaagatcata cctattagaa cgattaagga 6360
gaaatacaat tcgaatgaga aggatgtgcc gcttgttata ataaacagcc acacgacgta 6420
aacgtaaaat gaccacatga tgggccaata gacatggacc gactactaat aatagtaagt 6480
tacattttag gatggaataa atatcatacc gacatcagtt tgaaagaaaa gggaaaaaaa 6540
gaaaaaataa ataaaagata tactaccgac atgagttcca aaaagcaaaa aaaaagatca 6600
agccgacaca gacacgcgta gagagcaaaa tgactttgac gtcacaccac gaaaacagac 6660
gcttcatacg tgtcccttta tctctctcag tctctctata aacttagtga gaccctcctc 6720
tgttttactc acaaatttaa ttaaagatct cccgggcctt cacctgcgga gggtaagatc 6780
cgatcaccat cttctgaatt tctgttcttg atctgtcatg tataataact gtctagtctt 6840
ggtgttggtg agatggaaat tcggtggatc tcggaaggga tattgttcgt ttgctggggt 6900
tttttttgtg tgttgtgatc cgtagagaat ttgtgtttat ccatgttgtt gatcttggta 6960
tgtattcatg acatattgac atgcatgtgt tgtatgtgtc atatgtgtgc ctctccttgg 7020
gatttgtttt ggataataga acatgttatg gactcaatag tctgtgaaca aatctttttt 7080
tagatggtgg ccaaatctga tgatgatctt tcttgagagg aaaaagttca tgatagaaaa 7140
atcttttttg agatggtggc ttaatgtgat gatgatcttt cttgagagga aaaaaaagat 7200
tcattatagg agattttgat ttagctcctt tccaccgata ttaaatgagg agcatgcatg 7260
ctgattgctg ataaggatct gatttttta tcccctcttc tttgaacaga caagaaatag 7320
gctctgaatt tctgattgat tatttgtaca tgcagatgca agatgtacaa cggtccgcct 7380
cagcgcgatc gccttaaggt ttgggtaggt cagtccctta tgttacgtcc tgtagaaacc 7440
ccaacccgtg aaatcaaaaa actcgacggc ctgtgggcat tcagtctgga tcgcgaaaac 7500

tgtggaattg gtcagcgttg gtgggaaagc gcgttacaag aaagccgggc aattgctgtg 7560
ccaggcagtt ttaacgatca gttcgccgat gcagatattc gtaattatgc gggcaacgtc 7620
tggtatcagc gcgaagtctt tataccgaaa ggttgggcag gccagcgtat cgtgctgcgt 7680
ttcgatgcgg tcactcatta cggcaaagtg tgggtcaata atcaggaagt gatggagcat 7740
cagggcggct atacgccatt tgaagccgat gtcacgccgt atgttattgc cgggaaaagt 7800
gtacgtaagt ttctgcttct acctttgata tatatataat aattatcatt aattagtagt 7860
aatataatat ttcaaatatt tttttcaaaa taaaagaatg tagtatatag caattgcttt 7920
tctgtagttt ataagtgtgt atattttaat ttataacttt tctaatatat gaccaaaatt 7980
tgttgatgtg caggtatcac cgtttgtgtg aacaacgaac tgaactggca gactatcccg 8040
ccgggaatgg tgattaccga cgaaaacggc aagaaaaagc agtcttactt ccatgatttc 8100
tttaactatg ccggaatcca tcgcagcgta atgctctaca ccacgccgaa cacctgggtg 8160
gacgatatca ccgtggtgac gcatgtcgcg caagactgta accacgcgtc tgttgactgg 8220
caggtggtgg ccaatggtga tgtcagcgtt gaactgcgtg atgcggatca acaggtggtt 8280
gcaactggac aaggcactag cgggactttg caagtggtga atccgcacct ctggcaaccg 8340
ggtgaaggtt atctctatga actgtgcgtc acagccaaaa gccagacaga gtgtgatatc 8400
tacccgcttc gcgtcggcat ccggtcagtg gcagtgaagg gcgaacagtt cctgattaac 8460
cacaaaccgt tctactttac tggctttggt cgtcatgaag atgcggactt gcgtggcaaa 8520
ggattcgata acgtgctgat ggtgcacgac cacgcattaa tggactggat tggggccaac 8580
tcctaccgta cctcgcatta cccttacgct gaagagatgc tcgactgggc agatgaacat 8640
ggcatcgtgg tgattgatga aactgctgct gtcggcttta acctctcttt aggcattggt 8700
ttcgaagcgg gcaacaagcc gaaagaactg tacagcgaag aggcagtcaa cggggaaact 8760
cagcaagcgc acttacaggc gattaaagag ctgatagcgc gtgacaaaaa ccacccaagc 8820
gtggtgatgt ggagtattgc caacgaaccg gatacccgtc cgcaaggtgc acgggaatat 8880
ttcgcgccac tggcggaagc aacgcgtaaa ctcgacccga cgcgtccgat cacctgcgtc 8940
aatgtaatgt tctgcgacgc tcacaccgat accatcagcg atctctttga tgtgctgtgc 9000
ctgaaccgtt attacggatg gtatgtccaa agcggcgatt tggaaacggc agagaaggta 9060
ctggaaaaag aacttctggc ctggcaggag aaactgcatc agccgattat catcaccgaa 9120
tacggcgtgg atacgttagc cgggctgcac tcaatgtaca ccgacatgtg gagtgaagag 9180
tatcagtgtg catggctgga tatgtatcac cgcgtctttg atcgcgtcag cgccgtcgtc 9240
ggtgaacagg tatggaattt cgccgatttt gcgacctcgc aaggcatatt gcgcgttggc 9300
ggtaacaaga aagggatctt cactcgcgac cgcaaaccga agtcggcggc ttttctgctg 9360
caaaaacgct ggactggcat gaacttcggt gaaaaaccgc agcagggagg caaacaatga 9420
atcaacaact ctcctggcgc accatcgtcg gctacagcct cgggaattgc taccgagctc 9480
gaatttcccc gatcgttcaa acatttggca ataaagtttc ttaagattga atcctgttgc 9540

cggtcttgcg atgattatca tataatttct gttgaattac gttaagcatg taataattaa 9600
catgtaatgc atgacgttat ttatgagatg ggtttttatg attagagtcc cgcaattata 9660
catttaatac gcgatagaaa acaaaatata gcgcgcaaac taggataaat tatcgcgcgc 9720
ggtgtcatct atgttactag atcgggaatt ggcatgcaag cttggcactg gccgtcgttt 9780
tacaacgtcg tgactgggaa aaccctggcg ttacccaact taatcgcctt gcagcacatc 9840
ccccctttcgc cagctggcgt aatagcgaag aggcccgcac cgatcgccct tcccaacagt 9900
tgcgcagcct gaatggcgaa tgctagagca gcttgagctt ggatcagatt gtcgtttccc 9960
gccttcagtt taaacgatag cggtgaaggg ggcggccgcg gagcctgctt ttttgtacaa 10020
acttgtgata aacgggccgc tctagattag tgtacggaat aaaagtccta attcattctt 10080
tttcttatac gtcaccgttt tctacattta gaaaaatgaa gtgggaatca attgaaacaa 10140
ttgatagctt taaatatcaa gctgtcttct ccaggaccag gccccagcac atcggcgtgg 10200
aaagcgctag gccccagaac aactcgtcaa tcgtcatggc aaataatgac acattgagcc 10260
gatttgatgc atggaacaga ctaataagga actcaaatct ctttggagat tgaatgattg 10320
agatgaaaat gaattaatat tttttctcaa tcccctccaa tgctaagaaa gtttgagttt 10380
ccaaattagc tttagagggc gtttagatcc cttcgtttta gagaaattag aattcactca 10440
ataaaataac ttatttaatt tggaatttga tattcaacca cttttcaaag tttagatata 10500
ggtctatctc aaattcatag ggtggatgat ggaaatgatt ttatgcatta atagaatttg 10560
tttctactgt gtaacttaca tgacactctt catctcactc ctgtatagta aaaatgtagc 10620
atataaatat ctccgacatc ttgataataa tagtatacaa atatattttg cataaaaccg 10680
aattaactta attgatatat gccaaaatta ctattattag aatggaattt aattccaatg 10740
atccaaacca cgaaaatgga tcaggtaact aattcagtca aatgcctcat ttttttttcac 10800
tcccctcaaa ccacgaaaat gccattctgg tttgtaaaat agtttgaaat tgcagcccta 10860
gcattattcc atacatcata tgttgagttg aaattaccaa tataataata actaaaaaag 10920
gaaaaaaata gcgaaaacaa aagcaaggac cagttggaca cttaaatttg gcaacagaag 10980
ccaattcaga accactgcat agcagtgctt attatcttat ttatatgtag caaaaggcac 11040
ttaaatgatc tcttatgaca catgccagaa ggaaaacaac agactacaca attacaaggt 11100
cggaagctac ataggattac cataacagat agctgacggc ctacaaaaaa agatagaata 11160
caggagaaca tcacacagat aaaaacatat cacccttgta ctagcaggga ggcggtgctt 11220
gctggatttt agatcagttg cttgctggat tttagatcag tacacagtcc tgccatcacc 11280
atccaggatc atatccttga aagccccacc attagggatc ataggcaaca catgctcctg 11340
gtgtgggacg attatatcca agaggtacgg ccctggagtc tcgagcatct tctttatcgc 11400
tgcgcggact tcgttcttct ttgtcacacg gaccgctgga atgttgaacc ctttggcgat 11460
cgtcacgaaa tctggatata tctcactttc attctctggg tttcccaagt atgtgtgcgc 11520
tctgttggcc ttatagaacc tgtcctccca ctgcaccacc atccccaggt gctggttgtt 11580
tagcacaaag accttcaccg ggaggttctc aattcggatc atagctagct cctgaacgtt 11640

catgagaaag ctaccatctc catcgatgtc aacaacagta acacctgggt tggccacaga 11700
agcaccagca gcagccggca aaccaaatcc catagcccca agaccagctg aagacaacca 11760
ctgccttggc cgcttgtaag tgtagtactg tgccgcccac atctggtgct gcccaacacc 11820
tgtgccgatg atggcctcgc ctttcgtcag ctcatcaaga acctgaatag catattgtgg 11880
ctggatctcc tcattagatg ttttataccc aagggggaat tccctcttct gctgatccaa 11940
ctcatcgttc catgagccaa agtcaaagct cttctttgat gtgcttcctt caagaagagc 12000
attcatgccc tgcaaagcaa gcttaacatc tgcacagatg gacacatgtg gctgcttgtt 12060
cttgccaatc tcagccggat caatatcaac gtgcacaatc ttagccctgc ttgcaaaagc 12120
ctcaatcttc cctgtcacac gatcatcaaa ccgcacacca agtgcaagca acagatcggc 12180
cttatccact gcataatttg catacaccgt gccatgcata cctagcatgc gcagagacag 12240
tgggtcgtcg ctggggaagt tgccgaggcc cataagagta gttgtgaccg ggattccagt 12300
cagctccaca aagcgtcgca actcctcacc agatgctgcg cagccaccgc caacataaag 12360
aacagggcgc cgggattcac caacaagacg cagcacctgc tcaagcaact cagtcgcagg 12420
gggcttggga aggcgcgcaa tgtacccagg cagactcatg ggcttgtccc agacaggcac 12480
cgccatctgc tgctggatgt ccttggggat gtcgacaagc accggccccg gtcgaccaga 12540
ggaggcgagg aagaaagcct cctgcacgac gcgggggatg tcgtcgacgt cgaggaccag 12600
gtagttgtgc ttggtgatgg agcgggtgac ctcgacgatg ggcgtctcct ggaaggcgtc 12660
ggtgccaatc atgcgtcgcg gcacctgtcc cgtgatggcg accatgggga cggaatcgag 12720
cagcgcgtcg gcgagcgcgg agacaaggtt ggtggcgccg gggccggagg tggcgatgca 12780
gacgccgacg cggcccgagg agcgcgcgta gccggaggcc gcaaaggcct ccccttgctc 12840
gtggcggaag aggtggttgg cgatgacggg ggagcgggtg agtgcctggt ggatctccat 12900
ggacgcgccg ccggggtagg cgaagacgtc gcggacgccg cagcgctcga gggactcgac 12960
gaggatgtcg gcgcccttgc ggggatcggt ggggccccac ggccggagcg gggtggccgg 13020
gggagccatc ggcatggcgg gtgacgccgc tgagcacctg atgggcgcgg cgagggcgcg 13080
gcgggtggcc aggaggtgcg cccggcgcct cgccttgggc gcagcggtag tggcgccagt 13140
gagcgcggta gacgcggcgg cggcggtggc catggtttct agaactagtg gatcccccgg 13200
gggtaccctg cagaagtaac accaaacaac agggtgagca tcgacaaaag aaacagtacc 13260
aagcaaataa atagcgtatg aaggcagggc taaaaaaatc cacatatagc tgctgcatat 13320
gccatcatcc aagtatatca agatcgaaat aattataaaa catacttgtt tattataata 13380
gataggtact caaggttaga gcatatgaat agatgctgca tatgccatca tgtatatgca 13440
tcagtaaaac ccacatcaac atgtatacct atcctagatc gatatttcca tccatcttaa 13500
actcgtaact atgaagatgt atgacacaca catacagttc caaaattaat aaatacacca 13560
ggtagtttga aacagtattc tactccgatc tagaacgaat gaacgaccgc ccaaccacac 13620
cacatcatca caaccaagcg aacaaaaagc atctctgtat atgcatcagt aaaacccgca 13680

tcaacatgta tacctatcct agatcgatat ttccatccat catcttcaat tcgtaactat 13740
gaatatgtat ggcacacaca tacagatcca aaattaataa atccaccagg tagtttgaaa 13800
cagaattcta ctccgatcta gaacgaccgc ccaaccagac cacatcatca caaccaagac 13860
aaaaaaaagc atgaaaagat gacccgacaa acaagtgcac ggcatatatt gaaataaagg 13920
aaaagggcaa accaaaccct atgcaacgaa acaaaaaaaa tcatgaaatc gatcccgtct 13980
gcggaacggc tagagccatc ccaggattcc ccaaagagaa acactggcaa gttagcaatc 14040
agaacgtgtc tgacgtacag gtcgcatccg tgtacgaacg ctagcagcac ggatctaaca 14100
caaacacgga tctaacacaa acatgaacag aagtagaact accgggccct aaccatggac 14160
cggaacgccg atctagagaa ggtagagagg gggggggggg gggaggacga gcggcgtacc 14220
ttgaagcgga ggtgccgacg ggtggatttg ggggagatct ggttgtgtgt gtgtgcgctc 14280
cgaacaacac gaggttgggg aaagagggtg tggagggggt gtctatttat tacggcgggc 14340
gaggaaggga aagcgaagga gcggtgggaa aggaatcccc cgtagctgcc ggtgccgtga 14400
gaggaggagg aggccgcctg ccgtgccggc tcacgtctgc cgctccgcca cgcaatttct 14460
ggatgccgac agcggagcaa gtccaacggt ggagcggaac tctcgagagg ggtccagagg 14520
cagcgacaga gatgccgtgc cgtctgcttc gcttggcccg acgcgacgct gctggttcgc 14580
tggttggtgt ccgttagact cgtcgacggc gtttaacagg ctggcattat ctactcgaaa 14640
caagaaaaat gtttccttag ttttttaat ttcttaaagg gtatttgttt aatttttagt 14700
cactttattt tattctattt tatatctaaa ctattaaata aaaaaactaa aatagagttt 14760
tagttttctt aatttagagg ctaaaataga ataaaataga tgtactaaaa aaattagtct 14820
ataaaaacca ttaaccctaa accctaaatg gatgtactaa taaaatggat gaagtattat 14880
ataggtgaag ctatttgcaa aaaaaaagga gaacacatgc acactaaaaa gataaaactg 14940
tagagtcctg ttgtcaaaat actcaattgt cctttagacc atgtctaact gttcatttat 15000
atgattctct aaaacactga tattattgta gtactataga ttatattatt cgtagagtaa 15060
agtttaaata tatgtataaa gatagataaa ctgcacttca aacaagtgtg acaaaaaaaa 15120
tatgtggtaa tttttataa cttagacatg caatgctcat tatctctaga gaggggcacg 15180
accgggtcac gctgcactgc agccgcggaa gcttgcatgc ctgcaggcat gcaagcttgg 15240
cgcgccttaa ttaacacgtg ggcgcgccac tagtcaattc agtacattaa aaacgtccgc 15300
aatgtgttat taagttgtct aagcgtcaat ttgtttacac cacaatatat cctgccacca 15360
gccagccaac agctccccga ccggcagctc ggcacaaaat caccactcga tacaggcagc 15420
ccatcagtcc gggacggcgt cagcgggaga gccgttgtaa ggcggcagac tttgctcatg 15480
ttaccgatgc tattcggaag aacggcaact aagctgccgg gtttgaaaca cggatgatct 15540
cgcggagggt agc 15553

<210> 3
<211> 583
<212> DNA
<213> oryza sativa

<220>
<221> Intron
<222> (1)..(583)
<223> BPSI.1 intron

<400> 3

```
gtaagatccg atcaccatct tctgaatttc tgttcttgat ctgtcatgta taataactgt     60
ctagtcttgg tgttggtgag atggaaattc ggtggatctc ggaagggata ttgttcgttt    120
gctggggttt tttttgtgtg ttgtgatccg tagagaattt gtgtttatcc atgttgttga    180
tcttggtatg tattcatgac atattgacat gcatgtgttg tatgtgtcat atgtgtgcct    240
ctccttggga tttgttttgg ataatagaac atgttatgga ctcaatagtc tgtgaacaaa    300
tcttttttta gatggtggcc aaatctgatg atgatctttc ttgagaggaa aaagttcatg    360
atagaaaaat ctttttgag atggtggctt aatgtgatga tgatctttct tgagaggaaa     420
aaaaagattc attataggag attttgattt agctcctttc caccgatatt aaatgaggag    480
catgcatgct gattgctgat aaggatctga tttttttatc ccctcttctt tgaacagaca    540
agaaataggc tctgaatttc tgattgatta tttgtacatg cag                      583
```

<210> 4
<211> 1006
<212> DNA
<213> zea mays

<220>
<221> Intron
<222> (1)..(1006)

<400> 4

gtacgccgct cgtcctcccc cccccccccc ctctctacct tctctagatc ggcgttccgg 60
tccatggtta gggcccggta gttctacttc tgttcatgtt tgtgttagat ccgtgtttgt 120
gttagatccg tgctgctagc gttcgtacac ggatgcgacc tgtacgtcag acacgttctg 180
attgctaact tgccagtgtt tctctttggg gaatcctggg atggctctag ccgttccgca 240
gacgggatcg atttcatgat ttttttgtt tcgttgcata gggtttggtt tgcccttttc 300
ctttatttca atatatgccg tgcacttgtt tgtcgggtca tcttttcatg cttttttttg 360
tcttggttgt gatgatgtgg tctggttggg cggtcgttct agatcggagt agaattctgt 420
ttcaaactac ctggtggatt tattaatttt ggatctgtat gtgtgtgcca tacatattca 480
tagttacgaa ttgaagatga tggatggaaa tatcgatcta ggataggtat acatgttgat 540
gcgggtttta ctgatgcata tacagagatg ctttttgttc gcttggttgt gatgatgtgg 600
tgtggttggg cggtcgttca ttcgttctag atcggagtag aatactgttt caaactacct 660
ggtgtattta ttaattttgg aactgtatgt gtgtgtcata catcttcata gttacgagtt 720
taagatggat ggaaatatcg atctaggata ggtatacatg ttgatgtggg ttttactgat 780
gcatatacat gatggcatat gcagcatcta ttcatatgct ctaaccttga gtacctatct 840
attataataa acaagtatgt tttataatta tttcgatctt gatatacttg gatgatggca 900

tatgcagcag ctatatgtgg attttttag ccctgccttc atacgctatt tatttgcttg 960
gtactgtttc ttttgtcgat gctcaccctg ttgtttggtg ttactt 1006

<210> 5
<211> 27
<212> DNA
<213> arabidopsis thaliana

<220>
<221> primer_bind
<222> (1)..(27)

<400> 5
gcaagaatct caaacacgga gatctca 27

<210> 6
<211> 29
<212> DNA
<213> Arabidopsis thaliana

<220>
<221> primer_bind
<222> (1)..(29)

<400> 6
atttgtgagt aaaacagagg agggtctca 29

<210> 7
<211> 35
<212> DNA
<213> oryza sativa

<220>
<221> primer_bind
<222> (1)..(35)

<400> 7
cccgggcacc ctgcggaggg taagatccga tcacc 35

<210> 8
<211> 30
<212> DNA
<213> oryza sativa

<220>
<221> primer_bind
<222> (1)..(30)

<400> 8
cggaccggta catcttgcat ctgcatgtac 30

<210> 9
<211> 11
<212> DNA
<213> unknown

<220>
<223> AG-motif binding protein

<400> 9
ggagatctca a 11

<210> 10
<211> 11
<212> DNA
<213> unknown

<220>
<223> AG-motif binding protein 1 core seq

<220>
<221> misc_feature
<222> (1)..(3)
<223> n is a, c, g, or t

<220>
<221> misc_feature
<222> (8)..(11)
<223> n is a, c, g, or t

<400> 10
nnngatcnnn n 11

<210> 11
<211> 17
<212> DNA
<213> Petunia hybrida

<400> 11
gtaagtttgt tttgagt 17

<210> 12
<211> 17
<212> DNA
<213> Petunia hybrida

<220>
<221> misc_feature
<222> (1)..(6)
<223> n is a, c, g, or t

<220>
<221> misc_feature
<222> (11)..(17)
<223> n is a, c, g, or t

<400> 12
nnnnnnttgt nnnnnnn 17

<210> 13
<211> 21
<212> DNA
<213> Arabidopsis thaliana

<400> 13
acttacgaaa tttaggtaga a 21

<210> 14
<211> 21
<212> DNA
<213> Arabidopsis thaliana

<220>
<221> misc_feature
<222> (1)..(3)
<223> n is a, c, g, or t

<220>
<221> misc_feature
<222> (8)..(21)
<223> n is a, c, g, or t

<400> 14
nnntacgnnn nnnnnnnnnn n 21

<210> 15
<211> 21
<212> DNA
<213> Arabidopsis thaliana

<400> 15
aattacaata taatgtatat a 21

<210> 16
<211> 21
<212> DNA
<213> Arabidopsis thaliana

<400> 16

atatacatta tattgtaatt t 21

<210> 17
<211> 21
<212> DNA
<213> Arabidopsis thaliana

<220>
<221> misc_feature
<222> (1)..(3)
<223> n is a, c, g, or t

<220>
<221> misc_feature
<222> (8)..(21)
<223> n is a, c, g, or t

<400> 17
nnntacannn nnnnnnnnnn n 21

<210> 18
<211> 17
<212> DNA
<213> Petunia hybrida

<400> 18
aacattttgt tacaaaa 17

<210> 19
<211> 11
<212> DNA
<213> Glycine max

<400> 19
tgtttttatt a 11

<210> 20
<211> 11
<212> DNA
<213> Glycine max

<220>
<221> misc_feature
<222> (1)..(2)
<223> n is a, c, g, or t

<220>
<221> misc_feature
<222> (7)..(11)
<223> n is a, c, g, or t

<400> 20
nnttttnnnn n 11

<210> 21
<211> 11
<212> DNA
<213> Helianthus annuus

<400> 21
tttattatta t 11

<210> 22
<211> 11
<212> DNA
<213> Helianthus annuus

<220>
<221> misc_feature
<222> (1)..(6)
<223> n is a, c, g, or t

<220>
<221> misc_feature
<222> (11)..(11)
<223> n is a, c, g, or t

<400> 22
nnnnnnatta n 11

<210> 23
<211> 11
<212> DNA
<213> Helianthus annuus

<400> 23
attattatta t 11

<210> 24
<211> 17
<212> DNA
<213> unknown

<220>
<223> SBF-1

<400> 24 17
ttactggtta aattaaa 17

<210> 25
<211> 17
<212> DNA
<213> unknown

<220>
<223> SBF-1

<220>
<221> misc_feature
<222> (1)..(7)
<223> n is a, c, g, or t

<220>
<221> misc_feature
<222> (12)..(17)
<223> n is a, c, g, or t

<400> 25 17
nnnnnnntta annnnnn 17

<210> 26
<211> 15
<212> DNA
<213> unknwon

<400> 26 15
aaaaatgaat agaaa 15

<210> 27
<211> 15
<212> DNA
<213> unknown

<220>
<223> Cis-element

<220>
<221> misc_feature
<222> (1)..(4)
<223> n is a, c, g, or t

<220>
<221> misc_feature
<222> (9)..(15)
<223> n is a, c, g, or t

<400> 27 15
nnnnatgann nnnnn 15

<210> 28
<211> 17
<212> DNA
<213> unknown

<220>
<223> prolamin box

<400> 28
tgaatagaaa aggtgaa 17

<210> 29
<211> 17
<212> DNA
<213> unknown

<220>
<223> prolamin box

<220>
<221> misc_feature
<222> (1)..(8)
<223> n is a, c, g, or t

<220>
<221> misc_feature

<222> (13)..(17)
<223> n is a, c, g, or t

<400> 29
nnnnnnnnaa agnnnnn 17

<210> 30
<211> 17
<212> DNA
<213> unknown

<220>
<223> I-box in rbc genes and other light regulated genes

<400> 30
gaatagaaaa ggtgaat 17

<210> 31
<211> 17
<212> DNA
<213> unknown

<220>
<223> I-BOX in rbcs genes and other light regulated genes

<220>
<221> misc_feature
<222> (1)..(5)
<223> n is a, c, g, or t

<220>
<221> misc_feature
<222> (10)..(17)
<223> n is a, c, g, or t

<400> 31
nnnnngaaan nnnnnnn 17

<210> 32
<211> 11
<212> DNA
<213> unknown

<220>
<223> nodulin consensus sequence 1

<400> 32
gaaaaggtga a 11

<210> 33
<211> 11
<212> DNA
<213> unknown

<220>
<223> nodulin consensus sequence 1

<220>

<221> misc_feature
<222> (1)..(1)
<223> n is a, c, g, or t

<220>
<221> misc_feature
<222> (6)..(11)
<223> n is a, c, g, or t

<400> 33
naaaannnnn n 11

<210> 34
<211> 21
<212> DNA
<213> unknown

<220>
<223> OCS-like elements

<400> 34
agaagaaaat gtttacctcc t 21

<210> 35
<211> 21
<212> DNA
<213> unknown

<220>
<223> OCS-like elements

<220>
<221> misc_feature
<222> (1)..(14)
<223> n is a, c, g, or t

<220>
<221> misc_feature
<222> (19)..(21)
<223> n is a, c, g, or t

<400> 35
nnnnnnnnnn nnnnacctnn n 21

<210> 36
<211> 11
<212> DNA
<213> unknown

<220>
<223> nodulin consensus sequence 1

<400> 36 11
aaaatgttta c 11

<210> 37
<211> 11
<212> DNA

<213> unknown

<220>
<223> nodulin consensus sequence 1

<220>
<221> misc_feature
<222> (1)..(1)
<223> n is a, c, g, or t

<220>
<221> misc_feature
<222> (6)..(11)
<223> n is a, c, g, or t

<400> 37 11
naaatnnnnn n 11

<210> 38
<211> 21
<212> DNA
<213> unknown

<220>
<223> MADS-box protein

<400> 38 21
ttcttctatt ttttcatatt t 21

<210> 39
<211> 21
<212> DNA
<213> unknown

<220>
<223> MADS-box protein

<220>
<221> misc_feature
<222> (1)..(7)
<223> n is a, c, g, or t

<220>
<221> misc_feature
<222> (12)..(21)
<223> n is a, c, g, or t

<400> 39 21
nnnnnnnatt tnnnnnnnnn n 21

<210> 40
<211> 15
<212> DNA
<213> unknown

<220>
<223> cis-element in the GAPDH promoters conferring light inducibility

<400> 40
aaatatgaaa aaata 15

<210> 41
<211> 17
<212> DNA
<213> Solanum tuberosum

<400> 41
taatttatcc tgaaaat 17

<210> 42
<211> 17
<212> DNA
<213> Solanum tuberosum

<220>
<221> misc_feature
<222> (1)..(6)
<223> n is a, c, g, or t

<220>
<221> misc_feature
<222> (11)..(17)
<223> n is a, c, g, or t

<400> 42
nnnnnnatcc nnnnnnn 17

<210> 43
<211> 17
<212> DNA
<213> unknown

<220>
<223> class I GATA factors

<400> 43
ttcaggataa attattg 17

<210> 44
<211> 17
<212> DNA
<213> unknown

<220>
<223> class I GATA factors

<220>
<221> misc_feature
<222> (1)..(5)
<223> n is a, c, g, or t

<220>
<221> misc_feature
<222> (10)..(17)
<223> n is a, c, g, or t

<400> 44
nnnnngatan nnnnnnn 17

<210> 45
<211> 11
<212> DNA
<213> Arabidopsis thaliana

<400> 45
taaattattg t 11

<210> 46
<211> 11
<212> DNA
<213> Arabidopsis thaliana

<220>
<221> misc_feature
<222> (1)..(3)
<223> n is a, c, g, or t

<220>
<221> misc_feature
<222> (8)..(11)
<223> n is a, c, g, or t

<400> 46
nnnattannn n 11

<210> 47
<211> 11
<212> DNA
<213> unknown

<220>
<223> HDZip class I protein ATHB5

<400> 47
acaataattt a 11

<210> 48
<211> 11
<212> DNA
<213> unknown

<220>
<223> HDZip class I protein ATHB5

<220>
<221> misc_feature
<222> (1)..(3)
<223> n is a, c, g, or t

<220>
<221> misc_feature
<222> (8)..(11)
<223> n is a, c, g, or t

<400> 48
nnnataannn n 11

<210> 49
<211> 17
<212> DNA
<213> unknown

<220>
<223> trihelix DNA-binding factor GT-3a

<400> 49
taaactttta caataat 17

<210> 50
<211> 17
<212> DNA
<213> unknown

<220>
<223> trihelix DNA-binding factor GT-3a

<220>
<221> misc_feature
<222> (1)..(6)
<223> n is a, c, g, or t

<220>
<221> misc_feature
<222> (11)..(17)
<223> n is a, c, g, or t

<400> 50
nnnnnnttta nnnnnnn 17

<210> 51
<211> 11
<212> DNA
<213> unknown

<220>
<223> nodulin consensus sequence 1

<400> 51
taaaagttta c 11

<210> 52
<211> 17
<212> DNA
<213> unknown

<220>
<223> GAAA motif

<400> 52
aaatggaaat cttgtaa 17

<210> 53

<211> 17
<212> DNA
<213> unknown

<220>
<223> GAAA motif

<220>
<221> misc_feature
<222> (1)..(5)
<223> n is a, c, g, or t

<220>
<221> misc_feature
<222> (10)..(17)
<223> n is a, c, g, or t

<400> 53
nnnnngaaan nnnnnnn 17

<210> 54
<211> 17
<212> DNA
<213> Spinacia oleracea

<400> 54
tcaaatggaa atcttgt 17

<210> 55
<211> 17
<212> DNA
<213> Spinacia oleracea

<220>
<221> misc_feature
<222> (1)..(4)
<223> n is a, c, g, or t

<220>
<221> misc_feature
<222> (9)..(17)
<223> n is a, c, g, or t

<400> 55
nnnnatggnn nnnnnnn 17

<210> 56
<211> 17
<212> DNA
<213> unknown

<220>
<223> WRKY plant specific zinc-finger-type factor

<400> 56
tccatttgac tagtgta 17

<210> 57

<211> 17
<212> DNA
<213> unknown

<220>
<223> WRKY plant specific zinc-finger-type factor

<220>
<221> misc_feature
<222> (1)..(5)
<223> n is a, c, g, or t

<220>
<221> misc_feature
<222> (10)..(17)
<223> n is a, c, g, or t

<400> 57
nnnnnttgan nnnnnnn 17

<210> 58
<211> 17
<212> DNA
<213> Triticum aestivum

<400> 58
gagaatattc ctcattt 17

<210> 59
<211> 17
<212> DNA
<213> Triticum aestivum

<220>
<221> misc_feature
<222> (1)..(4)
<223> n is a, c, g, or t

<220>
<221> misc_feature
<222> (9)..(17)
<223> n is a, c, g, or t

<400> 59
nnnnatatnn nnnnnnn 17

<210> 60
<211> 17
<212> DNA
<213> Triticum aestivum

<400> 60
aggaatattc tctagta 17

<210> 61
<211> 11
<212> DNA
<213> Helianthus annuus

<220>
<221> misc_feature
<223> n is a, c, g, or t

<400> 61
aagatcatta t 11

<210> 62
<211> 11
<212> DNA
<213> unknown

<220>
<223> motif involved in carotenoid and tocopherol biosynthesis and in the expression of photosynthesis related genes

<400> 62
tcatctactt c 11

<210> 63
<211> 11
<212> DNA
<213> unknown

<220>
<223> motif involved in carotenoid and tocopherol biosynthesis and in the expression of photosynthesis-related genes

<220>
<221> misc_feature
<222> (1)..(2)
<223> n is a, c, g, or t

<220>
<221> misc_feature
<222> (7)..(11)
<223> n is a, c, g, or t

<400> 63
nnatctnnnn n 11

<210> 64
<211> 17
<212> DNA
<213> unknown

<220>
<223> classs I GATA factors

<400> 64
tagaagataa aagaagt 17

<210> 65
<211> 15
<212> DNA
<213> unknown

<220>
<223> heat shock element

<400> 65
agaagataaa agaag 15

<210> 66
<211> 15
<212> DNA
<213> unknown

<220>
<223> heat shock element

<220>
<221> misc_feature
<222> (1)..(10)
<223> n is a, c, g, or t

<220>
<221> misc_feature
<222> (15)..(15)
<223> n is a, c, g, or t

<400> 66
nnnnnnnnnn agaan 15

<210> 67
<211> 21
<212> DNA
<213> Arabidopsis thaliana

<400> 67
ttattcctct actggtagaa g 21

<210> 68
<211> 21
<212> DNA
<213> Arabidopsis thaliana

<220>
<221> misc_feature
<222> (1)..(3)
<223> n is a, c, g, or t

<220>
<221> misc_feature
<222> (8)..(21)
<223> n is a, c, g, or t

<400> 68
nnnttccnnn nnnnnnnnnn n 21

<210> 69
<211> 21
<212> DNA
<213> Arabidopsis thaliana

<400> 69
ttctaccagt agaggaataa a 21

<210> 70
<211> 21
<212> DNA
<213> Arabidopsis thaliana

<220>
<221> misc_feature
<222> (1)..(3)
<223> n is a, c, g, or t

<220>
<221> misc_feature
<222> (8)..(21)
<223> n is a, c, g, or t

<400> 70
nnntaccnnn nnnnnnnnnn n 21

<210> 71
<211> 17
<212> DNA
<213> Triticum aestivum

<400> 71
ttgtttattc ctctact 17

<210> 72
<211> 17
<212> DNA
<213> Triticum aestivum

<220>
<221> misc_feature
<222> (1)..(4)
<223> n is a, c, g, or t

<220>
<221> misc_feature
<222> (9)..(17)
<223> n is a, c, g, or t

<400> 72
nnnnttatnn nnnnnnn 17

<210> 73
<211> 17
<212> DNA
<213> unknown

<220>
<223> prolamin box, conserved in cereal seed storage protein gene promoters

<400> 73
tcctttgtaa atacaaa 17

<210> 74
<211> 17
<212> DNA

<213> unknown

<220>
<223> prolamin box, conserved in cereal seed storage protein gene promoters

<220>
<221> misc_feature
<222> (1)..(8)
<223> n is a, c, g, or t

<220>
<221> misc_feature
<222> (13)..(17)
<223> n is a, c, g, or t

<400> 74
nnnnnnnnaa atnnnnn 17

<210> 75
<211> 17
<212> DNA
<213> unknown

<220>
<223> SBF-1

<400> 75
cgtaaaatta aaattga 17

<210> 76
<211> 23
<212> DNA
<213> Triticum aestivum

<400> 76
cttttatttt atacgtaaaa tta 23

<210> 77
<211> 23
<212> DNA
<213> Triticum aestivum

<220>
<221> misc_feature
<222> (1)..(11)
<223> n is a, c, g, or t

<220>
<221> misc_feature
<222> (16)..(23)
<223> n is a, c, g, or t

<400> 77
nnnnnnnnnn ntacgnnnnn nnn 23

<210> 78
<211> 15
<212> DNA

<213> unknown

<220>
<223> high mobility group I/Y-like proteins

<400> 78
cttttatttt atacg 15

<210> 79
<211> 15
<212> DNA
<213> unknown

<220>
<223> high mobility group I/Y like proteins

<220>
<221> misc_feature
<222> (1)..(4)
<223> n is a, c, g, or t

<220>
<221> misc_feature
<222> (9)..(15)
<223> n is a, c, g, or t

<400> 79
nnnntattnn nnnnn 15

<210> 80
<211> 11
<212> DNA
<213> unknown

<220>
<223> nodulin consensus sequence 1

<400> 80
taaaagatca t 11

<210> 81
<211> 11
<212> DNA
<213> unknown

<220>
<223> homeodomain protein WUSCHEL

<400> 81
ctccttaatc g 11

<210> 82
<211> 11
<212> DNA
<213> unknown

<220>
<223> homeodomain protein WUSCHEL

<220>
<221> misc_feature
<222> (1)..(5)
<223> n is a, c, g, or t

<220>
<221> misc_feature
<222> (10)..(11)
<223> n is a, c, g, or t

<400> 82
nnnnntaatn n 11

<210> 83
<211> 17
<212> DNA
<213> unknown

<220>
<223> GT1-Box binding factors with a trihelix DNA-binding domain

<400> 83
catgtggtca ttttacg 17

<210> 84
<211> 17
<212> DNA
<213> unknown

<220>
<223> GT1-Box binding factors with a trihelix DNA-binding domain

<220>
<221> misc_feature
<222> (1)..(6)
<223> n is a, c, g, or t

<220>
<221> misc_feature
<222> (11)..(17)
<223> n is a, c, g, or t

<400> 84
nnnnnngtca nnnnnnn 17

<210> 85
<211> 13
<212> DNA
<213> Arabidopsis thaliana

<400> 85
attggcccat cat 13

<210> 86
<211> 13
<212> DNA
<213> Arabidopsis thaliana

<220>
<221> misc_feature
<222> (1)..(4)
<223> n is a, c, g, or t

<220>
<221> misc_feature
<222> (9)..(13)
<223> n is a, c, g, or t

<400> 86
nnnngcccnn nnn 13

<210> 87
<211> 15
<212> DNA
<213> unknown

<220>
<223> C-repeat/dehydration response element

<400> 87
atggaccgac tacta 15

<210> 88
<211> 15
<212> DNA
<213> unknown

<220>
<223> C-repeat/dehydration response element

<220>
<221> misc_feature
<222> (1)..(5)
<223> n is a, c, g, or t

<220>
<221> misc_feature
<222> (10)..(15)
<223> n is a, c, g, or t

<400> 88
nnnnnccgan nnnnn 15

<210> 89
<211> 21
<212> DNA
<213> Arabidopsis thaliana

<400> 89 21
acttactatt attagtagtc g 21

<210> 90
<211> 21
<212> DNA
<213> Arabidopsis thaliana

<220>
<221> misc_feature
<222> (1)..(3)
<223> n is a, c, g, or t

<220>
<221> misc_feature
<222> (8)..(21)
<223> n is a, c, g, or t

<400> 90 21
nnntactnnn nnnnnnnnnn n 21

<210> 91
<211> 21
<212> DNA
<213> Arabidopsis thaliana

<400> 91 21
gactactaat aatagtaagt t 21

<210> 92
<211> 11
<212> DNA
<213> Helianthus annuus

<400> 92
actattatta g 11

<210> 93
<211> 13
<212> DNA
<213> Ipomoea batatas

<400> 93 13
cttactatta tta 13

<210> 94
<211> 13
<212> DNA
<213> Ipomoea batatas

<220>
<221> misc_feature
<222> (1)..(2)
<223> n is a, c, g, or t

<220>
<221> misc_feature
<222> (7)..(13)
<223> n is a, c, g, or t

<400> 94
nntactnnnn nnn 13

<210> 95
<211> 17
<212> DNA

<213> unknown

<220>
<223> trihelix DNA-binding factor GT-3a

<400> 95
tagtaagtta catttta 17

<210> 96
<211> 17
<212> DNA
<213> unknown

<220>
<223> trihelix DNA-binding factor GT-3a

<220>
<221> misc_feature
<222> (1)..(6)
<223> n is a, c, g, or t

<220>
<221> misc_feature
<222> (11)..(17)
<223> n is a, c, g, or t

<400> 96
nnnnnngtta nnnnnnn 17

<210> 97
<211> 9
<212> DNA
<213> unknown

<220>
<223> TEIL

<400> 97
atgtaactt 9

<210> 98
<211> 9
<212> DNA
<213> unknown

<220>
<223> TEIL

<220>
<221> misc_feature
<222> (1)..(1)
<223> n is a, c, g, or t

<220>
<221> misc_feature
<222> (6)..(9)
<223> n is a, c, g, or t

<400> 98
ntgtannnn 9

<210> 99
<211> 17
<212> DNA
<213> spinacia oleracea

<400> 99
taggatggaa taaatat 17

<210> 100
<211> 15
<212> DNA
<213> unknown

<220>
<223> C-repeat/dehydration response element

<220>
<221> misc_feature
<223> n is a, c, g, or t

<400> 100
tcataccgac atcag 15

<210> 101
<211> 17
<212> DNA
<213> unknown

<220>
<223> PBF

<400> 101
ttgaaagaaa agggaaa 17

<210> 102
<211> 17
<212> DNA
<213> unknown

<220>
<223> PBF

<220>
<221> misc_feature
<222> (1)..(8)
<223> n is a, c, g, or t

<220>
<221> misc_feature
<222> (13)..(17)
<223> n is a, c, g, or t

<400> 102
nnnnnnnnaa agnnnnn 17

<210> 103
<211> 21
<212> DNA
<213> unknown

<220>
<223> TEF cis acting elements

<400> 103
aaaagggaaa aaaagaaaaa a 21

<210> 104
<211> 21
<212> DNA
<213> unknown

<220>
<223> TEF cis acting elements

<220>
<221> misc_feature
<222> (1)..(2)
<223> n is a, c, g, or t

<220>
<221> misc_feature
<222> (7)..(21)
<223> n is a, c, g, or t

<400> 104
nnaaggnnnn nnnnnnnnnn n 21

<210> 105
<211> 11
<212> DNA
<213> unknown

<220>
<223> nodulin consensus sequence 1

<400> 105
taaaagatat a 11

<210> 106
<211> 15
<212> DNA
<213> unknown

<220>
<223> C-repeat/dehydration response elements

<400> 106
tactaccgac atgag 15

<210> 107
<211> 17
<212> DNA
<213> unknown

<220>
<223> Dof3-single zinc finger transcription factor

<400> 107
agttccaaaa agcaaaa 17

<210> 108
<211> 17
<212> DNA
<213> unknown

<220>
<223> DOF3-single zinc finger transcription factor

<220>
<221> misc_feature
<222> (1)..(8)
<223> n is a, c, g, or t

<220>
<221> misc_feature
<222> (13)..(17)
<223> n is a, c, g, or t

<400> 108
nnnnnnnnaa agnnnnn 17

<210> 109
<211> 11
<212> DNA
<213> unknown

<220>
<223> nodulin consensus sequence 1

<400> 109
aaaaagatca a 11

<210> 110
<211> 15
<212> DNA
<213> unknown

<220>
<223> C-repeat/dehydration response element

<400> 110
tcaagccgac acaga 15

<210> 111
<211> 19
<212> DNA
<213> unknown

<220>
<223> coupling element 3 (CE3)

<400> 111

tctctacgcg tgtctgtgt 19

<210> 112
<211> 19
<212> DNA
<213> unknown

<220>
<223> coupling element 3 (CE3)

<220>
<221> misc_feature
<222> (1)..(6)
<223> n is a, c, g, or t

<220>
<221> misc_feature
<222> (11)..(19)
<223> n is a, c, g, or t

<400> 112
nnnnnncgcg nnnnnnnnn 19

<210> 113
<211> 11
<212> DNA
<213> Arabidopsis thaliana

<400> 113
ctacgcgtgt c 11

<210> 114
<211> 11
<212> DNA
<213> Arabidopsis thaliana

<220>
<221> misc_feature
<222> (1)..(3)
<223> n is a, c, g, or t

<220>
<221> misc_feature
<222> (8)..(11)
<223> n is a, c, g, or t

<400> 114
nnncgcgnnn n 11

<210> 115
<211> 11
<212> DNA
<213> Arabidopsis thaliana

<400> 115
acacgcgtag a 11

<210> 116

<211> 21
<212> DNA
<213> Arabidopsis thaliana

<400> 116
gtggtgtgac gtcaaagtca t 21

<210> 117
<211> 21
<212> DNA
<213> Arabidopsis thaliana

<220>
<221> misc_feature
<222> (1)..(6)
<223> n is a, c, g, or t

<220>
<221> misc_feature
<222> (11)..(21)
<223> n is a, c, g, or t

<400> 117
nnnnnntgac nnnnnnnnnn n 21

<210> 118
<211> 21
<212> DNA
<213> Arabidopsis thaliana

<400> 118
tgactttgac gtcacaccac g 21

<210> 119
<211> 17
<212> DNA
<213> unknown

<220>
<223> WRKY plant specific zinc-finger-type factor associated with pathogen defence

<400> 119
tgactttgac gtcacac 17

<210> 120
<211> 17
<212> DNA
<213> oryza sativa

<400> 120
actttgacgt cacacca 17

<210> 121
<211> 17
<212> DNA
<213> oryza sativa

<220>

<221> misc_feature
<222> (1)..(6)
<223> n is a, c, g, or t

<220>
<221> misc_feature
<222> (11)..(17)
<223> n is a, c, g, or t

<400> 121
nnnnnnacgt nnnnnnn 17

<210> 122
<211> 15
<212> DNA
<213> alfalfa

<400> 122
ttttcgtggt gtgac 15

<210> 123
<211> 15
<212> DNA
<213> unknown

<220>
<223> Zinc-finger protein core motif

<220>
<221> misc_feature
<222> (1)..(5)
<223> n is a, c, g, or t

<220>
<221> misc_feature
<222> (10)..(15)
<223> n is a, c, g, or t

<400> 123
nnnnngtggn nnnnn 15

<210> 124
<211> 21
<212> DNA
<213> unknown

<220>
<223> bZIP protein G-Box binding factor 1

<400> 124
cgcttcatac gtgtcccttt a 21

<210> 125
<211> 21
<212> DNA
<213> unknown

<220>

<223> bZIP protein G-Box binding factor 1

<220>
<221> misc_feature
<222> (1)..(8)
<223> n is a, c, g, or t

<220>
<221> misc_feature
<222> (13)..(21)
<223> n is a, c, g, or t

<400> 125
nnnnnnnnac gtnnnnnnnn n 21

<210> 126
<211> 17
<212> DNA
<213> unknown

<220>
<223> ABA response elements

<400> 126
gcttcatacg tgtccct 17

<210> 127
<211> 17
<212> DNA
<213> unknown

<220>
<223> ABA response elements

<220>
<221> misc_feature
<222> (1)..(7)
<223> n is a, c, g, or t

<220>
<221> misc_feature
<222> (12)..(17)
<223> n is a, c, g, or t

<400> 127
nnnnnnnacg tnnnnnn 17

<210> 128
<211> 21
<212> DNA
<213> unknown

<220>
<223> OCS-like elements

<400> 128
agagataaag ggacacgtat g 21

<210> 129
<211> 21
<212> DNA
<213> unknown

<220>
<223> OCS-like elements

<220>
<221> misc_feature
<222> (1)..(14)
<223> n is a, c, g, or t

<220>
<221> misc_feature
<222> (19)..(21)
<223> n is a, c, g, or t

<400> 129
nnnnnnnnnn nnnnacgtnn n 21

<210> 130
<211> 25
<212> DNA
<213> unknown

<220>
<223> (GA)n/(CT)n binding proteins

<400> 130
actgagagag ataaagggac acgta 25

<210> 131
<211> 25
<212> DNA
<213> unknown

<220>
<223> (GA)n/(CT)n binding proteins

<220>
<221> misc_feature
<222> (1)..(6)
<223> n is a, c, g, or t

<220>
<221> misc_feature
<222> (11)..(25)
<223> n is a, c, g, or t

<400> 131
nnnnnnagag nnnnnnnnnn nnnnn 25

<210> 132
<211> 17
<212> DNA
<213> unknown

<220>
<223> class I GATA factors

<400> 132
agagagataa agggaca 17

<210> 133
<211> 25
<212> DNA
<213> unknown

<220>
<223> (GA)n/(CT)n binding proteins

<400> 133
atagagagac tgagagagat aaagg 25

<210> 134
<211> 25
<212> DNA
<213> unknown

<220>
<223> (GA)n/(CT)n binding proteins

<400> 134
ttatagagag actgagagag ataaa 25

<210> 135
<211> 25
<212> DNA
<213> unknown

<220>
<223> (GA)n/(CT)n binding proteins

<400> 135
gtttatagag agactgagag agata 25

<210> 136
<211> 15
<212> DNA
<213> unknown

<220>
<223> plant TATA box

<400> 136
tctctataaa cttag 15

<210> 137
<211> 15
<212> DNA
<213> unknown

<220>
<223> plant tata box

<220>
<221> misc_feature
<222> (1)..(4)
<223> n is a, c, g, or t

<220>
<221> misc_feature
<222> (9)..(15)
<223> n is a, c, g, or t

<400> 137
nnnntatann nnnnn 15

<210> 138
<211> 25
<212> DNA
<213> artificial

<220>
<223> core motif of GAGA/GAGABP.01 motif2

<220>
<221> misc_feature
<222> (1)..(6)
<223> n is a, c, g, or t

<220>
<221> misc_feature
<222> (11)..(25)
<223> n is a, c, g, or t

<400> 138
nnnnnnagac nnnnnnnnnn nnnnn 25

<210> 139
<211> 1800
<212> DNA
<213> Arabidopsis thaliana

<400> 139

```
atggagtcac agttgacacg tccttatggt catgagcaag cagaagaacc aatcagaatt      60
caccatccag aagaagagca tcatgagaag ggagcatcca aagtgttgaa gaaagtaaaa     120
gaaaaggcta agaaaatcaa gaacagtctc actaaacatg gaaatggtca tgatcacgat     180
gtggaagatg atgatgatga gtatgacgag caagacccag aagttcacgg cgcaccagtg     240
tatgaatcct ctgccgtgag aggtggtgta acgggtaaac ctaagtctct tagtcatgcc     300
ggagaaacta atgttccggc atcggaggag attgttcctc cagggacaaa agtttttcct     360
gtcgtgtctt ctgaccacac caaacccatt gagcctgtat cattacaaga tacctcttac     420
ggacatgagg cactggctga tcctgtaaga acgacggaaa catcggactg ggaagcgaaa     480
agagaggcac cgactcatta tcctctcgga gtgtcagaat tttcagacag aggagagagc     540
agagaggctc atcaagagcc attgaacact cctgtgtctc tgctttcagc aacagaggac     600
gtgactagga cgtttgctcc tggtggtgaa gatgactatc tcggtggtca acggaaagtc     660
aacgtcgaga cgccaaaacg tttggaggaa gatccggctg ctccaggagg aggatcggat     720
tatctcagtg gtgtatctaa ttatcagtcc aaagttactg atcccacgca taaaggtgga     780
gaagctggag taccagagat tgctgagtct cttggtagaa tgaaagtgac tgatgagtct     840
cctgatcaga aatcaagaca aggacgcgaa gaagactttc cgacgagaag ccatgagttt     900
gatctgaaga aggaatctga tatcaacaag aattctccgg caagatttgg aggggaatca     960
aaagctggga tggaggaaga ttttccgaca agaggtgatg tgaaagtaga gagtggattg    1020
ggaagagact taccgacggg aactcatgat cagttctcac cagaactatc tcgtcccaaa    1080
gagagagatg attctgagga aaccaaagat gagtcgacac atgagacaaa accaagcacc    1140
tacacagagc agttagcttc agctacatca gccataacta acaaagctat agccgcaaag    1200
aacgtcgttg cctcaaagct aggttacacc ggagagaatg gcggcgggca aagcgagagc    1260
cctgtaaaag atgaaactcc gagatctgtt actgcttacg ggcagaaagt ggcgggaact    1320
gttgctgaga agttgactcc ggtttacgaa aaagtcaaag aaacaggatc aacggtgatg    1380
acaaagctac ctctctccgg aggtggaagt ggagtgaagg agacgcaaca aggggaagag    1440
aaaggtgtga cggctaaaaa ttatatatca gagaagctga aacctggaga agaggacaaa    1500
gctttatcgg aaatgatagc tgagaaactt cattttggag gaggaggaga gaagaagaca    1560
acggctacaa aggaggtgga agtgacggtt gagaagatac cttccgacca gatagcggag    1620
gggaaaggac atggtgaggc ggttgcagag gaaggaaaag gtggagaagg aatggtgggg    1680
aaagttaaag gagcggtcac ttcttggctc ggtggtaaac cgaagtcgcc acggtccgtt    1740
gaagagtctc cacaatcact tggcaccacc gttgggacta tggggttttc ggattccggt    1800
```

<210> 140
<211> 618
<212> PRT
<213> Arabidopsis thaliana

<400> 140

```
Met Glu Ser Gln Leu Thr Arg Pro Tyr Gly His Glu Gln Ala Glu Glu
1               5                   10                  15

Pro Ile Arg Ile His His Pro Glu Glu Glu His His Glu Lys Gly Ala
            20                  25                  30

Ser Lys Val Leu Lys Lys Val Lys Glu Lys Ala Lys Lys Ile Lys Asn
        35                  40                  45

Ser Leu Thr Lys His Gly Asn Gly His Asp His Asp Val Glu Asp Asp
    50                  55                  60

Asp Asp Glu Tyr Asp Glu Gln Asp Pro Glu Val His Gly Ala Pro Val
65                  70                  75                  80

Tyr Glu Ser Ser Ala Val Arg Gly Gly Val Thr Gly Lys Pro Lys Ser
                85                  90                  95

Leu Ser His Ala Gly Glu Thr Asn Val Pro Ala Ser Glu Glu Ile Val
            100                 105                 110

Pro Pro Gly Thr Lys Val Phe Pro Val Val Ser Ser Asp His Thr Lys
        115                 120                 125

Pro Ile Glu Pro Val Ser Leu Gln Asp Thr Ser Tyr Gly His Glu Ala
    130                 135                 140

Leu Ala Asp Pro Val Arg Thr Thr Glu Thr Ser Asp Trp Glu Ala Lys
145                 150                 155                 160

Arg Glu Ala Pro Thr His Tyr Pro Leu Gly Val Ser Glu Phe Ser Asp
                165                 170                 175

Arg Gly Glu Ser Arg Glu Ala His Gln Glu Pro Leu Asn Thr Pro Val
            180                 185                 190

Ser Leu Leu Ser Ala Thr Glu Asp Val Thr Arg Thr Phe Ala Pro Gly
        195                 200                 205

Gly Glu Asp Asp Tyr Leu Gly Gly Gln Arg Lys Val Asn Val Glu Thr
    210                 215                 220

Pro Lys Arg Leu Glu Glu Asp Pro Ala Ala Pro Gly Gly Gly Ser Asp
225                 230                 235                 240

Tyr Leu Ser Gly Val Ser Asn Tyr Gln Ser Lys Val Thr Asp Pro Thr
                245                 250                 255
```

His Lys Gly Gly Glu Ala Gly Val Pro Glu Ile Ala Glu Ser Leu Gly
260 265 270

Arg Met Lys Val Thr Asp Glu Ser Pro Asp Gln Lys Ser Arg Gln Gly
275 280 285

Arg Glu Glu Asp Phe Pro Thr Arg Ser His Glu Phe Asp Leu Lys Lys
290 295 300

Glu Ser Asp Ile Asn Lys Asn Ser Pro Ala Arg Phe Gly Gly Glu Ser
305 310 315 320

Lys Ala Gly Met Glu Glu Asp Phe Pro Thr Arg Gly Asp Val Lys Val
325 330 335

Glu Ser Gly Leu Gly Arg Asp Leu Pro Thr Gly Thr His Asp Gln Phe
340 345 350

Ser Pro Glu Leu Ser Arg Pro Lys Glu Arg Asp Asp Ser Glu Glu Thr
355 360 365

Lys Asp Glu Ser Thr His Glu Thr Lys Pro Ser Thr Tyr Thr Glu Gln
370 375 380

Leu Ala Ser Ala Thr Ser Ala Ile Thr Asn Lys Ala Ile Ala Ala Lys
385 390 395 400

Asn Val Val Ala Ser Lys Leu Gly Tyr Thr Gly Glu Asn Gly Gly Gly
405 410 415

Gln Ser Glu Ser Pro Val Lys Asp Glu Thr Pro Arg Ser Val Thr Ala
420 425 430

Tyr Gly Gln Lys Val Ala Gly Thr Val Ala Glu Lys Leu Thr Pro Val
435 440 445

Tyr Glu Lys Val Lys Glu Thr Gly Ser Thr Val Met Thr Lys Leu Pro
450 455 460

Leu Ser Gly Gly Gly Ser Gly Val Lys Glu Thr Gln Gln Gly Glu Glu
465 470 475 480

Lys Gly Val Thr Ala Lys Asn Tyr Ile Ser Glu Lys Leu Lys Pro Gly
485 490 495

Glu Glu Asp Lys Ala Leu Ser Glu Met Ile Ala Glu Lys Leu His Phe
500 505 510

Gly Gly Gly Gly Glu Lys Lys Thr Thr Ala Thr Lys Glu Val Glu Val
515 520 525

Thr Val Glu Lys Ile Pro Ser Asp Gln Ile Ala Glu Gly Lys Gly His

530 535 540

Gly Glu Ala Val Ala Glu Glu Gly Lys Gly Gly Glu Gly Met Val Gly
545 550 555 560

Lys Val Lys Gly Ala Val Thr Ser Trp Leu Gly Gly Lys Pro Lys Ser
565 570 575

Pro Arg Ser Val Glu Glu Ser Pro Gln Ser Leu Gly Thr Thr Val Gly
580 585 590

Thr Met Gly Phe Ser Asp Ser Gly Gly Ser Glu Leu Gly Gly Ser Gly
595 600 605

Gly Gly Lys Gly Val Gln Asp Ser Gly Asn
610 615

<210> 141
<211> 844
<212> DNA
<213> Arabidopsis thaliana

<400> 141

```
cgcgccgcaa gaatctcaaa cacggagatc tcaaagtttg aaagaaaatt tatttcttcg      60
actcaaaaca aacttacgaa atttaggtag aacttatata cattatattg taattttttg     120
taacaaaatg tttttattat tattatagaa ttttactggt taaattaaaa atgaatagaa     180
aaggtgaatt aagaggagag aggaggtaaa cattttcttc tatttttttca tattttcagg    240
ataaattatt gtaaaagttt acaagatttc catttgacta gtgtaaatga ggaatattct     300
ctagtaagat cattatttca tctacttctt ttatcttcta ccagtagagg aataaacaat     360
atttagctcc tttgtaaata caaattaatt ttcgttcttg acatcattca attttaattt     420
tacgtataaa ataaaagatc atacctatta gaacgattaa ggagaaatac aattcgaatg     480
agaaggatgt gccgcttgtt ataataaaca gccacacgac gtaaacgtaa aatgaccaca     540
tgatgggcca atagacatgg accgactact aataatagta agttacattt taggatggaa     600
taaatatcat accgacatca gtttgaaaga aaagggaaaa aaagaaaaaa taaataaaag     660
atatactacc gacatgagtt ccaaaaagca aaaaaaaaga tcaagccgac acagacacgc     720
gtagagagca aaatgacttt gacgtcacac cacgaaaaca gacgcttcat acgtgtccct     780
ttatctctct cagtctctct ataaacttag tgagaccctc ctctgtttta ctcacaaatt     840
taat                                                                 844
```

<210> 142
<211> 710
<212> PRT
<213> Arabidopsis thaliana

<400> 142

```
Met Asp Gln Thr Glu Glu Pro Pro Leu Asn Thr His Gln Gln His Pro
1               5                   10                  15

Glu Glu Val Glu His His Glu Asn Gly Ala Thr Lys Met Phe Arg Lys
            20                  25                  30

Val Lys Ala Arg Ala Lys Lys Phe Lys Asn Ser Leu Thr Lys His Gly
        35                  40                  45

Gln Ser Asn Glu His Glu Gln Asp His Asp Leu Val Glu Glu Asp Asp
    50                  55                  60

Asp Asp Asp Glu Leu Glu Pro Glu Val Ile Asp Ala Pro Gly Val Thr
65                  70                  75                  80

Gly Lys Pro Arg Glu Thr Asn Val Pro Ala Ser Glu Glu Ile Ile Pro
                85                  90                  95

Pro Gly Thr Lys Val Phe Pro Val Val Ser Ser Asp Tyr Thr Lys Pro
            100                 105                 110

Thr Glu Ser Val Pro Val Gln Glu Ala Ser Tyr Gly His Asp Ala Pro
        115                 120                 125

Ala His Ser Val Arg Thr Thr Phe Thr Ser Asp Lys Glu Glu Lys Arg
    130                 135                 140

Asp Val Pro Ile His His Pro Leu Ser Glu Leu Ser Asp Arg Glu Glu
145                 150                 155                 160

Ser Arg Glu Thr His His Glu Ser Leu Asn Thr Pro Val Ser Leu Leu
                165                 170                 175

Ser Gly Thr Glu Asp Val Thr Ser Thr Phe Ala Pro Ser Gly Asp Asp
            180                 185                 190

Glu Tyr Leu Asp Gly Gln Arg Lys Val Asn Val Glu Thr Pro Ile Thr
        195                 200                 205

Leu Glu Glu Glu Ser Ala Val Ser Asp Tyr Leu Ser Gly Val Ser Asn
    210                 215                 220

Tyr Gln Ser Lys Val Thr Asp Pro Thr Lys Glu Glu Thr Gly Gly Val
225                 230                 235                 240

Pro Glu Ile Ala Glu Ser Phe Gly Asn Met Glu Val Thr Asp Glu Ser
                245                 250                 255

Pro Asp Gln Lys Pro Gly Gln Phe Glu Arg Asp Leu Ser Thr Arg Ser
            260                 265                 270

Lys Glu Phe Lys Glu Phe Asp Gln Asp Phe Asp Ser Val Leu Gly Lys
        275                 280                 285
```

Asp Ser Pro Ala Lys Phe Pro Gly Glu Ser Gly Val Val Phe Pro Val
290 295 300

Gly Phe Gly Asp Glu Ser Gly Ala Glu Leu Glu Lys Asp Phe Pro Thr
305 310 315 320

Arg Ser His Asp Phe Asp Met Lys Thr Glu Thr Gly Met Asp Thr Asn
325 330 335

Ser Pro Ser Arg Ser His Glu Phe Asp Leu Lys Thr Glu Ser Gly Asn
340 345 350

Asp Lys Asn Ser Pro Met Gly Phe Gly Ser Glu Ser Gly Ala Glu Leu
355 360 365

Glu Lys Glu Phe Asp Gln Lys Asn Asp Ser Gly Arg Asn Glu Tyr Ser
370 375 380

Pro Glu Ser Asp Gly Gly Leu Gly Ala Pro Leu Gly Gly Asn Phe Pro
385 390 395 400

Val Arg Ser His Glu Leu Asp Leu Lys Asn Glu Ser Asp Ile Asp Lys
405 410 415

Asp Val Pro Thr Gly Phe Asp Gly Glu Pro Asp Phe Leu Ala Lys Gly
420 425 430

Arg Pro Gly Tyr Gly Glu Ala Ser Glu Glu Asp Lys Phe Pro Ala Arg
435 440 445

Ser Asp Asp Val Glu Val Glu Thr Glu Leu Gly Arg Asp Pro Lys Thr
450 455 460

Glu Thr Leu Asp Gln Phe Ser Pro Glu Leu Ser His Pro Lys Glu Arg
465 470 475 480

Asp Glu Phe Lys Glu Ser Arg Asp Asp Phe Glu Glu Thr Arg Asp Glu
485 490 495

Lys Thr Glu Glu Pro Lys Gln Ser Thr Tyr Thr Glu Lys Phe Ala Ser
500 505 510

Met Leu Gly Tyr Ser Gly Glu Ile Pro Val Gly Asp Gln Thr Gln Val
515 520 525

Ala Gly Thr Val Asp Glu Lys Leu Thr Pro Val Asn Glu Lys Asp Gln
530 535 540

Glu Thr Glu Ser Ala Val Thr Thr Lys Leu Pro Ile Ser Gly Gly Gly
545 550 555 560

Ser Gly Val Glu Glu Gln Arg Gly Glu Asp Lys Ser Val Ser Gly Arg
565 570 575

Asp Tyr Val Ala Glu Lys Leu Thr Thr Glu Glu Glu Asp Lys Ala Phe
580 585 590

Ser Asp Met Val Ala Glu Lys Leu Gln Ile Gly Gly Glu Glu Glu Lys
595 600 605

Lys Glu Thr Thr Thr Lys Glu Val Glu Lys Ile Ser Thr Glu Lys Ala
610 615 620

Ala Ser Glu Glu Gly Glu Ala Val Glu Glu Glu Val Lys Gly Gly Gly
625 630 635 640

Gly Met Val Gly Arg Ile Lys Gly Trp Phe Gly Gly Gly Ala Thr Asp
645 650 655

Glu Val Lys Pro Glu Ser Pro His Ser Val Glu Glu Ala Pro Lys Ser
660 665 670

Ser Gly Trp Phe Gly Gly Gly Ala Thr Glu Glu Val Lys Pro Lys Ser
675 680 685

Pro His Ser Val Glu Glu Ser Pro Gln Ser Leu Gly Ser Thr Val Val
690 695 700

Pro Val Gln Lys Glu Leu
705 710

<210> 143
<211> 3533
<212> DNA
<213> Arabidopsis thaliana

<400> 143

gatctcaaag tttgaaagaa aatttatttc ttcgactcaa aacaaactta cgaaatttag 60
gtagaactta tatacattat attgtaattt tttgtaacaa aatgttttta ttattattat 120
agaattttac tggttaaatt aaaaatgaat agaaaaggtg aattaagagg agagaggagg 180
taaacatttt cttctatttt ttcatatttt caggataaat tattgtaaaa gtttacaaga 240
tttccatttg actagtgtaa atgaggaata ttctctagta agatcattat ttcatctact 300
tcttttatct tctaccagta gaggaataaa caatatttag ctcctttgta aatacaaatt 360
aattttcgtt cttgacatca ttcaatttta attttcgtat aaaataaaag atcataccta 420
ttagaacgat taaggagaaa tacaattcga atgagaagga tgtgccgttt gttataataa 480
acagccacac gacgtaaacg taaaatgacc acatgatggg ccaatagaca tggaccgact 540
actaataata gtaagttaca ttttaggatg gaataaatat cataccgaca tcagtttgaa 600
aagaaaaagg gaaaaaaaga aaaaataaat aaaagatata ctaccgacat gagttccaaa 660
aagcaaaaaa aaagatcaag ccgacacaga cacgcgtaga gagcaaaatg actttgacgt 720

cacaccacga aaacagacgc ttcatacgtg tccctttatc tctctcagtc tctctataaa 780
cttagtgaga ccctcctctg ttttactcac aaatatgcaa actagaaaac aatcatcagg 840
aataaagggt ttgattactt ctattggaaa gaaaaaaact ttggaaaatg gatcaaacag 900
aggaaccacc actcaacaca caccagcagc acccaggtag attctaattt cagaaactta 960
tatttttttt aagtgacaat cctctgaatt tacttaaact tattgtgatt tatggataca 1020
gaagaagttg aacatcatga gaatggtgcg actaagatgt ttaggaaagt aaaggctaga 1080
gctaagaagt tcaagaacag tctcactaaa catggacaaa gcaatgagca tgagcaagat 1140
catgatttgg ttgaagaaga tgatgatgat gacgagctag aacctgaagt gatcgatgca 1200
ccaggttact ttcttttgta gtttcattca acttatgatc taaaaactat tggttattca 1260
attttgcgtg acattaacgg tttggtatct gtatatgcag cgtaacaggt aaacctagag 1320
aaactaatgt tccagcatcg gaggaaatta ttccaccagg gacaaaggtg tttcctgtcg 1380
tgtcttccga ttacaccaaa cccactgaat ctgtaccagt acaagaggcc tcttacggac 1440
acgatgcacc ggctcattct gtaaggacga cgtttacatc ggacaaggaa gagaaaagag 1500
atgtaccgat tcatcatcct ctgtccgaat tgtcagacag agaagagagt agagagactc 1560
atcatgagtc attgaacact ccggtctctc tgctttctgg aacagaggat gtaacgagta 1620
cgtttgctcc aagtggtgat gatgaatatc ttgatggtca acggaaggtc aacgtcgaga 1680
ccccgataac gttggaggaa gagtcggctg tttcagacta tcttagtggt gtatctaatt 1740
atcagtccaa agttactgat cccaccaaag aaggtaagaa ctttgacctt ttaagattgt 1800
gtttttcttt agtgattatg aatatgtaat aactctgtta cgttgtgttt ggtttagaaa 1860
ctggaggagt accggagatt gctgagtctt ttggtaatat ggaagtgact gatgagtctc 1920
ctgatcagaa gccaggacaa tttgaaagag acttgtcgac gagaagcaaa gaattcaaag 1980
agtttgatca ggactttgac tctgttctcg gtaaggattc gccggcgaaa tttccaggtg 2040
aatcaggagt tgtttttcccg gtgggctttg gtgacgagtc aggagctgag ctggaaaaag 2100
attttccgac gagaagtcat gattttgata tgaagactga aactggaatg gacacgaatt 2160
ctccatcaag aagccatgaa tttgatctga agactgaatc tggaaacgac aagaattctc 2220
cgatgggctt tggtagtgaa tcaggagctg agctggaaaa agaatttgat cagaagaacg 2280
attctggaag aaacgagtat tcgccggaat ctgacggcgg tttaggagct ccgttgggag 2340
gaaattttcc ggtgagaagt catgagttgg atctgaagaa cgaatctgat atcgacaagg 2400
atgtgccgac gggatttgac ggagaaccag attttctggc gaagggaaga cctggatacg 2460
gtgaggcatc agaagaggat aaatttccgg cgagaagtga tgatgtggaa gtagagactg 2520
agctgggaag agacccaaag acggagactc ttgatcaatt ctcaccggaa ctttctcatc 2580
ctaaagaaag agatgagttt aaggagtcca gagatgattt tgaggagacg agagatgaga 2640
aaacagagga gccaaaacag agcacttaca cagagaagtt tgcttcaatg ctaggttact 2700
ccggagaaat tccggtggga gatcaaactc aagtggcggg aactgttgat gagaggttga 2760

```
ctccggtcaa tgagaaggat caagaaacag agtctgccgt gacgacgaag ttacctatct   2820
ccggaggtgg aagtggagta gaggagcaac gaggggaaga taaaagtgtg tcgggtagag   2880
attatgtggc ggagaaactg acaactgaag aagaagacaa agccttttct gatatggttg   2940
ccgagaaact tcagattgga ggagaagaag agaagaagga aacgacgaca aaggaagtgg   3000
agaagatctc taccgagaag gcagcatcgg aggagggtga ggcggtggaa gaggaagtga   3060
aaggaggagg aggaatggtt gggaggatta aaggatggtt cggtggtggt gcgactgatg   3120
aggtgaagcc agaatcgcca cattctgttg aagaggctcc aaaatcatct ggctggtttg   3180
gtggtggtgc gacggaggag gtgaagccaa aatcgcctca ttccgttgaa gagtctccac   3240
aatcacttgg ctccactgtt gttccggtgc agaaggagct ttaagaatat gagaactgag   3300
attttcaagt ttcactttgg atgtttatgt gtgttttgtt tgacgtcttt gatgtattat   3360
ggtataattc cttgtttgtg tgaaaaaagg acatttggtt aataaattgt tcggctttgg   3420
attaagaagt tcctccatac cagctactag gtctaaagtg ggtaaaatca ttggatttat   3480
tcccttcaaa gttcttagaa ttattcacag gatttacatt atgagctagt agt          3533
```

<210> 144
<211> 249
<212> DNA
<213> Oryza sativa

<400> 144

```
atgtcgtgct gcggaggaaa ctgcggctgc ggatccggct gccagtgcgg cagcggctgc     60
ggaggatgca agatgtaccc ggagatggct gaggaggtga ccactaccca gactgtcatc    120
atgggtgttg caccttccaa gggtcatgcc gaggggttgg aggccggcgc cgccgccgga    180
gcaggagcag agaacgggtg caagtgcggc gacaactgca cctgcaaccc ctgcaactgc    240
ggcaagtga                                                            249
```

## Description

### FIELD OF THE INVENTION

The present invention relates to the field of agricultural biotechnology. Disclosed herein are expression constructs with expression specificity for the germinating embryo, transgenic plants comprising such expression constructs, and methods of making and using such DNA constructs and transgenic plants.

### BACKGROUND OF THE INVENTION

In grain crops of agronomic importance, seed formation is the ultimate goal of plant development. Seeds are harvested for use in food, feed, and industrial products. The utility and value of those seeds are determined by the quantity and quality of protein, oil, and starch contained therein. In turn, the quality and quantity of seed produced may be affected by environmental conditions at any point prior to fertilization through seed maturation. In particular, stress at or around the time of fertilization may have substantial impact on seed development. Members of the grass family (Poaceae), which include the cereal grains, produce dry, one-seeded fruits. This type of fruit is, strictly speaking, a caryopsis but is commonly called a kernel or grain. The caryopsis of a fruit coat or pericarp surrounds the seed and adheres tightly to a seed coat. The seed consists of an embryo or germ and an endosperm enclosed by a nucellar epidermis and a seed coat. Accordingly the grain comprises the seed and its coat or pericarp. The seed comprises the embryo and the endosperm.

A fertile corn plant contains both male and female reproductive tissues, commonly known as the tassel and the ear, respectively. The tassel tissues form the haploid pollen grains with two nuclei in each grain, which, when shed at anthesis, contact the silks of a female ear. The ear may be on the same plant as that which shed the pollen, or on a different plant. The pollen cell develops a structure known as a pollen tube, which extends down through an individual female silk to the ovule. The two male nuclei travel through this tube to reach the haploid female egg at the base of the silk. One of the male nuclei fuses with and fertilizes the female haploid egg nuclei to form the zygote, which is diploid in chromosome number and will become the embryo within the kernel. The remaining male nucleus fuses with and fertilizes a second female nucleus to form the primary endosperm nucleus, which is triploid in number and will become the endosperm of the kernel, or seed, of the corn plant. Non-fertilized ovules do not produce kernels and the unfertilized tissues eventually degenerate.

The kernel consists of a number of parts, some derived from maternal tissue and others from the fertilization process. Maternally, the kernel inherits a number of tissues, including a protective, surrounding pericarp and a pedicel. The pedicel is a short stalk-like tissue which attaches the kernel to the cob and provides nutrient transfer from maternal tissue into the kernel. The kernel contains tissues resulting from the fertilization activities, including the new embryo as well as the endosperm. The embryo is the miniature progenitor of the next generation, containing cells for root and shoot growth of a new, young corn plant. It is also one tissue in which oils and proteins are stored in the kernel. The endosperm functions more as a nutritive tissue and provides the energy in the form of stored starch, proteins and oil, needed for the germination and initial growth of the embryo.

Considering the complex regulation that occurs during embryo and kernel development in higher plants, and considering that it is commonly grain that is a primary source of nutrition for animals and humans, key tools needed to improve such a nutritional source include genetic promoters that can drive the expression of nutrition enhancing genes. On the other hand the embryo is highly sensitive toward stresses. Stresses to plants may be caused by both biotic and abiotic agents. For example, biotic causes of stress include infection with a pathogen, insect feeding, and parasitism by another plant such as mistletoe, and grazing by ruminant animals. Abiotic stresses include, for example, excessive or insufficient available water, insufficient light, temperature extremes, synthetic chemicals such as herbicides, excessive wind, extremes of soil pH, limited nutrient availability, and air pollution. Yet plants survive and often flourish, even under unfavorable conditions, using a variety of internal and external mechanisms for avoiding or tolerating stress. Plants' physiological responses to stress reflect changes in gene expression.

While manipulation of stress-induced genes may play an important role in improving plant tolerance to stresses, it has been shown that constitutive expression of stress-inducible genes has a severe negative impact on plant growth and development when the stress is not present. (Kasuga 1999) Therefore, there is a need in the art for promoters driving expression which is temporally- and/or spatially-differentiated, to provide a means to control and direct gene expression in specific cells or tissues at critical times, especially to provide stress tolerance or avoidance. In particular, drought and/or density stress of maize often results in reduced yield. To stabilize plant development and grain yield under unfavorable environments, manipulation of hormones and nutritional supply to embryo (axis and scutellum) during seed germination is of interest. Thus there is a need for transcription regulating sequences which drive gene expression in embryo or scutellum under abiotic stress conditions.

One other well-known problem in the art of plant biotechnology is marker-deletion. Selectable marker are useful during the transformation process to select for, and identify, transformed organisms, but typically provide no useful function once the transformed organism has been identified and contributes substantially to the lack of acceptance of these "gene food" products among consumers (Kuiper 2001), and few markers are available that are not based on these mechanisms (Hare 2002). Thus, there are multiple attempts to develop techniques by means of which marker DNA can be excised from plant genome (Ow 1995; Gleave 1999). The person skilled in the art is familiar with a variety of systems for the site-directed removal of recombinantly introduced nucleic acid sequences. They are mainly based on the use of sequence specific recombinases. Various sequence-specific recombination systems are described, such as the Cre/lox system of the bacteriophage P1 (Dale 1991; Russell 1992; Osborne 1995), the yeast FLP/FRT system (Kilby 1995; Lyznik1996), the Mu phage Gin recombinase, the *E. coli* Pin recombinase, the R/RS system of the plasmid pSR1 (Onouchi1995; Sugita2000), the attP/bacteriophage Lambda system (Zubko 2000). It is one known disadvantage of these methods known in the prior art that excision is not homogenous through the entire plants thereby leading to mosaic-like excision patterns, which require laborious additional rounds of selection and regeneration.

Promoters that confer enhanced expression during seed or grain maturation are described (such as the barley hordein promoters; see US patent application 20040088754). Promoters which direct embryo-specific or seed-specific expression in dicots (*e.g.,* the soybean conglycinin promoter; Chen 1988; the napin promoter, Kridl 1991) are in general not capable to direct similar expression in monocots. Unfortunately, relatively few promoters specifically directing this aspect of physiology have been identified (see for example US20040163144).

WO2006/094976 discloses sequences homolog to SEQ ID 1 and 3 of the present invention. WO2005/103075, Al-Abed et al (2005) and WO2006/034479 disclose the use of a rd29A promoter for gene expression in monocotyledonous plants.

Since seed- or grain-specific promoters, which are described include those associated with genes encoding plant seed storage proteins such as genes encoding: barley hordeins, rice glutelins, oryzins, prolamines, or globulins; wheat gliadins or glutenins; maize zeins or glutelins; oat glutelins; sorghum kafirins; millet pennisetins; or rye secalins. However, on the one hand expression of these promoters is often leaky or of low expression level. Furthermore, it has been noted that improvement of crop plants with multiple transgenes ("stacking") is of increasing interest. For example, a single maize hybrid may comprise recombinant DNA constructs conferring not only insect resistance, but also resistance to a specific herbicide. Importantly, appropriate regulatory sequences are needed to drive the desired expression of each of these or other transgenes of interest. Furthermore, it is important that regulatory elements be distinct from each other. Concerns associated with the utilization of similar regulatory sequences to drive expression of multiple genes include, but are not restricted to: (a) pairing along homologous regions, crossing-over and loss of the intervening region either within a plasmid prior to integration, or within the plant genome, post-integration; (b) hairpin loops caused by two copies of the sequence in opposite orientation adjacent to each other, again with possibilities of excision and loss of these regulatory regions; (c) competition among different copies of the same promoter region for binding of promoter-specific transcription factors or other regulatory DNA-binding proteins.

There is, therefore, a great need in the art for the identification of novel sequences that can be used for expression of selected transgenes in economically important plants, especially in monocotyledonous plants. Also there is a need in the art for transcription regulating sequences which allow for expression in embryo or scutellum during the early germinating seed. It is thus an objective of the present invention to provide new and alternative expression cassettes for embryo-preferential or specific expression. The objective is solved by the present invention.

## SUMMARY OF THE INVENTION

The invention relates to an isolated nucleic acid molecule comprising a polynucleotide encoding a plant transcription regulating sequence comprising

i) a first nucleic acid selected from the group consisting of

a) a polynucleotide as defined in SEQ ID NO:1;
b) a polynucleotide having at least 70% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1mM EDTA at 50°C with washing in 0,5xSSC, 0.1% SDS at 50°C to a nucleic acid comprising at least 50 nucleotides of a polynucleotide as defined in SEQ ID NO:1, or the complement thereof, and

ii) a second nucleic acid selected from the group consisting of

a) a polynucleotide as defined in SEQ ID NO:3;
b) a polynucleotide having at least 60% sequence identity to the polynucleotide of SEQ ID NO:3;

c) a fragment of at least 50 consecutive bases, preferably at least 200 consecutive bases of the polynucleotide of SEQ ID NO:3; and

d) a polynucleotide hybridizing under conditions equivalent to hybridization in 7% sodium dodecyl sulfate (SDS), 0.5M $NaPO_4$, 1 mM EDTA at 50°C with washing in 0.5xSSC, 0.1 % SDS at 50°C to a nucleic acid comprising at least 50 nucleotides of a sequence described by SEQ ID NO:3, or the complement thereof,

wherein said first and said second nucleic acid sequences are functionally linked and heterologous to each other and wherein the isolated nucleic acid molecule comprising said first and second nucleic acid sequence is regulating embryo-specific expression of an operably linked nucleic acid sequence.

Promoter sequence of a drought, cold responsive and/or ABA regulated gene comprise promoters of a well known class of proteins. Preferably, it is the promoter sequence of a drought, cold responsive and ABA regulated gene. Examples of genes controlled by drought, cold responsive and/or ABA regulated gene are rd29A, rd29B, lti, or cor78.

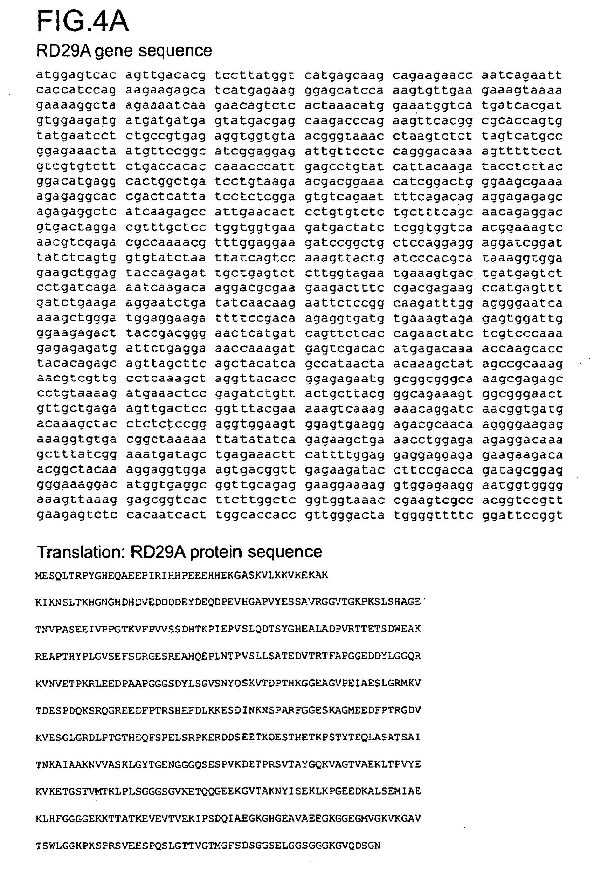

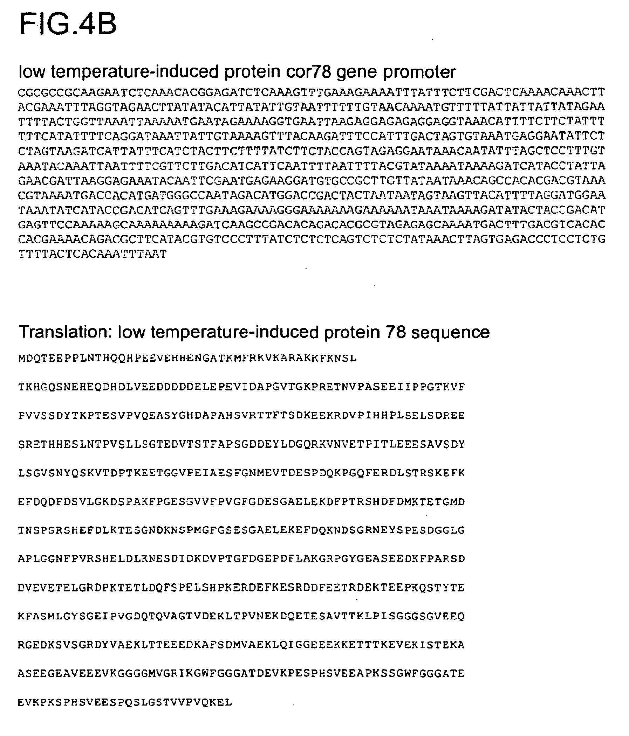

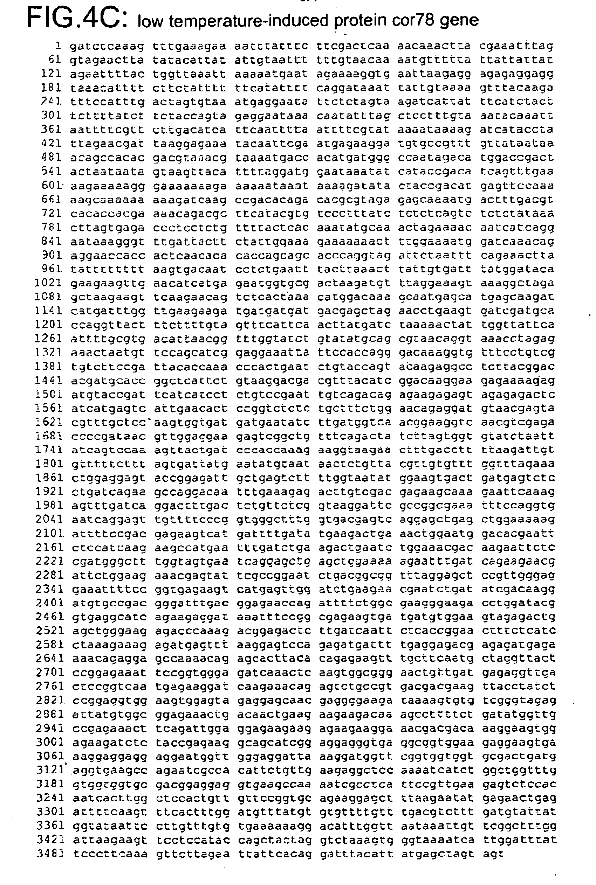

The Fig 4 sequence represents RD29A and low temperature-induced protein 78. The cor78 promoter is also known as rd29 promoter sequence. A BLAST with Genbank database refers for example to accession number Genbank AB019226, which comprises further genes. In this genome sequence, the cor78 promoter resides bp11743 to bp12328 in 5'-3' direction. The gene which resides downstream of this promoter region is low-temperature-induced protein 78 (e.g. bp 12650 to 12698,12784 to 12966,13063 to 13536,and 13621 to 15047).

The desiccation-responsive RD29 plant gene is for example published under Accession NM_001036984 with a 2131 bp mRNA, dated PLN 09-JUN-2006, VERSION NM_001036984.1 GI:79330663 and is described as a gene that encodes a protein that is induced in expression in response to water deprivation such as cold, high-salt, and dessication. The response appears to be via abscisic acid. The promoter region contains two ABA-responsive elements (ABREs) that are required for the dehydration-responsive expression of rd29B as cis-acting elements. Protein is a member of a gene family with other members found plants, animals and fungi. It is described as similar to stress-responsive protein-related [Arabidopsis thaliana] (TAIR:At4g25580.1). RD29 is also published as cor78 under Accession number GB: AAA32776.1.

Thus, in one embodiment, the promoter controlling the transcription of an mRNA encoding one of said proteins in a plant is used, e.g. the promoter of a gene encoding a stress-responsive protein, for example of Arabidopsis thaliana (TAIR:At4g25580.1), e.g. rd29A, rd29B, lti or cor78 (GB:AAA32776.1). Preferably, the promoter has the sequence shown in Fiq 4 or of a homolog thereof, e.g. of an ortholog.

Preferably, the cor78 promoter is derived from a dicotyledonous plant.

In one embodiment the homolog is a homolog predicted as ortholog found in Viridiplantae; preferably found in Streptophyta, more preferred in Embryophyta; Tracheophyta, even more preferred found in Spermatophyta. More preferred the homolog is identified from Magnoliophyta, more preferred it is form eudicotyledons, even more preferred the homolog is from core eudicotyledons, Thus, it is preferably from rosids; more prefered from eurosids II; even more preferred from Brassicales, even more preferred the homolog is e.g. form Brassicaceae, preferably form Arabbidopsis.

In one embodiment the homolog is from an Arabidopsis thaliana cultivar, e.g. from C24, or from a ecotype like "Columbia"

One further indication that two polypeptides are substantially similar to each other, besides having substantially the same function, is that an agent, *e.g.*, an antibody, which specifically binds to one of the polypeptides, also specifically binds to the other. Thus, in one embodiment, the homolog of RD29 is a protein which can be identified in an Western Blot Assay by binding to a monoclonal antibody generated for specifi binding to a protein having the amino acid shown in Fig. 4, respectively.

In one embodiment, the percentage of the gene which promoter is used is a nucleic acid or a protein with an amino acid sequence or nucleic acid identity of at least 60%, 61%, 62%, 63%, 64%, 65%, 66%, 67%, 68%, 69%, 70%, e.g., 71%, 72%, 73%, 74%, 75%, 76%, 77%, 78%, 79%, 80%, 81 %, 82%, 83%, 84%, 85%, 86%, 87%, 88%, 89%, and even 90% or more, e.g., 91 %, 92%, 93%, 94%, 95%, 96%, 97%, 98%, up to at least 99% to the amino acid sequence or the nucleic acid sequence shown in Fig. 4.

Preferably the gene which promoter is used, is a gene showing the expression pattern of a low-temperature-induced protein 78 or a RD29 gene. Preferably the promoter sequence connected to said gene is 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99% to the nucleic acid sequence as described by SEQ ID NO.: 1. Said promoters can be obtained by using at least 10, 15, 20, 30 or more consecutive nucleotides of SEQ ID NO.: 1, 5 or 6 or of a low-temperature-induced protein 78 or a RD29 gene e.g. as shown in Figures 4a to 4c using standard techniques for identification or cloning of nucleic acid sequences like, but not excluding others, database searches, hybridization or PCR based techniques.

The Met1 gene is published in Sasaki, T., et al., Nature 420 (6913), 312-316 (2002) and under Accession number AP002540 with a 249 bp DNA, linear, PLN 19-OCT-2004, VERSION AP002540.2 GI:13872872.

The sequence was submitted Submitted 21-JUN-2000 and on Apr 27, 2001 this sequence version replaced gi:8698578.

Genes were predicted from the integrated results of the following GENSCAN, FGENESH, GeneMark.hmm, GlimmerM, RiceHMM, SplicePredictor, sim4, gap2, BLASTN and BLASTX. The genomic sequence was searched against NCBI NonRedundant Protein database, nr and the cDNA sequence database at RGP or DDBJ. Protein homologies of the coding regions were searched against NCBI NonRedundant Protein database with BLASTP. ESTs represent the identified cDNA sequences using BLASTN with the corresponding DDBJ accession no. and RGP clone ID. Full-length cDNAs represent the identified cDNA sequences using BLASTN with the corresponding DDBJ accession no. A gene with identity or significant homology to a protein is classified based on the protein name to indicate the homology level, such as same name, 'putative-' and '-like protein'. A gene without significant homology to any protein but with full-length cDNA or EST homology (covering almost the entire length of partial sequence) is classified as an 'unknown' protein. A gene predicted by two or more gene prediction programs is classified as a 'hypothetical' protein according to IRGSP standard. A gene predicted by a single gene prediction program is also classified as a probable 'hypothetical' protein and is included as a miscellaneous feature of the sequence. The orientation of the sequence is from T7 to SP6 of the PAC clone. This sequence of P0434B04 clone has an overlap with P0416D03(DDBJ: AP002872) clone at 5' end and with P0009G03 (DDBJ: AP002522) clone at 3' end. Detailed information on overlap and assembly quality together with annotation of this entry is available at GenomeSeq.

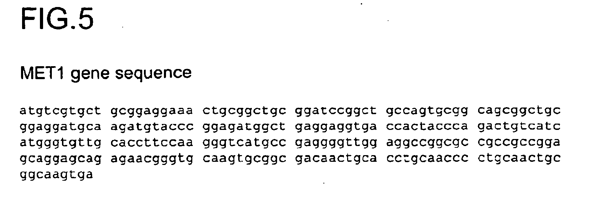

Preferably, an intron of the MET1 gene shown in Fig. 5 or a homolog thereof is used, e.g. of an ortholog.

Preferably, said first intron is derived from a MET1 gene from a monocotyledonous plant. The homolog is preferably an ortholog, e.g from Viridiplantae; e.g. from Streptophyta, more preferred from Embryophyta, even more preferred from Tracheophyta. The homolog is for example form Spermatophyta, preferably form Magnoliophyta, more preferred from Liliopsida, even more preferred Poales, even more preferred from Poaceae. In one embodiment the homolog is from BEP, clade, e.g. from Ehrhartoideae, more preferred from Oryzeae, e.g. from Oryza.

In one embodiment, an otholog is used, which is derived from Oryza sativa, e.g. form a cultivar like the japonica cultivar-group.

Peferably the homolog is an ortholog and shows substantially the same phenotype in a depletion assay as the wild type.

One further indication that two polypeptides are substantially similar to each other, besides having substantially the same function, is that an agent, *e.g.*, an antibody, which specifically binds to one of the polypeptides, also specifically binds to the other. Thus, in one embodiment, the homolog of MET1 is a protein which can be identified in an Western Blot Assay by binding to a monoclonal antibody generated for specifi binding to a protein having the amino acid shown in Fig. 5.

In one embodiment, the percentage homolog of a gene which intron is used, e.g. the first intron, is encoding a protein with an amino acid sequence or nucleic acid identity of at least 60%, 61 %, 62%, 63%, 64%, 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99% to the amino acid sequence shown in Fig. 5.

Preferably the homolog of a gene which intron is used is encoding a protein showing the expression pattern of the MET1 gene. Preferably the nucleic acid sequence of the intron of said gene is 60%, 61%, 62%, 63%, 64%, 65%, 66%, 67%, 68%, 69%, 70%, e.g., 71%, 72%, 73%, 74%, 75%, 76%, 77%, 78%, 79%, 80%, 81 %, 82%, 83%, 84%, 85%, 86%, 87%, 88%, 89%, and even 90% or more, e.g., 91%, 92%, 93%, 94%, 95%, 96%, 97%, 98%, up to at least 99% to the nucleic acid sequence as described by SEQ ID NO.: 3. Said promoters can be obtained by using at least 10, 15, 20, 30 or more consecutive nucleotides of SEQ ID NO.: 3, 7 or 8 or of a MET1 gene, e.g. as shown in Figure 5 in standard techniques for identification or cloning of nucleic acid sequences like, but not excluding others, database searches, hybridization or PCR based techniques.

Sequence comparisons maybe carried out using a Smith-Waterman sequence alignment algorithm (see *e.g.*, Waterman 1995). The localS program, version 1.16, is preferably used with following parameters: match: 1, mismatch penalty: 0.33, open-gap penalty: 2, extended-gap penalty: 2.

The distance between the cor78 promoter and the intron, e.g. the first intron of MET1 is preferably short. In one embodiment, the nucleic acid molecule comprises for example a linker sequence which is located between the cor78 promoter and and the first nucleotide of said intron with for example a length of 10 bp to 100bp, e.g. 10bp to 90 bp, or 20 to 80bp, e.g. 20, 30, 40, 50, 60, 70, 80 or 90 bp..

In an other embodiment of the present invention, said intron, e.g. the first intron of MET1, is located in the sequence of an other intron of a nucleotide sequence transcripted under the control of the transcription regulating nucleotide sequence.

Further, in one embodiment, said nucleic acid molecule comprises for example a 5'UTR which is located between the cor78 promoter sequence and the first nucleotide of the MET1 intron.

One possible arrangement is represented by base pairs 8199 to 9656 of SEQ ID NO: 2, comprising the cor79 promoter and the first intron of the MET1 gene, or by base pairs 8134 to 9656, comprising the cor79 promoter, the first intron of the MET1 gene and the 5'UTR.

The intron of the nucleotide sequence transcripted under the control of the transcription regulationg nucleotide sequence is for example located in the 5'UTR or in an other location close to the cor78 promoter sequence, e.g. it is located within the first 100bp of the 5' end of a coding region or within the first 100bp after the transcription start codon.

Accordingly, in one embodiment, tThe invention relates to an isolated nucleic acid molecule comprising a polynucleotide encoding a plant transcription regulating sequence comprising

i) a first nucleic acid selected from the group consisting of

a) a polynucleotide as defined in SEQ ID NO:1;
b) a polynucleotide having at least 70% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1mM EDTA at 50°C with washing in 0,5xSSC, 0.1 % SDS at 50°C to a nucleic acid comprising at least 50 nucleotides of a polynucleotide as defined in SEQ ID NO:1, or the complement thereof, and

ii) a second nucleic acid selected from the group consisting of

a) a polynucleotide as defined in SEQ ID NO:3;
b) a polynucleotide having at least 60% sequence identity to the polynucleotide of SEQ ID NO:3;
c) a fragment of at least 50 consecutive bases, preferably at least 200 consecutive bases of the polynucleotide of SEQ ID NO:3; and
d) a polynucleotide hybridizing under conditions equivalent to hybridization in 7% sodium dodecyl sulfate (SDS), 0.5M $NaPO_4$, 1mM EDTA at 50°C with washing in 0.5xSSC, 0.1 % SDS at 50°C to a nucleic acid comprising at least 50 nucleotides of a sequence described by SEQ ID NO:3, or the complement thereof,

wherein said first and said second nucleic acid sequences are functionally linked and heterologous to each other and wherein the isolated nucleic acid molecule comprising said first and second nucleic acid sequence is regulating embryo-specific expression of an operably linked nucleic acid sequence.

Preferably, the nucleic acid molecule comprising a polynucleotide encoding a transcription regulating sequence is capable to mediate transcription of an operably linked nucleic acid sequence in a plant or plant cell. More preferably, the nucleic acid molecule comprising a polynucleotide encoding a transcription regulating sequence is able to mediate transcription of a nucleic acid sequence in a monocotyledonous plant or monocotyledonous plant cell. It is preferred that the nucleic acid molecule comprising a polynucleotide encoding a transcription regulating sequence is tissue-specific. More preferably, the nucleic acid molecule comprising a polynucleotide encoding a transcription regulating sequence is preferentially expressed in embryo or scutellum. It is also preferred that the nucleic acid molecule comprising a polynucleotide encoding a transcription regulating sequence is preferentially expressed under abiotic or biotic stress conditions. The biotic stress conditions are selected from the group consisting of fungal, nematode, insect, virus, and bacteria and combinations thereof. The abiotic stress conditions are selected from the group consisting of drought, cold, heat, salt, salinity, high plant population density, nitrogen, UV light, and combinations thereof. Most preferably, the nucleic acid molecule comprising a polynucleotide encoding a transcription regulating sequence is preferentially expressed under drought condition.

Another embodiment of the current invention relates to the nucleic acid molecule comprising a polynucleotide encoding a transcription regulating sequence as described above, wherein the nucleic acid comprises at least two, preferably more, e.g. three, four, five, six, seven, eight, nine, ten or more, e.g. up to all, core promoter motifs selected from the group consisting of the sequences as defined in SEQ ID NOs: 10, 12, 14, 17, 20, 22, 25, 27, 29, 31, 33, 35, 37, 39, 42, 44, 46, 48, 50, 53, 55, 57, 59, 63, 66, 68, 70, 72, 74, 77, 79, 82, 84, 86, 88, 90, 94, 96, 98, 102, 104, 108, 112, 114, 117, 121, 123, 125, 127, 129, 131, 137, and 138. Preferably said promoter motifs are ananged on the minus or plus strand of the transcription regulating sequence as described in table 8 and 9. Even more preferred said promoter motifs are positioned at the same or similar position in the transcription regulating sequence as described in table 8 and9.

Yet another embodiment of the current invention relates to the nucleic acid molecule comprising a polynucleotide encoding a transcription regulating sequence as described above, wherein the nucleic acid comprises at least two, preferably more, e.g. three, four, five, six, seven, eight, nine, ten or more, e.g. up to all,promoter motifs selected from the group consisting of the sequences as defined in SEQ ID NOs: 9, 11, 13, 15, 16, 18, 19, 21, 23, 24, 26, 28, 30, 32, 34, 36, 38, 40, 41, 43, 45, 47, 49, 51, 52, 54, 56, 58, 60, 61, 62, 64, 65, 67, 69, 71, 73, 75, 76, 78, 80, 81, 83, 85, 87, 89, 91, 92, 93, 95, 97, 99, 100, 101, 103, 105, 106, 107, 109, 110, 111, 113, 115, 116, 118, 119, 120, 122, 124, 126, 128, 130, 132, 133, 134, 135, and 136.. Preferably said promoter motifs are aranged on the minus or plus strand of the transcription regulating sequence as described in table 8 and 9. Even more preferred said promoter motifs are positioned at the same or similar position in the transcription regulating sequence as described in table 8 and 9.

Another embodiment of the current invention relates to an expression construct comprising the nucleic acid molecule comprising a polynucleotide encoding a transcription regulating sequence functionally linked thereto a nucleic acid sequence. Preferably the functionally linked nucleic acid sequence confers to a plant a trait or property selected from the group consisting of increased yield, increased resistance under stress conditions, increased nutritional quality and/or oil content of a seed or a sprout, and selection marker excision. The increased nutritional quality and/or oil content may comprise an increased content of at least one compound selected from the group consisting of vitamins, carotinoids, antioxidants, unsaturated fatty acids, polyunsaturated fatty acids, or proteins with altered amino acid content. It is also preferred that the transcription of the functionally linked nucleic acid sequence in the expression construct results in expression of a protein or expression of a functional ribonucleotide sequence capable to impart function of at least one gene in the target plant. The functional RNA comprises at least one from the group consisting of: antisense RNA, sense RNA, dsRNA, microRNA, ta-siRNA, snR-NA, RNAi, or combinations thereof.

The embodiment of the current invention provides a plant or a seed produced by a transgenic plant transformed with a nucleic acid molecule comprising a polynucleotide encoding the transcription regulating sequence functionally linked to a nucleic acid. In a further preferred embodiment, the seed produced by the transgenic plant expresses a protein or a functional RNA sequence capable to impart function of at least one gene in the target plant, wherein the seed or plant has increased resistance under stress conditions and/or increased yield, and/or increased nutritional quality and/or oil content of a seed or a sprout. More preferably, the seed or plant is a monocot. Preferably, the seed or plant is selected from the group consisting of maize, wheat, rice barley, oat, rye, sorghum, ryegrass or coix. More preferably, the seed or plant is a cereal plant selected from the group consisting of maize, wheat, barley, rice, oat, rye, and sorghum, even more preferably from maize, wheat, and rice, most preferably the seed or plant is maize. Further embodiments of the invention relate to seeds, parts and cells of the monocotyledonous plant of the invention. Preferably, the plant parts are selected from the group consisting of: cells, protoplasts, cell tissue cultures, callus, cell clumps, embryos, pollen, ovules, seeds, flowers, kernels, ears, cobs, leaves, husks, stalks, roots, root tips, anthers, and silk.

Another embodiment of the invention relates to a method for increased yield and/or increased stress tolerance in a plant, wherein the method comprises the steps of

A) introducing into a plant an expression construct comprising

i) a first nucleic acid sequence selected from the group consisting of

a) a polynucleotide as defined in SEQ ID NO:1;
b) a polynucleotide having at least 70% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1mM EDTA at 50°C with washing in 0.5xSSC, 0.1 % SDS at 50°C to a nucleic acid comprising at least 50 nucleotides of a polynucleotide as defined in SEQ ID NO:1, or the complement thereof, and operably linked thereto a

ii) a second nucleic acid selected from the group consisting of

a) a polynucleotide as defined in SEQ ID NO:3;
b) a polynucleotide having at least 60% sequence identity to the polynucleotide of SEQ ID NO:3;
c) a fragment of at least 200 consecutive bases of the polynucleotide of SEQ ID NO:3; and
d) a polynucleotide hybridizing under conditions equivalent to hybridization in 7% sodium dodecyl sulfate (SDS), 0.5M $NaPO_4$, 1mM EDTA at 50°C with washing in 0.5xSSC, 0.1 % SDS at 50°C to a nucleic acid comprising at least 50 nucleotides of a sequence described by SEQ ID NO:3, or the complement thereof, wherein the isolated nucleic acid molecule comprising said first and second nucleic acid sequence is regulating embryo-specific expression of an operably linked nucleic acid sequence.

and operably linked to at least one nucleic acid which is heterologous in relation to the first or second nucleic acid sequence and is capable to confer to a plant an increased yield and/or increased stress tolerance, and

B) selecting transgenic plants, wherein the plants have increased yield and/or increased stress tolerance under stress conditions as compared to the wild type or null segregant plants.

Various nucleic acids are known to the person skilled in the art to obtain yield and/or stress resistance. The nucleic acids may include, but are not limited to, polynucleotides encoding a polypeptide involved in phytohormone biosynthesis, phytohormone regulation, cell cycle regulation, or carbohydrate metabolism.

Another embodiment of the invention relates to a method for conferring increased nutritional quality and/or oil content of a seed or a sprout to a plant, wherein the method comprises the steps of

A) introducing into a plant an expression construct comprising

i) a first nucleic acid selected from the group consisting of

a) a polynucleotide as defined in SEQ ID NO:1;
b) a polynucleotide having at least 70% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1 mM EDTA at 50°C with washing in 0.5xSSC, 0.1 % SDS at 50°C to a nucleic acid comprising at least 50 nucleotides of a polynucleotide as defined in SEQ ID NO:1, or the complement thereof, and operably linked to

ii) a second nucleic acid selected from the group consisting of

a) a polynucleotide as defined in SEQ ID NO:3;
b) a polynucleotide having at least 60% sequence identity to the polynucleotide of SEQ ID NO:3;
c) a fragment of at least 200 consecutive bases of the polynucleotide of SEQ ID NO:3; and
d) a polynucleotide hybridizing under conditions equivalent to hybridization in 7% sodium dodecyl sulfate (SDS), 0.5M $NaPO_4$, 1 mM EDTA at 50°C with washing in 0.5xSSC, 0.1 % SDS at 50°C to a nucleic acid comprising at least 50 nucleotides of a sequence described by SEQ ID NO:3, or the complement thereof, wherein the isolated nucleic acid molecule comprising said first and second nucleic acid sequence is regulating embryo-specific expression of an operably linked nucleic acid sequence.

and operably linked to at least one nucleic acid which is heterologous in relation to said first or said second nucleic acid sequence and is suitable to confer to a plant an increased nutritional quality and/or oil content of a seed or a sprout, and

B) selecting transgenic plants, wherein the plants have increased nutritional quality and/or oil content of a seed or a sprout as compared to the wild type or null segregant plants.

The nutritional quality and/or oil content are defined as above. More specific examples are given herein below. The transcription regulating sequence is described above, more preferably the transcription regulating sequence is embryo-specific.

Yet another embodiment of the invention relates to a method for excision of a target sequence, e.g. a marker sequence from a plant, said method comprising the steps of

A) constructing an expression cassette by operably linking a transcription regulating nucleotide sequence comprising

i) a first nucleic acid selected from the group consisting of

a) a polynucleotide as defined in SEQ ID NO:1;
b) a polynucleotide having at least 70% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1 mM EDTA at 50°C with washing in 0.5xSSC, 0.1 % SDS at 50°C to a nucleic acid comprising at least 50 nucleotides of a polynucleotide as defined in SEQ ID NO:1, or the complement thereof, and

ii) a second nucleic acid selected from the group consisting of

a) a polynucleotide as defined in SEQ ID NO:3;
b) a polynucleotide having at least 60% sequence identity to the polynucleotide of SEQ ID NO:3;
c) a fragment of at least 200 consecutive bases of the polynucleotide of SEQ ID NO:3; and
d) a polynucleotide hybridizing under conditions equivalent to hybridization in 7% sodium dodecyl sulfate

(SDS), 0.5M $NaPO_4$, 1 mM EDTA at 50°C with washing in 0.5xSSC, 0.1 % SDS at 50°C to a nucleic acid comprising at least 50 nucleotides of a sequence described by SEQ ID NO:3, or the complement thereof, wherein the isolated nucleic acid molecule comprising said first and second nucleic acid sequence is regulating embryo-specific expression of an operably linked nucleic acid sequence.

and operably linked to at least one nucleic acid which is heterologous in relation to said first or said second nucleic acid sequence and is suitable to induce excision of target sequence, e.g. a marker sequence from a plant, and

B) inserting said expression cassette into a plant comprising at least one target sequence, e.g. a marker sequence to provide a transgenic plant, wherein said plant expresses said heterologous nucleic acid sequence, and
C) selecting transgenic plants, which demonstrate excision of said target sequence, e.g. the marker sequence.

Preferably the target sequence is a marker sequence, e.g. a antibotic or herbicid resistance gene.
In one preferred embodiment of the invention the nucleotide sequence expressed from the chimeric transcription regulating sequence of the invention is not encoding a beta-glucuronidase (GUS), or is not a method for expression of a GUS gene for the purpose of achieving a GUS-mediating staining.

## BRIEF DESCRIPTION OF THE DRAWINGS

Fig. 1. Map of plasmid pBPSMM368:At.Cor78::BPS1.1::GUS::NOS

Fig. 2. Histochemical GUS staining of At.Cor78 promoter constructs. Shown are representative examples for the typical staining patterns of the constructs. A: At.Cor78::BPS1.1::GUS::NOS (pBPSMM368); B: At.Cor78::GUS:: NOS (pBPSMM250); C: At.Cor78::Ubi-intron1::GUS::NOS (pBPSMM346)

Fig. 3. Drought-stress-induced or stable expression controlled by pBPSMM346 promoter construct in maize. Transgenic plants at 5-leaf stage were drought-stressed by withholding water. Samples were taken from leaves at the indicated timepoints. RNA was isolated from leaf samples and analyzed with quantitative RT-PCR. GUS expression was normalized against an internal control gene in each sample. Results are shown as fold increase of expression levels compared to the 0-timepoint which is set as 1.

Fig. 4a to 4c: Nucleotide sequence and amino acid sequence of the RD29A gene and the low temperature-induced protein 78 gene.

Fig. 5: Nucleotide sequence of the MET1 gene.

## DEFINITIONS

Unless otherwise noted, technical terms are used according to conventional usage. Definitions of common terms in molecular biology may be found in Lewin, Genes V published by Oxford University Press, 1994 (ISBN 0-19-854187-9); Kendrew et al (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).
It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a" and "the" include plural reference unless the context clearly dictates otherwise. Thus, for example, reference to "a vector" is a reference to one or more vectors and includes equivalents thereof known to those skilled in the art, and so forth.
The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower).
As used herein, the word "or" means any one member of a particular list and also includes any combination of members of that list.

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes can include non-coding DNA sequences and/or the regulatory sequences, wherein the non-coding regions can be transcribed into, such as microRNA. Genes also include non-expressed segments that, for example, form recognition sequences for other proteins and/or RNA. Genes can be obtained from a variety of sources, including cloning from a source of interest or synthesizing from known or predicted sequence information via chemical or molecular biology approaches, and may include sequences designed to have desired parameters.

The term "native" or "wild type" gene refers to a gene that is present in the genome of an untransformed cell, *i.e.,* a cell not having a known mutation.

A "marker gene" or "marker sequence" encodes a selectable or screenable trait.

The term "chimeric gene" refers to any gene that contains

1) DNA sequences, including regulatory and coding sequences, that are not found together in nature, or
2) sequences encoding parts of coding sequences, e.g. coding for proteins, not naturally adjoined, or
3) parts of regulatory sequeces, e.g. promoters, that are not naturally adjoined.

Accordingly, a chimeric gene may comprise regulatory sequences and coding sequences that are derived from different sources, or comprise regulatory sequences and coding sequences derived from the same source, but arranged in a manner different from that found in nature.

A "transgene" refers to a gene that has been introduced into the genome by transformation and is stably maintained. Transgenes may include, for example, genes that are either heterologous or homologous to the genes of a particular plant to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

An "oligonucleotide" corresponding to a nucleotide sequence of the invention, *e.g*., for use in probing or amplification reactions, may be about 30 or fewer nucleotides in length (*e.g*., 9, 12, 15, 18, 20, 21 or 24, or any number between 9 and 30). Generally specific primers are upwards of 14 nucleotides in length. For optimum specificity and cost effectiveness, primers of 16 to 24 nucleotides in length may be preferred. Those skilled in the art are well versed in the design of primers for use processes such as PCR. If required, probing can be done with entire restriction fragments of the gene disclosed herein which may be 100's or even 1,000's of nucleotides in length.

The terms "polypeptide", "peptide", "oligopeptide", "polypeptide", "gene product", "expression product" and "protein" are used interchangeably herein to refer to a polymer or oligomer of consecutive amino acid residues. As used herein, the term "amino acid sequence" or a "polypeptide sequence" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. The abbreviations used herein are conventional one letter codes for the amino acids: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid (see L. Stryer, Biochemistry, 1988, W. H. Freeman and Company, New York. The letter "x" as used herein within an amino acid sequence can stand for any amino acid residue.

"Coding sequence" refers to a DNA or RNA sequence that codes for a specific amino acid sequence and excludes the non-coding sequences. It may constitute an "uninterrupted coding sequence", i.e., lacking an intron, such as in a cDNA or it may include one or more introns bounded by appropriate splice junctions. An "intron" is a sequence of RNA which is contained in the primary transcript but which is removed through cleavage and re-ligation of the RNA within the cell to create the mature mRNA that can be translated into a protein.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

A "functional RNA" refers to an antisense RNA, ribozyme, or other RNA that is not translated.

The term "RNA transcript" refers to the product resulting from RNA polymerase catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA" (mRNA) refers to the RNA that is without introns and that can be translated into protein by the cell. "cDNA" refers to a single- or a double-stranded DNA that is complementary to and derived from mRNA.

"Transcription regulating nucleotide sequence", "regulatory sequences", and "suitable regulatory sequences", each refers to nucleotide sequences influencing the transcription, RNA processing or stability, or translation of the associated (or functionally linked) nucleotide sequence to be transcribed. The transcription regulating nucleotide sequence may have various localizations with the respect to the nucleotide sequences to be transcribed. The transcription regulating nucleotide sequence may be located upstream (5' non-coding sequences), within, or downstream (3' non-coding sequences) of the sequence to be transcribed (*e.g.*, a coding sequence). The transcription regulating nucleotide sequences may be selected from the group comprising enhancers, promoters, translation leader sequences, introns, 5'-untranslated sequences, 3'-untranslated sequences, and polyadenylation signal sequences. They include natural and synthetic sequences as well as sequences, which may be a combination of synthetic and natural sequences. As is noted above, the term "transcription regulating nucleotide sequence" is not limited to promoters. However, preferably a transcription regulating nucleotide sequence of the invention comprises at least one promoter sequence (*e.g.*, a sequence localized upstream of the transcription start of a gene capable to induce transcription of the downstream sequences). In one preferred embodiment the transcription regulating nucleotide sequence of the invention comprises the promoter sequence of the corresponding gene and - optionally and preferably - the native 5'-untranslated region of said gene. Furthermore, the 3'-untranslated region and/or the polyadenylation region of said gene may also be employed.

"Promoter" refers to a nucleotide sequence, usually upstream (5') to its coding sequence, which controls the expression of the coding sequence by providing the recognition for RNA polymerase and other factors (*e.g.*, trans-acting transcription factors) required for proper transcription. "Promoter" includes a minimal promoter that is a short DNA sequence comprised of a TATA box and other sequences that serve to specify the site of transcription initiation, to which regulatory elements (*e.g.*, *cis*-elements) are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements are often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements, derived from different promoters found in nature, or even be comprised of synthetic DNA segments. A promoter may also contain DNA sequences that are involved in the binding of protein factors which control the effectiveness of transcription initiation in response to physiological or developmental conditions. As used herein, the term "cis-element" refers to a *cis*-acting transcriptional regulatory element that confers an aspect of the overall control of gene expression. A *cis*-element may function to bind transcription factors, *trans*-acting protein factors that regulate transcription. Some *cis*-elements bind more than one transcription factor, and transcription factors may interact with different affinities with more than one cis-element. The promoters of the present invention desirably contain *cis*-elements that can confer or modulate gene expression. *Cis*-elements can be identified by a number of techniques, including deletion analysis, *i.e.*, deleting one or more nucleotides from the 5' end or internal to a promoter; DNA binding protein analysis using DNase I footprinting, methylation interference, electrophoresis mobility-shift assays, *in vivo* genomic footprinting by ligation-mediated PCR, and other conventional assays; or by DNA sequence similarity analysis with known *cis*-element motifs by conventional DNA sequence comparison methods. The fine structure of a *cis*-element can be further studied by mutagenesis (or substitution) of one or more nucleotides or by other conventional methods. *Cis*-elements can be obtained by chemical synthesis or by isolation from promoters that include such elements, and they can be synthesized with additional flanking nucleotides that contain useful restriction enzyme sites to facilitate subsequence manipulation.

The "initiation site" is the position surrounding the first nucleotide that is part of the transcribed sequence, which is also defined as position +1. With respect to this site all other sequences of the gene and its controlling regions are numbered. Downstream sequences (*i.e.*, further protein encoding sequences in the 3' direction) are denominated positive, while upstream sequences (mostly of the controlling regions in the 5' direction) are denominated negative.

Promoter elements, particularly a TATA element, that are inactive or that have greatly reduced promoter activity in the absence of upstream activation are referred to as "minimal or core promoters." In the presence of a suitable transcription factor, the minimal promoter functions to permit transcription. A "minimal or core promoter" thus consists only of all basal elements needed for transcription initiation, *e.g.*, a TATA box and/or an initiator.

The term "intron" refers to sections of DNA (intervening sequences) within a gene that do not encode part of the protein that the gene produces, and that is spliced out of the mRNA that is transcribed from the gene before it is exported from the cell nucleus. Intron sequence refers to the nucleic acid sequence of an intron. Thus, introns are those regions of DNA sequences that are transcribed along with the coding sequence (exons) but are removed during the formation of mature mRNA. Introns can be positioned within the actual coding region or in either the 5' or 3' untranslated leaders of the pre-mRNA (unspliced mRNA). Introns in the primary transcript are excised and the coding sequences are simultaneously and precisely ligated to form the mature mRNA. The junctions of introns and exons form the splice site. The sequence of an intron begins with GU and ends with AG. Furthermore, in plants, two examples of AU-AC introns have been described: intron 14 of the RecA-like protein gene and intron 7 of the G5 gene from Arabidopsis thaliana are AT-AC introns, Pre-mRNAs containing introns have three short sequences that are -beside other sequences- essential for the intron to be accurately spliced. These sequences are the 5'splice-site, the 3' splice-site, and the branchpoint. mRNA splicing is the removal of intervening sequences (introns) present in primary mRNA transcripts and joining or ligation of exon sequences. This is also known as cis-splicing which joins two exons on the same RNA with the removal of the intervening sequence (intron). The functional elements of an intron comprising sequences that are recognized and bound by the specific protein components of the spliceosome (*e.g.* splicing consensus sequences at the ends of introns). The interaction of the functional elements with the spliceosome results in the removal of the intron sequence from the premature mRNA and the rejoining of the exon sequences. Introns have three short sequences that are essential -although not sufficient- for the intron to be accurately spliced. These sequences are the 5' splice site, the 3' splice site and the branchpoint The branchpoint sequence is important in splicing and splice-site selection in plants. The branchpoint sequence is usually located 10-60 nucleotides upstream of the 3' splice site. Plant sequences exhibit sequence deviations in the branchpoint, the consensus sequences being CURAY or YURAY.

"Constitutive expression" refers to expression using a constitutive or regulated promoter. "Conditional" and "regulated expression" refer to expression controlled by a regulated promoter.

"Constitutive promoter" refers to a promoter that is able to express the open reading frame (ORF) that it controls in all or nearly all of the plant tissues during all or nearly all developmental stages of the plant. Each of the transcription-activating elements does not exhibit an absolute tissue-specificity, but mediate transcriptional activation in most plant parts at a level of at least 1 % of the level reached in the part of the plant in which transcription is most active.

"Regulated promoter" refers to promoters that direct gene expression not constitutively, but in a temporally- and/or spatially-regulated manner, and includes both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered, numerous examples may be found in the compilation by Okamuro *et al.* (1989). Typical regulated promoters useful in plants include but are not limited to safener-inducible promoters, promoters derived from the tetracycline-inducible system, promoters derived from salicylate-inducible systems, promoters derived from alcohol-inducible systems, promoters derived from glucocorticoid-inducible system, promoters derived from pathogen-inducible systems, and promoters derived from ecdysone-inducible systems.

"Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of senescence.

"Inducible promoter" refers to those regulated promoters that can be turned on in one or more cell types by an external stimulus, such as a chemical, light, hormone, stress, or a pathogen.

"Operably-linked" or "functionally linked" refers preferably to the association of nucleic acid sequences on single nucleic acid fragment so that the function of one is affected by the other. More specifically, it refers to a first sequence (s) being positioned sufficiently proximal to a second sequence(s) so that the first sequence(s) can exert influence over the second sequence(s) or a region under control of that second sequence. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (*i.e.*, that the coding sequence or functional RNA is under the transcriptional control of the promoter).

Most activating sequences, e.g. an intron, are within about 300 to 400 base pairs of the promoter that is enhanced.

In one embodiment, the location of the intron, e.g. an INME intron, is in the 5' UTR of the controlled gene. (Rose et al. RNA 2002, 8:1444-1453; Callis et al., 1987, Genes & Dev 1:1183-1200; PF56400).

The linker between the promoter region and the intron is for example below 400 base pairs, e.g. 200, 100, 50 or less. Preferably the fist sequence of the nucleic acid molecule is linked to the second sequence by a linker of around 100 nucleotides or less.

In a further embodiment of the invention more than one activating sequence is employed and the activating sequences are within about 16 to about 80 base pairs of each other. The coding sequence can be operably-linked to regulatory sequences in a sense or antisense orientation, e.g. being transcripted into a mRNA encoding a polypeptide or a fragment of a protein or being transcripted into a regulatory or enzymatic RNA molecule, e.g. a antisense RNA, a RNAi, a Ribozyme, a miRNA, a ta-siRNA, a dsRNA, snRNA or others like described herein or in relevant literature, or combinations thereof.

"Expression" refers to the transcription and/or translation of an endogenous gene, ORF or portion thereof, or a transgene in plants. For example, in the case of antisense constructs, expression may refer to the transcription of the antisense DNA only. In addition, expression refers to the transcription and stable accumulation of sense (mRNA) or functional RNA. Expression may also refer to the production of protein.

"Specific expression" is the expression of gene products which is limited to one or a few plant tissues (spatial limitation) and/or to one or a few plant developmental stages (temporal limitation). It is acknowledged that hardly a true specificity exists: promoters seem to be preferably switch on in some tissues, while in other tissues there can be no or only little activity. This phenomenon is known as leaky expression. However, with specific expression in this invention is meant preferable expression in one or a few plant tissues.

The "expression pattern" of a promoter (with or without enhancer) is the pattern of expression levels which shows where in the plant and in what developmental stage transcription is initiated by said promoter. Expression patterns of a set of promoters are said to be complementary when the expression pattern of one promoter shows little overlap with the expression pattern of the other promoter. The level of expression of a promoter can be determined by measuring the 'steady state' concentration of a standard transcribed reporter mRNA. This measurement is indirect since the concentration of the reporter mRNA is dependent not only on its synthesis rate, but also on the rate with which the mRNA is degraded. Therefore, the steady state level is the product of synthesis rates and degradation rates. The rate of degradation can however be considered to proceed at a fixed rate when the transcribed sequences are identical, and thus this value can serve as a measure of synthesis rates. When promoters are compared in this way, techniques available to those skilled in the art are hybridization, S1-RNAse analysis, northern blots and competitive RT-PCR. This list of techniques in no way represents all available techniques, but rather describes commonly used procedures to analyze transcription activity and expression levels of mRNA. The analysis of transcription start points in practically all promoters has revealed that there is usually no single base at which transcription starts, but rather a more or less clustered set of initiation sites, each of which accounts for some start points of the mRNA. Since this distribution varies from promoter to promoter the sequences of the reporter mRNA in each of the populations would differ from each other. Since each mRNA species is more or less prone to degradation, no single degradation rate can be expected for different reporter mRNAs. It has been shown for various eukaryotic promoter sequences that the sequence surrounding the initiation site ('initiator') plays an important role in determining the level of RNA expression directed by that specific promoter. This includes also part of the transcribed sequences. The direct fusion of promoter to reporter sequences would therefore lead to suboptimal levels of transcription. A commonly used procedure to analyze expression patterns and levels is through determination of the 'steady state' level of protein accumulation in a cell. Commonly used candidates for the reporter gene, known to those skilled in the art are beta-glucuronidase (GUS), chloramphenicol acetyl transferase (CAT) and proteins with fluorescent properties, such as green fluorescent protein (GFP) from Aequora victoria. In principle, however, many more proteins are suitable for this purpose, provided the protein does not interfere with essential plant functions. For quantification and determination of localization a number of tools are suited. Detection systems can readily be created or are available which are based on, *e.g.*, immunochemical, enzymatic, fluorescent detection and quantification. Protein levels can be determined in plant tissue extracts or in intact tissue using in situ analysis of protein expression. Generally, individual transformed lines with one chimeric promoter reporter construct will vary in their levels of expression of the reporter gene. Also frequently observed is the phenomenon that such transformants do not express any detectable product (RNA or protein). The variability in expression is commonly ascribed to 'position effects', although the molecular mechanisms underlying this inactivity are usually not clear.

"Overexpression" refers to the level of expression in transgenic cells or organisms that exceeds levels of expression in normal or untransformed (non-transgenic) cells or organisms.

"5' non-coding sequence" or "5'-untranslated sequence" or "-region" refers to a nucleotide sequence located 5' (upstream) to the coding sequence. It is present in the fully processed mRNA upstream of the initiation codon and may affect processing of the primary transcript to mRNA, mRNA stability or translation efficiency (Turner 1995).

"3' non-coding sequence" or "3'-untranslated sequence" or "-region" refers to nucleotide sequences located 3' (downstream) to a coding sequence and include polyadenylation signal sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht *et al.*, 1989.

The term "translation leader sequence" refers to that DNA sequence portion of a gene between the promoter and coding sequence that is transcribed into RNA and is present in the fully processed mRNA upstream (5') of the translation start codon. The translation leader sequence may affect processing of the primary transcript to mRNA, mRNA stability or translation efficiency.

"Signal peptide" refers to the amino terminal extension of a polypeptide, which is translated in conjunction with the polypeptide forming a precursor peptide and which is required for its entrance into the secretory pathway. The term "signal sequence" refers to a nucleotide sequence that encodes the signal peptide. The term "transit peptide" as used herein refers to a part of an expressed polypeptide (preferably to the amino terminal extension of a polypeptide), which is translated in conjunction with the polypeptide forming a precursor peptide and which is required for its entrance into a cell organelle (such as the plastids (*e.g.*, chloroplasts) or mitochondria). The term "transit sequence" refers to a nucleotide sequence that encodes the transit peptide.

"Antisense inhibition" refers to the production of antisense RNA transcripts capable of suppressing the expression of protein from an endogenous gene or a transgene.

"Gene silencing" refers to homology-dependent suppression of viral genes, transgenes, or endogenous nuclear genes. Gene silencing may be transcriptional, when the suppression is due to decreased transcription of the affected genes, or post-transcriptional, when the suppression is due to increased turnover (degradation) of RNA species homologous to the affected genes (English 1996). Gene silencing includes virus-induced gene silencing (Ruiz *et al.* 1998).

The terms "heterologous DNA sequence", "exogenous DNA segment" or "heterologous nucleic acid," as used herein, each refers to a sequence that originates from a source foreign to the particular host cell or, if from the same source, is modified from its original form. Thus, a heterologous gene in a host cell includes a gene that is endogenous to the particular host cell but has been modified through, for example, the use of DNA shuffling. The terms also include non-naturally occurring multiple copies of a naturally occurring DNA sequence. Thus, the terms refer to a DNA segment that is foreign or heterologous to the cell, or homologous to the cell but in a position within the host cell nucleic acid in which the element is not ordinarily found. Exogenous DNA segments are expressed to yield exogenous polypeptides. A "homologous" DNA sequence is a DNA sequence that is naturally associated with a host cell into which it is introduced.

"Homologous to" in the context of nucleotide sequence identity refers to the similarity between the nucleotide sequence of two nucleic acid molecules or between the amino acid sequences of two protein molecules. Estimates of such homology are provided by either DNA-DNA or DNA-RNA hybridization under conditions of stringency as is well understood by those skilled in the art (as described in Haines and Higgins (eds.), Nucleic Acid Hybridization, IRL Press, Oxford, U.K.), or by the comparison of sequence similarity between two nucleic acids or proteins.

The term "substantially similar" refers to nucleotide and amino acid sequences that represent functional and/or structural equivalents or orthologs of sequences disclosed herein, in particular of *Oryza sativa, Arabidopsis thaliana or Brassica napus* sequences disclosed herein.

In its broadest sense, the term "substantially similar" when used herein with respect to a nucleotide sequence means that the nucleotide sequence is part of a gene which encodes a polypeptide having substantially the same structure and function as a polypeptide encoded by a gene for the reference nucleotide sequence, *e.g.*, the nucleotide sequence comprises a promoter from a gene that is the ortholog of the gene corresponding to the reference nucleotide sequence, as well as promoter sequences that are structurally related to the promoter sequences particularly exemplified herein, *i.e.*, the substantially similar promoter sequences hybridize to the complement of the promoter sequences exemplified herein under high or very high stringency conditions. For example, altered nucleotide sequences which simply reflect the degeneracy of the genetic code but nonetheless encode amino acid sequences that are identical to a particular amino acid sequence are substantially similar to the particular sequences. The term "substantially similar" also includes nucleotide sequences wherein the sequence has been modified, for example, to optimize expression in particular cells, as well as nucleotide sequences encoding a variant polypeptide having one or more amino acid substitutions relative to the (unmodified) polypeptide encoded by the reference sequence, which substitution(s) does not alter the activity of the variant polypeptide relative to the unmodified polypeptide.

In its broadest sense, the term "substantially similar" when used herein with respect to polypeptide means that the polypeptide has substantially the same structure and function as the reference polypeptide. Preferbly the homolog is an ortholog. Whether a gene with a substantially similar sequence is a ortholog of gene, e.g. or cor78 oder Met1 gene can be tested in a simple assay: The endogenous gene, e.g. cor78 or Met is first deleted and than the the potential ortholog is introduced into the depleted cell or plant. A ortholog should re-establish a similar or substantially similar phenotype as the wild type, preferably the phenotype is identical to the wild type. , The term can in one embodiment mean that the amino acid sequences that are substantially similar to a particular sequence are those wherein overall amino acid identity is at least 60% or greater to the instant sequences. Modifications that result in equivalent nucleotide or amino acid sequences are well within the routine skill in the art. In one embodiment, the percentage of amino acid sequence identity between the substantially similar and the reference polypeptide is at least 60%, 61%, 62%, 63%, 64%, 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99%. One further indication that two polypeptides are substantially similar to each other, besides having substantially the same function, is that an agent, *e.g.*, an antibody, which specifically binds to one of the polypeptides, also specifically binds to the other.

Sequence comparisons maybe carried out using a Smith-Waterman sequence alignment algorithm (see *e.g.*, Waterman 1995). The localS program, version 1.16, is preferably used with following parameters: match: 1, mismatch penalty: 0.33, open-gap penalty: 2, extended-gap penalty: 2.

Moreover, a nucleotide sequence that is "substantially similar" to a reference nucleotide sequence is said to be "equivalent" to the reference nucleotide sequence. The skilled artisan recognizes that equivalent nucleotide sequences encompassed by this invention can also be defined by their ability to hybridize, under low, moderate and/or stringent conditions (*e.g.*, 0.1 X SSC, 0.1 % SDS, 65°C), with the nucleotide sequences that are within the literal scope of the instant claims.

What is meant by "substantially the same activity" when used in reference to a polynucleotide or polypeptide fragment is that the fragment has at least 60%, 61%, 62%, 63%, 64%, 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99% of the activity of the full length polynucleotide or full length polypeptide.

"Target gene" refers to a gene e.g. to an gene on the replicon, that expresses the desired target coding sequence, functional RNA, or protein. In one embodiment, the target gene is not essential for replicon replication. Additionally, target genes may comprise native non-viral genes inserted into a non-native organism, or chimeric genes, and will be under the control of suitable regulatory sequences. Thus, the regulatory sequences in the target gene may come from any source, including the virus Target genes may include coding sequences that are either heterologous or homologous to the genes of a particular plant to be transformed. In one embodiment, however, target genes can be genes that do not include native viral genes.Typical target genes include, but are not limited to genes encoding a structural protein, a seed storage protein, a protein that conveys herbicide resistance, and a protein that conveys insect resistance. Proteins encoded by target genes are known as "foreign proteins". The expression of a target gene in a plant will typically produce an altered plant trait.

The term "altered plant trait" means any phenotypic or genotypic change in a transgenic plant relative to the wild-type or non-transgenic plant host.

The term "transformation" refers to the transfer of a nucleic acid fragment into the genome of a host cell, resulting in genetically stable inheritance. Host cells containing the transformed nucleic acid fragments are referred to as "transgenic" cells, and organisms comprising transgenic cells are referred to as "transgenic organisms". Examples of methods of transformation of plants and plant cells include *Agrobacterium*-mediated transformation (De Blaere 1987) and particle bombardment technology (US 4,945,050). Whole plants may be regenerated from transgenic cells by methods well known to the skilled artisan (see, for example, Fromm 1990).

"Transformed," "transgenic," and "recombinant" refer to a host organism such as a bacterium or a plant into which a heterologous nucleic acid molecule has been introduced. The nucleic acid molecule can be stably integrated into the genome. For example, "transformed," "transformant," and "transgenic" plants or calli have been through the transformation process and contain a foreign gene integrated into their chromosome. The term "untransformed" refers to normal plants that have not been through the transformation process.

"Transiently transformed" refers to cells in which transgenes and foreign DNA have been introduced (for example, by such methods as *Agrobacterium*-mediated transformation or biolistic bombardment), but not selected for stable maintenance.

"Stably transformed" refers to cells that have been selected and regenerated on a selection media following transformation.

"Chromosomally-integrated" refers to the integration of a foreign gene or DNA construct into the host DNA by covalent bonds. In case the genes are not "chromosomally integrated" they may be "transiently expressed." Transient expression of a gene refers to the expression of a gene that is not integrated into the host chromosome but functions independently, either as part of an autonomously replicating plasmid or expression cassette, for example, or as part of another biological system such as a virus.

"Transient expression" refers to expression in cells in which a virus or a transgene is introduced by viral infection or by such methods as *Agrobacterium-mediated* transformation, electroporation, or biolistic bombardment, but not selected for its stable maintenance.

"Genetically stable" and "heritable" refer to chromosomally-integrated genetic elements that are stably maintained in the plant and stably inherited by progeny through successive generations.

"Primary transformant" and "T0 generation" refer to transgenic plants that are of the same genetic generation as the tissue, which was initially transformed (*i.e.*, not having gone through meiosis and fertilization since transformation).

"Secondary transformants" and the "T1, T2, T3, *etc.* generations" refer to transgenic plants derived from primary transformants through one or more meiotic and fertilization cycles. They may be derived by self-fertilization of primary or secondary transformants or crosses of primary or secondary transformants with other transformed or untransformed plants.

"Wild-type" refers to a virus or organism found in nature without any known mutation.

A null segregant is progeny (or lines derived from the progeny) of a transgenic plant that does not contain the transgene due to Mendelian segregation.

The terms "genome" or "genomic DNA" is referring to the heritable genetic information of a host organism. Said genomic DNA comprises the DNA of the nucleus (also referred to as chromosomal DNA) but also the DNA of the plastids (*e.g.*, chloroplasts) and other cellular organelles (*e.g.*, mitochondria). Preferably the terms genome or genomic DNA is referring to the chromosomal DNA of the nucleus.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like *e.g.*, polymerase chain reaction (PCR) analysis, Southern blot analysis, fluorescence *in situ* hybridization (FISH), and *in situ* PCR.

The term "nucleic acid" refers to deoxyribonucleotides or ribonucleotides and polymers thereof in either single- or double-stranded form, composed of monomers (nucleotides) containing a sugar, phosphate and a base, which is either a purine or pyrimidine. Unless specifically limited, the term encompasses nucleic acids containing known analogs of natural nucleotides, which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.*, degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer 1991; Ohtsuka 1985; Rossolini 1994). A "nucleic acid fragment" is a fraction of a given nucleic acid molecule. In higher plants, deoxyribonucleic acid (DNA) is the genetic material while ribonucleic acid (RNA) is involved in the transfer of information contained within DNA into proteins. The term "nucleotide sequence" refers to a polymer of DNA or RNA which can be single- or double-stranded, optionally containing synthetic, non-natural or altered nucleotide bases capable of incorporation into DNA or RNA polymers. The terms "nucleic acid" or "nucleic acid sequence" may also be used interchangeably with gene, cDNA, DNA and RNA encoded by a gene.

The invention encompasses isolated or substantially purified nucleic acid or protein compositions. In the context of the present invention, an "isolated" or "purified" DNA molecule or an "isolated" or "purified" polypeptide is a DNA molecule or polypeptide that, by the hand of man, exists apart from its native environment and is therefore not a product of nature. An isolated DNA molecule or polypeptide may exist in a purified form or may exist in a non-native environment such as, for example, a transgenic host cell. For example, an "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (*i.e.*, sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein or polypeptide having less than about 30%, 20%, 10%, 5%, (by dry weight) of contaminating protein. When the protein of the invention, or biologically active portion thereof, is recombinantly produced, preferably culture medium represents less than about 30%, 20%, 10%, or 5% (by dry weight) of chemical precursors or non-protein of interest chemicals. The nucleotide sequences of the invention include both the naturally occurring sequences as well as mutant (variant) forms. Such variants will continue to possess the desired activity, *i.e.*, either promoter activity or the activity of the product encoded by the open reading frame of the non-variant nucleotide sequence.

The term "variant" with respect to a sequence (*e.g.*, a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence.

"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations" which are one species of "conservatively modified variations." Every nucleic acid sequence described herein which encodes a polypeptide also describes every possible silent variation, except where otherwise noted. One of skill will recognize that each codon in a nucleic acid (except ATG, which is ordinarily the only codon for methionine) can be modified to yield a functionally identical molecule by standard techniques. Accordingly, each "silent variation" of a nucleic acid which encodes a polypeptide is implicit in each described sequence.

The nucleic acid molecules of the invention can be "optimized" for enhanced expression in plants of interest (see, for example, WO 91/16432; Perlak 1991; Murray 1989). In this manner, the open reading frames in genes or gene fragments can be synthesized utilizing plant-preferred codons (see, for example, Campbell & Gowri, 1990 for a discussion of host-preferred codon usage). Thus, the nucleotide sequences can be optimized for expression in any plant. It is recognized that all or any part of the gene sequence may be optimized or synthetic. That is, synthetic or partially optimized sequences may also be used. Variant nucleotide sequences and proteins also encompass, sequences and protein derived from a mutagenic and recombinogenic procedure such as DNA shuffling. With such a procedure, one or more different coding sequences can be manipulated to create a new polypeptide possessing the desired properties. In this manner, libraries of recombinant polynucleotides are generated from a population of related sequence polynucleotides comprising sequence regions that have substantial sequence identity and can be homologously recombined in vitro or in vivo. Strategies for such DNA shuffling are known in the art (see, for example, Stemmer 1994; Stemmer 1994; Crameri 1997; Moore 1997; Zhang 1997; Crameri 1998; and US 5,605,797, 9, 11, 13, 15, and 17,837,458).

By "variant" polypeptide is intended a polypeptide derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Such variants may result from, for example, genetic polymorphism or from human manipulation. Methods for such manipulations are generally known in the art.

Thus, the polypeptides may be altered in various ways including amino acid substitutions, deletions, truncations, and insertions. Methods for such manipulations are generally known in the art. For example, amino acid sequence variants of the polypeptides can be prepared by mutations in the DNA. Methods for mutagenesis and nucleotide sequence alterations are well known in the art (see, for example, Kunkel 1985; Kunkel 1987; US 4,873,192; Walker & Gaastra, 1983 and the references cited therein). Guidance as to appropriate amino acid substitutions that do not affect biological activity of the protein of interest may be found in the model of Dayhoff *et al.* (1978). Conservative substitutions, such as exchanging one amino acid with another having similar properties, are preferred. Individual substitutions deletions or additions that alter, add or delete a single amino acid or a small percentage of amino acids (typically less than 5%, more typically less than 1 %) in an encoded sequence are "conservatively modified variations," where the alterations result in the substitution of an amino acid with a chemically similar amino acid. Conservative substitution tables providing functionally similar amino acids are well known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). See also, Creighton, 1984. In addition, individual substitutions, deletions or additions which alter, add or delete a single amino acid or a small percentage of amino acids in an encoded sequence are also "conservatively modified variations."

"Expression cassette" or "expression construct" as used herein means a DNA sequence capable of directing expression of a particular nucleotide sequence in an appropriate host cell, comprising a promoter operably linked to a nucleotide sequence of interest, which is - optionally - operably linked to termination signals and/or other regulatory elements. An expression cassette may also comprise sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the nucleotide sequence of interest may be chimeric, meaning that at least one of its components is heterologous with respect to at least one of its other components. The expression cassette may also be one, which is naturally occurring but has been obtained in a recombinant form useful for heterologous expression. An expression cassette may be assembled entirely extracellularly (*e.g.*, by recombinant cloning techniques). However, an expression cassette may also be assembled using in part endogenous components. For example, an expression cassette may be obtained by placing (or inserting) a promoter sequence upstream of an endogenous sequence, which thereby becomes functionally linked and controlled by said promoter sequences. Likewise, a nucleic acid sequence to be expressed may be placed (or inserted) downstream of an endogenous promoter sequence thereby forming an expression cassette. In general, the expression of the nucleotide sequence in the expression cassette may be under the control of a constitutive promoter or of an inducible promoter which initiates transcription only when the host cell is exposed to some particular external stimulus. The expression cassettes of the invention, the transcription regulating sequence or the promoter can also be specific to a particular tissue or organ or stage of development, in particular to the embryo or the scutellum.. In a preferred embodiment, such expression cassettes will comprise the transcriptional initiation region of the invention linked to a nucleotide sequence of interest. Such an expression cassette is preferably provided with a plurality of restriction sites for insertion of the gene of interest to be under the transcriptional regulation of the regulatory regions. The expression cassette may additionally contain selectable marker genes. The cassette will include in the 5'-3' direction of transcription, a transcriptional and translational initiation region, a DNA sequence of interest, and a transcriptional and translational termination region functional in plants. The termination region may be native with the transcriptional initiation region, may be native with the DNA sequence of interest, or may be derived from another source. Convenient termination regions are available from the Ti-plasmid of *A. tumefaciens,* such, as the octopine synthase and nopaline synthase termination regions and others described below (see also, Guerineau 1991; Proudfoot 1991; Sanfacon 1991; Mogen 1990; Munroe 1990; Ballas 1989; Joshi 1987).

"Vector" is defined to include, inter alia, any plasmid, cosmid, phage or *Agrobacterium* binary vector in double or single stranded linear or circular form which may or may not be self transmissible or mobilizable, and which can transform prokaryotic or eukaryotic host either by integration into the cellular genome or exist extrachromosomally (*e.g.* autonomous replicating plasmid with an origin of replication).

Specifically included are shuttle vectors by which is meant a DNA vehicle capable, naturally or by design, of replication in two different host organisms, which may be selected, e.g. from actinomycetes and related species, such like *Sacharomyces cerevisiae,* bacteria and eukaryotic (*e.g.* higher plant, mammalian, yeast or fungal cells).

Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance.

A "transgenic plant" is a plant having one or more plant cells that contain an expression vector or an recombinant expression cassette, like the expression cassettes of the invention.

"Plant tissue" includes differentiated and undifferentiated tissues or plants, including but not limited to roots, stems, shoots, leaves, pollen, seeds, tumor tissue and various forms of cells and culture such as single cells, protoplast, embryos, and callus tissue. The plant tissue may be in plants or in organ, tissue or cell culture.

The following terms are used to describe the sequence relationships between two or more nucleic acids or polynucleotides: (a) "reference sequence", (b) "comparison window", (c) "sequence identity", (d) "percentage of sequence identity", and (e) "substantial identity".

(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.

(b) As used herein, "comparison window" makes reference to a contiguous and specified segment of a polynucleotide sequence, wherein the polynucleotide sequence in the comparison window may comprise additions or deletions (*i.e.*, gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well known in the art. Thus, the determination of percent identity between any two sequences can be accomplished using a mathematical algorithm. Preferred, non-limiting examples of such mathematical algorithms are the algorithm of Myers and Miller, 1988; the local homology algorithm of Smith *et al.* 1981; the homology alignment algorithm of Needleman and Wunsch 1970; the search-for-similarity-method of Pearson and Lipman 1988; the algorithm of Karlin and Altschul, 1990, modified as in Karlin and Altschul, 1993.

Computer implementations of these mathematical algorithms can be utilized for comparison of sequences to determine sequence identity. Such implementations include, but are not limited to: CLUSTAL in the PC/Gene program (available from Intelligenetics, Mountain View, Calif.); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described (Higgins 1988, 1989; Corpet 1988; Huang 1992; Pearson 1994). The ALIGN program is based on the algorithm of Myers and Miller, supra. The BLAST programs of Altschul *et al.,* 1990, are based on the algorithm of Karlin and Altschul, supra. Multiple aligments (*i.e.* of more than 2 sequences) are preferably performed using the Clustal W algorithm (Thompson 1994; *e.g.*, in the software VectorNTI™, version 9;

Invitrogen Inc.) with the scoring matrix BLOSUM62MT2 with the default settings (gap opening penalty 15/19, gap extension penalty 6.66/0.05; gap separation penalty range 8; % identity for alignment delay 40; using residue specific gaps and hydrophilic residue gaps).

Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences (see, *e.g.*, Karlin & Altschul (1993). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

To obtain gapped alignments for comparison purposes, Gapped BLAST (in BLAST 2.0) can be utilized as described in Altschul *et al.* 1997. Alternatively, PSI-BLAST (in BLAST 2.0) can be used to perform an iterated search that detects distant relationships between molecules. See Altschul *et al.,* supra. When utilizing BLAST, Gapped BLAST, PSI-BLAST, the default parameters of the respective programs (*e.g.* BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, 1989). See http://www.ncbi.nlm.nih.gov. Alignment may also be performed manually by inspection.

For purposes of the present invention, comparison of nucleotide sequences for determination of percent sequence identity to the promoter sequences disclosed herein is preferably made using the BlastN program (version 1.4.7 or later) with its default parameters or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.

(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (*e.g.*, charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g.*, as implemented in the program PC/GENE (Intelligenetics, Mountain View, Calif.).

(d) As used herein, "percentage of sequence identity" means the value determined by comparing two optimally aligned sequences over a comparison window, wherein the portion of the polynucleotide sequence in the comparison window may comprise additions or deletions (*i.e.*, gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

(e)

(i) The term "substantial identity" of polynucleotide sequences means that a polynucleotide comprises a sequence that has at least 60%, 61%, 62%, 63%, 64%, 65%, 66%, 67%, 68%, 69%, 70%, 71%, 72%, 73%, 74%, 75%, 76%, 77%, 78%, or 79%, preferably at least 80%, 81%, 82%, 83%, 84%, 85%, 86%, 87%, 88%, or 89%, more preferably at least 90%, 91 %, 92%, 93%, or 94%, and most preferably at least 95%, 96%, 97%, 98%, or 99% sequence identity, compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 60% or 70%, more preferably at least 80%, 90%, and most preferably at least 95%.

Another indication that nucleotide sequences are substantially identical is if two molecules hybridize to each other under stringent conditions (see below). Generally, stringent conditions are selected to be about 5°C lower than the thermal melting point ($T_m$) for the specific sequence at a defined ionic strength and pH. However, stringent conditions encompass temperatures in the range of about 1°C to about 20°C, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.

(ii) The term "substantial identity" in the context of a peptide indicates that a peptide comprises a sequence with at least 60%, 61%, 62%, 63%, 64%, 65%, 66%, 67%, 68%, 69%, 70%, 71%, 72%, 73%, 74%, 75%, 76%, 77%, 78%, or 79%, preferably 80%, 81%, 82%, 83%, 84%, 85%, 86%, 87%, 88%, or 89%, more preferably at least 90%, 91%, 92%, 93%, or 94%, or even more preferably, 95%, 96%, 97%, 98% or 99%, sequence identity to the reference sequence over a specified comparison window. Preferably, optimal alignment is conducted using the homology alignment algorithm of Needleman and Wunsch (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (*e.g.*, total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridization are sequence dependent, and are different under different environmental parameters. The $T_m$ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the $T_m$ can be approximated from the equation of Meinkoth and Wahl, 1984:

$$T_m = 81.5°C + 16.6 (\log_{10} M)+0.41 (\%GC) - 0.61 (\% form) - 500 / L$$

where M is the molarity of monovalent cations, %GC is the percentage of guanosine and cytosine nucleotides in the DNA, % form is the percentage of formamide in the hybridization solution, and L is the length of the hybrid in base pairs. $T_m$ is reduced by about 1°C for each 1 % of mismatching; thus, $T_m$, hybridization, and/or wash conditions can be adjusted to hybridize to sequences of the desired identity. For example, if sequences with >90% identity are sought, the $T_m$ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. An extensive guide to the hybridization of nucleic acids is found in Tijssen, 1993. Generally, highly stringent hybridization and wash conditions are selected to be about 5°C lower than the thermal melting point $T_m$ for the specific sequence at a defined ionic strength and pH.

An example of highly stringent wash conditions is 0.15 M NaCl at 72°C for about 15 minutes. An example of stringent wash conditions is a 0.2 X SSC wash at 65°C for 15 minutes (see, Sambrook, infra, for a description of SSC buffer). Often, a high stringency wash is preceded by a low stringency wash to remove background probe signal. An example medium stringency wash for a duplex of, *e.g.*, more than 100 nucleotides, is 1 X SSC at 45°C for 15 minutes. An example low stringency wash for a duplex of, e.g., more than 100 nucleotides, is 4 to 6 X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (*e.g.*, >50 nucleotides). Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, *e.g.*, when a copy of a nucleic acid is created using the maximum codon degeneracy permitted by the genetic code.

Very stringent conditions are selected to be equal to the $T_m$ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0.1 x SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1 X to 2 X SSC (20 X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5 X to 1 X SSC at 55 to 60°C.

The following are examples of sets of hybridization/wash conditions that may be used to clone orthologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M $NaPO_4$, 1 mM EDTA at 50°C with washing in 2 X SSC, 0. 1% SDS at 50°C, more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0.1 % SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C.

"DNA shuffling" is a method to introduce mutations or rearrangements, preferably randomly, in a DNA molecule or to generate exchanges of DNA sequences between two or more DNA molecules, preferably randomly. The DNA molecule resulting from DNA shuffling is a shuffled DNA molecule that is a non-naturally occurring DNA molecule derived from at least one template DNA molecule. The shuffled DNA preferably encodes a variant polypeptide modified with respect to the polypeptide encoded by the template DNA, and may have an altered biological activity with respect to the polypeptide encoded by the template DNA.

"Recombinant DNA molecule" is a combination of DNA sequences that are joined together using recombinant DNA technology and procedures used to join together DNA sequences as described, for example, in Sambrook *et al.,* 1989.

The present invention is especially useful for applications in monocotyledonous plants. The term "monocotyledonous plant" includes plants of a variety of ploidy levels, including aneuploid, polyploid, diploid, haploid and hemizygous. Included are furthermore the mature plants, seed, shoots and seedlings, and parts, propagation material (for example seeds and fruit) and cultures, for example cell cultures, derived therefrom. Annual and perennial monocotyledonous plants are preferred host organisms for the generation of transgenic plants.

Furthermore, the preset invention includes mature plants, seed, shoots and seedlings, and parts, propagation material and cultures derived therefrom, for example, cell cultures. Mature plants refers to plants at any developmental stage beyond that of the seedling. The term seedling refers to a young immature plant in an early developmental stage, at which it is still dependent upon assimilates stored within the seed (*e.g.* in the endosperm, perisperm or cotyledons. Included are all genera of the subfamilies Bambusoideae (*e.g.*, the genus bamboo), Andropogonoideae (*e.g.*, the genera Saccharum, *Sorghum,* or *Zea*), Arundineae (*e.g.*, the genus *Phragmites*), Oryzoideae (*e.g.*, the genus *Oryza*), Panicoideae (*e.g.*, the genera *Panicum, Pennisetum,* and *Setaria*), Pooideae (Festuciadeae) (*e.g.*, the genera *Poa, Festuca, Lolium, Trisetum, Agrostis, Phleum, Dactylis, Alopecurus, Avena, Triticum, Secale,* and *Hordeum).* Preferred are *Avena sativa* (oats), Bambusa sp. and Bambusa bambos (bamboo), *Saccharum officinarum* (sugarcane), *Triticum dicoccum* (Emmer wheat), *Triticum monococcum* (Einkorn wheat), *Triticum spelta* (spelt wheat), *Triticum durum* (wheat), *Triticum turgidum, Triticum aestivum* (wheat), *Zea mays* (maize/corn), *Panicum miliaceum* (common millet), *Pennisetum thiphoides* (Bulrush millet), *Hordeum vulgare* or *H. sativum* (barley), *Oryza sativa* (rice), *Zizania aquatica* (wild rice), *Secale cereale (rye), Sorghum bicolor (S. vulgare)* (sorghum). More preferred are wheat (*Triticum* spp.), rice (*Oryza* spp.), barley (*Hordeum* spp.), oats (*Avena* spp.), rye (*Secale* spp.), corn (*Zea mays*), sorghum and millet (*Pennisettum* spp). Preferred are all wheat species especially of the Triticum family (including both winter and spring wheat), more especially *Triticum* spp.: common (*T. aestivum*), durum (*T. durum*), spelt (*T. spelta*), *Triticum dicoccum* (Emmer wheat), *Triticum turgidum,* and *Triticum monococcum* (Einkorn wheat), with *T. aestivum* being particularly preferred. The method of the invention can be used to produce transgenic plants from spring wheats, such as, for example, Bobwhite, Marshall, PIVOT1, UC702, and Panewawa as well as from winter wheats, such as, for example, HY368, Neeley, FL302, RH91, R332, R1269 and R585. Other suitable wheat genotypes are including, but not limited to Yecora Rojo, Karl and Anza. However, it should be pointed out, that the invention is not limited to certain varities but is highly genotype-independent.

The word "plant" refers to any plant, particularly to agronomically useful plants (*e.g.*, seed plants), and "plant cell" is a structural and physiological unit of the plant, which comprises a cell wall but may also refer to a protoplast. The plant cell may be in form of an isolated single cell or a cultured cell, or as a part of higher organized unit such as, for example, a plant tissue, or a plant organ differentiated into a structure that is present at any stage of a plant's development.

Such structures include one or more plant organs including, but are not limited to, fruit, shoot, stem, leaf, flower petal, etc. Preferably, the term "plant" includes whole plants, shoot vegetative organs/structures (*e.g.* leaves, stems and tubers), roots, flowers and floral organs/structures (*e.g.* bracts, sepals, petals, stamens, carpels, anthers and ovules), seeds (including embryo, endosperm, and seed coat) and fruits (the mature ovary), plant tissues (*e.g.* vascular tissue, ground tissue, and the like) and cells (*e.g.* guard cells, egg cells, trichomes and the like), and progeny of same.

"Significant increase" is an increase that is larger than the margin of error inherent in the measurement technique, preferably an increase by about 2-fold or greater.

"Significantly less" means that the decrease is larger than the margin of error inherent in the measurement technique, preferably a decrease by about 2-fold or greater.

## DETAILED DESCRIPTION OF THE INVENTION

One first embodiment of the invention relates to
a plant or plant cell comprising an expression cassette, said expression cassette comprising

a) a first nucleic acid molecule elected from the group consisting of

- i) a polynucleotide as defined in SEQ ID NO:1;
- ii) a polynucleotide having at least 70%, or 80%, more preferably at least 85% or 90%, most preferaly at least 95%, 98% or 99% sequence identity to the polynucleotide of SEQ ID NO:1; and
- iii) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a polynucleotide as defined in SEQ ID NO:1, or the complement thereof, and operably linked thereto

b) a second nucleic acid molecule selected from the group consisting of

i) a polynucleotide as defined in SEQ ID NO:3;

ii) a polynucleotide having at least 60%, 70%, or 80%, more preferably at least 85% or 90%, most preferaly at least 95%, 98% or 99% sequence identity to the polynucleotide of SEQ ID NO:3; and

iii) a polynucleotide hybridizing under conditions equivalent to hybridization in 7% sodium dodecyl sulfate (SDS), 0.5M $NaPO_4$, 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a sequence described by SEQ ID NO:3, or the complement thereof,

and operably linked to at least one nucleic acid which is heterologous in relation to said first or said second nucleic acid sequence of the transcription regulating sequence and is capable suitable to confer to a plant an increased yield, and/or increased stress tolerance, and/or increased nutritional quality and/or oil content of a seed or a sprout.

Preferably, the chimeric transcription regulating nucleotide sequence causes said heterologous DNA to be predominantly expressed in the germinating embryo. This expression profile is especially useful for the following applications:

i) enhanced resistance against stress factors: as described above in the prior art section the embryo is very sensitive against all kinds of biotic and abiotc stress factors (drought, cold, diseases *etc.*). These stress factors have an immediate effect on yield and crop quality. Most promoters known in the art have no or low expression capacity during this stage. The transcription regulating specificity disclosed herein is especially useful to express stress-resistance genes "on-demand" *i.e.* at the right time to high levels. Furthermore, because of the specificity in the starchy endosperm it is possible to pursue new ways of stress-resistance. Because the starchy endosperm is the tissue, which nourishes the embryo, one can increase stress-resistance via improved supplementation of the embryo with nutrients.

ii) increased nutritional quality of a seed or a sprout: The expression profile of the chimeric transcription regulating nucleic acid sequences allows for conversion of seed (kernel) ingredients or for changing the distribution of the ingredients in the seed. For example one can convert carbohydrates (starch) into oil or other high-value ingredients (*e.g.*, vitamins) or can shift localization of ingredients from the endosperm towards the embryo thereby providing sprouts with improved nutritional value.

iii) increased yield: Increased yield is partially related to stress resistance (see above under i)). However, the expression profile of the chimeric transcription regulating nucleic acid sequences allows even without stress factors to increase yield by optimizing growth of the embryo, which will directly affect growth of the seedling. One can also achieve earlier germination under field conditions and other traits, which will lead to higher or earlier yield.

iv) targeted sequence excision. As described above homogenous excision of sequences, especially marker sequences, is a yet unsolved issue in the field of biotechnology. Most plants demonstrate mosaic-like excision patterns, which areas of successful excision and areas of no excision. To achieve homogenous or substantially homogenous excision, the excision mechanism needs to be activated preferably at an early stage of development, when the organism does not consist of many plants. Furthermore the activation (*i.e.* expression of the excision mediating enzyme) needs to be strong. Both requirements are met by the expression profile of the chimeric transcription regulating nucleic acid sequences disclosed herein. The strong transcription activity in early embryo germination allows for efficient marker excision in this stage, from which a target sequence free (e.g., marker-free) plant is generated.

"Germinating embryo-specific transcription" in the context of this invention means the transcription of a nucleic acid sequence by a transcription regulating element in a way that transcription of said nucleic acid sequence in the germinating plant, preferably the germinating embryo contribute to more than 90%, preferably more than 95%, more preferably more than 99% of the entire quantity of the RNA transcribed from said nucleic acid sequence in the entire plant, seed or sprout during the specified developmental stage.

**1. The chimeric transcription regulating nucleic acid sequence**

In its most general form the nucleic acid molecule comprising a polynucleotide encoding a chimeric transcription regulating nucleotide sequence comprises

i) a first nucleic acid molecule selected from the group consisting of

a) a polynucleotide as defined in SEQ ID NO:1;

b) a polynucleotide having at least 70%, or 80%, more preferably at least 85% or 90%, most preferaly at least 95%, 98% or 99% sequence identity to the polynucleotide of SEQ ID NO:1; and

c) a polynucleotide hybridizing under conditions equivalent to hybridization in 7% sodium dodecyl sulfate (SDS), 0.5M $NaPO_4$, 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a polynucleotide as defined in SEQ ID NO:1, or the complement thereof, and

ii) a second nucleic acid molecule selected from the group consisting of

a) a polynucleotide as defined-in SEQ ID NO:3;

b) a polynucleotide having at least 60%, 70%, or 80%, more preferably at least 85% or 90%, most preferaly at least 95%, 98% or 99% sequence identity to the polynucleotide of SEQ ID NO:3; and

c) a polynucleotide hybridizing under conditions equivalent to hybridization in 7% sodium dodecyl sulfate (SDS), 0.5M $NaPO_4$, 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a sequence described by SEQ ID NO:3, or the complement thereof,

wherein said first and said second nucleic acid sequences are functionally linked and heterologous to each other.

The term "derived" when used in the context of DNA regions like promoters, transcription regulating nucleic acid sequences, or upstream activating sequences refers to situations where the DNA region that is "derived" is obtained from or based upon a naturally-occurring DNA region or other source DNA region. The DNA region that is "derived" can differ, usually through deliberate mutation, from the naturally-occurring DNA region or other source DNA region.

### 1.1 Derivatives and variants of the chimeric transcription regulating nucleotide sequence of the invention and its functional elements

The invention disclosed herein contemplates that in addition to the specific chimeric transcription regulating nucleotide sequences and their specific elements disclosed herein, derivatives and variants of said sequences can be employed.

Derivatives of the specific chimeric transcription regulating nucleotide sequences and their specific elements may include, but are not limited to, deletions of sequence, single or multiple point mutations, alterations at a particular restriction enzyme site, addition of functional elements, or other means of molecular modification. This modification may or may not enhance, or otherwise alter the transcription regulating activity of said sequences.

For example, one of skill in the art may delimit the functional elements within the sequences and delete any non-essential elements. Functional elements may be modified or combined to increase the utility or expression of the sequences of the invention for any particular application. Functionally equivalent fragments of a transcription regulating nucleotide sequence of the invention can also be obtained by removing or deleting non-essential sequences without deleting the essential one. Narrowing the transcription regulating nucleotide sequence to its essential, transcription mediating elements can be realized in vitro by trial-and-error deletion mutations, or in silico using promoter element search routines. Regions essential for promoter activity often demonstrate clusters of certain, known promoter elements. Such analysis can be performed using available computer algorithms such as PLACE ("Plant Cis-acting Regulatory DNA Elements"; Higo 1999), the BIOBASE database "Transfac" (Biologische Datenbanken GmbH, Braunschweig; Wingender 2001) or the database PlantCARE (Lescot 2002). Especially preferred are equivalent fragments of transcription regulating nucleotide sequences, which are obtained by deleting the region encoding the 5'-untranslated region of the mRNA, thus only providing the (untranscribed) promoter region. The 5'-untranslated region can be easily determined by methods known in the art (such as 5'-RACE analysis). Accordingly, some of the transcription regulating nucleotide sequences of the invention are equivalent fragments of other sequences.

As indicated above, deletion mutants of the promoter of the invention can be randomly prepared and then assayed. With this strategy, a series of constructs are prepared, each containing a different portion of the clone (a subclone), and these constructs are then screened for activity. A suitable means for screening for activity is to attach a deleted promoter construct, which contains a deleted segment to a selectable or screenable marker, and to isolate only those cells expressing the marker gene. In this way, a number of different, deleted promoter constructs are identified which still retain the desired, or even enhanced, activity. The smallest segment, which is required for activity, is thereby identified through comparison of the selected constructs. This segment may then be used for the construction of vectors for the expression of exogenous genes.

The means for mutagenizing or creating deletions in a DNA segment encoding any promoter sequence are well known to those of skill in the art and are disclosed, for example, in US 6,583,338. Certain variant nucleotide sequences of the present invention retain biological activity (*i.e.* regulate transcription with a profile as defined above). One example of a regulatory sequence variant is a promoter formed by one or more deletions from a larger promoter. The 5' portion of a promoter up to the TATA box near the transcription start site can sometimes be deleted without abolishing promoter activity, as described by Zhu et al., (1995) The Plant Cell 7:1681-1689. A routine way to remove part of a DNA sequence is to use an exonuclease in combination with DNA amplification to produce unidirectional nested deletions of double-stranded DNA clones. A commercial kit for this purpose is sold under the trade name Exo-Size.TM. (New England Biolabs, Beverly, Mass.). Biologically active variants also include, for example, the native promoter sequences of the invention having one or more nucleotide substitutions, deletions or insertions.

Derivatives and variants also include homologs, paralogs and orthologs from other species, such as but not limited to, bacteria, fungi, and plants. "Homolog" is a generic term used in the art to indicate a polynucleotide or polypeptide sequence possessing a high degree of sequence relatedness to a reference sequence. Such relatedness may be quantified by determining the degree of identity and/or similarity between the two sequences as hereinbefore defined. Falling within this generic term are the terms "ortholog", and "paralog". "Paralog" refers to a polynucleotide or polypeptide that within the same species which is functionally similar. "Ortholog" refers to a polynucleotide or polypeptide that is the functional equivalent of the polynucleotide or polypeptide in another species. An orthologous gene means preferably a gene, which is encoding a orthologous protein. More specifically, the term "ortholog" denotes a polypeptide or protein obtained from one species that is the functional counterpart of a polypeptide or protein from a different species. Sequence differences among orthologs are the result of speciation.

Preferably, the transcription regulating activity of a variant or derivative of a chimeric transcription regulating nucleotide sequences is substantially the same (or equivalent) than for the chimeric transcription regulating nucleotide sequences specifically disclosed herein, *i.e.* that expression is regulated in the germinating embryo-specific fashion as described above. In addition to this, the transcription regulating activity of a derivative or variant may vary from the activity of its parent sequence, especially with respect to expression level. The expression level may be higher or lower than the expression level of the parent sequence. Both derivations may be advantageous depending on the nucleic acid sequence of interest to be expressed. Preferred are such functional equivalent sequences, which - in comparison with its parent sequence - does, not derivate from the expression level of said parent sequence by more than 50%, preferably 25%, more preferably 10% (as to be preferably judged by either mRNA expression or protein (*e.g.,* reporter gene) expression). Furthermore preferred are equivalent sequences which demonstrate an increased expression in comparison to its parent sequence, preferably an increase by at least 50%, more preferably by at least 100%, most preferably by at least 500%. Such expression profile is preferably demonstrated using reporter genes operably linked to said transcription regulating nucleotide sequence. Preferred reporter genes (Schenborn 1999) in this context are green fluorescence protein (GFP) (Chui 1996; Leffel 1997), chloramphenicol transferase, luciferase (Millar 1992), β-glucuronidase or · -galactosidase. Especially preferred is β-glucuronidase (Jefferson 1987). Other methods to assay transcriptional regulation are well known in the art and include Northern blots, and RT-PCR (see, for example, Sambrook et *al.*, supra).

In one preferred embodiment the transcription regulating nucleotide sequence is described by a isolated nucleic acid molecule comprising a polynucleotide encoding a sequence selected from the group consisting of

i) the sequence described by SEQ ID NO:1;
ii) a fragment of at least 200 consecutive bases of the sequence described by SEQ ID NOs:1,
iii) a nucleotide sequence having a sequence identity of at least 50%, preferably at least 70% or 80%, more preferably at least 85% or 90%, most preferably at least 95%, 98% or 99% to the sequence described by SEQ ID NO:1,
iv) a nucleotide sequence capable of hybridizing under conditions equivalent to hybridization in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C) to the sequence described by SEQ ID NO: 1, or the complement thereof;
v) a nucleotide sequence capable of hybridizing under conditions equivalent to hybridization in 7% sodium dodecyl sulfate (SDS), 0.5 M $NaPO_4$, 1 mM EDTA at 50°C with washing in 0.5 X SSC, 0. 1 % SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C) to a nucleic acid comprising 50 to 200 or more consecutive nucleotides (such as 50 or 100, preferably 150 or 200, more preferably 250 or 400 consecutive nucleotides, most preferably the entire sequence) of a sequence described by SEQ ID NO: 1, or the complement thereof;
vi) a nucleotide sequence which is the complement or reverse complement of any of the previously mentioned nucleotide sequences under i) to v).

In another preferred embodiment the upstream activating sequence is described by a nucleic acid molecule comprising a polynucleotide encoding a sequence selected from the group consisting of

i) the sequence described by SEQ ID NO: 3,
ii) a fragment of at least 200 consecutive bases of the sequence described by SEQ ID NO:3,
iii) a nucleotide sequence having a sequence identity of at least 60%, 70% or 80%, more preferably at least 85% or 90%, most preferably at least 95%, 98% or 99% to the sequence described by SEQ ID NO: 3,
iv) a nucleotide sequence capable of hybridizing under conditions equivalent to hybridization in 7% sodium dodecyl sulfate (SDS), 0.5 M $NaPO_4$, 1 mM EDTA at 50°C with washing in 0.5 X SSC, 0. 1 % SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C) to the sequence described by SEQ ID NO: 3, or the complement thereof;
v) a nucleotide sequence capable of hybridizing under conditions equivalent to hybridization in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1 % SDS at 65°C) to a nucleic acid comprising 50 to 200 or more consecutive nucleotides (such as 50 or 100, preferably 150 or 200, more preferably 250 or 400 consecutive nucleotides, most preferably the entire sequence) of a sequence described by SEQ ID NO: 3, or the complement thereof;
vi) a nucleotide sequence which is the complement or reverse complement of any of the previously mentioned nucleotide sequences under i) to v).

The sequences specified under ii), iii), iv) v) and vi) of any of the specified chimeric transcription regulating sequences defined above are preferably capable to modify transcription in a monocotyledonous plant cell or organism, more preferably they are capable to induce embryo specific expression. Preferably, the sequences specified under iv) or v) are hybridizing under stringent conditions with the specified target sequence.

Preferably, the nucleotide sequences identity is determined by using the BlastN program (version 1.4.7 or later) with its default parameters (wordlength (W) of 11, an expectation (E) of 10, a cutoff of 100, M=5, N=-4, and a comparison of both strands) or any equivalent program.

In hybridization techniques, all or part of a known nucleotide sequence is used as a probe that selectively hybridizes to other corresponding nucleotide sequences present in a population of cloned genomic DNA fragments or cDNA fragments (*i.e.*, genomic or cDNA libraries) from a chosen organism. The hybridization probes may be genomic DNA fragments, cDNA fragments, RNA fragments, or other oligonucleotides, and may be labeled with a detectable group such as $^{32}P$, or any other detectable marker. Thus, for example, probes for hybridization can be made by labeling synthetic oligonucleotides based on the sequence of the invention. Methods for preparation of probes for hybridization and for construction of cDNA and genomic libraries are generally known in the art and are disclosed in Sambrook *et al.* (1989). In general, sequences that hybridize to the sequences disclosed herein will have at least about 60% to 70% and even about 80% 85%, 90%, 95% to 98% or more identity with the disclosed sequences. That is, the sequence similarity of sequences may range, sharing at least about 60% to 70%, and even about 80%, 85%, 90%, 95% to 98% sequence similarity.

A typical core promoter motif of the current invention, as shown in Tables 8 and 9, comprises four well-defined nucleotides flanked by a string of any nucleotides at both the 5' and 3' ends of the promoter motif. For example, GAPB/GAP. 01 promoter motif comprises 15 nucleotides "aaaaATGAatagaaa" as defined in SEQ ID NO:26, wherein "nnnnATGAnnnnnnn" is the core GAPB/GAP.01 promoter motif as described in SEQ ID NO:27, wherein n is selected from the group consisting of a, c, t and g. The number of n at either the 5' end or the 3' end of the core promoter motif corresponds to the number of nucleotides flanking the core promoter motif in the corresponding promoter motif at the 5' or 3' end, respectively. The identity percentage of the core promoter motif to the corresponding promoter motif is calculated, for example, as illustrated below. When two "n" in the core GAPB/GAP.01 promoter motif of SEQ ID NO:26 are identical to the nucleotides at the corresponding positions of the GAPB/GAP.01 promoter motif of SEQ ID NO:27, for example, n(2) = a and n(4) = a, the percentage identity is determined by dividing the total number of the identical nucleotides (including the four core nucleotides) by the entire length of the promoter motif, 6/15 = 40%. When five "n" in the core GAPB/GAP.01 promoter motif of SEQ ID NO:26 are identical to the nucleotides at the corresponding positions of the GAPB/GAP.01 promoter motif of SEQ ID NO:27, for example, n(1) = a, n(3) = a, n(10) = t, n(12) = g, and n(14) = a, the percentage identity is 9/15 = 60%.

The promoter motifs and core promoter motifs identified in Ar.cor78 are shown in Tables 8 and 9. More specifically, SEQ ID NOs: 10, 12, 14, 17, 20, 22, 25, 27, 29, 31, 33, 35, 37, 39, 42, 44, 46, 48, 50, 53, 55, 57, 59, 63, 66, 68, 70, 72, 74, 77, 79, 82, 84, 86, 88, 90, 94, 96, 98, 102, 104, 108, 112, 114, 117, 121, 123, 125, 127, 129, 131, 137, and 138 are core promoter motifs identified in At.cor78 promoter. SEQ ID NOs: 9, 11, 13, 15, 16, 18, 19, 21, 23, 24, 26, 28, 30, 32, 34, 36, 38, 40, 41, 43, 45, 47, 49, 51, 52, 54, 56, 58, 60, 61, 62, 64, 65, 67, 69, 71, 73, 75, 76, 78, 80, 81, 83, 85, 87, 89, 91, 92, 93, 95, 97, 99, 100, 101, 103, 105, 106, 107, 109, 110, 111, 113, 115, 116, 118, 119, 120, 122, 124, 126, 128, 130, 132, 133, 134, 135, and 136 are promoter motifs identified in At.sor78 promoter. Arabidopsis cor78 promoter (SEQ ID NO:1) is described e.g. in (Horvath 1993)and (Gilmour *et al.* 1991; Nordin *et al.* 1991). In some cases, one core promoter motif corresponds to promoter motifs located at different positions of At.cor78 with different flanking 5' and 3' nucleotides, as shown in Table 9.

The current invention relates further to a nucleic acid molecule comprising a polynucleotide encoding a transcription regulating sequence comprising at least two core promoter motifs selected from the group consisting of the sequences as defined in SEQ ID NOs: 10, 12, 14, 17, 20, 22, 25, 27, 29, 31, 33, 35, 37, 39, 42, 44, 46, 48, 50, 53, 55, 57, 59, 63, 66, 68, 70, 72, 74, 77, 79, 82, 84, 86, 88, 90, 94, 96, 98, 102, 104, 108, 112, 114, 117, 121, 123, 125, 127, 129, 131, 137, and 138. Preferably the core promoter motifs of SEQ ID NOs: 10, 12, 14, 17, 20, 22, 25, 27, 29, 31, 33, 35, 37, 39, 42, 44, 46, 48, 50, 53, 55, 57, 59, 63, 66, 68, 70, 72, 74, 77, 79, 82, 84, 86, 88, 90, 94, 96, 98, 102, 104, 108, 112, 114, 117, 121, 123, 125, 127, 129, 131, 137, and 138 are 30-40%, preferably 40-50%, or more preferably 50-60% or more identical to SEQ ID NOs: 9, (11,18), 13, (15, 16), 19, (21, 23, 61, 92), (24, 75), (26, 40), 28, 30, (32, 51, 80, 105, 109), 34, 36, 38, 41, (43, 64, 132), 45, 47, 49, 52, (54, 99), (56, 119), (58, 60), 62, 65, 67, 69, 71, 73, 76, 78, 81, 83, 85, (87,100,106, 110), (89, 91), 93, 95, 97, 101, 103, 107, 111, (113, 115), (116,118), 120, 122, 124, 126, 128, (130, 134, 135), 136, and 133, respectively. More preferably the core promoter motifs of SEQ ID NOs: 10, 12, 14, 16, 19, 22, 24, 27, 29, 31, 33, 35, 37, 39, 41, 44, 46, 48, 50, 52, 55, 57, 59, 61, 65, 68, 70, 72, 74, 76, 79, 81, 84, 86, 88, 90, 92, 96, 98, 100, 104, 106, 110, 114, 116, 119, 123, 125, 127, 129, 131, 133, 139, and 140 are 60-70%, preferably 70-80%, or more preferably 80-90% or more identical to SEQ ID NOs:9, (11,18), 13, (15, 16), 19, (21, 23, 61, 92), (24, 75), (26, 40), 28, 30, (32, 51, 80, 105, 109), 34, 36, 38, 41, (43, 64, 132), 45, 47, 49, 52, (54, 99), (56, 119), (58, 60), 62, 65, 67, 69, 71, 73, 76, 78, 81, 83, 85, (87,100,106, 110), (89, 91), 93, 95, 97, 101, 103, 107, 111, (113, 115), (116,118), 120, 122, 124, 126, 128, (130, 134, 135), 136, and 133, respectively. Most preferably the core promoter motifs of SEQ ID NOs: 10, 12, 14, 16, 19, 22, 24, 27, 29, 31, 33, 35, 37, 39, 41, 44, 46, 48, 50, 52, 55, 57, 59, 61, 65, 68, 70, 72, 74, 76, 79, 81, 84, 86, 88, 90, 92, 96, 98, 100, 104, 106, 110, 114, 116, 119, 123, 125, 127, 129, 131, 133, 139, and 140 are 90-95% or preferably 95-99% identical to SEQ ID NOs:9, (11,18), 13, (15, 16), 19, (21, 23, 61, 92), (24, 75), (26, 40), 28, 30, (32, 51, 80, 105, 109), 34, 36, 38, 41, (43, 64, 132), 45, 47, 49, 52, (54, 99), (56, 119), (58, 60), 62, 65, 67, 69, 71, 73, 76, 78, 81, 83, 85, (87,100,106, 110), (89, 91), 93, 95, 97, 101, 103, 107, 111, (113, 115), (116,118), 120, 122, 124, 126, 128, (130, 134, 135), 136, and 133, respectively.

### 1.2 Additional regulatory and functional elements for the expression cassette and vectors of the invention

An expression cassette of the invention may comprise further regulatory elements. The term in this context is to be understood in a broad meaning comprising all sequences which may influence construction or function of the expression cassette. Regulatory elements may for example modify transcription and/or translation in prokaryotic or eukaryotic organism. In a preferred embodiment the expression cassette of the invention comprised transcription regulating sequence and - optionally additional regulatory elements - each operably liked to the nucleic acid sequence to be expressed (or the transcription regulating nucleotide sequence).

A variety of 5' and 3' transcriptional regulatory sequences are available for use in the present invention. Transcriptional terminators are responsible for the termination of transcription and correct mRNA polyadenylation. The 3' untranslated regulatory DNA sequence preferably includes from about 50 to about 1,000, more preferably about 100 to about 1,000, nucleotide base pairs and contains plant transcriptional and translational termination sequences. Appropriate transcriptional terminators and those which are known to function in plants include the CaMV 35S terminator, the tml terminator, the nopaline synthase terminator, the pea rbcS E9 terminator, the terminator for the T7 transcript from the octopine synthase gene of *Agrobacterium tumefaciens,* and the 3' end of the protease inhibitor I or II genes from potato or tomato, although other 3' elements known to those of skill in the art can also be employed. Alternatively, one also could use a gamma coixin, oleosin 3 or other terminator from the genus Coix

As the DNA sequence between the transcription initiation site and the start of the coding sequence, *i.e.,* the untranslated leader sequence, can influence gene expression, one may also wish to employ a particular leader sequence. Preferred leader sequences are contemplated to include those, which include sequences, predicted to direct optimum expression of the attached gene, *i.e.,* to include a preferred consensus leader sequence, which may increase or maintain mRNA stability and prevent inappropriate initiation of translation. The choice of such sequences will be known to those of skill in the art in light of the present disclosure. Sequences that are derived from genes that are highly expressed in plants will be most preferred.

Preferred regulatory elements also include the 5'-untranslated region, introns and the 3'-untranslated region of genes.

Such sequences that have been found to enhance gene expression in transgenic plants include intron sequences (see below for details) and viral leader sequences (*e.g.*, from TMV, MCMV and AMV; Gallie 1987). For example, a number of untranslated leader sequences derived from viruses are known to enhance expression. Specifically, leader sequences from Tobacco Mosaic Virus (TMV), Maize Chlorotic Mottle Virus (MCMV), and Alfalfa Mosaic Virus (AMV) have been shown to be effective in enhancing expression (*e.g.*, Gallie 1987; Skuzeski 1990). Other leaders known in the art include but are not limited to: Picornavirus leaders, for example, EMCV leader (Encephalomyocarditis 5' noncoding region) (Elroy-Stein 1989); Potyvirus leaders, for example, TEV leader (Tobacco Etch Virus); MDMV leader (Maize Dwarf Mosaic Virus); Human immunoglobulin heavy-chain binding protein (BiP) leader, (Macejak 1991); Untranslated leader from the coat protein mRNA of alfalfa mosaic virus (AMV RNA 4), (Jobling 1987; Tobacco mosaic virus leader (TMV), (Gallie 1989; and Maize Chlorotic Mottle Virus leader (MCMV) (Lommel 1991. See also, Della-Cioppa 1987. Regulatory elements such as the TMV omega element (Gallie 1989), may further be included where desired. Additional examples of enhancers include elements from the CaMV 35S promoter, octopine synthase genes (Ellis *el al.*, 1987), the rice actin I gene, the maize alcohol dehydrogenase gene (Callis 1987), the maize shrunken I gene (Vasil 1989), TMV Omega element (Gallie 1989) and promoters from non-plant eukaryotes (*e.g.* yeast; Ma 1988). Vectors for use in accordance with the present invention may be constructed to include the ocs enhancer element. This element was first identified as a 16 bp palindromic enhancer from the octopine synthase (ocs) gene of ultilane (Ellis 1987), and is present in at least 10 other promoters (Bouchez 1989). The use of an enhancer element, such as the ocs elements and particularly multiple copies of the element, will act to increase the level of transcription from adjacent promoters when applied in the context of plant transformation.

Additional preferred regulatory elements are enhancer sequences or polyadenylation sequences. Preferred polyadenylation sequences are those from plant genes or Agrobacterium T-DNA genes (such as for example the terminator sequences of the OCS (octopine synthase) or NOS (nopaline synthase) genes).

An expression cassette of the invention (or a vector derived therefrom, i. e. a vector comprising a expression cassette of the invention) may comprise additional functional elements, which are to be understood in the broad sense as all elements which influence construction, propagation, or function of an expression cassette or a vector or a transgenic organism comprising them. Such functional elements may include origin of replications (to allow replication in bacteria; for the ORI of pBR322 or the P15A ori; Sambrook 1989), or elements required for Agrobacterium T-DNA transfer (such as for example the left and/or rights border of the T-DNA).

Additionally, the expression cassettes may be constructed and employed in the intracellular targeting of a specific gene product within the cells of a transgenic plant or in directing a protein to the extracellular environment. This will generally be achieved by joining a DNA sequence encoding a transit or signal peptide sequence to the coding sequence of a particular gene. The resultant transit or signal peptide will transport the protein to a particular intracellular or extracellular destination, respectively, and will then be post-translationally removed. Transit or signal peptides act by facilitating the transport of proteins through intracellular membranes, *e.g.*, vacuole, vesicle, plastid and mitochondrial membranes, whereas signal peptides direct proteins through the extracellular membrane. By facilitating the transport of the protein into compartments inside and outside the cell, these sequences may increase the accumulation of gene product protecting them from proteolytic degradation. These sequences also allow for additional mRNA sequences from highly expressed genes to be attached to the coding sequence of the genes. Since mRNA being translated by ribosomes is more stable than naked mRNA, the presence of translatable mRNA in front of the gene may increase the overall stability of the mRNA transcript from the gene and thereby increase synthesis of the gene product. Since transit and signal sequences are usually post-translationally removed from the initial translation product, the use of these sequences allows for the addition of extra translated sequences that may not appear on the final polypeptide. Targeting of certain proteins may be desirable in order to enhance the stability of the protein (US 5,545,818).

### 1.3 Assembly of the chimeric transcription regulating nucleic acid sequence, expression cassettes, and vectors of the invention

An operable linkage in relation to any chimeric transcription regulating nucleic acid sequence, expression cassette or vector of the invention may be realized by various methods known in the art, comprising both *in vitro* and *in vivo* procedure. Thus, any chimeric transcription regulating nucleic acid sequence, expression cassette or vector of the invention may by realized using standard recombination and cloning techniques well known in the art (see *e.g.*, Maniatis 1989; Silhavy 1984; Ausubel 1987). Many approaches or methods have been developed and used for gene cloning. Examples of these are cloning by restriction enzyme digestion and ligation of compatible ends, T-A cloning directly from PCR product, TOPO-attached unidirectional cloning, and recombination-based cloning. Recombination-based cloning is one of the most versatile cloning methods available due to its high cloning efficiency and its broad application for cloning a variety of genes regardless of available restriction enzyme sites. Recombination cloning uses the lambda recombination system to clone genes into vectors that contain recombination sequences for the lambda recombinase machinery. Recombination cloning uses site-specific recombinases, which along with associated proteins in some cases, recognize specific sequences of bases in a nucleic acid molecule and exchange the nucleic acid segments flanking those sequences. The recombinases and associated proteins are collectively referred to as "recombination proteins."

Site-specific recombinases are proteins that are present in many organisms (*e.g.*, viruses and bacteria) and have been characterized as having both endonuclease and ligase properties. Many of the known site-specific recombinases belong to the integrase family of recombinases including the Integrase/att system from bacteriophage lambda. An example of one application of the Integrase/att system from bacteriophage lambda is the LR cloning reaction as disclosed in US 5,888,732 and US 6,277,608 and U.S. published patent application 2002/0007051 A1 and International application WO 02/081711 A1. The LR cloning reaction is commercially available as the GATEWAY™ cloning technology (available from Invitrogen Corporation, Carlsbad, California). The LR cloning reaction is catalyzed by the LR Clonase Enzyme mix, which comprises lambda recombination proteins Int, Xis, and the E. coli-encoded protein IHF.

An expression cassette may also be assembled by inserting a chimeric transcription regulating nucleic acid sequence of the invention into the plant genome. Such insertion will result in an operable linkage to a nucleic acid sequence of interest, which as such already existed in the genome. By the insertion the nucleic acid of interest is expressed in a germinating embryo-specific way due to the transcription regulating properties of the chimeric transcription regulating nucleotide sequence. The insertion may be directed or by chance. Preferably the insertion is directed and realized by for example homologous recombination. By this procedure a natural promoter may be exchanged against the chimeric transcription regulating nucleotide sequence of the invention, thereby modifying the expression profile of an endogenous gene. The transcription regulating nucleotide sequence may also be inserted in a way, that antisense mRNA of an endogenous gene is expressed, thereby inducing gene silencing.

An operable linkage may - for example - comprise an sequential arrangement of the chimeric transcription regulating nucleotide sequence of the invention (for example the super-promoter) with a nucleic acid sequence to be expressed, and - optionally - additional regulatory elements such as for example polyadenylation or transcription termination elements, enhancers, introns *etc,* in a way that the transcription regulating nucleotide sequence can fulfill its function in the process of expression the nucleic acid sequence of interest under the appropriate conditions. The term "appropriate conditions" mean preferably the presence of the expression cassette in a plant cell. Preferred are arrangements, in which the nucleic acid sequence of interest to be expressed is placed down-stream (*i.e.*, in 3'-direction) of the chimeric transcription regulating nucleotide sequence of the invention in a way, that both sequences are covalently linked. Optionally additional sequences may be inserted in-between the two sequences. Such sequences may be for example linker or multiple cloning sites. Furthermore, sequences can be inserted coding for parts of fusion proteins (in case a fusion protein of the protein encoded by the nucleic acid of interest is intended to be expressed). Preferably, the distance between the nucleic acid sequence of interest to be expressed and the transcription regulating nucleotide sequence of the invention is not more than 200 base pairs, preferably not more than 100 base pairs, more preferably not more than 50 base pairs.

Virtually any DNA composition may be used for delivery to recipient monocotyledonous plants or plant cells, to ultimately produce fertile transgenic plants in accordance with the present invention. For example, DNA segments or fragments in the form of vectors and plasmids, or linear DNA segments or fragments, in some instances containing only the DNA element to be expressed in the plant, and the like, may be employed. The construction of vectors, which may be employed in conjunction with the present invention, will be known to those of skill of the art in light of the present disclosure (see, *e.g.*, Sambrook 1989; Gelvin 1990).

The present invention further provides a recombinant vector or other DNA construct suitable for plant transformation (including but not limited to cosmids, YACs (yeast artificial chromosomes), BACs (bacterial artificial chromosomes), and plant artificial chromosomes) containing the expression cassette of the invention, and monocotyledonous host cells comprising the expression cassette or vector, *e.g.*, comprising a plasmid. The expression cassette or vector may (preferably) augment the genome of a transformed monocotyledonous plant or may be maintained extra chromosomally. The expression cassette or vector of the invention may be present in the nucleus, chloroplast, mitochondria and/or plastid of the cells of the plant. Preferably, the expression cassette or vector of the invention is comprised in the chromosomal DNA of the plant nucleus. In certain embodiments, it is contemplated that one may wish to employ replication-competent viral vectors in monocot transformation. Such vectors include, for example, wheat dwarf virus (WDV) "shuttle" vectors, such as pW1-11 and PW1-GUS (Ugaki 1991). These vectors are capable of autonomous replication in maize cells as well as *E. coli*, and as such may provide increased sensitivity for detecting DNA delivered to transgenic cells. A replicating vector may also be useful for delivery of genes flanked by DNA sequences from transposable elements such as Ac, Ds, or Mu.

The DNA construct according to the invention and any vectors derived therefrom may comprise further functional elements. The term "further functional elements" is to be understood in the broad sense. It preferably refers to all those elements which affect the generation, multiplication, function, use or value of said DNA construct or vectors comprising said DNA construct, or cells or organisms comprising the beforementioned. These further functional elements may include but shall not be limited to:

i) Origins of replication which ensure replication of the expression cassettes or vectors according to the invention in, for example, *E. coli.* Examples which may be mentioned are ORI (origin of DNA replication), the pBR322 ori or the P15A ori (Sambrook *et al.* 1989).

ii) Multiple cloning sites (MCS) to enable and facilitate the insertion of one or more nucleic acid sequences.

iii) Sequences which make possible homologous recombination or insertion into the genome of a host organism.

iv) Elements, for example border sequences, which make possible the Agrobacterium-mediated transfer in plant cells for the transfer and integration into the plant genome, such as, for example, the right or left border of the T-DNA or the vir region.

The introduced recombinant DNA molecule used for transformation herein may be circular or linear, double-stranded or single-stranded. Generally, the DNA is in the form of chimeric DNA, such as plasmid DNA, that can also contain coding regions flanked by regulatory sequences, which promote the expression of the recombinant DNA present in the resultant plant. Generally, the introduced recombinant DNA molecule will be relatively small, *i.e.,* less than about 30 kb to minimize any susceptibility to physical, chemical, or enzymatic degradation which is known to increase as the size of the nucleotide molecule increases. As noted above, the number of proteins, RNA transcripts or mixtures thereof, which is introduced into the plant genome, is preferably preselected and defined, *e.g.*, from one to about 5-10 such products of the introduced DNA may be formed.

The present invention also provides a monocotyledonous plant (preferably a transgenic plant), seed and parts from such a plant, and progeny plants from such a plant, including hybrids and inbreds.

The invention also provides a method of plant breeding, *e.g.*, to prepare a crossed fertile transgenic plant. The method comprises crossing a fertile transgenic plant comprising a particular expression cassette of the invention with itself or with a second plant, *e.g.*, one lacking the particular expression cassette, to prepare the seed of a crossed fertile transgenic plant comprising the particular expression cassette. The seed is then planted to obtain a crossed fertile transgenic plant. The plant may be preferably a monocot (preferably as defined above). The crossed fertile transgenic plant may have the particular expression cassette inherited through a female parent or through a male parent. The second plant may be an inbred plant. The crossed fertile transgenic may be a hybrid. Also included within the present invention are seeds of any of these crossed fertile transgenic plants.

## 2. Advantageous traits or properties to be expressed by the expression cassette of the invention

The chimeric transcription regulating nucleotide sequences of the invention are useful to modify the phenotype of a plant. Various changes in the phenotype of a transgenic plant are desirable and can be achieved using the advantageous expression profile (*i.e.* germinating embryo-specific expression) of the transcription regulating nucleotide sequences disclosed herein. These results can be achieved by providing expression of heterologous products or increased expression of endogenous products in plants. Alternatively, the results can be achieved by providing for a reduction of expression of one or more endogenous products, particularly enzymes or cofactors in the plant. Generally, the chimeric transcription regulating nucleotide sequences may be employed to express a nucleic acid segment that is operably linked to said promoter such as, for example, an open reading frame, or a portion thereof, an anti-sense sequence, a sequence encoding for a sense or double-stranded RNA sequence, a sequence encoding for a micro RNA sequence, or a transgene in plants. These changes result in an alteration in the phenotype of the transformed plant.

The choice of a heterologous DNA for expression in a monocotyledonous plant host cell in accordance with the invention will depend on the purpose of the transformation. One of the major purposes of transformation of crop plants is to add commercially desirable, agronomically important or end-product traits to the plant.

Although numerous nucleic acid sequences are suitable to be expressed by the chimeric transcription regulating nucleic acid sequence of the invention most preferably the nucleic acid is conferring upon expression to the monocotyledonous plant a trait or property selected from the group consisting of

i) enhanced resistance against at least one stress factor,
ii) increased nutritional quality of a seed or a sprout,
iii) increased yield, and
iv) excision of a target sequence, e.g. excision of a selection marker sequence.

### 2.1 Basic Principles

Two principal methods for the control of expression are known, overexpression and underexpression. Overexpression can be achieved by insertion of one or more than one extra copy of the selected gene. It is, however, not unknown for plants or their progeny, originally transformed with one or more than one extra copy of a nucleotide sequence, to exhibit the effects of underexpression as well as overexpression. For underexpression there are two principle methods, which are commonly referred to in the art as "antisense downregulation" and "sense downregulation" (sense downregulation is also referred to as "cosuppression"). Generically these processes are referred to as "gene silencing". Both of these methods lead to an inhibition of expression of a target gene.

Thus, expression of the nucleic acid sequence under the chimeric transcription regulating sequence may result in transcription of a mRNA and expression of a protein, or expression of an antisense RNA, sense RNA, dsRNA, microRNA, ta-siRNA, snRNA, RNAi, or any combination thereof.

The mRNA or the regulatory RNA expressed/transcripted in the method of the invention can be for example

a) a double-stranded RNA nucleic acid sequence (dsRNA) as described above;
b) an antisense nucleic acid sequence. Encompassed are those methods in which the antisense nucleic acid sequence is directed against a gene (i.e. genomic DNA sequences including the promoter sequence) or a gene transcript (i.e. RNA sequences) including the 5'and 3'non-translated regions. Also encompassed are $\alpha$-anomeric nucleic acid sequences;
c) an antisense nucleic acid sequence in combination with a ribozyme;
d) a sense nucleic acid sequences for inducing cosuppression;
e) a nucleic acid sequence encoding dominant-negative factor;
f) a DNA-, RNA- or protein-binding factor or antibodies against genes, RNA's or proteins;
g) a viral nucleic acid sequences which bring about the degradation of RNA;
h) a microRNA or micro-RNA (miRNA) that has been designed to target the gene of interest in order to induce a breakdown of the mRNA or translation inhibition of the gene of interest and thereby silence gene expression;
i) a ta-siRNA that has been designed to target the gene of interest in order to induce breakdown of the mRNA (or maybe translational inhibition) of the gene of interest and thereby silence gene expression; and/or

What follows is a brief description of the individual preferred methods. In general, herein, the meaning of the term "expression" shall include the meaning of the terms "transcription" and/or "translation" where appropriate.

a) The regulatory RNA expressed in the method of the invention can be for example a double-stranded RNA nucleic acid sequence (dsRNA) e.g. for the reduction or deletion of activity of the nucleic acid molecule or polypeptide which activity is to be reduced in the process of the invention.

As an alternative to antisense polynucleotides and sense polynucleotides, double stranded RNA (dsRNA) can be used to reduce expression of a gene. The term dsRNA, as used herein, refers to RNA hybrids comprising two stands of RNA. The dsRNA can be linear or circular in structure. The hybridizing RNAs may be substantially or completely complementary. By "substantially complementary", is meant that when the two hybridizing RNAs are optimally aligned using the Needleman and Wunsch algorithm as described above, the hybridizing portions are at least 95% complementary. Preferably, the dsRNA will be at least 100 se pairs in length. Methods for making and using dsRNA are known in the art. One method comprises the simultaneous transcription of two complementary DNA strands in vivo (See, for example, U.S. Patent No. 5,795,715). DsRNA can be introduced into a plant or plant cell directly by standard transformation procedures. Alternatively, dsRNA can be express in a plant by transcribing two complementary RNAs.

The method of regulating genes by means of double-stranded RNA ("double-stranded RNA interference"; dsRNAi) has been described extensively for animal, yeast, fungi and plant organisms, e.g. for Neurospora, Zebrafish, Drosophila, mice, planaria, humans, Trypanosoma, petunia or Arabidopsis (for example Matzke MA et al. (2000) Plant Mol. Biol. 43: 401-415; Fire A. et al. (1998) Nature 391: 806-811; WO 99/32619; WO 99/53050; WO 00/68374; WO 00/44914; WO 00/44895; WO 00/49035; WO 00/63364). In addition, RNAi is also documented as an advantageously tool for the repression of genes in bacteria, such as E. coli, for example by Tchurikov et al. [J. Biol. Chem., 2000, 275 (34): 26523-26529]. Fire et al. named the phenomenon RNAi for RNA interference. The techniques and methods described in the above references are expressly referred to. Efficient gene suppression can also be observed in the case of transient expression or following transient transformation, for example, as the consequence of a biolistic transformation (Schweizer P et al. (2000) Plant J 2000 24: 895-903). dsRNAi methods are based on the phenomenon that the simultaneous introduction of complementary strand and counterstrand of a gene transcript brings about highly effective suppression of the expression of the gene in question. The resulting phenotype is very similar to that of an analogous knock-out mutant (Waterhouse PM et al. (1998) Proc. Natl. Acad. Sci. USA 95: 13959-64).

Tuschl et al., Gens Dev., 1999, 13 (24): 3191-3197, were able to show that the efficiency of the RNAi method is a function of the length of the duplex, the length of the 3'-end overhangs, and the sequence in these overhangs.

Based on the work of Tuschl et al. and assuming that the underlining principles are conserved between different species, the following guidelines can be given to the skilled worker. Accordingly, the dsRNA molecule of the invention or used in the process of the invention preferable fulfils at least one of the following principles:

- to achieve good results, the 5' and 3' untranslated regions of the nucleic acid sequence in use and regions close to the start codon should be, in general, avoided as these regions are richer in regulatory protein binding sites and interactions between RNAi sequences and such regulatory proteins might lead to undesired interactions;
- in plants, the 5' and 3' untranslated regions of the nucleic acid sequence in use and regions close to the start codon, preferably 50 to 100 nt upstream of the start codon, give good results and therefore should not be avoided;
- preferably a region of the used mRNA is selected, which is 50 to 100 nt (= nucleotides or bases) downstream from the AUG start codon;
- only dsRNA (= double-stranded RNA) sequences from exons are useful for the method, as sequences from introns have no effect;
- the G/C content in this region should be greater than 30% and less than 70%, ideally around 50%;
- a possible secondary structure of the target mRNA is less important for the effect of the RNAi method.

The dsRNAi method can be particularly effective and advantageous for reducing the expression of the nucleic acid molecule which activity is to be reduced in the process of the invention. As described inter alia in WO 99/32619, dsRNAi approaches are clearly superior to traditional antisense approaches.

Accordingly, the invention can be used fot the expression of double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid molecule which activity is to be reduced in the process of the invention.

In a double-stranded RNA molecule, e.g. a dsRNA for reducing the expression of a protein encoded by a nucleic acid molecule which activity is to be reduced in the process of the invention, one of the two RNA strands is essentially identical to at least part of a nucleic acid sequence, and the respective other RNA strand is essentially identical to at least part of the complementary strand of a nucleic acid sequence.

The term "essentially identical" refers to the fact that the dsRNA sequence may also include insertions, deletions and individual point mutations in comparison to the target sequence while still bringing about an effective reduction in expression. Preferably, the homology as defined above amounts to at least 30%, preferably at least 40%, 50%, 60%, 70% or 80%, very especially preferably at least 90%, most preferably 100%, between the "sense" strand of an inhibitory dsRNA and a part-segment of a nucleic acid sequence of the invention including in a preferred embodiment of the invention their endogenous 5'- and 3'untranslated regions or between the "antisense" strand and the complementary strand of a nucleic acid sequence, respectively. The part-segment amounts to at least 10 bases, preferably at least 17, 18, 19, 20, 21, 22, 23, 24, 25, 26, 27, 28, 29 or 30 bases, especially preferably at least 40, 50, 60, 70, 80 or 90 bases, very especially preferably at least 100, 200, 300 or 400 bases, most preferably at least 500, 600, 700, 800, 900 or more bases or at least 1000 or 2000 bases or more in length. In another preferred embodiment of the invention the part-segment amounts to 17, 18, 19, 20, 21, 22, 23, 24, 25, 26 or 27 bases, preferably to 20, 21, 22, 23, 24 or 25 bases. These short sequences are preferred in animals and plants. The longer sequences preferably between 200 and 800 bases are preferred in nonmammalian animals, preferably in invertebrates, in yeast, fungi or bacteria, but they are also useable in plants. Long double-stranded RNAs are processed in the organisms into many siRNAs (= small/short interfering RNAs) for example by the protein Dicer, which is a ds-specific Rnase III enzyme. As an alternative, an "essentially identical" dsRNA may also be defined as a nucleic acid sequence, which is capable of hybridizing with part of a gene transcript (for example in 400 mM NaCl, 40 mM PIPES pH 6.4, 1 mM EDTA at 50°C or 70°C for 12 to 16 h).

The dsRNA may consist of one or more strands of polymerized ribonucleotides. Modification of both the sugar-phosphate backbone and of the nucleosides may furthermore be present. For example, the phosphodiester bonds of the natural RNA can be modified in such a way that they encompass at least one nitrogen or sulfur hetero atom. Bases may undergo modification in such a way that the activity of, for example, adenosine deaminase, is restricted. These and other modifications are described herein below in the methods for stabilizing antisense RNA.

The dsRNA can be prepared enzymatically; it may also be synthesized chemically, either in full or in part. Short dsRNA up to 30 bp, which effectively mediate RNA interference, can be for example, efficiently generated by partial digestion of long dsRNA templates using E. coli ribonuclease III (RNase III). (Yang, D., et al. (2002) Proc. Natl. Acad. Sci. USA 99, 9942.)

The double-stranded structure can be formed starting from a single, self-complementary strand or starting from two complementary strands. In a single, self-complementary strand, "sense" and "antisense" sequence can be linked by a linking sequence ("linker") and form, for example, a hairpin structure. Preferably, the linking sequence may take the form of an intron, which is spliced out following dsRNA synthesis. The nucleic acid sequence encoding a dsRNA may contain further elements such as, for example, transcription termination signals or polyadenylation signals. If the two strands of the dsRNA are to be combined in a cell or an organism advantageously in a plant, this can be brought about in a variety of ways:

a) transformation of the cell or of the organism, advantageously of a plant, with a vector encompassing the two expression cassettes;

b) cotransformation of the cell or of the organism, advantageously of a plant, with two vectors, one of which encompasses the expression cassettes with the "sense" strand while the other encompasses the expression cassettes with the "antisense" strand;

c) supertransformation of the cell or of the organism, advantageously of a plant, with a vector encompassing the expression cassettes with the "sense" strand, after the cell or the organism had already been transformed with a vector encompassing the expression cassettes with the "antisense" strand or vice versa;

d) hybridization e.g. crossing of two organisms, advantageously of plants, each of which has been transformed with one vector, one of which encompasses the expression cassette with the "sense" strand while the other encompasses the expression cassette with the "antisense" strand;

e) introduction of a construct comprising two promoters that lead to transcription of the desired sequence from both directions; and/or

f) infecting of the cell or of the organism, advantageously of a plant, with an engineered virus, which is able to produce the desired dsRNA molecule.

Formation of the RNA duplex can be initiated either outside the cell or within the cell. If the dsRNA is synthesized outside the target cell or organism, it can be introduced into the organism or a cell of the organism by injection, microinjection, electroporation, high velocity particles, by laser beam or mediated by chemical compounds (DEAE-dextran, calciumphosphate, liposomes).

Accordingly, in one embodiment, the present invention relates to a dsRNA whereby the sense strand of said double-stranded RNA nucleic acid molecule has a homology of at least 30%, 35%, 40%, 45%, 50%, 55% or 60%, preferably 65%, 70%, 75% or 80%, more preferably 85%, 90%, 95%, 96%, 97%, 98% or 99% to the nucleic acid sequence.

Another embodiment of the invention is a dsRNA molecule, comprising a fragment of at least 10 base pairs (= bases, nt, nucleotides), preferably at least 17, 18, 19, 20, 21, 22, 23, 24, 25, , 26, 27, 28, 29, 30, 35, 40, 45 or 50, especially preferably at least 55, 60, 70, 80 or 90 base pairs, very especially preferably at least 100, 200, 300 or 400 base pairs, most preferably at least 500, 600, 700, 800, 900 or more base pairs or at least 1000 or 2000 base pairs of a nucleic acid molecule with a homology of at least 50%, 60%, 70%, 80% or 90%, preferably 100% to a nucleic acid molecule.

In another preferred embodiment of the invention the encoded sequence or its part-segment of the dsRNA molecule amounts to 17, 18, 19, 20, 21, 22, 23, 24, 25, 26 or 27 bases, preferably to 20, 21, 22, 23, 24 or 25 bases, whereby the homology of the sequence is essentially 95%, 96%, 97%, 98%, or preferred 99% or 100%.

In a preferred embodiment of the invention the sense and antisense strand of the double-stranded RNA are covalently bound or are bound by other, e.g. weak chemical bonds such as hydrogen bonds to each other and the antisense strand is essentially the complement of the sense-RNA strand.

As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.

The expression cassettes encoding the "antisense" or the "sense" strand of the dsRNA or the self-complementary strand of the dsRNA are preferably inserted into a vector and stably inserted into the genome of a plant, using the methods described herein below (for example using selection markers), in order to ensure permanent expression of the dsRNA. Transient expression with bacterial or viral vectors are similarly useful.

The dsRNA can be introduced using an amount which makes possible at least one copy per cell. A larger amount (for example at least 5, 10, 100, 500 or 1 000 copies per cell) may bring about more efficient reduction.

As has already been described, 100 % sequence identity between the dsRNA and a gene transcript of a nucleic acid molecule to be reduced, is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a nucleic acid molecule to be reduced according to the process of the invention.

The dsRNA can be synthesized either in vivo or in vitro. To this end, a DNA sequence encoding a dsRNA can be introduced into an expression cassette under the control of at least one genetic control element (such as, for example, promoter, enhancer, silencer, splice donor or splice acceptor or polyadenylation signal). Suitable advantageous constructs are described herein below. Polyadenylation is not required, nor do elements for initiating translation have to be present.

A dsRNA can be synthesized chemically or enzymatically. Cellular RNA polymerases or bacteriophage RNA polymerases (such as, for example T3, T7 or SP6 RNA polymerase) can be used for this purpose. Suitable methods for the in-vitro expression of RNA are described (WO 97/32016; US 5,593,874; US 5,698,425, US 5,712,135, US 5,789,214, US 5,804,693). Prior to introduction into a cell, tissue or organism, a dsRNA which has been synthesized in vitro either chemically or enzymatically can be isolated to a higher or lesser degree from the reaction mixture, for example, by extraction, precipitation, electrophoresis, chromatography or combinations of these methods. The dsRNA can be introduced directly into the cell or else be applied extracellularly (for example into the interstitial space). In one embodiment of the invention the RNAi method leads to only a partial loss of gene function and therefore enables the skilled worker to study a gene dose effect in the desired organism and to fine tune the process of the invention. In another preferred embodiment it leads to a total loss of function and therefore increases the production of the fine chemical. Furthermore, it enables a person skilled in the art to study multiple functions of a gene.

Stable transformation of the plant with an expression construct, which brings about the expression of the dsRNA is preferred, however. Suitable methods are described herein below. b) The regulatory RNA expressed in the method of the invention can be for example an antisense nucleic acid sequence, e.g. for the reduction or deletion of the nucleic acid molecule or polypeptide which activity is to be reduced in the process of the invention.

An exogenous DNA sequence may be designed to down-regulate a specific nucleic acid sequence. This is for example accomplished by operably linking with the chimeric transcription regulating nucleic acid sequence of the invention, an exogenous DNA in an antisense orientation or a DNA designed such that a hairpin-forming RNA molecule is generated upon transcription. Gene suppression may be effective against a native plant gene associated with a trait, e.g. to provide plants with reduced levels of a protein encoded by the native gene or with enhanced or reduced levels of an affected metabolite. For example, the chimeric transcription regulating nucleic acid sequence of the invention may be operably linked to a heterologous DNA designed such that a hairpin-shaped RNA is formed for suppression of a native gene in maize embryos. Different types of exogenous DNA arrangements resulting in gene suppression are known to those of skill in the art and include but are not limited to the following. International Publication WO 94/01550 discloses DNA constructs where the anti-sense RNA was stabilized with a self-complementary 3' segment. Other double-stranded hairpin-forming elements in transcribed RNA are disclosed in WO 98/05770 where the anti-sense RNA is stabilized by hairpin forming repeats of poly(CG) nucleotides and Patent Application Publication No. 2002/0048814 A1 describes sense or anti-sense RNA stabilized by a poly(T)-poly(A) tail. U.S. Patent Application Publication No. 2003/0018993 A1 discloses sense or anti-sense RNA that is stabilized by an inverted repeat of a subsequence of 3' untranslated region of the NOS gene. U.S. Patent Application Publication No. 2003/0036197 A1 describes an RNA stabilized by two complementary RNA regions having homology to a target sequence.

Methods for suppressing a specific protein by preventing the accumulation of its mRNA by means of "antisense" technology can be used widely and has been described extensively, including for plants; Sheehy et al. (1988) Proc. Natl. Acad. Sci. USA 85: 8805-8809;

US 4,801,34100; Mol JN et al. (1990) FEBS Lett 268(2): 427-430. The antisense nucleic acid molecule hybridizes with, or binds to, the cellular mRNA and/or the genomic DNA encoding the target protein to be suppressed. This process suppresses the transcription and/or translation of the target protein. Hybridization can be brought about in the conventional manner via the formation of a stable duplex or, in the case of genomic DNA, by the antisense nucleic acid molecule binding to the duplex of the genomic DNA by specific interaction in the large groove of the DNA helix.

In one embodiment, an "antisense" nucleic acid molecule comprises a nucleotide sequence, which is at least in part complementary to a "sense" nucleic acid molecule encoding a protein, e.g., complementary to the coding strand of a double-stranded cDNA molecule or complementary to an encoding mRNA sequence. Accordingly, an antisense nucleic acid molecule can bind via hydrogen bonds to a sense nucleic acid molecule. The antisense nucleic acid molecule can be complementary to an entire coding strand of a nucleic acid molecule conferring the expression of the polypeptide to be reduced in the process of the invention or comprising the nucleic acid molecule which activity is to be reduced in the process of the invention or to only a portion thereof. Accordingly, an antisense nucleic acid molecule can be antisense to a "coding region" of the coding strand of a nucleotide sequence of a nucleic acid molecule of the present invention.

The term "coding region" refers to the region of the nucleotide sequence comprising codons, which are translated into amino acid residues.

In another embodiment, the antisense nucleic acid molecule is antisense to a "noncoding region" of the mRNA flanking the coding region of a nucleotide sequence. The term "noncoding region" refers to 5' and 3' sequences which flank the coding region that are not translated into a polypeptide, i.e., also referred to as 5' and 3' untranslated regions (5'-UTR or 3'-UTR). Advantageously, the noncoding region is in the area of 50 bp, 100 bp, 200bp or 300 bp, preferably 400 bp, 500 bp, 600 bp, 700 bp, 800 bp, 900 bp or 1000 bp up- and/or downstream from the coding region.

Given the coding strand sequences encoding the polypeptide or the nucleic acid molecule to be reduced in the process of the invention, e.g. having above mentioned activity, e.g. the activity of a polypeptide with the *activity* of the protein which activity is to be reduced in the process of the invention as disclosed herein, antisense nucleic acid molecules can be designed according to the rules of Watson and Crick base pairing.

In a further embodiment, the antisense nucleic acid molecule can be an $\alpha$-anomeric nucleic acid. Such $\alpha$-anomeric nucleic acid molecules form specific double-stranded hybrids with complementary RNA in which - as opposed to the conventional $\beta$-nucleic acids - the two strands run in parallel with one another (Gautier C et al. (1987) Nucleic Acids Res. 15: 6625-6641). Furthermore, the antisense nucleic acid molecule can also comprise 2'-O- methylribonucleotides (Inoue et al. (1987) Nucleic Acids Res. 15: 6131-6148), or chimeric RNA-DNA analogs (Inoue et al. (1987) FEBS Lett 215: 327-330).

The antisense nucleic acid molecules of the invention are typically administered to a cell or generated *in situ* such that they hybridize with or bind to cellular mRNA and/or genomic DNA encoding a polypeptide having the *activity* of protein which activity is to be reduced in the process of the invention or encoding a nucleic acid molecule having the *activity* of the nucleic acid molecule which activity is to be reduced in the process of the invention and thereby inhibit expression of the protein, e.g., by inhibiting transcription and/or translation and leading to the aforementioned fine chemical increasing activity.

The antisense molecule of the present invention comprises also a nucleic acid molecule comprising a nucleotide sequences complementary to the regulatory region of an nucleotide sequence encoding the natural occurring polypeptide of the invention, e.g. the polypeptide sequences shown in the sequence listing, or identified according to the methods described herein, e.g., its promoter and/or enhancers, e.g. to form triple helical structures that prevent transcription of the gene in target cells. See generally, Helene, C. (1991) Anticancer Drug Des. 6(6):569-84; Helene, C. et al. (1992) Ann. N.Y. Acad. Sci. 660:27-36; and Maher, L.J. (1992) Bioassays 14(12):807-15.

c) The regulatory RNA expressed in the method of the invention can be for example an antisense nucleic acid sequence combined with a ribozyme, e.g. for the reduction or deletion of activity of the nucleic acid molecule or polypeptide which activity is to be reduced in the process of the invention.

It is advantageous to combine the above-described antisense strategy with a ribozyme method. Catalytic RNA molecules or ribozymes can be adapted to any target RNA and cleave the phosphodiester backbone at specific positions, thus functionally deactivating the target RNA (Tanner NK (1999) FEMS Microbiol. Rev. 23(3): 257-275). The ribozyme per se is not modified thereby, but is capable of cleaving further target RNA molecules in an analogous manner, thus acquiring the properties of an enzyme. The incorporation of ribozyme sequences into "antisense" RNAs imparts this enzyme-like RNA-cleaving property to precisely these "antisense" RNAs and thus increases their efficiency when inactivating the target RNA. The preparation and the use of suitable ribozyme "antisense" RNA molecules is described, for example, by Haseloff et al. (1988) Nature 33410: 585-591.

Further the antisense nucleic acid molecule of the invention can be also a ribozyme. Ribozymes are catalytic RNA molecules with ribonuclease activity, which are capable of cleaving a single-stranded nucleic acid, such as an mRNA, to which they have a complementary region. In this manner, ribozymes (for example "Hammerhead" ribozymes; Haselhoff and Gerlach (1988) Nature 33410: 585-591) can be used to catalytically cleave the mRNA of an enzyme to be suppressed and to prevent translation. The ribozyme technology can increase the efficacy of an antisense strategy. Methods for expressing ribozymes for reducing specific proteins are described in (EP 0 291 533, EP 0 321 201, EP 0 360 257). Ribozyme expression has also been described for plant cells (Steinecke P et al. (1992) EMBO J 11 (4): 1525-1530; de Feyter R et al. (1996) Mol. Gen. Genet. 250(3): 329-338). Suitable target sequences and ribozymes can be identified for example as described by Steinecke P, Ribozymes, Methods in Cell Biology 50, Galbraith et al. eds, Academic Press, Inc. (1995), pp. 449-460 by calculating the secondary structures of ribozyme RNA and target RNA and by their interaction [Bayley CC et al. (1992) Plant Mol. Biol. 18(2): 353-361; Lloyd AM and Davis RW et al. (1994) Mol. Gen. Genet. 242(6): 653-657]. For example, derivatives of the tetrahymena L-19 IVS RNA, which have complementary regions to the mRNA of the protein to be suppressed can be constructed (see also US 4,987,071 and US 5,116,742). As an alternative, such ribozymes can also be identified from a library of a variety of ribozymes via a selection process (Bartel D and Szostak JW (1993) Science 261: 1411-1418).

d) The regulatory RNA expressed in the method of the invention can be for example a (sense) nucleic acid sequence for inducing cosuppression, e.g. for the reduction or deletion of activity of the nucleic acid molecule or polypeptide which activity is to be reduced in the process of the invention.

As used herein "gene suppression" means any of the well-known methods for suppressing an RNA transcript or production of protein translated from an RNA transcript, including post-transcriptional gene suppression and transcriptional suppression. Post-transcriptional gene suppression is mediated by double-stranded RNA having homology to a gene targeted for suppression. Gene suppression by RNA transcribed from an exogenous DNA construct comprising an inverted repeat of at least part of a transcription unit is a common feature of gene suppression methods known as anti-sense suppression, co-suppression and RNA interference. More particularly, post transcriptional gene suppression by inserting an exogenous DNA construct with anti-sense oriented DNA to regulate gene expression in plant cells is disclosed in US 5,107,065 and US 5,759,829. Transcriptional suppression can be mediated by a transcribed double-stranded RNA having homology to promoter DNA sequence to effect what is called promoter trans-suppression. Post transcriptional gene suppression by inserting an exogneous DNA construct with sense-oriented DNA to regulate gene expression in plants is disclosed in US 5,283,184 and US 5,231,020.

The expression of a nucleic acid sequence in sense orientation can lead to cosuppression of the corresponding homologous, endogenous genes. The expression of sense RNA with homology to an endogenous gene can reduce or indeed eliminate the expression of the endogenous gene, in a similar manner as has been described for the following antisense approaches: Jorgensen et al. (1996) Plant Mol. Biol. 31(5): 957-973, Goring et al. (1991) Proc. Natl. Acad. Sci. USA 88: 1770-1774], Smith et al. (1990) Mol. Gen. Genet. 224: 447-481, Napoli et al. (1990) Plant Cell 2: 279-289 or Van der Krol et al. (1990) Plant Cell 2: 291-99. In this context, the construct introduced may represent the homologous gene to be reduced either in full or only in part. The application of this technique to plants has been described for example by Napoli et al. (1990) The Plant Cell 2: 279-289 and in US 5,03410,323. Furthermore the above described cosuppression strategy can advantageously be combined with the RNAi method as described by Brummell et al., 2003, Plant J. 33, pp793-800. At least in plants it is advantageously to use strong or very strong promoters in cosuppression approaches. Recent work for example by. Schubert et al., (Plant Journal 2004, 16, 2561-2572) has indicated that cosuppression effects are dependent on a gene specific threshold level, above which cosuppression occurs.

e) The mRNA expressed in the method of the invention can be for example nucleic acid sequences encoding a dominant-negative protein, e.g. for the reduction or deletion of activity of the polypeptide which activity is to be reduced in the process of the invention The function or activity of a protein can efficiently also be reduced by expressing a dominant-negative variant of said protein. The skilled worker is familiar with methods for reducing the function or activity of a protein by means of coexpression of its dominant-negative form [Lagna G and Hemmati-Brivanlou A (1998) Current Topics in Developmental Biology 36: 75-98; Perlmutter RM and Alberola-Ila J (1996) Current Opinion in Immunology 8 (2): 285-90; Sheppard D (1994) American Journal of Respiratory Cell & Molecular Biology 11(1): 1-6; Herskowitz I (1987) Nature 329 (6136): 219-22].

A dominant-negative variant can be realized for example by changing of an amino acid of a polypeptide.

This change can be determined for example by computer-aided comparison ("alignment"). These mutations for achieving a dominant-negative variant are preferably carried out at the level of the nucleic acid sequences. A corresponding mutation can be performed for example by PCRmediated in-vitro mutagenesis using suitable oligonucleotide primers by means of which the desired mutation is introduced. To this end, methods are used with which the skilled worker is familiar. For example, the "LA PCR in vitro Mutagenesis Kit" (Takara Shuzo, Kyoto) can be used for this purpose. It is also possible and known to those skilled in the art that deleting or changing of functional domains, e. g. TF or other signaling components which can bind but not activate may achieve the reduction of protein activity. f) Yet another embodiment of the invention the controlled mRNA encodes a DNA- or protein-binding factor.

These factors attach to the genomic sequence of the endogenous target gene, preferably in the regulatory regions, and bring about repression of the endogenous gene. The use of such a method makes possible the reduction in the expression of an endogenous gene without it being necessary to recombinantly manipulate the sequence of the latter. Such methods for the preparation of relevant factors are described in Dreier B et al. (2001) J. Biol. Chem. 276 (31): 29466-78 and (2000) J. Mol. Biol. 303(4): 489-502, Beerli RR et al. (1998) Proc. Natl. Acad. Sci. USA 95(25): 14628-14633; (2000) Proc. Natl. Acad. Sci. USA 97(4): 1495-1500 and (2000) J. Biol. Chem. 275(42): 32617-32627), Segal DJ and Barbas CF, 3rd (2000) Curr. Opin. Chem. Biol. 4(1): 3410-39, Kang JS and Kim JS (2000) J. Biol. Chem. 275(12): 8742-8748, Kim JS et al. (1997) Proc. Natl. Acad. Sci. USA 94(8): 3616-3620, Klug A (1999) J. Mol. Biol. 293 (2): 215-218, Tsai SY et al. (1998) Adv. Drug Deliv. Rev. 30(1-3): 23-31, Mapp AK et al. (2000) Proc. Natl. Acad. Sci. USA 97(8): 3930-3935, Sharrocks AD et al. (1997) Int. J. Biochem. Cell Biol. 29(12): 1371-1387 and Zhang L et al. (2000) J. Biol. Chem. 275(43): 33850-33860. Examples for the application of this technology in plants have been described in WO 01/52620, Ordiz MI et al., (Proc. Natl. Acad. Sci. USA, Vol. 99, Issue 20, 13290 - 13295, 2002) or Guan et al., (Proc. Natl. Acad. Sci. USA, Vol. 99, Issue 20, 13296 - 13301, 2002)

These factors can be selected using any portion of a gene. This segment is preferably located in the promoter region. For the purposes of gene suppression, however, it may also be located in the region of the coding exons or introns. The skilled worker can obtain the relevant segments from Genbank by database search or starting from a cDNA whose gene is not present in Genbank by screening a genomic library for corresponding genomic clones.

It is also possible to first identify sequences in a target crop, which encompass the nucleic acid molecule or which encode the polypeptide which activity is to be reduced in the process of the invention, then find the promoter and reduce expression by the use of the abovementioned factors.

The skilled worker is familiar with the methods required for doing so.

Furthermore, factors which are introduced into a cell may also be those which themselves inhibit the target protein. The protein-binding factors can, for example, be aptamers (Famulok M and Mayer G (1999) Curr. Top Microbiol. Immunol. 243: 123-36) or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants (Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272).

Gene expression may also be suppressed by tailor-made low-molecular-weight synthetic compounds, for example of the polyamide type Dervan PB and Bürli RW (1999) Current Opinion in Chemical Biology 3: 688-693; Gottesfeld JM et al. (2000) Gene Expr. 9(1-2): 77-91. These oligomers consist of the units 3-(dimethylamino)propylamine, N-methyl-3-hydroxypyrrole, N-methylimidazole and N-methylpyrroles; they can be adapted to each portion of double-stranded DNA in such a way that they bind sequence-specifically to the large groove and block the expression of the gene sequences located in this position. Suitable methods have been described in Bremer RE et al. (2001) Bioorg. Med.

Chem. 9(8): 2093-103], Ansari AZ et al. [(2001) Chem. Biol. 8(6): 583-92, Gottesfeld JM et al. (2001) J. Mol. Biol. 309 (3): 615-29, Wurtz NR et al. (2001) Org. Lett 3(8): 1201-3, Wang CC et al. (2001) Bioorg. Med. Chem. 9(3): 653-7], Urbach AR and Dervan PB (2001) Proc. Natl. Acad. Sci. USA 98(8): 434103-8 and Chiang SY et al. (2000) J. Biol. Chem. 275(32): 24246-54.

g) The regulatory RNA expressed in the method of the invention can be for example a viral nucleic acid sequence which brings about the degradation of RNA, e.g. for the reduction or deletion of activity of the nucleic acid molecule or polypeptide which activity is to be reduced in the process of the invention.

Inactivation or downregulation can also be efficiently brought about by inducing specific RNA degradation by the organism, advantageously in the plant, with the aid of a viral expression system (Amplikon) (Angell, SM et al. (1999) Plant J. 20(3): 357-362). Nucleic acid sequences with homology to the transcripts to be suppressed are introduced into the plant by these systems - also referred to as "VIGS" (viral induced gene silencing) with the aid of viral vectors. Then, transcription is switched off, presumably mediated by plant defense mechanisms against viruses. Suitable techniques and methods are described in Ratcliff F et al. (2001) Plant J. 25(2): 237-45, Fagard M and Vaucheret H (2000) Plant Mol. Biol. 43(2-3): 285-93, Anandalakshmi R et al. (1998) Proc. Natl. Acad. Sci. USA 95(22): 13079-84 and Ruiz MT (1998) Plant Cell 10(6): 937-46.

g) The regulatory RNA expressed in the method of the invention can be for example a microRNA (or micro-RNA) that has been designed to target the gene of interest in order to induce a breakdown or translational inhibition of the mRNA of the gene of intrest and thereby silence gene expression or of an expression cassette ensuring the expression of the former, e.g. for the reduction or deletion of activity of the nucleic acid molecule or polypeptide which activity is to be reduced in the process of the invention.

Regulation of gene expression can also be achieved by modifying expression of microRNAs. Growing evidence demonstrates that plant miRNAs have a wide range of regulatory functions in meristem identity, cell division, organ separation, organ polarity and abiotic stress response (Bartel D 2004). Many plant miRNAs have unique spatial or temporal expression pattern. Over-expression or ectopic expression of plant miRNA can change the morphology and physiology of a plant. Plant miRNA precursor can also be engineered to target a reporter gene in Arabidopsis (Parizotto E A et al., 2004).

MicroRNAs (miRNAs) have emerged as evolutionarily conserved, RNA-based regulators of gene expression in plants and animals. MiRNAs (- 21 to 25 nt) arise from larger precursors with a stem loop structure that are transcribed from non-protein-coding genes. miRNA targets a specific mRNA to suppress gene expression at post-transcriptional (*i.e.* degrades mRNA) or translational levels (*i.e.* inhibits protein synthesis) (Bartel D 2004, Cell 116, 281-297). MiRNAs can be efficiently design to specifally target and down regulated selected genes. Determinants of target selection of natural plant miRNAs have been analysed by Schwab and coworkers (Schwab et al. 2005,. 2005 Dev. Cell 8, 517-527). This work has been extended to the design and use of artificial miRNAs (amiRNAs) to efficiently down regulate target genes, resulting in concepts and rules for the design of effective amiRNAs for directed gene silencing (Highly Specific Gene Silencing by Artificial microRNAs in Arabidopsis, Schwab et al., Plant Cell 2006 18 (4)) and a web based tool for efficient amiRNA design (http://wmd.weigelworld.org)).

i) The regulatory RNA expressed in the method of the invention can be for example a transacting small interfering RNA (ta-siRNA) or of an expression cassette ensuring the expression of the former, e.g. for the reduction or deletion of activity of the nucleic acid molecule or maybe a polypeptide which activity is to be reduced in the process of the invention.

Trans-acting siRNA (ta-siRNA) is a recently identified endogenous siRNA (Peragine et al. 2004; Vazquez et al. 2004). The generation of ta-siRNA requires miRNA-mediated mRNA cleavage and subsequent dsRNA synthesis by an RNA-dependent RNA polymerase (RdRP), RDR6 (Allen et al. 2005). ta-siRNA regulates gene expression in a way similar to that of miRNA.

A transacting small interfering RNA (ta-siRNA) can be designed to target the gene of interest in order to induce a breakdown of the mRNA of the gene of interest and thereby silence gene expression.

Methods employing ta-siRNAs useful for the repression or inactivation of a gene product according to the process of the present invention are described in US 60/672976 and 60/718645. Nucleic acid sequences as described in above items are expressed in the cell or organism by transformation/transfection of the cell or organism or are introduced in the cell or organism by known methods, for example as disclosed in item A).

**2.2 Agronomically relevant traits**

The chimeric transcription regulating nucleotide sequences can be preferably employed to confer to the transformed monocotyledonous plant an agronomically relevant trait. Such traits include, but are not limited to, herbicide resistance, herbicide tolerance, insect resistance, insect tolerance, disease resistance, disease tolerance (viral, bacterial, fungal, nematode), stress tolerance, stress resistance, as exemplified by resistance or tolerance to drought, heat, chilling, freezing, excessive moisture, salt stress and oxidative stress, increased yield, food content and value, increased feed content and value, physical appearance, male sterility, female sterility, drydown, standability, prolificacy, starch quantity and quality, oil quantity and quality, protein quality and quantity, amino acid composition, and the like. Although numerous nucleic acid sequences are suitable to be expressed by the chimeric transcription regulating nucleic acid sequence of the invention (*e.g.*, in combination with preferred promoters, for example with the super-promoter) most preferably the nucleic acid is conferring upon expression to the monocotyledonous plant an agronomically relevant trait selected from the group consisting of

i) enhanced resistance or tolerance against at least one stress factor,
ii) increased nutritional quality of a seed or a sprout,
iii) increased yield.

One of the most economically relevant traits is yield. Yield is heavily affected by damage in any kind to the embryo and young seedling. Accordingly, any kind of trait which protects the young seedling and embryo or enhances its performance is advantageous with respect to yield. Thus, a trait resulting in stress resistance (see below) can also result in increased yield. Thus, another embodiment of the invention relates to a method for conferring increased yield and/or increased stress tolerance to a plant, said method comprising the steps of

A) introducing into a plant an expression construct comprising a polynucleotide encoding a plant transcription regulating sequence, wherein the polynucleotide encoding the transcription regulating sequence comprises

i) a first nucleic acid molecule selected from the group consisting of

a) a polynucleotide as defined in SEQ ID NO:1;
b) a polynucleotide having at least 70% or 80%, more preferably at least 85% or 90%, most preferably at least 95%, 98% or 99% sequence identity to the polynucleotide of SEQ ID NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1mM EDTA at 50°C with washing in 0.5 X SSC, 0.1% SDS at 50°C, more preferably in 7% sodium dodecyl sulfate (SDS), 0.5 M $Na\text{-}PO_4$, 1 mM EDTA at 50°C with washing in 0.1 X SSC, 0.1% SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C, to a nucleic acid comprising at least 50 nucleotides of a polynucleotide as defined in SEQ ID NO:1, or the complement thereof, and operably linked thereto

ii) a second nucleic acid molecule selected from the group consisting of

a) a polynucleotide as defined in SEQ ID NO:3;
b) a polynucleotide having at least 60%, 70% or 80%, more preferably at least 85% or 90%, most preferably at least 95%, 98% or 99% sequence identity to the polynucleotide of SEQ ID NO:3;
c) a fragment of at least 200 consecutive bases of the polynucleotide of SEQ ID NO:3; and
d) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C, to a nucleic acid comprising at least 50 nucleotides of a sequence described by SEQ ID NO:3, or the complement thereof,

and operably linked to at least one nucleic acid which is heterologous in relation to said first or said second nucleic acid sequence the transcription regulating sequence and is capable to confer to a plant an increased yield and/or increased stress tolerance, and
B) selecting transgenic plants, wherein the plants have increased yield and/or increased stress tolerance as compared to the wild type or null segregant plants.

The increased yield and/or increased stress tolerance and the corresponding heterologous nucleic acid sequence to be expressed are defined as above. More specific examples are given herein below. Preferred chimeric transcription regulating nucleotide sequence are described above.

### 2.2.1 Increase stress resistance or tolerance

The transcription regulating nucleotide sequences can be preferably employed to confer to the transformed monocotyledonous plant an increased (or enhanced) stress resistance (preferably to achieve a stress-resistant or stress tolerant plant). By "resistant" is meant a plant, which exhibits substantially no phenotypic changes as a consequence of agent administration, infection with a pathogen, or exposure to stress. By "tolerant" is meant a plant, which, although it may exhibit some phenotypic changes as a consequence of infection, does not have a substantially decreased reproductive capacity or substantially altered metabolism.

Various nucleic acids sequences are known to the person skilled in the art to obtain such stress resistance. Said sequences may include but are not limited to polynucleotides encoding a polypeptide involved in phytohormone biosynthesis, phytohormone regulation, cell cycle regulation, or carbohydrate metabolism. The stress factor is preferably defined as above. The heterologous nucleic acid sequence to be expressed (e.g., either as a sense, antisense or double-stranded RNA) may encode a stress-related polypeptide as desribed above (or a part thereof; preferably a part of at least 5, more preferably at least 10, most preferably at least 30 consecutive amino acids). Preferred chimeric transcription regulating nucleotide sequence are described above.

The stress factor and the heterologous nucleic acid sequence to be expressed are preferably defined as above. Preferred chimeric transcription regulating nucleotide sequence are described above.

The stress resistance, which can be advantageously obtained, is preferably against an abiotic or biotic stress factor. The biotic stress factor may be selected from the group consisting of fungal resistance, nematode resistance, insect resistance, virus resistance, and bacteria resistance. Preferably, the biotic stress factor is a seed-borne disease (mainly fungal diseases *e.g.* common bunt (*Tilletia tritici*) mainly in wheat; leaf stripe (*Pyrenophora graminea*), and loose smut (*Ustilago nuda*) mainly in barley).

The abiotic stress factor may be selected from the group consisting of drought, excessive moisture, heat, chilling, freezing, cold, salt, nitrogen, high plant population density, UV light and oxidative stress. Preferably, the stress resistance is achieved by inducing early vigor.

Various nucleic acids sequences are known to the person skilled in the art to obtain such stress resistance. Said sequences may include but are not limited to polynucleotides encoding a polypeptide involved in phytohormone biosynthesis, phytohormone regulation, cell cycle regulation, or carbohydrate metabolism. More specific examples are given below.

The invention is applicable to all monocotyledonous plants such as maize, wheat, rice, barley, oat, rye, sorghum, millet, triticale, ryegrass or coix, but is preferably applicable to kernel producing cereal plants of the Pooideae family such as maize, wheat, rice, barley, oat, rye, sorghum, millet, or triticale, preferably to maize, barley and wheat, most preferably to maize.

Further embodiments of the invention relate to seeds, parts and cells of the monocotyledonous plant of the invention. Preferably, the plant parts are selected from the group consisting of: cells, protoplasts, cell tissue cultures, callus, cell clumps, embryos, pollen, ovules, seeds, flowers, kernels, ears, cobs, leaves, husks, stalks, roots, root tips, anthers, and silk.

Indirectly, the increased stress tolerance may cause one or more traits which promote aspects of enhanced grain agronomic characteristics, grain fill, decreased kernel abortion, increased transport of nutrients and the like.

### 2.2.1.1 Insect Resistance and Tolerance

An important aspect of the present invention concerns the introduction of insect resistance-conferring genes into plants. Potential insect resistance genes, which can be introduced, include *Bacillus thuringiensis* crystal toxin genes or Bt genes (Watrud 1985). Bt genes may provide resistance to lepidopteran or coleopteran pests such as European Corn Borer (ECB) and corn rootworm (CRW). Preferred Bt toxin genes for use in such embodiments include the Cry-IA (b) and CryIA(c) genes. Endotoxin genes from other species of B. thuringiensis, which affect insect growth or development, may also be employed in this regard. Protease inhibitors may also provide insect resistance (Johnson 1989), and will thus have utility in plant transformation. The use of a protease inhibitor II gene, pinII, from tomato or potato is envisioned to be particularly useful. Even more advantageous is the use of a pinII gene in combination with a Bt toxin gene, the combined effect of which has been discovered by the present inventors to produce synergistic insecticidal activity. Other genes, which encode inhibitors of the insects' digestive system, or those that encode enzymes or co-factors that facilitate the production of inhibitors, may also be useful. Cystatin and amylase inhibitors, such as those from wheat and barley, may exemplify this group.

Also, genes encoding lectins may confer additional or alternative insecticide properties. Lectins (originally termed phytohemagglutinins) are multivalent carbohydrate-binding proteins, which have the ability to agglutinate red blood cells from a range of species. Lectins have been identified recently as insecticidal agents with activity against weevils, ECB and rootworm (Murdock 1990; Czapla & Lang, 1990). Lectin genes contemplated to be useful include, for example, barley and wheat germ agglutinin (WGA) and rice lectins (Gatehouse 1984), with WGA being preferred.

Genes controlling the production of large or small polypeptides active against insects when introduced into the insect pests, such as, *e.g.*, lytic peptides, peptide hormones and toxins and venoms, form another aspect of the invention.

For example, it is contemplated, that the expression of juvenile hormone esterase, directed towards specific insect pests, may also result in insecticidal activity, or perhaps cause cessation of metamorphosis (Hammock 1990).

Transgenic plants expressing genes, which encode enzymes that affect the integrity of the insect cuticle form yet another aspect of the invention. Such genes include those encoding, *e.g.*, chitinase, proteases, lipases and also genes for the production of nikkomycin, a compound that inhibits chitin synthesis, the introduction of any of which is contemplated to produce insect resistant maize plants. Genes that code for activities that affect insect molting, such those affecting the production of ecdysteroid UDP-glucosyl transferase, also fall within the scope of the useful transgenes of the present invention.

Genes that code for enzymes that facilitate the production of compounds that reduce the nutritional quality of the host plant to insect pests are also encompassed by the present invention. It may be possible, for instance, to confer insecticidal activity on a plant by altering its sterol composition. Sterols are obtained by insects from their diet and are used for hormone synthesis and membrane stability. Therefore alterations in plant sterol composition by expression of novel genes, *e.g.*, those that directly promote the production of undesirable sterols or those that convert desirable sterols into undesirable forms, could have a negative effect on insect growth and/or development and hence endow the plant with insecticidal activity. Lipoxygenases are naturally occurring plant enzymes that have been shown to exhibit anti-nutritional effects on insects and to reduce the nutritional quality of their diet. Therefore, further embodiments of the invention concern transgenic plants with enhanced lipoxygenase activity which may be resistant to insect feeding.

The present invention also provides methods and compositions by which to achieve qualitative or quantitative changes in plant secondary metabolites. One example concerns transforming plants to produce DIMBOA which, it is contemplated, will confer resistance to European corn borer, rootworm and several other maize insect pests. Candidate genes that are particularly considered for use in this regard include those genes at the bx locus known to be involved in the synthetic DIMBOA pathway (Dunn 1981). The introduction of genes that can regulate the production of maysin, and genes involved in the production of dhurrin in sorghum, is also contemplated to be of use in facilitating resistance to earworm and rootworm, respectively.

Tripsacum dactyloides is a species of grass that is resistant to certain insects, including corn rootworm. It is anticipated that genes encoding proteins that are toxic to insects or are involved in the biosynthesis of compounds toxic to insects will be isolated from Tripsacum and that these novel genes will be useful in conferring resistance to insects. It is known that the basis of insect resistance in Tripsacum is genetic, because said resistance has been transferred to Zea mays via sexual crosses (Branson & Guss, 1972).

Further genes encoding proteins characterized as having potential insecticidal activity may also be used as transgenes in accordance herewith. Such genes include, for example, the cowpea trypsin inhibitor (CpTI; Hilder 1987) which may be used as a rootworm deterrent; genes encoding avermectin (Campbell 1989; Ikeda 1987) which may prove particularly useful as a corn rootworm deterrent; ribosome inactivating protein genes; and even genes that regulate plant structures. Transgenic maize including anti-insect antibody genes and genes that code for enzymes that can covert a non-toxic insecticide (pro-insecticide) applied to the outside of the plant into an insecticide inside the plant are also contemplated.

### 2.2.1.2 Environment or Stress Resistance and Tolerance

Improvement of a plant's ability to tolerate various environmental stresses such as, but not limited to, drought, excess moisture, nitrogen, chilling, freezing, high temperature, salt, and oxidative stress, can also be effected through expression of heterologous, or overexpression of homologous genes. Benefits may be realized in terms of increased resistance to freezing temperatures through the introduction of an "antifreeze" protein such as that of the Winter Flounder (Cutler 1989) or synthetic gene derivatives thereof. Improved chilling tolerance may also be conferred through increased expression of glycerol-3-phosphate acetyltransferase in chloroplasts (Murata 1992; Wolter 1992). Resistance to oxidative stress (often exacerbated by conditions such as chilling temperatures in combination with high light intensities) can be conferred by expression of superoxide dismutase (Gupta 1993), and may be improved by glutathione reductase (Bowler 1992). Such strategies may allow for tolerance to freezing in newly emerged fields as well as extending later maturity higher yielding varieties to earlier relative maturity zones.

Expression of novel genes that favorably effect plant water content, total water potential, osmotic potential, and turgor can enhance the ability of the plant to tolerate drought. As used herein, the terms "drought resistance" and "drought tolerance" are used to refer to a plant's increased resistance or tolerance to stress induced by a reduction in water availability, as compared to normal circumstances, and the ability of the plant to function and survive in lower-water environments, and perform in a relatively superior manner. In this aspect of the invention it is proposed, for example, that the expression of a gene encoding the biosynthesis of osmotically active solutes can impart protection against drought. Within this class of genes are DNAs encoding mannitol dehydrogenase (Lee and Saier, 1982) and trehalose-6-phosphate synthase (Kaasen 1992). Through the subsequent action of native phosphatases in the cell or by the introduction and coexpression of a specific phosphatase, these introduced genes will result in the accumulation of either mannitol or trehalose, respectively, both of which have been well documented as protective compounds able to mitigate the effects of stress. Mannitol accumulation in transgenic tobacco has been verified and preliminary results indicate that plants expressing high levels of this metabolite are able to tolerate an applied osmotic stress (Tarczynski 1992).

Similarly, the efficacy of other metabolites in protecting either enzyme function (*e.g.* alanopine or propionic acid) or membrane integrity (*e.g.,* alanopine) has been documented (Loomis 1989), and therefore expression of gene encoding the biosynthesis of these compounds can confer drought resistance in a manner similar to or complimentary to mannitol. Other examples of naturally occurring metabolites that are osmotically active and/or provide some direct protective effect during drought and/or desiccation include sugars and sugar derivatives such as fructose, erythritol (Coxson 1992), sorbitol, dulcitol (Karsten 1992), glucosylglycerol (Reed 1984; Erdmann 1992), sucrose, stachyose (Koster & Leopold 1988; Blackman 1992), ononitol and pinitol (Vernon & Bohnert 1992), and raffinose (Bernal-Lugo & Leopold 1992). Other osmotically active solutes, which are not sugars, include, but are not limited to, proline and glycine-betaine (Wyn-Jones and Storey, 1981). Continued canopy growth and increased reproductive fitness during times of stress can be augmented by introduction and expression of genes such as those controlling the osmotically active compounds discussed above and other such compounds, as represented in one exemplary embodiment by the enzyme myoinositol 0-methyltransferase.

It is contemplated that the expression of specific proteins may also increase drought tolerance. Three classes of Late Embryogenic Proteins have been assigned based on structural similarities (see Dure 1989). All three classes of these proteins have been demonstrated in maturing (*i.e.*, desiccating) seeds. Within these 3 types of proteins, the Type-II (dehydrin-type) have generally been implicated in drought and/or desiccation tolerance in vegetative plant parts (*e.g.* Mundy and Chua, 1988; Piatkowski 1990; Yamaguchi-Shinozaki 1992). Recently, expression of a Type-III LEA (HVA-1) in tobacco was found to influence plant height, maturity and drought tolerance (Fitzpatrick, 1993). Expression of structural genes from all three groups may therefore confer drought tolerance. Other types of proteins induced during water stress include thiol proteases, aldolases and transmembrane transporters (Guerrero 1990), which may confer various protective and/or repair-type functions during drought stress. The expression of a gene that effects lipid biosynthesis and hence membrane composition can also be useful in conferring drought resistance on the plant.

Many genes that improve drought resistance have complementary modes of action. Thus, combinations of these genes might have additive and/or synergistic effects in improving drought resistance in maize. Many of these genes also improve freezing tolerance (or resistance); the physical stresses incurred during freezing and drought are similar in nature and may be mitigated in similar fashion. Benefit may be conferred via constitutive expression or tissue-specific of these genes, but the preferred means of expressing these novel genes may be through the use of a turgor-induced promoter (such as the promoters for the turgor-induced genes described in Guerrero et al. 1990 and Shagan 1993). Spatial and temporal expression patterns of these genes may enable maize to better withstand stress.

Expression of genes that are involved with specific morphological traits that allow for increased water extractions from drying soil would be of benefit. For example, introduction and expression of genes that alter root characteristics may enhance water uptake. Expression of genes that enhance reproductive fitness during times of stress would be of significant value. For example, expression of DNAs that improve the synchrony of pollen shed and receptiveness of the female flower parts, *i.e.,* silks, would be of benefit. In addition, expression of genes that minimize kernel abortion during times of stress would increase the amount of grain to be harvested and hence be of value. Regulation of cytokinin levels in monocots, such as maize, by introduction and expression of an isopentenyl transferase gene with appropriate regulatory sequences can improve monocot stress resistance and yield (Gan 1995).

Given the overall role of water in determining yield, it is contemplated that enabling plants to utilize water more efficiently, through the introduction and expression of novel genes, will improve overall performance even when soil water availability is not limiting. By introducing genes that improve the ability of plants to maximize water usage across a full range of stresses relating to water availability, yield stability or consistency of yield performance may be realized.

Improved protection of the plant to abiotic stress factors such as drought, heat or chill, can also be achieved - for example - by overexpressing antifreeze polypeptides from Myoxocephalus Scorpius (WO 00/00512), Myoxocephalus octodecemspinosus, the Arabidopsis thaliana transcription activator CBF1, glutamate dehydrogenases (WO 97/12983, WO 98/11240), calcium-dependent protein kinase genes (WO 98/26045), calcineurins (WO 99/05902), casein kinase from yeast (WO 02/052012), farnesyltransferases (WO 99/06580; Pei ZM *et al.* 1998), ferritin (Deak M *et al.* 1999), oxalate oxidase (WO 99/04013; Dunwell JM 1998), DREB1A factor ("dehydration response element B 1A"; Kasuga M *et al.* 1999), genes of mannitol or trehalose synthesis such as trehalose-phosphate synthase or trehalose-phosphate phosphatase (WO 97/42326) or by inhibiting genes such as trehalase (WO 97/50561).

One use for the chimeric transcription regulating sequences is to protect the embryo from cold damage during germination. One important factor is oxidative damage. The super-promoter could drive *i.e.* catalase, ascorbate peroxidase, superoxide dismutase and alike. The cold affects the COX enzyme activity also through a rigid membrane. For drought-stress expression of glutamine synthase and glycine betain synthase might be beneficial.

### 2.2.1.3 Disease Resistance and Tolerance

It is proposed that increased resistance to diseases may be realized through introduction of genes into plants. It is possible to produce resistance to diseases caused, by viruses, bacteria, fungi, root pathogens, insects and nematodes. It is also contemplated that control of mycotoxin producing organisms may be realized through expression of introduced genes.

Resistance to viruses may be produced through expression of novel genes. For example, it has been demonstrated that expression of a viral coat protein in a transgenic plant can impart resistance to infection of the plant by that virus and perhaps other closely related viruses (Cuozzo 1988, Hemenway 1988, Abel 1986). It is contemplated that expression of antisense genes targeted at essential viral functions may impart resistance to said virus. For example, an antisense gene targeted at the gene responsible for replication of viral nucleic acid may inhibit said replication and lead to resistance to the virus. It is believed that interference with other viral functions through the use of antisense genes may also increase resistance to viruses. Further it is proposed that it may be possible to achieve resistance to viruses through other approaches, including, but not limited to the use of satellite viruses.

It is proposed that increased resistance to diseases caused by bacteria and fungi may be realized through introduction of novel genes. It is contemplated that genes encoding so-called "peptide antibiotics," pathogenesis related (PR) proteins, toxin resistance, and proteins affecting host-pathogen interactions such as morphological characteristics will be useful. Peptide antibiotics are polypeptide sequences, which are inhibitory to growth of bacteria and other microorganisms. For example, the classes of peptides referred to as cecropins and magainins inhibit growth of many species of bacteria and fungi. It is proposed that expression of PR proteins in plants may be useful in conferring resistance to bacterial disease. These genes are induced following pathogen attack on a host plant and have been divided into at least five classes of proteins (Bol 1990). Included amongst the PR proteins are beta-1,3-glucanases, chitinases, and osmotin and other proteins that are believed to function in plant resistance to disease organisms. Other genes have been identified that have antifungal properties, *e.g.*, UDA (stinging nettle lectin) and hevein (Broakgert 1989; Barkai-Golan 1978). It is known that certain plant diseases are caused by the production of phytotoxins. Resistance to these diseases could be achieved through expression of a novel gene that encodes an enzyme capable of degrading or otherwise inactivating the phytotoxin. Expression novel genes that alter the interactions between the host plant and pathogen may be useful in reducing the ability the disease organism to invade the tissues of the host plant, *e.g.*, an increase in the waxiness of the leaf cuticle or other morphological characteristics.

Plant parasitic nematodes are a cause of disease in many plants. It is proposed that it would be possible to make the plant resistant to these organisms through the expression of novel genes. It is anticipated that control of nematode infestations would be accomplished by altering the ability of the nematode to recognize or attach to a host plant and/or enabling the plant to produce nematicidal compounds, including but not limited to proteins.

Furthermore, a resistance to fungi, insects, nematodes and diseases, can be achieved by targeted accumulation of certain metabolites or proteins. Such proteins include but are not limited to glucosinolates (defense against herbivores), chitinases or glucanases and other enzymes which destroy the cell wall of parasites, ribosome-inactivating proteins (RIPs) and other proteins of the plant resistance and stress reaction as are induced when plants are wounded or attacked by microbes, or chemically, by, for example, salicylic acid, jasmonic acid or ethylene, or lysozymes from nonplant sources such as, for example, T4-lysozyme or lysozyme from a variety of mammals, insecticidal proteins such as *Bacillus thuringiensis* endotoxin, alpha-amylase inhibitor or protease inhibitors (cowpea trypsin inhibitor), lectins such as wheatgerm agglutinin, RNAses or ribozymes. Further examples are nucleic acids which encode the Trichoderma harzianum chit42 endochitinase (GenBank Acc. No.: S78423) or the N-hydroxylating, multi-functional cytochrome P-450 (CYP79) protein from Sorghum bicolor (GenBank Acc. No.: U32624), or functional equivalents of these. The accumulation of glucosinolates as protection from pests (Rask L *et al.* 2000; Menard R *et al.* 1999), the expression of *Bacillus thuringiensis* endotoxins (Vaeck *et al.* 1987) or the protection against attack by fungi, by expression of chitinases, for example from beans (Broglie et al. 1991), is advantageous. Resistance to pests such as, for example, the rice pest *Nilaparvata lugens* in rice plants can be achieved by expressing the snowdrop (*Galanthus nivalis*) lectin agglutinin (Rao *et al.* 1998).The expression of synthetic cryIA(b) and cryIA(c) genes, which encode lepidoptera-specific *Bacillus thuringiensis* D-endotoxins can bring about a resistance to insect pests in various plants (Goyal RK *et al.* 2000). Further genes which are suitable for pathogen defense comprise "polygalacturonase-inhibiting protein" (PGIP), thaumatine, invertase and antimicrobial peptides such as lactoferrin (Lee TJ *et al.* 2002). Other nucleic acid sequences which may be advantageously used herein include traits for insect control (U.S. Nos. 6,063,597; 6,063,756; 6,093,695; 5,942,664; and 6,110,464), fungal disease resistance (U.S. Pat. Nos. 5,516,671; 5,773,696; 6,121,436; 6,316,407; and 6,506,962), virus resistance (U.S. Pat. Nos. 5,304,730 and 6,013,864), nematode resistance (US 6,228,992), and bacterial disease resistance (US 5,516,671).

The heterologous nucleic acid sequence to be expressed may encode a stress-related polypeptide (or a part thereof; preferably a part of at least 5, more preferably at least 10, most preferably at least 30 consecutive amino acids). Preferred chimeric transcription regulating nucleotide sequence are described above.

### 2.2.2 Increased nutritional quality of a seed or a sprout

The chimeric transcription regulating nucleotide sequences can be preferably employed to confer to the transformed monocotyledonous plant an increased (or enhanced) nutritional quality of a seed or a sprout. Accordingly another embodiment of the invention relates to a method for conferring increased nutritional quality and/or oil content of a seed or a sprout to a plant, said method comprising the steps of

A) introducing into a plant an expression construct comprising a polynucleotide encoding a plant transcription regulating sequence, wherein the polynucleotide encoding the transcription regulating sequence comprises

i) a first nucleic acid molecule selected from the group consisting of

a) a polynucleotide as defined in SEQ ID NO:1;
b) a polynucleotide having at least 70% or 80%, more preferably at least 85% or 90%, most preferably at least 95%, 98% or 99% sequence identity to the polynucleotide of SEQ ID NO:1; and
c) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1% SDS at 50°C, more preferably in 7% sodium dodecyl sulfate (SDS), 0.5 M $NaPO_4$ 1 mM EDTA at 50°C with washing in 0.1 X SSC, 0.1% SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a polynucleotide as defined in SEQ ID NO:1, or the complement thereof, and operably linked to

ii a second nucleic acid molecule selected from the group consisting of

a) a polynucleotide as defined in SEQ ID NO:3;
b) a polynucleotide having at least 60%, 70% or 80%, more preferably at least 85% or 90%, most preferably at least 95%, 98% or 99% sequence identity to the polynucleotide of SEQ ID NO:3; and
c) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1% SDS at 50°C, more preferably in 7% sodium dodecyl sulfate (SDS), 0.5 M $NaPO_4$ 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a sequence described by SEQ ID NO:3, or the complement thereof,

and operably linked to at least one nucleic acid which is heterologous in relation to said first or said second nucleic acid sequence and is suitable to confer to a plant an increased nutritional quality and/or oil content of a seed or a sprout, and
B) selecting transgenic plants, wherein the plants have increased nutritional quality and/or oil content of a seed or a sprout as compared to the wild type or null segregant plants.

The nutritional quality and/or oil content may comprise an increased content of at least one compound selected from the group consisting of vitamins, carotinoids, antioxidants, unsaturated fatty acids, and poly-unsaturated fatty acids. The heterologous nucleic acid sequence to be expressed (e.g., either as a sense, antisense or double-stranded RNA) may encode a trait-related polypeptide (or a part thereof; preferably a part of at least 5, more preferably at least 10, most preferably at least 30 consecutive amino acids) as described below, or a functional equivalent thereof, which is capable to bring about the same phenotype than any of said polypeptide. Preferred chimeric transcription regulating nucleotide sequence are described above.
The nutritional quality and the corresponding heterologous nucleic acid sequence to be expressed are defined herein below. Preferred chimeric transcription regulating nucleotide sequence are described above, most preferred is the super-promoter. The monocotyledonous plant to which the methods of this invention are preferably applied to may be selected from the group consisting of maize, wheat, rice, barley, oat, rye, sorghum, ryegrass or coix. Preferably the plant is a cereal plant selected from the group consisting of maize, wheat, barley, rice, oat, rye, and sorghum, even more preferably from maize, wheat, and rice, most preferably the plant is a maize plant.
An increased nutritional quality may - for example - result in one or more of the following properties: modifying the fatty acid composition in a plant, altering the amino acid content of a plant, increases the concentration of a plant metabolite.
Genes may be introduced into monocotyledonous plants, particularly commercially important cereals such as maize, wheat or rice, to improve the grain for which the cereal is primarily grown. A wide range of novel transgenic plants produced in this manner may be envisioned depending on the particular end use of the grain.

For example, the largest use of maize grain is for feed or food. Introduction of genes that alter the composition of the grain may greatly enhance the feed or food value. The primary components of maize grain are starch, protein, and oil. Each of these primary components of maize grain may be improved by altering its level or composition. Several examples may be mentioned for illustrative purposes but in no way provide an exhaustive list of possibilities.

The protein of many cereal grains is suboptimal for feed and food purposes especially when fed to pigs, poultry, and humans. The protein is deficient in several amino acids that are essential in the diet of these species, requiring the addition of supplements to the grain. Limiting essential amino acids may include lysine, methionine, tryptophan, threonine, valine, arginine, and histidine. Some amino acids become limiting only after the grain is supplemented with other inputs for feed formulations. For example, when the grain is supplemented with soybean meal to meet lysine requirements, methionine becomes limiting. The levels of these essential amino acids in seeds and grain may be elevated by mechanisms which include, but are not limited to, the introduction of genes to increase the biosynthesis of the amino acids, decrease the degradation of the amino acids, increase the storage of the amino acids in proteins, or increase transport of the amino acids to the seeds or grain.

One mechanism for increasing the biosynthesis of the amino acids is to introduce genes that deregulate the amino acid biosynthetic pathways such that the plant can no longer adequately control the levels that are produced. This may be done by deregulating or bypassing steps in the amino acid biosynthetic pathway that are normally regulated by levels of the amino acid end product of the pathway. Examples include the introduction of genes that encode deregulated versions of the enzymes aspartokinase or dihydrodipicolinic acid (DHDP)-synthase for increasing lysine and threonine production, and anthranilate synthase for increasing tryptophan production. Reduction of the catabolism of the amino acids may be accomplished by introduction of DNA sequences that reduce or eliminate the expression of genes encoding enzymes that catalyse steps in the catabolic pathways such as the enzyme lysine-ketoglutarate reductase.

The protein composition of the grain may be altered to improve the balance of amino acids in a variety of ways including elevating expression of native proteins, decreasing expression of those with poor composition, changing the composition of native proteins, or introducing genes encoding entirely new proteins possessing superior composition. DNA may be introduced that decreases the expression of members of the zein family of storage proteins. This DNA may encode ribozymes or antisense sequences directed to impairing expression of zein proteins or expression of regulators of zein expression such as the opaque-2 gene product. The protein composition of the grain may be modified through the phenomenon of cosuppression, *i.e.,* inhibition of expression of an endogenous gene through the expression of an identical structural gene or gene fragment introduced through transformation (Goring 1991). Additionally, the introduced DNA may encode enzymes, which degrade zeines. The decreases in zein expression that are achieved may be accompanied by increases in proteins with more desirable amino acid composition or increases in other major seed constituents such as starch. Alternatively, a chimeric gene may be introduced that comprises a coding sequence for a native protein of adequate amino acid composition such as for one of the globulin proteins or 10 kD zein of maize and a promoter or other regulatory sequence designed to elevate expression of said protein. The coding sequence of said gene may include additional or replacement codons for essential amino acids. Further, a coding sequence obtained from another species, or, a partially or completely synthetic sequence encoding a completely unique peptide sequence designed to enhance the amino acid composition of the seed may be employed.

The introduction of genes that alter the oil content of the grain may be of value. Increases in oil content may result in increases in metabolizable energy content and density of the seeds for uses in feed and food. The introduced genes may encode enzymes that remove or reduce rate-limitations or regulated steps in fatty acid or lipid biosynthesis. Such genes may include, but are not limited to, those that encode acetyl-CoA carboxylase, ACP-acyltransferase, beta-ketoacyl-ACP synthase, plus other well-known fatty acid biosynthetic activities. Other possibilities are genes that encode proteins that do not possess enzymatic activity such as acyl carrier protein. Additional examples include 2-acetyltransferase, oleosin pyruvate dehydrogenase complex, acetyl CoA synthetase, ATP citrate lyase, ADP-glucose pyrophosphorylase and genes of the carnitine-CoA-acetyl-CoA shuttles. It is anticipated that expression of genes related to oil biosynthesis will be targeted to the plastid, using a plastid transit peptide sequence and preferably expressed in the seed embryo. Genes may be introduced that alter the balance of fatty acids present in the oil providing a heathier or nutritious feedstuff. The introduced DNA may also encode sequences that block expression of enzymes involved in fatty acid biosynthesis, altering the proportions of fatty acids present in the grain such as described below.

Genes may be introduced that enhance the nutrition value of the starch component of the grain, for example by increasing the degree of branching, resulting in improved utilization of the starch in cows by delaying its metabolism.

Besides affecting the major constituents of the grain, genes may be introduced that affect a variety of other nutrient, processing, or other quality aspects of the grain as used for feed or food. For example, pigmentation of the grain may be increased or decreased. Enhancement and stability of yellow pigmentation is desirable in some animal feeds and may be achieved by introduction of genes that result in enhanced production of xanthophylls and carotenes by eliminating rate-limiting steps in their production. Such genes may encode altered forms of the enzymes phytoene synthase, phytoene desaturase, or lycopene synthase. Alternatively, unpigmented white corn is desirable for production of many food products and may be produced by the introduction of DNA, which blocks or eliminates steps in pigment production pathways.

Feed or food comprising some cereal grains possesses insufficient quantities of vitamins and must be supplemented to provide adequate nutrition value. Introduction of genes that enhance vitamin biosynthesis in seeds may be envisioned including, for example, vitamins A, E, $B_{12}$, choline, and the like. For example, maize grain also does not possess sufficient mineral content for optimal nutrition value. Genes that affect the accumulation or availability of compounds containing phosphorus, sulfur, calcium, manganese, zinc, and iron among others would be valuable. An example may be the introduction of a gene that reduced phytic acid production or encoded the enzyme phytase, which enhances phytic acid breakdown. These genes would increase levels of available phosphate in the diet, reducing the need for supplementation with mineral phosphate.

Numerous other examples of improvement of cereals for feed and food purposes might be described. The improvements may not even necessarily involve the grain, but may, for example, improve the value of the grain for silage. Introduction of DNA to accomplish this might include sequences that alter lignin production such as those that result in the "brown midrib" phenotype associated with superior feed value for cattle.

In addition to direct improvements in feed or food value, genes may also be introduced which improve the processing of grain and improve the value of the products resulting from the processing. The primary method of processing certain grains such as maize is via wetmilling. Maize may be improved though the expression of novel genes that increase the efficiency and reduce the cost of processing such as by decreasing steeping time.

Improving the value of wetmilling products may include altering the quantity or quality of starch, oil, corn gluten meal, or the components of corn gluten feed. Elevation of starch may be achieved through the identification and elimination of rate limiting steps in starch biosynthesis or by decreasing levels of the other components of the grain resulting in proportional increases in starch. An example of the former may be the introduction of genes encoding ADP-glucose pyrophosphorylase enzymes with altered regulatory activity or which are expressed at higher level. Examples of the latter may include selective inhibitors of, for example, protein or oil biosynthesis expressed during later stages of kernel development.

Oil is another product of wetmilling of corn and other grains, the value of which may be improved by introduction and expression of genes. The quantity of oil that can be extracted by wetmilling may be elevated by approaches as described for feed and food above. Oil properties may also be altered to improve its performance in the production and use of cooking oil, shortenings, lubricants or other oil-derived products or improvement of its health attributes when used in the food-related applications. Novel fatty acids may also be synthesized which upon extraction can serve as starting materials for chemical syntheses. The changes in oil properties may be achieved by altering the type, level, or lipid arrangement of the fatty acids present in the oil. This in turn may be accomplished by the addition of genes that encode enzymes that catalyze the synthesis of novel fatty acids and the lipids possessing them or by increasing levels of native fatty acids while possibly reducing levels of precursors. Alternatively DNA sequences may be introduced which slow or block steps in fatty acid biosynthesis resulting in the increase in precursor fatty acid intermediates. Genes that might be added include desaturases, epoxidases, hydratases, dehydratases, and other enzymes that catalyze reactions involving fatty acid intermediates. Representative examples of catalytic steps that might be blocked include the desaturations from stearic to oleic acid and oleic to linolenic acid resulting in the respective accumulations of stearic and oleic acids.

Improvements in the other major cereal wetmilling products, gluten meal and gluten feed, may also be achieved by the introduction of genes to obtain novel plants. Representative possibilities include but are not limited to those described above for improvement of food and feed value.

In addition it may further be considered that the plant be used for the production or manufacturing of useful biological compounds that were either not produced at all, or not produced at the same level, in the plant previously. The novel plants producing these compounds are made possible by the introduction and expression of genes by transformation methods. The possibilities include, but are not limited to, any biological compound which is presently produced by any organism such as proteins, nucleic acids, primary and intermediary metabolites, carbohydrate polymers, etc. The compounds may be produced by the plant, extracted upon harvest and/or processing, and used for any presently recognized useful purpose such as pharmaceuticals, fragrances, industrial enzymes to name a few.

Further possibilities to exemplify the range of grain traits or properties potentially encoded by introduced genes in transgenic plants include grain with less breakage susceptibility for export purposes or larger grit size when processed by dry milling through introduction of genes that enhance gamma-zein synthesis, popcorn with improved popping, quality and expansion volume through genes that increase pericarp thickness, corn with whiter grain for food uses though introduction of genes that effectively block expression of enzymes involved in pigment production pathways, and improved quality of alcoholic beverages or sweet corn through introduction of genes which affect flavor such as the shrunken gene (encoding sucrose synthase) for sweet corn.

Useful nucleic acid sequences that can be combined with the promoter nucleic acid sequence of the present invention and provide improved end-product traits include, without limitation, those encoding seed storage proteins, fatty acid pathway enzymes, tocopherol biosynthetic enzymes, amino acid biosynthetic enzymes, and starch branching enzymes. A discussion of exemplary heterologous DNAs useful for the modification of plant phenotypes may be found in, for example, U.S. Nos. 6,194,636; 6,207,879; 6,232,526; 6,426,446; 6,429,357; 6,433,252; 6,437,217; 6,515,201; and 6,583,338 and WO 02/057471. Such traits include but are not limited to:

- Expression of metabolic enzymes for use in the food-and-feed sector, for example of phytases and cellulases. Especially preferred are nucleic acids such as the artificial cDNA which encodes a microbial phytase (GenBank Acc. No. A19451) or functional equivalents thereof.
- Expression of genes which bring about an accumulation of fine chemicals such as of tocopherols, tocotrienols or carotenoids. An example which may be mentioned is phytoene desaturase. Preferred are nucleic acids which encode the Narcissus pseudonarcissus photoene desaturase (GenBank Acc. No.X78815) or functional equivalents thereof. Preferred tocopherol biosynthetic enzymes include tyrA, slr1736, ATPT2, dxs, dxr, GGPPS, HPPD, GMT, MT1, tMT2, AANT1, slr 1737, and an antisense construct for homogentisic acid dioxygenase (Kridl *et al.* (1991); Keegstra (1989); Nawrath *et al.* (1994); Xia *et al.* (1992); Lois *et al.* (1998); Takahashi *et al.* (1998); Norris *et al.* (1998); Bartley and Scolnik (1994); Smith *et al.* (1997); WO 00/32757; WO 00/10380; Saint Guily *et al.* (1992); Sato *et al.* (2000).
- starch production (U.S. Nos. 5,750,876 and 6,476,295), high protein production (US 6,380,466), fruit ripening (US 5,512,466), enhanced animal and human nutrition (U.S. Nos. 5,985,605 and 6,171,640), biopolymers (US 5,958,745 and U.S. Patent Publication No. 2003/0028917), environmental stress resistance (US 6,072,103), pharmaceutical peptides (US 6,080,560), improved processing traits (US 6,476,295), improved digestibility (US 6,531,648), low raffinose (US 6,166,292), industrial enzyme production (US 5,543,576), improved flavor (US 6,011,199), nitrogen fixation (US 5,229,114), hybrid seed production (US 5,689,041), and biofuel production (US 5,998,700). Preferred starch branching enzymes (for modification of starch properties) include those set forth in U.S. Nos. 6,232,122 and 6,147,279; and WO 97/22703.
- Modified oils production (US 6,444,876), high oil production (U.S. Nos. 5,608,149 and 6,476,295), or modified fatty acid content (US 6,537,750). Preferred fatty acid pathway enzymes include thioesterases (U.S. Nos. 5,512,482; 5,530,186; 5,945,585; 5,639,790; 5,807,893; 5,955,650; 5,955,329; 5,759,829; 5,147,792; 5,304,481; 5,298,421; 5,344,771; and 5,760,206), diacylglycerol acyltransferases (U.S. Patent Publications 20030115632A1 and 20030028923A1), and desaturases (U.S. Nos. 5,689,050; 5,663,068; 5,614,393; 5,856,157; 6,117,677; 6,043,411; 6,194,167; 5,705,391; 5,663,068; 5,552,306; 6,075,183; 6,051,754; 5,689,050; 5,789,220; 5,057,419; 5,654,402; 5,659,645; 6,100,091; 5,760,206; 6,172,106; 5,952,544; 5,866,789; 5,443,974; and 5,093,249).
- Preferred amino acid biosynthetic enzymes include anthranilate synthase (US 5,965,727 and WO 97/26366, WO 99/11800, WO 99/49058), tryptophan decarboxylase (WO 99/06581), threonine decarboxylase (U.S. Nos. 5,534,421 and 5,942,660; WO 95/19442), threonine deaminase (WO 99/02656 and WO 98/55601), dihydrodipicolinic acid synthase (US 5,258,300), and aspartate kinase (U.S. Nos. 5,367,110; 5,858,749; and 6,040,160).
- Production of nutraceuticals such as, for example, polyunsaturated fatty acids (for example arachidonic acid, eicosapentaenoic acid or docosahexaenoic acid) by expression of fatty acid elongases and/or desaturases, or production of proteins with improved nutritional value such as, for example, with a high content of essential amino acids (for example the high-methionine 2S albumin gene of the brazil nut). Preferred are nucleic acids which encode the Bertholletia excelsa high-methionine 2S albumin (GenBank Acc. No. AB044391), the Physcomitrella patents · 6-acyl-lipid desaturase (GenBank Acc. No.AJ222980; Girke *et al.* 1998), the Mortierella alpina · 6-desaturase (Sakuradani *et al.* 1999), the Caenorhabditis elvegans · 5-desaturase (Michaelson *et al.* 1998), the Caenorhabditis elegans · 5-fatty acid desaturase (des-5) (GenBank Acc. No.AF078796), the Mortierella alpine · 5-desaturase (Michaelson et al. JBC 273:19055-19059), the Caenorhabditis elegans · 6-elongase (Beaudoin *et al.* 2000), the Physcomitrella patents · 6-elongase (Zank *et al.* 2000), or functional equivalents of these.
- Production of high-quality proteins and enzymes for industrial purposes (for example enzymes, such as lipases) or as pharmaceuticals (such as, for example, antibodies, blood clotting factors, interferons, lymphokins, colony stimulation factor, plasminogen activators, hormones or vaccines, as described by Hood EE and Jilka JM 1999). For example, it has been possible to produce recombinant avidin from chicken albumen and bacterial · - glucuronidase (GUS) on a large scale in transgenic maize plants (Hood *et al.* 1999).
- Obtaining an increased storability in cells which normally comprise fewer storage proteins or storage lipids, with the purpose of increasing the yield of these substances, for example by expression of acetyl-CoA carboxylase. Preferred nucleic acids are those which encode the Medicago sativa acetyl-CoA carboxylase (ACCase) (GenBank Acc. No. L25042), or functional equivalents thereof. Alterenatively, in some scenarios an increased storage protein content might be advantageous for high-protein product production. Preferred seed storage proteins include zeins (U.S. Nos. 4,886,878; 4,885,357; 5,215,912; 5,589,616; 5,508,468; 5,939,599; 5,633,436; and 5,990,384; WO 90/01869, WO 91/13993, WO 92/14822, WO 93/08682, WO 94/20628, WO 97/28247, WO 98/26064, and WO 99/40209), 7S proteins (U.S. 5,003,045 and 5,576,203), brazil nut protein (US 5,850,024), phenylalanine free proteins (WO 96/17064), albumin (WO 97/35023), beta-conglycinin (WO 00/19839), 11S (US 6,107,051), alpha-hordothionin (U.S.

5,885,802 and 5,885,801), arcelin seed storage proteins (US 5,270,200), lectins (US 6,110,891), and glutenin (U.S. 5,990,389 and 5,914,450).

- Reducing levels of · -glucan L-type tuber phosphorylase (GLTP) or · -glucan H-type tuber phosphorylase (GHTP) enzyme activity preferably within the potato tuber (see US 5,998,701). The conversion of starches to sugars in potato tubers, particularly when stored at temperatures below 7°C., is reduced in tubers exhibiting reduced GLTP or GHTP enzyme activity. Reducing cold-sweetening in potatoes allows for potato storage at cooler temperatures, resulting in prolonged dormancy, reduced incidence of disease, and increased storage life. Reduction of GLTP or GHTP activity within the potato tuber may be accomplished by such techniques as suppression of gene expression using homologous antisense or double-stranded RNA, the use of co-suppression, regulatory silencing sequences. A potato plant having improved cold-storage characteristics, comprising a potato plant transformed with an expression cassette having a TPT promoter sequence operably linked to a DNA sequence comprising at least 20 nucleotides of a gene encoding an · -glucan phosphorylase selected from the group consisting of · · glucan L-type tuber phosphorylase (GLTP) and · -glucan H-type phosphorylase (GHTP).

Further examples of advantageous genes are mentioned for example in Dunwell JM, Transgenic approaches to crop improvement, J Exp Bot. 2000; 51 Spec No; pages 487-96. A discussion of exemplary heterologous DNAs useful for the modification of plant phenotypes may be found in, for example, U.S. 6,194,636;

Another aspect of the invention provides a DNA construct in which the promoter with starchyendosperm and/or germinating embryo-specific or -preferential expression drives a gene suppression DNA element, *e.g.* to suppress an amino acid catabolizing enzyme.

Seed maturation or grain development refers to the period starting with fertilization in which metabolizable food reserves (*e.g.*, proteins, lipids, starch, *etc.*) are deposited in the developing seed, particularly in storage organs of the seed, including the endosperm, testa, aleurone layer, embryo, and scutellar epithelium, resulting in enlargement and filling of the seed and ending with seed desiccation.

Embryo-specific promoters of this invention may be useful in minimizing yield drag and other potential adverse physiological effects on maize growth and development that might be encountered by high-level, non-inducible, constitutive expression of a transgenic protein or other molecule in a plant. When each transgene is fused to a promoter of the invention, the risk of DNA sequence homology dependent transgene inactivation (co-suppression) can be minimized.

It may be useful to target DNA itself within a cell. For example, it may be useful to target introduced DNA to the nucleus as this may increase the frequency of transformation. Within the nucleus itself it would be useful to target a gene in order to achieve site-specific integration. For example, it would be useful to have a gene introduced through transformation replace an existing gene in the cell. Other elements include those that can be regulated by endogenous or exogenous agents, *e.g.*, by zinc finger proteins, including naturally occurring zinc finger proteins or chimeric zinc finger proteins (see, *e.g.*, US 5,789,538, WO 99/48909; WO 99/45132; WO 98/53060; WO 98/53057; WO 98/53058; WO 00/23464; WO 95/19431; and WO 98/54311) or myb-like transcription factors. For example, a chimeric zinc finger protein may include amino acid sequences, which bind to a specific DNA sequence (the zinc finger) and amino acid sequences that activate (*e.g.*, GAL 4 sequences) or repress the transcription of the sequences linked to the specific DNA sequence.

General categories of genes of interest for the purposes of the present invention include, for example, those genes involved in information, such as Zinc fingers, those involved in communication, such as kinases, and those involved in housekeeping, such as heat shock proteins. More specific categories of transgenes include genes encoding important traits for agronomic quality, insect resistance, disease resistance, herbicide resistance, and grain characteristics. Still other categories of transgenes include genes for inducing expression of exogenous products such as enzymes, cofactors, and hormones from plants and other eukaryotes as well as from prokaryotic organisms. It is recognized that any gene of interest can be operably linked to the promoter of the invention and expressed under stress.

In a more preferred embodiment, the promoter of the instant invention modulates genes encoding proteins which act as cell cycle regulators, or which control carbohydrate metabolism or phytohormone levels, as has been shown in tobacco and canola with other tissue-preferred promoters. (Ma, Q. H. *et al.,* 1998; Roeckel, P. *et al.,* 1997) For example, genes encoding isopentenyl transferase or IAA-M may be useful in modulating development of the female florets. Other important genes encode growth factors and transcription factors. Expression of selected endogenous or heterologous nucleotides under the direction of the promoter may result in continued or improved development of the female florets under adverse conditions.

Seed production may be improved by altering expression of genes that affect the response of seed growth and development during environmental stress (Cheikh-N *et al.* 1994) and genes controlling carbohydrate metabolism to reduce seed abortion in maize (Zinselmeier *et al.* 1995).

**2.3 Targeted Sequence Excision**

The specificity of the chimeric transcription regulating nucleic acid sequences of the invention in monocotyledonous plants makes it especially useful for targeted excision or deletion of sequences (such as marker sequences) from the genome of said monocotyledonous plant. It is one known disadvantage of the methods known in the prior art that excision is not homogenous through the entire plants thereby leading to mosaic-like excision patterns, which require laborious additional rounds of selection and regeneration. The specificity of the promoters of the invention in the early embryo allows for homogenous excision throughout the entire embryo, which will then provide a plant homogenous target-sequence (*e.g.*, marker) free plant.

Another embodiment of the invention relates to a method for excision of target sequences (*e.g.*, marker sequences) from a plant, preferably a monocotyledonous plant, said method comprising the steps of

A) constructing an expression cassette by operably linking polynucleotide encoding a chimeric transcription regulating nucleotide sequence comprising

i) a first nucleic acid molecule selected from the group consisting of

a) a polynucleotide as defined in SEQ ID NO:1;
b) a polynucleotide having at least 60%, 70% or 80%, more preferably at least 85% or 90%, most preferably at least 95%, 98% or 99% sequence identity to the polynucleotide of SEQ I D NO:1;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1 % SDS at 50°C, more preferably in 7% sodium dodecyl sulfate (SDS), 0.5 M $NaPO_4$ 1 mM EDTA at 50°C with washing in 0.1 X SSC, 0.1 % SDS at 50° C, and most preferably in 7% sodium dodecyl sulfate (SDS), 0.5 M $NaPO_4$ 1 mM EDTA at 50°C with washing in 0.1 X SSC, 0.1% SDS at 65°C to a nucleic acid comprising at least 50 nucleotides of a polynucleotide as defined in SEQ ID NO:1, or the complement thereof, and operably linked to

ii) a second nucleic acid molecule selected from the group consisting of

a) a polynucleotide as defined in SEQ ID NO:3;
b) a polynucleotide having at least 60%, 70% or 80%, more preferably at least 85% or 90%, most preferably at least 95%, 98% or 99% sequence identity to the polynucleotide of SEQ ID NO:3;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$ 1 mM EDTA at 50°C with washing in 0.5 X SSC, 0. 1 % SDS at 50°C, more preferably in 7% sodium dodecyl sulfate (SDS), 0.5 M $NaPO_4$, 1 mM EDTA at 50°C with washing in 0.1 X SSC, 0.1 % SDS at 50° C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a sequence described by SEQ ID NO:3, or the complement thereof,

to at least one nucleic acid sequence which is heterologous in relation to said chimeric transcription regulating nucleotide sequence and is suitable to induce excision of a target sequences from a monocotyledonous plant, and

B) inserting said expression cassette into a monocotyledonous plant comprising at least one target sequence to provide a transgenic plant, wherein said plant expresses said heterologous nucleic acid sequence, and
C) selecting transgenic plants, which demonstrate excision of said marker.

The excision is realized by various means, including but not limited to:

- induction of sequence deletion by side specific recombination using site-specific recombinases, wherein said site-specific recombinase is expressed by the chimeric transcription regulating nucleotide sequence of the invention,
- induction of sequence deletion by induced homologous recombination, wherein the sequences to be deleted are flanked by sequences, said sequences having an orientation, a sufficient length and a homology to each other to allow for homologous recombination between them, wherein homologous recombination is induced by a site-specific double-strand break made by a site-specific endonuclease (preferably a homing endonuclease, more preferably the homing endonuclease I-SceI), wherein said site-specific endonuclease is expressed by the chimeric transcription regulating nucleotide sequence of the invention.

Another embodiment of the invention relates to a plant, preferably a monocotyledonous plant or plant cell comprising

A) at least one target sequence, which is stably inserted into the plant genome, wherein said target sequence is flanked by excision-sequences which are capable to mediate upon interaction with a sequence specific excision-mediating enzyme excision of said target sequence from the plant genome, and
B) an expression cassette comprising at least one nucleic acid sequence encoding an excision-mediating enzyme, which is capable to interact with said excision-sequences of i), operably linked to a chimeric transcription regulating nucleotide sequence comprising

i) a first nucleic acid molecule selected from the group consisting of

a) a polynucleotide as defined in SEQ ID NO:1;
b) a polynucleotide having at least 70% or 80%, more preferably at least 85% or 90%, most preferably at least 95%, 98% or 99% sequence identity to the polynucleotide of SEQ ID NO:1; a
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$ 1 mM EDTA at 50°C with washing in 0.1 X SSC, 0.1% SDS at 65°C to a nucleic acid comprising at least 50 nucleotides of a polynucleotide as defined in SEQ ID NO:1, or the complement thereof, and operably linked to

ii) a second nucleic acid molecule selected from the group consisting of

a) a polynucleotide as defined in SEQ ID NO:3;
b) a polynucleotide having at least 60%, 70% or 80%, more preferably at least 85% or 90%, most preferably at least 95%, 98% or 99% sequence identity to the polynucleotide of SEQ ID NO:3;
c) a fragment of at least 200 consecutive bases of the polynucleotide of SEQ ID NO:1; and
d) a polynucleotide hybridizing under conditions equivalent to hybridization in 7% sodium dodecyl sulfate (SDS), 0.5M $NaPO_4$, 1 mM EDTA at 50°C with washing in 0.5 X SSC, 0. 1% SDS at 50°C, more preferably in 7% sodium dodecyl sulfate (SDS), 0.5 M $NaPO_4$, 1 mM EDTA at 50°C with washing in 0.1 X SSC, 0.1 % SDS at 50°C, and most preferably in 7% sodium dodecyl sulfate (SDS), 0.5 M $NaPO_4$, 1 mM EDTA at 50°C with washing in 0.1 X SSC, 0.1 % SDS at 65°C to a nucleic acid comprising at least 50 nucleotides of a sequence described by SEQ ID NO:3, or the complement thereof.

Preferred nucleic acid molecules comprising a polynucleotide encoding a chimeric transcription regulating nucleotide sequence are described above. Preferred heterologous nucleic acid sequence to be expressed to achieve sequence excision (*e.g.*, encoding for a site-specific recombinase or endonuclease) are described herein below.
The monocotyledonous plant to which the methods of this invention are preferrably applied to may be selected from the group consisting of maize, wheat, rice, barley, rye, millet, sorghum, ryegrass or coix, triticale or oats and sugar cane. Preferably the plant is a cereal plant selected from the group consisting of maize, wheat, barley, rice, oat, rye, and sorghum, even more preferably from maize, wheat, and rice, most preferably the plant is a maize plant.
The chimeric transcription regulating nucleotide sequence is preferably defined as above. The target sequence in the above defined monocotyledonous pant or plant cell will be excised as soon as seeds of said plant are germinated and the embryo starts to grow. From this embryo a target-sequence free plant will result.
The target-sequence and the expression cassette for the excision-mediating enzyme may be combined on one DNA or on different construct. The different DNA constructs may be combined by other means in the genome of the monocotyledonous plant of plant cell such as - for example - crossing of distinct parental lines comprising said target sequence and said expression cassette for the excision-mediating enzyme, respectively, or co-transformation or subsequent transformation.
Accordingly, another embodiment of the invention relates to a method for excising at least one target sequence from the genome of a monocotyledonous plant or plant cell comprising the steps of

A) stably inserting into the genome a nucleic acid construct at least one target sequence, which is stably inserted into the plant genome, wherein said target sequence is flanked by excision-sequences, which are capable to mediate upon interaction with a sequence specific excision-mediating enzyme excision of said target sequence from the plant genome, and

B) introducing into said monocotyledonous plants or plant cells an expression cassette comprising at least one nucleic acid sequence encoding an excision-mediating enzyme, which is capable to interact with said excision-sequences of i), operably linked to polynucleotide encoding a chimeric transcription regulating nucleotide sequence comprising

i) a first nucleic acid molecule selected from the group consisting of

a) a polynucleotide as defined in SEQ ID NO:1;
b) a polynucleotide having at least 70% sequence identity to the polynucleotide of SEQ ID NO:1; and
c) a polynucleotide hybridizing under conditions equivalent to hybridization in 7% sodium dodecyl sulfate (SDS), 0.5M NaPOa, 1mM EDTA at 50°C with washing in 0.5xSSC, 0.1 % SDS at 50°C to a nucleic acid comprising at least 50 nucleotides of a polynucleotide as defined in SEQ ID NO:1, or the complement thereof, and operably linked to

ii) a second nucleic acid molecule selected from the group consisting of

a) a polynucleotide as defined in SEQ ID NO:3;
b) a polynucleotide having at least 60% sequence identity to the polynucleotide of SEQ ID NO:3; and
c) a polynucleotide hybridizing under conditions equivalent to hybridization in 7% sodium dodecyl sulfate (SDS), 0.5M $NaPO_4$, 1mM EDTA at 50°C with washing in 0.5xSSC, 0.1 % SDS at 50°C to a nucleic acid comprising at least 50 nucleotides of a sequence described by SEQ ID NO:3, or the complement thereof,

C) generating seeds of said monocotyledonous plant or plant cells comprising both said target sequence and said expression cassette, germinating said seeds and growing plants therefrom, and
D) selecting plants from which said target sequence has be excised.

In a preferred embodiment the method of the invention further comprises the step of regeneration of a fertile plant. The method may further include sexually or asexually propagating or growing off-spring or a descendant of the plant regenerated from said plant cell.

Preferably, excision (or deletion) of the target sequence can be realized by various means known as such in the art, including but not limited to one or more of the following methods:

a) recombination induced by a sequence specific recombinase, wherein said target sequence is flanked by corresponding recombination sites in a way that recombination between said flanking recombination sites results in deletion of the target-sequences in-between from the genome,
b) homologous recombination between homology sequences A and A' flanking said target sequence, preferably induced by a sequence-specific double-strand break between said homology sequences caused by a sequence specific endonuclease, wherein said homology sequences A and A' have sufficient length and homology in order to ensure homologous recombination between A and A', and having an orientation which - upon recombination between A and A' - will lead to excision of said target sequence from the genome of said plant.

Preferred excision sequences and excision enzymes are specified below. In another preferred embodiment the mechanism of deletion/excision can be induced or activated in a way to prevent pre-mature deletion/excision of the dual-function marker. Preferably, thus expression and/or activity of an preferably employed sequence-specific recombinase or endonuclease can be induced and/or activated, preferably by a method selected from the group consisting of

a) inducible expression by operably linking the sequence encoding said excision enzyme (*e.g.*, recombinase or endonuclease) to the chimeric transcription regulating sequence combined with an inducible promoter or promoter element,
b) inducible activation, by employing an inducible, modified excision enzyme (*e.g.*, a recombinase or endonuclease) for example comprising a ligand-binding-domain, wherein activity of said modified excision enzyme can be modified by treatment of a compound having binding activity to said ligand-binding-domain.

Preferably, the target sequence is a marker, more preferably a selection marker (preferred marker sequences are specified below). Thus the method of the inventions results in a monocotyledonous plant cell or plant, which is selection marker-free.

### 2.3.1 Preferred excision sequences and excision enzymes

For ensuring target sequence deletion / excision the target sequence is flanked by excision sequences, which are capable to mediate upon interaction with a sequence specific excision-mediating enzyme excision of said target sequence from the plant genome. Preferably, deletion of the target sequence can be realized by various means known in the art, including but not limited to one or more of the following methods:

a) recombination induced by a sequence specific recombinase, wherein said target sequence is flanked by corresponding recombination sites in a way that recombination between said flanking recombination sites results in deletion of the target sequence in-between from the genome,
b) homologous recombination between homology sequences A and A' flanking said target sequence, preferably induced by a sequence-specific double-strand break between said homology sequences caused by a sequence specific endonuclease, wherein said homology sequences A and A' have sufficient length and homology in order to ensure homologous recombination between A and A', and having an orientation which - upon recombination between A and A' - will lead to excision of said target sequence from the genome of said plant.

Accordingly, for ensuring target sequence deletion / excision the target sequence is flanked by sequences which allow for specific deletion of said expression cassette. Said sequences may be recombination sites for a sequence specific recombinase, which are placed in a way the recombination induced between said flanking recombination sites results in deletion of the said target sequence from the genome. There are various recombination sites and corresponding sequence specific recombinases known in the art (described herein below), which can be employed for the purpose of the invention.
In another preferred embodiment, deletion / excision of the target sequence is performed by intramolecular (preferably intrachromosomal) homologous recombination. Homologous recombination may occur spontaneous but is preferably induced by a sequence-specific double-strand break (*e.g.*, between the homology sequences). The basic principals are disclosed in WO 03/004659. For this purpose the target sequence is flanked by homology sequences A and A', wherein said homology sequences have sufficient length and homology in order to ensure homologous recombination between A and A', and having an orientation which - upon recombination between A and A' - will lead to an excision said target sequence from the genome. Furthermore, the sequence flanked by said homology sequences further comprises at least one recognition sequence of at least 10 base pairs for the site-directed induction of DNA double-strand breaks by a sequence specific DNA double-strand break inducing enzyme, preferably a sequence-specific DNA-endonuclease, more preferably a homing-endonuclease, most preferably a endonuclease selected from the group consisting of I-SceI, I-CpaI, I-CpaII, I-CreI and I-ChuI or chimeras thereof with ligand-binding domains. Suitable endonucleases are described herein below.

#### 2.3.1.1 Recombination Sites and Recombinases

Sequence-specific recombinases and their corresponding recombination sites suitable within the present invention may include but are not limited to the Cre/lox system of the bacteriophage P1 (Dale EC and Ow DW 1991; Russell SH *et al.* 1992; Osborne BI *et al.* 1995), the yeast FLP/FRT system (Kilby NJ *et al.* 1995; Lyznik LA *et al.* 1996), the Mu phage Gin recombinase, the E. coli Pin recombinase or the R/RS system of the plasmid pSR1 (Onouchi H *et al.* 1995; Sugita Ket *et al.* 2000). The recombinase (for example Cre or FLP) interacts specifically with its corresponding recombination sequences (34 bp lox sequence and 47 bp FRT sequence, respectively) in order to delete or invert the interposed sequences. Deletion of standard selection marker in plants which was flanked by two lox sequences by the Cre is described (Dale EC and Ow DW 1991). The preferred recombination sites for suitable recombinases are described in Table 1 below:

Table 1. Suitable sequence-specific recombinases

| Recombinase | Organism of origin | Recombination Sites |
|---|---|---|
| CRE | Bacteriophage P1 | 5'-AACTCTCATCGCTTCGGATAACTTCCTGT-TATCCGAAA CATATCACTCACTTTGG-TGATTTCACCGTAACTGTCTATGATTAATG-3' |

(continued)

| Recombinase | Organism of origin | Recombination Sites |
|---|---|---|
| FLP | Saccharomyces cerevisiae | 5'-GAAGTTCCTATTCCGAAGTTCCTATTCTCTAGAA AGTATAGGAACTTC-3' |
| R | pSR1 Plasmids | 5'-CGAGATCATATCACTGTGGACGTTGATGAAAGAATAC GTTATTCTTTCATCAAATCGT |

### 2.3.1.2 The Homology Sequences

Referring to the homology sequences (*e.g.*, A, A') "sufficient length" preferably refers to sequences with a length of at least 20 base pairs, preferably at least 50 base pairs, especially preferably at least 100 base pairs, very especially preferably at least 250 base pairs, most preferably at least 500 base pairs.

Referring to the homology sequences (*e.g.*, A, A'), "sufficient homology" preferably refers to sequences with at least 70%, preferably 80%, by preference at least 90%, especially preferably at least 95%, very especially preferably at least 99%, most preferably 100%, homology within these homology sequences over a length of at least 20 base pairs, preferably at least 50 base pairs, especially preferably at least 100 base pairs, very especially preferably at least 250 base pairs, most preferably at least 500 base pairs.

The homology sequences A and A' are preferably organized in the form of a direct repeat. The term "direct repeat" means a subsequent localization of two sequences on the same strand of a DNA molecule in the same orientation, wherein these two sequences fulfill the above given requirements for homologous recombination between said two sequences.

In a preferred embodiment, the homology sequences may be a duplication of a sequence having additional use within the DNA construct. For example, the homology sequences may be two transcription terminator sequences. One of these terminator sequences may be operably linked to the agronomically valuable or relevant trait, while the other may be linked to the dual-function selection marker, which is localized in 3'-direction of the trait gene. Recombination between the two terminator sequences will excise the target sequence (*e.g.*, a marker gene) but will reconstitute the terminator of the trait gene. In another example, the homology sequences may be two promoter sequences. One of these promoter sequences may be operably linked to the agronomically valuable or relevant trait, while the other may be linked to the target sequence (*e.g.,* a selection marker), which is localized in 5'-direction of the trait gene. Recombination between the two promoter sequences will excise the target sequence (*e.g.*, a marker gene) but will reconstitute the promoter of the trait gene. The person skilled in the art will know that the homology sequences do not need to be restricted to a single functional element (*e.g.* promoter or terminator), but may comprise or extent to other sequences (*e.g.* being part of the coding region of the trait gene and the respective terminator sequence of said trait gene.

### 2.3.1.3. Double-Strand Break Inducing Enzyme

Preferably, deletion / excision of the target sequence (*e.g.*, a marker gene) is realized by homologous recombination between the above specified homology sequences induced by a sequence-specific double-strand break, preferably between the homology sequences which should recombine. General methods are disclosed for example in WO 03/004659. Various enzyme suitable for induction of sequence-specific double-strand breaks (hereinafter together "endonuclease") are known in the art. The endonuclease may be for example selected from the group comprising:

1. Restriction endonucleases (type II), preferably homing endonucleases as described in detail hereinbelow.
2. Transposases, for example the P-element transposase (Kaufman PD and Rio DC 1992) or AcDs (Xiao YL and Peterson T 2000). In principle, all transposases or integrases are suitable as long as they have sequence specificity (Haren L *et al.* 1999; Beall EL, Rio DC 1997).
3. Chimeric nucleases as described in detail hereinbelow.
4. Enzymes which induce double-strand breaks in the immune system, such as the RAG1/RAG2 system (Agrawal A *et al.* 1998).
5. Group II intron endonucleases. Modifications of the intron sequence allows group II introns to be directed to virtually any sequence in a double-stranded DNA, where group II introns can subsequently insert by means of a reverse splice mechanism (Mohr *et al.* 2000; Guo *et al.* 2000). During this reverse splice mechanism, a double-strand break is introduced into the target DNA, the excised intron RNA cleaving the sense strand while the protein portion of the group II intron endonuclease hydrolyses the antisense strand (Guo *et al.* 1997). If it is only desired to induce the double-strand break without achieving complete reverse splicing, as is the case in the present invention, it is possible to resort to, for example, group II intron endonucleases which lack the reverse transcriptase activity. While this does not prevent the generation of the double-strand break, the reverse splicing mechanism cannot proceed to completion.

Suitable enzymes are not only natural enzymes, but also synthetic enzymes. Preferred enzymes are all those endonucleases whose recognition sequence is known and which can either be obtained in the form of their proteins (for example by purification) or expressed using their nucleic acid sequence.

In a preferred embodiment a sequence-specific endonuclease is employed for specific induction of double-strand breaks and subsequent induced homologous recombination. The term "sequence specific DNA-endonuclease" generally refers to all those enzymes, which are capable of generating double-strand breaks in double stranded DNA in a sequence-specific manner at one or more recognition sequences. Said DNA cleavage may result in blunt ends, or so-called "sticky" ends of the DNA (having a 5'- or 3'-overhang). The cleavage site may be localized within or outside the recognition sequence. Various kinds of endonucleases can be employed. Endonucleases can be, for example, of the Class II or Class IIs type. Class IIs R-M restriction endonucleases catalyze the DNA cleavage at sequences other than the recognition sequence, i.e. they cleave at a DNA sequence at a particular number of nucleotides away from the recognition sequence (Szybalski *et al.* 1991). The following may be mentioned by way of example, but not by limitation:

1. Restriction endonucleases (*e.g.*, type II or IIs), preferably homing endonucleases as described in detail hereinbelow.
2. Chimeric or synthetic nucleases as described in detail hereinbelow.

Unlike recombinases, restriction enzymes typically do not ligate DNA, but only cleave DNA. Restriction enzymes are described, for instance, in the New England Biolabs online catalog (www.neb.com), Promega online catalog (www.promega.com) and Rao *et al.* (2000). Within this invention "ligation" of the DNA ends resulting from the cleavage by the endonuclease is realized by fusion by homologous recombination of the homology sequences.

Preferably, the endonuclease is chosen in a way that its corresponding recognition sequences are rarely, if ever, found in the unmodified genome of the target plant organism. Ideally, the only copy (or copies) of the recognition sequence in the genome is (or are) the one(s) introduced by the DNA construct of the invention, thereby eliminating the chance that other DNA in the genome is excised or rearranged when the sequence-specific endonuclease is expressed.

One criterion for selecting a suitable endonuclease is the length of its corresponding recognition sequence. Said recognition sequence has an appropriate length to allow for rare cleavage, more preferably cleavage only at the recognition sequence(s) comprised in the DNA construct of the invention. One factor determining the minimum length of said recognition sequence is - from a statistical point of view - the size of the genome of the host organism. In a preferred embodiment the recognition sequence has a length of at least 10 base pairs, preferably at least 14 base pairs, more preferably at least 16 base pairs, especially preferably at least 18 base pairs, most preferably at least 20 base pairs.

A restriction enzyme that cleaves a 10 base pair recognition sequence is described in Huang B *et al.* 1996.

Suitable enzymes are not only natural enzymes, but also synthetic enzymes. Preferred enzymes are all those sequence specific DNA-endonucleases whose recognition sequence is known and which can either be obtained in the form of their proteins (for example by purification) or expressed using their nucleic acid sequence.

Especially preferred are restriction endonucleases (restriction enzymes) which have no or only a few recognition sequences - besides the recognition sequences present in the transgenic recombination construct - in the chromosomal DNA sequence of a particular eukaryotic organism. This avoids further double-strand breaks at undesired loci in the genome. This is why homing endonucleases are especially preferred (Review: (Belfort M and Roberts RJ 1997; Jasin M 1996; Internet: http://rebase.neb.com/rebase/rebase.homing.html). Owing to their long recognition sequences, they have no, or only a few, further recognition sequences in the chromosomal DNA of eukaryotic organisms in most cases.

The sequences encoding for such homing endonucleases can be isolated for example from the chloroplast genome of Chlamydomonas (Turmel M *et al.* 1993). They are small (18 to 26 kD) and their open reading frames (ORF) have a "codon usage" which is suitable directly for nuclear expression in eukaryotes (Monnat RJ Jr *et al.* 1999). Homing endonucleases which are very especially preferably isolated are the homing endonucleases I-SceI (WO96/14408), I-SceII (Sarguiel B *et al.* 1990), I-SceIII (Sarguiel B *et al.* 1991), I-CeuI (Marshall 1991), I-CreI (Wang J *et al.* 1997), I-ChuI (Cote V *et al.* 1993), I-TevI (Chu *et al.* 1990; Bell-Pedersen *et al.* 1990), I-TevII (Bell-Pedersen *et al.* 1990), I-TevIII (Eddy *et al.* 1991), Endo SceI (Kawasaki *et al.* 1991), I-CpaI (Turmel M *et al.* 1995a) and I-CpaII (Turmel M *et al.* 1995b).

Further homing endonucleases are detailed in the abovementioned Internet website, and examples which may be mentioned are homing endonucleases such as F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-

CvuAIP, I-DdiI, I-DdiII, I-DirI, I-DmoI, I-HmuI, I-HmuII, I-HspNIP, I-LlaI, I-MsoI, I-NaaI, I-NanI, I-NcIIP, I-NgrIP, I-NitI, I-NjaI, I-Nsp2361P, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII , I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI, PI-TliII, H-DreI, I-BasI, I-BmoI, I-PogI, I-TwoI, PI-MgaI, PI-PabI, PI-PabII.

Preferred in this context are the homing endonucleases whose gene sequences are already known, such as, for example, F-SceI, I-CeuI, I-ChuI, I-DmoI, I-CpaI, I-CpaII, I-CreI, I-CsmI, F-TevI, F-TevII, I-TevI, I-TevII, I-AniI, I-CvuI, I-DdiI, I-HmuI, I-HmuII, I-LlaI, I-NanI, I-MsoI, I-NitI, I-NjaI, I-PakI, I-PorI, I-PpoI, I-ScaI, I-Ssp6803I, PI-PkoI, PI-PkoII, PI-PspI, PI-TfuI, PI-TliI. Especially preferred are commercially available homing endonucleases such as I-CeuI, I-SceI, I-DmoI, I-PpoI, PI-PspI or PI-SceI. Endonucleases with particularly long recognition sequences, and which therefore only rarely (if ever) cleave within a genome include: I-CeuI (26 bp recognition sequence), PI-PspI (30 bp recognition sequence), PI-SceI (39 bp recognition sequence), I-SceI (18 bp recognition sequence) and I-PpoI (15 bp recognition sequence). The enzymes can be isolated from their organisms of origin in the manner with which the skilled worker is familiar, and/or their coding nucleic acid sequence can be cloned. The sequences of various enzymes are deposited in GenBank. Especially preferred are the homing endonucleases I-SceI, I-CpaI, I-CpaII, I-CreI and I-ChuI. Sequences encoding said nucleases are known in the art and - for example - specified in WO 03/004659 (*e.g.*, as SEQ ID NO: 2, 4, 6, 8, and 10 of WP 03/004659).

The heterologous nucleic acid sequence to be expressed may preferably encode a polypeptide as described by any of SEQ ID NO: 22, or a functional equivalent thereof, which is capable to bring about the same phenotype than any of said polypeptide. Most preferably the nucleic acid sequence to be expressed is described by SEQ ID NO 21 or 23.

In a preferred embodiment, the sequences encoding said homing endonucleases can be modified by insertion of an intron sequence. This prevents expression of a functional enzyme in procaryotic host organisms and thereby facilitates cloning and transformation procedures (*e.g.*, based on *E.coli* or *Agrobacterium*). In plant organisms, expression of a functional enzyme is realized, since plants are able to recognize and "splice" out introns. Preferably, introns are inserted in the homing endonucleases mentioned as preferred above (*e.g.*, into I-SceI or I-CreI). In some aspects of the invention, molecular evolution can be employed to create an improved endonuclease. Polynucleotides encoding a candidate endonuclease enzyme can, for example, be modulated with DNA shuffling protocols. DNA shuffling is a process of recursive recombination and mutation, performed by random fragmentation of a pool of related genes, followed by reassembly of the fragments by a polymerase chain reaction-like process. See, *e.g.*, Stemmer (1994); Stemmer (1994); and US 5,605,793, US 5,837,458, US 5,830,721 and US 5, 811,238.

Other synthetic endonucleases which may be mentioned by way of example are chimeric nucleases which are composed of an unspecific nuclease domain and a sequence-specific DNA binding domain consisting of zinc fingers (Bibikova M *et al.* 2001). These DNA-binding zinc finger domaines can be adapted to suit any DNA sequence. Suitable methods for preparing suitable zinc finger domaines are described and known to the skilled worker (Beerli RR *et al.* 2000; Beerli RR *et al.* 2000; Segal DJ and Barbas CF 2000; Kang JS and Kim JS 2000; Beerli RR *et al.* 1998; Kim JS *et al.* 1997; Klug A 1999; Tsai SY *et al.* 1998; Mapp AK *et al.* 2000; Sharrocks AD *et al.* 1997; Zhang L *et al.* 2000).

The endonuclease is preferably expressed as a fusion protein with a nuclear localization sequence (NLS). This NLS sequence enables facilitated transport into the nucleus and increases the efficacy of the recombination system. A variety of NLS sequences are known to the skilled worker and described, inter alia, by Jicks GR and Raikhel NV 1995. Preferred for plant organisms is, for example, the NLS sequence of the SV40 large antigen. Examples are provided in WO 03/060133. However, owing to the small size of many DSBI enzymes (such as, for example, the homing endonucleases), an NLS sequence is not necessarily required. These enzymes are capable of passing through the nuclear pores even without any aid.

In a further preferred embodiment, the activity of the endonuclease can be induced. Suitable methods have been described for sequence-specific recombinases (Angrand PO *et al.* 1998; Logie C and Stewart AF 1995; Imai T *et al.* 2001). These methods employ fusion proteins of the endonuclease and the ligand binding domain for steroid hormone receptor (for example the human androgen receptor, or mutated variants of the human estrogen receptor as described therein). Induction may be effected with ligands such as, for example, estradiol, dexamethasone, 4-hydroxytamoxifen or raloxifen. Some endonucleases are active as dimers (homo- or heterodimers; I-CreI forms a homodimer; I-SecIV forms a heterodimerk) (Wernette CM 1998). Dimerization can be designed as an inducible feature, for example by exchanging the natural dimerization domains for the binding domain of a low-molecular-weight ligand. Addition of a dimeric ligand then brings about dimerization of the fusion protein. Corresponding inducible dimerization methods, and the preparation of the dimeric ligands, have been described (Amara JF *et al.* 1997; Muthuswamy SK *et al.* 1999; Schultz LW and Clardy J 1998; Keenan T *et al.* 1998).

Recognition sequences for sequence specific DNA endonuclease (*e.g.*, homing endonucleases) are described in the art. "Recognition sequence" refers to a DNA sequence that is recognized by a sequence-specific DNA endonuclease of the invention. The recognition sequence will typically be at least 10 base pairs long, is more usually 10 to 30 base pairs long, and in most embodiments, is less than 50 base pairs long.

"Recognition sequence" generally refers to those sequences which, under the conditions in a plant cell used within this invention, enable the recognition and cleavage by the sequence specific DNA-endonuclease. The recognition sequences for the respective sequence specific DNA-endonucleases are mentioned in Table 2 hereinbelow by way of example, but not by limitation.

Table 2. Recognition sequences and organisms of origin for endonucleases (*e.g.*, homing endonucleases; "^" indicates the cleavage site of the sequence specific DNA-endonuclease within a recognition sequence).

| DSBI Enzyme | Organism of origin | Recognition sequence |
|---|---|---|
| P-Element TransPosase | Drosophila | 5'-CTAGATGAAATAACATAAGGTGG-3' |
| I-AniI | Aspergillus nidulans | 5'-<br>TTGAG-<br>GAGGTT^TCTCTGTAAATAANNNNNNNNNNNNNN<br>3'-<br>AACTCCTCCAAAGAGA-<br>CATTTATTNNNNNNNNNNNNNNN^ |
| I-DdiI | Dictyostelium discoideumAX3 | 5'-TTTTTTGGTCATCCAGAAGTATAT<br>3'-AAAAAACCAGATAGGTCTTCATATA |
| I-CvuI | Chlorella vulgaris | 5'-CTGGGTTCAAAACGTCGTGA^GACAGTTTGG<br>3'-GACCCAAGTTTTGCAG^CACTCTGTCAAACC |
| I-CsmI | Chlamydomonas smithii | 5'-GTACTAGCATGGGGTCAAATGTCTTTCTGG |
| I-CmoeI | Chlamydomonasmoewusii | 5'-TCGTAGCAGCT^CACGGTT<br>3'-AGCATCG^TCGAGTGCCAA |
| I-CreI | Chlamydomonas reinhardtii | 5'-CTGGGTTCAAAACGTCGTGA^GACAGTTTGG<br>3'-GACCCAAGTTTTGCAG^CACTCTGTCAAACC |
| I-ChuI | Chlamydomonas humicola | 5'-GAAGGTTTGGCACCTCG^ATGTCGGCTCATC<br>3'-CTTCCAAACCGTG^GAGCTACAGCCGAGTAG |
| I-CpaI | Chlamydomonas pallidostigmatica | 5'-CGATCCTAAGGTAGCGAA^ATTCA<br>3'-GCTAGGATTCCATC^GCTTTAAGT |
| I-CpaII | Chlamydomonas pallidostigmatica | 5'-CCCGGCTAACTC^TGTGCCAG<br>3'-GGGCCGAT^TGAGACACGGTC |
| I-CeuI | Chlamydomonas eugametos | 5'-CGTAACTATAACGGTCCTAA^GGTAGCGAA<br>3'-GCATTGATATTGCCAG^GATTCCATCGCTT |
| I-DmoI | Desulfurococcus mobilis | 5'-ATGCCTTGCCGGGTAA^GTTCCGGCGCGCAT<br>3'-TACGGAACGGCC^CATTCAAGGCCGCGCGTA |
| I-SceI | Saccharomyces cerevisiae | 5'-AGTTACGCTAGGGATAA^CAGGGTAATATAG<br>3'-TCAATGCGATCCC^TATTGTCCCATTATATC<br>5'-TAGGGATAA^CAGGGTAAT<br>3'-ATCCC^TATTGTCCCATTA ("Core"-Sequence) |
| I-SceII | S.cerevisiae | 5'-TTTTGATTCTTTGGTCACCC^TGAAGTATA<br>3'-AAAACTAAGAAACCAG^TGGGACTTCATAT |
| I-SceIII | S.cerevisiae | 5'-ATTGGAGGTTTTGGTAAC^TATTTATTACC<br>3'-TAACCTCCAAAACC^ATTGATAAATAATGG |
| I-SceIV | S.cerevisiae | 5'-TCTTTTCTCTTGATTA^GCCCTAATCTACG |

(continued)

| DSBI Enzyme | Organism of origin | Recognition sequence |
|---|---|---|
| | | 3'-AGAAAAGAGAAC^TAATCGGGATTAGATGC |
| I-SceV | S.cerevisiae | 5'-AATAATTTTCT^TCTTAGTAATGCC<br>3'-TTATTAAAAGAAGAATCATTA^CGG |
| I-SceVI | S.cerevisiae | 5'-GTTATTTAATG^TTTTAGTAGTTGG<br>3'-CAATAAATTACAAAATCATCA^ACC |
| I-SceVII | S.cerevisiae | 5'-TGTCACATTGAGGTGCACTAGTTATTAC |
| PI-SceI | S.cerevisiae | 5'-ATCTATGTCGGGTGC^GGAGAAAGAGGTAAT<br>3'-TAGATACAGCC^CACGCCTCTTTCTCCATTA |
| F-SceI | S.cerevisiae | 5'-GATGCTGTAGGC^ATAGGCTTGGTT<br>3'-CTACGACA^TCCGTATCCGAACCAA |
| F-SceII | S.cerevisiae | 5'-CTTTCCGCAACA^GTAAAATT<br>3'-GAAAGGCG^TTGTCATTTTAA |
| I-HmuI | Bacillus subtilis bacteriophage SPO1 | 5'-AGTAATGAGCCTAACGCTCAGCAA<br>3'-TCATTACTCGGATTGC^GAGTCGTT |
| I-HmuII | Bacillus subtilis bacteriophage SP82 | 5'-<br>AGTAATGAGCCTAACGCT-<br>CAACAANNNNNNNNNNNNNNNN-<br>NNNNNNNNNNNNNNNNNNNNNNN |
| I-LlaI | Lactococcus lactis | 5'-CACATCCATAAC^CATATCATTTTT<br>3'-GTGTAGGTATTGGTATAGTAA^AAA |
| I-MsoI | Monomastix species | 5'-CTGGGTTCAAAACGTCGTGA^GACAGTTTGG<br>3'-GACCCAAGTTTTGCAG^CACTCTGTCAAACC |
| I-NanI | Naegleria andersoni | 5'-AAGTCTGGTGCCA^GCACCCGC<br>3'-TTCAGACC^ACGGTCGTGGGCG |
| I-NitI | Naegleria italica | 5'-AAGTCTGGTGCCA^GCACCCGC<br>3'-TTCAGACC^ACGGTCGTGGGCG |
| I-NjaI | Naegleria jamiesoni | 5'-AAGTCTGGTGCCA^GCACCCGC<br>3'-TTCAGACC^ACGGTCGTGGGCG |
| I-PakI | Pseudendoclonium akinetum | 5'-CTGGGTTCAAAACGTCGTGA^GACAGTTTGG<br>3'-GACCCAAGTTTTGCAG^CACTCTGTCAAACC |
| I-PorI | Pyrobaculum organotrophum | 5'-GCGAGCCCGTAAGGGT^GTGTACGGG<br>3'-CGCTCGGGCATT^CCCACACATGCCC |
| I-PpoI | Physarum polycephalum | 5'-TAACTATGACTCTCTTAA^GGTAGCCAAAT<br>3'-ATTGATACTGAGAG^AATTCCATCGGTTTA |
| I-ScaI | Saccharomyces capensis | 5'-TGTCACATTGAGGTGCACT^AGTTATTAC<br>3'-ACAGTGTAACTCCAC^GTGATCAATAATG |
| I-Ssp6803I | Synechocystis species | 5'-GTCGGGCT^CATAACCCGAA<br>3'-CAGCCCGAGTA^TTGGGCTT |
| PI-PfuI | Pyrococcus furiosus Vc1 | 5'-GAAGATGGGAGGAGGG^ACCGGACTCAACTT<br>3'-CTTCTACCCTCC^TCCCTGGCCTGAGTTGAA |
| PI-PfuII | Pyrococcus furiosus Vc1 | 5'-ACGAATCCATGTGGAGA^AGAGCCTCTATA<br>3'-TGCTTAGGTACAC^CTCTTCTCGGAGATAT |

(continued)

| DSBI Enzyme | Organism of origin | Recognition sequence |
|---|---|---|
| PI-PkoI | Pyrococcus kodakaraensis KOD1 | 5'-GATTTTAGAT^CCCTGTACC<br>3'-CTAAAA^TCTAGGGACATGG |
| PI-PkoII | Pyrococcus kodakaraensis KOD1 | 5'-CAGTACTACG^GTTAC<br>3'-GTCATG^ATGCCAATG |
| PI-PspI | Pyrococcus sp. | 5'-AAAATCCTGGCAAACAGCTATTAT^GGGTAT<br>3'-TTTTAGGACCGTTTGTCGAT^AATACCCATA |
| PI-TfuI | Thermococcus fumicolans ST557 | 5'-TAGATTTTAGGT^CGCTATATCCTTCC<br>3'-ATCTAAAA^TCCAGCGATATAGGAAGG |
| PI-TfuII | Thermococcus fumicolans ST557 | 5'-TAYGCNGAYACN^GACGGYTTYT<br>3'-ATRCGNCT^RTGNCTGCCRAARA |
| PI-ThyI | Thermococcus hydrothermalis | 5'-TAYGCNGAYACN^GACGGYTTYT<br>3'-ATRCGNCT^RTGNCTGCCRAARA |
| PI-TliI | Thermococcus | 5'-TAYGCNGAYACNGACGG^YTTYT |
| | litoralis | 3'-ATRCGNCTRTGNC^TGCCRAARA |
| PI-TliII | Thermococcus litoralis | 5'-AAATTGCTTGCAAACAGCTATTACGGCTAT |
| I-TevI | Bacteriophage T4 | 5'-AGTGGTATCAAC^GCTCAGTAGATG<br>3'-TCACCATAGT^TGCGAGTCATCTAC |
| I-TevII | Bacteriophage T4 | 5'-GCTTATGAGTATGAAGTGAACACGT^TATTC<br>3'-CGAATACTCATACTTCACTTGTG^ACAATAAG |
| F-TevI | Bacteriophage T4 | 5'-<br>GAAACACAA-<br>GA^AATGTTTAGTAAANNNNNNNNNNNNNN<br>3'-<br>CTTTGTGTTCTTTACAAATCATTTNNNNNNNNNNN-<br>NNN^ |
| F-TevII | Bacteriophage T4 | 5'-TTTAATCCTCGCTTC^AGATATGGCAACTG<br>3'-AAATTAGGAGCGA^AGTCTATACCGTTGAC |
| H-DreI | *E. coli pI-DreI* | 5'-CAAAACGTCGTAA^GTTCCGGCGCG<br>3'-GTTTTGCAG^CATTCAAGGCCGCGC |
| I-BasI | *Bacillus thuringiensis phage Bastille* | 5' AGTAATGAGCCTAACGCTCAGCAA<br>3'- TCATTACGAGTCGAACTCGGATTG |
| I-BmoI | *Bacillus mojavensis* s87-18 | 5'-GAGTAAGAGCCCG^TAGTAATGACATGGC<br>3'-CTCATTCTCG^GGCATCATTACTGTACCG |
| I-PogI | *Pyrobaculum oguniense* | 5'-CTTCAGTAT^GCCCCGAAAC<br>3'-GAAGT^CATACGGGGCTTTG |
| I-TwoI | *Staphylococcus aureus phage Twort* | 5'-TCTTGCACCTACACAATCCA<br>3'-AGAACGTGGATGTGTTAGGT |
| PI-MgaI | *Mycobacterium gastri* | 5'-CGTAGCTGCCCAGTATGAGTCA<br>3'-GCATCGACGGGTCATACTCAGT |
| PI-PabI | *Pyrococcus abyssi* | 5'-GGGGGCAGCCAGTGGTCCCGTT<br>3'-CCCCCGTCGGTCACCAGGGCAA |

(continued)

| DSBI Enzyme | Organism of origin | Recognition sequence |
|---|---|---|
| PI-PabII | *Pyrococcus abyssi* | 5'-ACCCCTGTGGAGAGGAGCCCCTC<br>3'-TGGGGACACCTCTCCTCGGGGAG |

Also encompassed are minor deviations (degenerations) of the recognition sequence which still enable recognition and cleavage by the sequence specific DNA-endonuclease in question. Such deviations - also in connection with different framework conditions such as, for example, calcium or magnesium concentration - have been described (Argast GM *et al.* 1998). Also encompassed are core sequences of these recognition sequences and minor deviations (degenerations) in there. It is known that the inner portions of the recognition sequences suffice for an induced double-strand break and that the outer ones are not absolutely relevant, but can code-termine the cleavage efficacy. Thus, for example, an 18bp core sequence can be defined for I-SceI.

**2.3.2 Initiation of Deletion / Excision**

There are various means to appropriately initiate deletion / excision of the target sequence. Preferably deletion is only initiated after successful integration of the target sequence into the plant genome. For example in cases, where the target sequence is a selection marker, excision is preferably initiated after the marker has successfully completed its function resulting in insertion of the DNA construct into the genome of the plant cell or organism to be transformed.

Various means are available for the person skilled in art to combine the excision enzyme with the target sequence flanked by the excision sequences. Preferably, an excision enzyme (*e.g.*, a recombinase or endonuclease) can be expressed or combined with its corresponding excision sequence (*e.g.*, a recombination or recognition site), respectively, by a method selected from the group consisting of:

a) incorporation of the expression cassette for the excision enzyme (*e.g.,* the recombinase or sequence-specific endonuclease) into a DNA construct, preferably together with the target sequence (*e.g.,* a marker gene) flanked by said excision sequences,
b) incorporation of the expression cassette for the excision enzyme (*e.g.,* the recombinase or sequence-specific endonuclease) into plant cells or plants, which are already comprising the target sequence (*e.g.,* a marker gene) flanked by said excision sequences,
c) incorporation of the expression cassette for the excision enzyme (*e.g.,* the recombinase or sequence-specific endonuclease) into plant cells or plants, which are subsequently used for as master plants or cells for transformation with constructs comprising the target sequence (*e.g.,* a marker gene) flanked by said excision sequences,
d) incorporation of the expression cassette for the excision enzyme (*e.g.,* the recombinase or sequence-specific endonuclease) into a separate DNA construct, which is transformed by way of co-transformation with a separate DNA construct comprising the target sequence (*e.g.,* a marker gene) flanked by said excision sequences.

Accordingly the target sequence and the excision enzyme (*e.g.,* the recombinase or endonuclease) can be combined in a plant organism, cell, cell compartment or tissue for example as follows:

1.) Plants comprising inserted into their genome the target sequence (*e.g.,* a marker gene) flanked by excision sequences (preferably into the chromosomal DNA) are generated in the customary manner. A further expression cassette for the excision enzyme is then combined with said DNA constructs by

a) a second transformation with said second expression cassette, or
b) crossing of the plants comprising the target sequence with master plants comprising the expression cassette for the excision enzyme.

2.) The expression cassette encoding for the excision enzyme can be integrated into the DNA construct which already bears the target sequence. It is preferred to insert the sequence encoding the excision enzyme between the sequences allowing for deletion and thus to delete it from the genomic DNA after it has fulfilled its function. Especially preferred, expression of the endonuclease is inducible in such a case (for example under the control of one of the inducible promoters described hereinbelow), in a development-dependent fashion using a development-dependent promoter, or else excision enzymes are employed whose activity is inducible in order to avoid premature deletion of the dual-function marker prior to its insertion into the genome.

3.) Relying on the co-transformation technique, the expression cassette for the excision enzyme can be transformed into the cells simultaneously with the DNA construct comprising the target sequence, but on a separate DNA molecule (*e.g.,* vector). Co-transformation can be stable or transient. In such a case, expression of the excision enzyme is preferably inducible (for example under the control of one of the inducible chimeric transcription regulating sequence as described above), although the development-dependent expression pattern of the unmodified super-promoter is already preventing premature excision.

4.) Plants expressing the excision enzyme may also act as parent individuals. In the progeny from the crossing between plants expressing the excision enzyme on the one hand and plants bearing the target sequence on the other hand, the desired target sequence excision (*e.g.,* by double-strand breaks and recombination between the homology sequences) are observed.

A preferred embodiment of the invention is related to DNA constructs comprising both the target sequence (*e.g.,* an expression cassette a selection marker; the first expression cassette) and a second expression cassette for the excision enzyme (*e.g.,* an endonuclease or recombinase encoding sequence linked to a plant promoter), preferably in a way that said second expression cassette is together with said first expression cassette flanked by said excision sequences, which allow for specific target sequence deletion.

In another preferred embodiment the mechanism of deletion/excision can be induced or activated in a way to prevent pre-mature deletion/excision of the dual-function marker. Preferably, thus expression and/or activity of a preferably employed excision enzyme can be induced, preferably by a method selected from the group consisting of

a) inducible expression by operably linking the sequence encoding said excision enzyme (*e.g.,* a recombinase or endonuclease) to an inducible promoter,

b) inducible activation, by employing a modified excision enzyme (*e.g.,* a recombinase or endonuclease) comprising a ligand-binding-domain, wherein activity of said modified excision enzyme can be modified by treatment of a compound having binding activity to said ligand-binding-domain.

Expression of the polynucleotide encoding the excision enzyme is preferably controlled by an excision promoter, which allows for expression in a timely manner so that the dual-function marker can perform its function as a negative selection marker before getting excised. Suitable promoters are for example described in the German Patent Application DE 03028884.9. Such promoters may have for example expression specificity for late developmental stages like *e.g.,* reproductive tissue. The excision promoter may be selected from one of the following groups of promoters:

### 2.3.3 The target sequence to be excised

Although various sequences are contemplated herein, where excision might be advantageous, the most preferred target sequence to be excised is a marker sequence. Various selectable and screenable marker sequences are comprised under the general term marker sequence. Thus, the methods of the invention results in a monocotyledonous plant cell or plant, which is marker-free. The terms "marker-free" or "selection marker free" as used herein with respect to a cell or an organism are intended to mean a cell or an organism which is not able to express a functional marker protein. The sequence encoding said marker protein may be absent in part or - preferably - entirely.

#### 2.3.3.1 Marker Genes

Marker genes (*e.g.,* selectable or screenable marker) are frequently used in order to improve the ability to identify transformants. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can 'select' for by chemical means, *i.e.,* through the use of a selective agent (*e.g.,* a herbicide, antibiotic, or the like), or whether it is simply a trait that one can identify through observation or testing, *i.e.*, by 'screening' (*e.g.,* the R-locus trait, the green fluorescent protein (GFP)). Of course, many examples of suitable marker genes are known to the art and can be employed in the practice of the invention. Included within the terms selectable or screenable marker genes are also genes which encode a "secretable marker" whose secretion can be detected as a means of identifying or selecting for transformed cells. Examples include markers, which encode a secretable antigen that can be identified by antibody interaction, or even secretable enzymes, which can be detected by their catalytic activity. Secretable proteins fall into a number of classes, including small, diffusible proteins detectable, *e.g.,* by ELISA; small active enzymes detectable in extracellular solution (*e.g.,* alpha-amylase, beta-lactamase, phosphinothricin acetyltransferase); and proteins that are inserted or trapped in the cell wall (*e.g.,* proteins that include a leader sequence such as that found in the expression unit of extensin or tobacco PR-S).

With regard to selectable secretable markers, the use of a gene that encodes a protein that becomes sequestered in the cell wall, and which protein includes a unique epitope is considered to be particularly advantageous. Such a secreted antigen marker would ideally employ an epitope sequence that would provide low background in plant tissue, a promoter-leader sequence that would impart efficient expression and targeting across the plasma membrane, and would produce protein that is bound in the cell wall and yet accessible to antibodies. A normally secreted wall protein modified to include a unique epitope would satisfy all such requirements. One example of a protein suitable for modification in this manner is extensin, or hydroxyproline rich glycoprotein (HPRG). For example, the maize HPRG (Steifel 1990) molecule is well characterized in terms of molecular biology, expression and protein structure. However, any one of a variety of ultilane and/or glycine-rich wall proteins (Keller 1989) could be modified by the addition of an antigenic site to create a screenable marker.

One exemplary embodiment of a secretable screenable marker concerns the use of a maize sequence encoding the wall protein HPRG, modified to include a 15 residue epitope from the pro-region of murine interleukin, however, virtually any detectable epitope may be employed in such embodiments, as selected from the extremely wide variety of antigen-antibody combinations known to those of skill in the art. The unique extracellular epitope can then be straightforwardly detected using antibody labeling in conjunction with chromogenic or fluorescent adjuncts.

Elements of the present disclosure may be exemplified in detail through the use of the bar and/or GUS genes, and also through the use of various other markers. Of course, in light of this disclosure, numerous other possible selectable and/or screenable marker genes will be apparent to those of skill in the art in addition to the one set forth herein below. Therefore, it will be understood that the following discussion is exemplary rather than exhaustive. In light of the techniques disclosed herein and the general recombinant techniques which are known in the art, the present invention renders possible the introduction of any gene, including marker genes, into a recipient cell to generate a transformed plant.

The marker sequence can be expressed by any transcription regulating sequence or promoter having expression capability in plant cells (suitable promoter sequences are described below). Most preferred are marker sequences, which are employed in plant transformation, screening and selection. Markers enable transgenic cells or organisms (e.g., plants or plant cells) to be identified after transformation. They can be divided into positive selection marker (conferring a selective advantage), negative selection marker (compensating a selection disadvantage), and counter-selection marker (conferring a selection disadvantage), respectively. Such markers may include but are not limited to:

### 2.3.3.1.1 Negative selection markers

Negative selection markers confer a resistance to a biocidal compound such as a metabolic inhibitor (*e.g.,* 2-deoxyglucose-6-phosphate, WO 98/45456), antibiotics (*e.g.,* kanamycin, G 418, bleomycin or hygromycin) or herbicides (*e.g.,* phosphinothricin or glyphosate). Transformed plant materials (*e.g.,* cells, tissues or plantlets), which express marker genes, are capable of developing in the presence of concentrations of a corresponding selection compound (*e.g.,* antibiotic or herbicide), which suppresses growth of an untransformed wild type tissue. Especially preferred negative selection markers are those, which confer resistance to herbicides. Examples, which may be mentioned, are:

- Phosphinothricin acetyltransferases (PAT; also named Bialophos ®resistance; bar; de Block 1987; Vasil 1992, 1993; Weeks 1993; Becker 1994; Nehra 1994; Wan & Lemaux 1994; EP 0 333 033; US 4,975,374). Preferred are the bar gene from *Streptomyces hygroscopicus* or the pat gene from *Streptomyces viridochromogenes.* PAT inactivates the active ingredient in the herbicide bialaphos, phosphinothricin (PPT). PPT inhibits glutamine synthetase, (Murakami 1986; Twell 1989) causing rapid accumulation of ammonia and cell death.
- altered 5-enolpyruvylshikimate-3-phosphate synthase (EPSPS) conferring resistance to Glyphosate® (N-(phosphonomethyl)glycine) (Hinchee 1988; Shah 1986; Della-Cioppa 1987). Where a mutant EPSP synthase gene is employed, additional benefit may be realized through the incorporation of a suitable chloroplast transit peptide, CTP (EP-A1 0 218 571).
- Glyphosate® degrading enzymes (Glyphosate® oxidoreductase; gox),
- Dalapon® inactivating dehalogenases (deh)
- sulfonylurea- and/or imidazolinone-inactivating acetolactate synthases (*ahas* or ALS; for example mutated ahas/ALS variants with, for example, the S4, XI12, XA17, and/or Hra mutation (EP-A1 154 204)
- Bromoxynil® degrading nitrilases (bxn; Stalker 1988)
- Kanamycin- or geneticin (G418) resistance genes (NPTII; NPT or neo; Potrykus 1985) coding e.g., for neomycin phosphotransferases (Fraley 1983; Nehra 1994)
- 2-Desoxyglucose-6-phosphate phosphatase ($DOG^R$1-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- altered dihydrofolate reductase (Eichholtz 1987) conferring resistance against methotrexat (Thillet 1988);

- mutated anthranilate synthase genes that confers resistance to 5-methyl tryptophan.

Additional negative selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Especially preferred are negative selection markers that confer resistance against the toxic effects imposed by D-amino acids like *e.g.,* D-alanine and D-serine (WO 03/060133; Erikson 2004). Especially preferred as negative selection markers in this contest are the *dao*I gene (EC: 1.4. 3.3: GenBank Acc.No. U60066) from the yeast *Rhodotorula gracilis* (*Rhodosporidium toruloides*) and the *E. coli* gene *dsdA* (D-serine dehydratase (D-serine deaminase) [EC: 4.3. 1.18; GenBank Acc.No. J01603).

Transformed plant materials (*e.g.,* cells, embryos, tissues or plantlets) which express such marker genes are capable of developing in the presence of concentrations of a corresponding selection compound (*e.g.,* antibiotic or herbicide) which suppresses growth of an untransformed wild type tissue. The resulting plants can be bred and hybridized in the customary fashion. Two or more generations should be grown in order to ensure that the genomic integration is stable and hereditary. Corresponding methods are described (Jenes 1993; Potrykus 1991).

Furthermore, reporter genes can be employed to allow visual screening, which may or may not (depending on the type of reporter gene) require supplementation with a substrate as a selection compound.

Various time schemes can be employed for the various negative selection marker genes. In case of resistance genes (*e.g.,* against herbicides or D-amino acids) selection is preferably applied throughout callus induction phase for about 4 weeks and beyond at least 4 weeks into regeneration. Such a selection scheme can be applied for all selection regimes. It is furthermore possible (although not explicitly preferred) to remain the selection also throughout the entire regeneration scheme including rooting.

For example, with the phosphinotricin resistance gene (bar) as the selective marker, phosphinotricin at a concentration of from about 1 to 50 mg/L may be included in the medium. For example, with the *dao1* gene as the selective marker, D-serine or D-alanine at a concentration of from about 3 to 100 mg/L may be included in the medium. Typical concentrations for selection are 20 to 40 mg/L. For example, with the mutated ahas genes as the selective marker, PURSUIT™ at a concentration of from about 3 to 100 mg/L may be included in the medium. Typical concentrations for selection are 20 to 40 mg/L.

### 2.3.3.1.2 Positive selection marker

Furthermore, positive selection marker can be employed. Positive selection markers are those, which do not result in detoxification of a biocidal compound, but confer an advantage by increased or improved regeneration, growth, propagation, multiplication as the like of the cell or organism comprising such kind of marker. Examples are isopentenyltransferase (a key enzyme of the cytokinin biosynthesis facilitating regeneration of transformed plant cells by selection on cytokinin-free medium; Ebinuma 2000a; Ebinuma 2000b; for example from strain:PO22; Genbank Acc.No. AB025109). Additional positive selection markers, which confer a growth advantage to a transformed plant cells in comparison with a non-transformed one, are described *e.g.,* in EP-A 0 601 092. Growth stimulation selection markers may include (but shall not be limited to) · -Glucuronidase (in combination with *e.g.,* a cytokinin glucuronide), mannose-6-phosphate isomerase (in combination with mannose), UDP-galactose-4-epimerase (in combination with *e.g.,* galactose), wherein mannose-6-phosphate isomerase in combination with mannose is especially preferred.

### 2.3.3.1.3 Counter-selection marker

The target sequence to be excised may not only comprise a negative selection marker or a positive selection marker (to facilitate selection and isolation of successfully transformed plants) but may also comprise a counter-selection marker to evaluate successful subsequent marker excision. In one preferred embodiment both the negative and/or positive selection marker and the counter selection marker are flanked by the excision sequences and are both deleted / excised by action of the excision enzyme. Counter-selection markers are especially suitable to select organisms with defined deleted sequences comprising said marker (Koprek 1999). Counter-selection markers are sequences encoding for enzymes which are able to convert a non-toxic compound into a toxic compound. In consequence, only cells will survive treatment with said non-toxic compound which are lacking said counter-selection marker, thereby allowing for selection of cells which have successfully undergone sequence (*e.g.,* marker) deletion. Typical counter-selection markers known in the art are for example

a) cytosine deaminases (CodA) in combination with 5-fluorocytosine (5-FC) (WO 93/01281; US 5,358,866; Gleave AP *et al.* 1999; Perera RJ *et al.* 1993; Stougaard J 1993; EP-A1 595 837; Mullen CA *et al.* 1992; Kobayashi T *et al.*

1995; Schlaman HRM & Hooykaas PFF 1997; Xiaohui Wang H *et al.* 2001; Koprek T *et al.* 1999; Gleave AP *et al.* 1999; Gallego ME 1999; Salomon S & Puchta H 1998; Thykjaer T *et al.* 1997; Serino G 1997; Risseeuw E 1997; Blanc V *et al.* 1996; Corneille S *et al.* 2001).

b) Cytochrome P-450 enzymes in combination with the sulfonylurea pro-herbicide R7402 (2-methylethyl-2-3-dihydro-N-[(4,6-dimethoxypyrimidine-2-yl)aminocarbonyl]-1,2-benzoisothiazol-7-sulfonamid-1,1-dioxide) (O'Keefe DP *et al.* 1994; Tissier AF *et al.* 1999; Koprek T *et al.* 1999; O'Keefe DP 1991).

c) Indoleacetic acid hydrolases like *e.g.,* the tms2 gene product from Agrobacterium tumefaciens in combination with naphthalacetamide (NAM) (Fedoroff NV & Smith DL 1993; Upadh-yaya NM *et al.* 2000; Depicker AG *et al.* 1988; Karlin-Neumannn GA *et al.* 1991; Sundaresan V *et al.* 1995; Cecchini E *et al.* 1998; Zubko E *et al.* 2000).

d) Haloalkane dehalogenases (dhlA gene product) from Xanthobacter autotropicus GJ10 in combination with 1,2-dichloroethane (DCE) (Naested H *et al.* 1999; Janssen DB *et al.* 1994; Janssen DB 1989).

e) Thymidine kinases (TK), *e.g.,* from Type 1 Herpes Simplex virus (TK HSV-1), in combination with acyclovir, ganciclovir or 1,2-deoxy-2-fluoro-· -D-arabinofuranosil-5-iodouracile (FIAU) (Czako M & Marton L 1994; Wigler M *et al.* 1977; McKnight SL *et al.* 1980; McKnight SL *et al.* 1980; Preston *et al.* 1981; Wagner *et al.* 1981; St. Clair *et al.* 1987).

Several other counter-selection systems are known in the art (see for example international application WO 04/013333; p.13 to 20 for a summary).

### 2.3.3.1.4. Screenable Markers

Screenable markers (also named reporter genes or proteins; Schenborn E, Groskreutz D. 1999) that may be employed include, but are not limited to, a beta-glucuronidase (GUS; Jefferson *et al.* 1987) or uidA gene which encodes an enzyme for which various chromogenic substrates are known; an R-locus gene, which encodes a product that regulates the production of anthocyanin pigments (red color) in plant tissues (Dellaporta 1988); a beta-lactamase gene (Sutcliffe 1978), which encodes an enzyme for which various chromogenic substrates are known (*e.g.,* PADAC, a chromogenic cephalosporin); a xylE gene (Zukowsky 1983) which encodes a catechol dioxy-genase that can convert chromogenic catechols; an · -amylase gene (Ikuta 1990); a tyrosinase gene (Katz 1983) which encodes an enzyme capable of oxidizing tyrosine to DOPA and dopaquinone which in turn condenses to form the easily detectable compound melanin; · - galactosidase gene, which encodes an enzyme for which there are chromogenic substrates; a luciferase (lux) gene (Ow 1986; Millar *et al.* 1992), which allows for bioluminescence detection; or even an aequorin gene (Prasher 1985), which may be employed in calcium-sensitive bioluminescence detection, or a green fluorescent protein gene (GFP) (Niedz 1995; Chui WL *et al.* 1996; Leffel SM *et al.* 1997; Sheen *et al.* 1995; Haseloff *et al.* 1997; Reichel *et al.* 1996; Tian *et al.* 1997).

Genes from the maize R gene complex are contemplated to be particularly useful as screenable markers. The R gene complex in maize encodes a protein that acts to regulate the production of anthocyanin pigments in most seed and plant tissue. A gene from the R gene complex was applied to maize transformation, because the expression of this gene in transformed cells does not harm the cells. Thus, an R gene introduced into such cells will cause the expression of a red pigment and, if stably incorporated, can be visually scored as a red sector. If a maize line is dominant for genes encoding the enzymatic intermediates in the anthocyanin biosynthetic pathway (C2, A1, A2, Bz1 and Bz2), but carries a recessive allele at the R locus, transformation of any cell from that line with R will result in red pigment formation. Exemplary lines include Wisconsin 22 which contains the rg-Stadler allele and TR112, a K55 derivative which is r-g, b, P1. Alternatively any genotype of maize can be utilized if the C1 and R alleles are introduced together.

It is further proposed that R gene regulatory regions may be employed in chimeric constructs in order to provide mechanisms for controlling the expression of chimeric genes. More diversity of phenotypic expression is known at the R locus than at any other locus (Coe 1988). It is contemplated that regulatory regions obtained from regions 5' to the structural R gene would be valuable in directing the expression of genes, *e.g.,* insect resistance, drought resistance, herbicide tolerance or other protein coding regions. For the purposes of the present invention, it is believed that any of the various R gene family members may be successfully employed (e.*g.,* P, S, Lc, *etc.*). However, the most preferred will generally be Sn (particularly Sn:bol3). Sn is a dominant member of the R gene complex and is functionally similar to the R and B loci in that Sn controls the tissue specific deposition of anthocyanin pigments in certain seedling and plant cells, therefore, its phenotype is similar to R.

A further screenable marker contemplated for use in the present invention is firefly luciferase, encoded by the lux gene. The presence of the lux gene in transformed cells may be detected using, for example, X-ray film, scintillation counting, fluorescent spectrophotometry, low-light video cameras, photon counting cameras or multiwell luminometry. It is also envisioned that this system may be developed for populational screening for bioluminescence, such as on tissue culture plates, or even for whole plant screening. Where use of a screenable marker gene such as lux or GFP is desired, benefit may be realized by creating a gene fusion between the screenable marker gene and a selectable marker gene, for example, a GFP-NPTII gene fusion. This could allow, for example, selection of transformed cells followed by screening of transgenic plants or seeds.

**2.3.3.1.5. Dual-function marker**

In one preferred embodiment of the invention the target sequence is a dual-function marker. The term dual-function marker relates to a marker which combines in one sequence the opportunity to be employed as negative or counter selection marker. The choice, which effect is achieved, depends on the substrate employed in the screening process. Most preferably the dual-function marker is a D-amino acid oxidase. This enzyme is capable to convert D-amino acids. Some D-amino acids are toxic to plants and are detoxified by action of the enzyme. Other D-amino acids are harmless to plants but are converted to toxic compounds by the enzyme.

The term D-amino acid oxidase (abbreviated DAAO, DAMOX, or DAO) is referring to the enzyme converting a D-amino acid into a 2-oxo acid, by - preferably - employing Oxygen ($O_2$) as a substrate and producing hydrogen peroxide ($H_2O_2$) as a co-product (Dixon M & Kleppe K. Biochim. Biophys. Acta 96 (1965) 357-367; Dixon M & Kleppe K Biochim. Biophys. Acta 96 (1965) 368-382; Dixon M & Kleppe Biochim. Biophys. Acta 96 (1965) 383-389; Massey V et al. Biochim. Biophys. Acta 48 (1961) 1-9. Meister A & Wellner D Flavoprotein amino acid oxidase. In: Boyer, P.D., Lardy, H. and Myrbäck, K. (Eds.), 1963)

DAAO can be described by the Nomenclature Committee of the International Union of Biochemistry and Molecular Biology (IUBMB) with the EC (Enzyme Commission) number EC 1.4.3.3. Generally a DAAO enzyme of the EC 1.4.3.3. class is an FAD flavoenzyme that catalyzes the oxidation of neutral and basic D-amino acids into their corresponding keto acids. DAAOs have been characterized and sequenced in fungi and vertebrates where they are known to be located in the peroxisomes. The term D-amino oxidase further comprises D-aspartate oxidases (EC 1.4.3.1) (DASOX) (Negri A *et al.* 1992), which are enzymes structurally related to DAAO catalyzing the same reaction but active only toward dicarboxylic D-amino acids. Within this invention DAAO of the EC 1.4.3.3. class is preferred.

In DAAO, a conserved histidine has been shown (Miyano M *et al.* 1991) to be important for the enzyme's catalytic activity. In a preferred embodiment of the invention a DAAO is referring to a protein comprising the following consensus motif:

[LIVM]-[LIVM]-H*-[NHA]-Y-G-x-[GSA]-[GSA]-x-G-$x_5$-G-x-A

wherein amino acid residues given in brackets represent alternative residues for the respective position, x represents any amino acid residue, and indices numbers indicate the respective number of consecutive amino acid residues. The abbreviation for the individual amino acid residues have their standard IUPAC meaning as defined above. Further potential DAAO enzymes comprising said motif are described in Table 3.

Table 3. Suitable D-amino acid oxidases from various organism. Acc.-No. refers to protein sequence from SwisProt database.

| Acc.-No. | Gene Name | Description | Source Organism | Length |
|---|---|---|---|---|
| Q19564 | F18E3.7 | Putative D-amino acid oxidase (EC 1.4.3.3) (DAMOX) (DAO) (DAAO) | Caenorhabditis elegans | 334 |
| P24552 | | D-amino acid oxidase (EC 1.4.3.3) (DAMOX) (DAO) (DAAO) | Fusarium solani (subsp. pisi) (Nectria haematococca) | 361 |
| P14920 | DAO, DAMOX | D-amino acid oxidase (EC 1.4.3.3) (DAMOX) (DAO) (DAAO) | Homo sapiens (Human) | 347 |
| P18894 | DAO, DAO1 | D-amino acid oxidase (EC 1.4.3.3) (DAMOX) (DAO) (DAAO) | Mus musculus (Mouse) | 346 |
| P00371 | DAO | D-amino acid oxidase (EC 1.4.3.3) (DAMOX) (DAO) (DAAO) | Sus scrofa (Pig) | 347 |

(continued)

| Acc.-No. | Gene Name | Description | Source Organism | Length |
|---|---|---|---|---|
| P22942 | DAO | D-amino acid oxidase (EC 1.4.3.3) (DAMOX) (DAO) (DAAO) | Oryctolagus cuniculus (Rabbit) | 347 |
| O35078 | DAO | D-amino acid oxidase (EC 1.4.3.3) (DAMOX) (DAO) (DAAO) | Rattus norvegicus (Rat) | 346 |
| P80324 | DAO1 | D-amino acid oxidase (EC 1.4.3.3) (DAMOX) (DAO) (DAAO) | Rhodosporidium toruloides (Yeast) (Rhodotorula gracilis) | 368 |
| U60066 | DAO | D-amino acid oxidase (EC 1.4.3.3) (DAMOX) (DAO) (DAAO) | Rhodosporidium toruloides, strain TCC 26217 | 368 |
| Q99042 | DAO1 | D-amino acid oxidase (EC 1.4.3.3) (DAMOX) (DAO) (DAAO) | Trigonopsis variabilis (Yeast) | 356 |
| P31228 | DDO | D-aspartate oxidase (EC 1.4.3.1) (DASOX) (DDO) | Bos taurus (Bovine) | 341 |
| Q99489 | DDO | D-aspartate oxidase (EC 1.4.3.1) (DASOX) (DDO) | Homo sapiens (Human) | 341 |
| Q9C1L2 | NCU06558.1 | (AF309689) putative D-amino acid oxidase G6G8.6 (Hypothetical protein) | Neurospora crassa | 362 |
| Q7SFW 4 | NCU03131.1 | Hypothetical protein | Neurospora crassa | 390 |
| Q8N552 | | Similar to D-aspartate oxidase | Homo sapiens (Human) | 369 |
| Q7Z312 | DKFZP686F0 4272 | Hypothetical protein DKFZp686F04272 | Homo sapiens (Human) | 330 |
| Q9VM8 0 | CG11236 | CG11236 protein (GH12548p) | Drosophila melanogaster (Fruit fly) | 341 |
| O01739 | F20H11.5 | F20H11.5 protein | Caenorhabditis elegans | 383 |
| O45307 | C47A10.5 | C47A10.5 protein | Caenorhabditis elegans | 343 |
| Q8SZN 5 | CG12338 | RE73481p | Drosophila melanogaster (Fruit fly) | 335 |
| Q9V5P1 | CG12338 | CG12338 protein (RE49860p) | Drosophila melanogaster (Fruit fly) | 335 |
| Q86JV2 | | Similar to Bos taurus (Bovine). D-aspartate oxidase (EC 1.4.3.1) (DASOX) (DDO) | Dictyostelium discoideum (Slime mold) | 599 |
| Q95XG 9 | Y69A2AR.5 | Hypothetical protein | Caenorhabditis elegans | 322 |

(continued)

| Acc.-No. | Gene Name | Description | Source Organism | Length |
|---|---|---|---|---|
| Q7Q7G 4 | AGCG53627 | AgCP5709 (Fragment) | Anopheles gambiae str. PEST | 344 |
| Q7PWY 8 | AGCG53442 | AgCP12432 (Fragment) | Anopheles gambiae str. PEST | 355 |
| Q7PWX 4 | AGCG45272 | AgCP12797 (Fragment) | Anopheles gambiae str. PEST | 373 |
| Q8PG9 5 | XAC3721 | D-amino acid oxidase | Xanthomonas axonopodis (pv. citri) | 404 |
| Q8P4M 9 | XCC3678 | D-amino acid oxidase | Xanthomonas campestris (pv. campestris) | 405 |
| Q9X7P6 | SCO6740, SC5F2A. 23C | Putative D-amino acid oxidase | Streptomyces coelicolor | 320 |
| Q82MI8 | DAO, SAV1672 | Putative D-amino acid oxidase | Streptomyces avermitilis | 317 |
| Q8VCW 7 | DAO1 | D-amino acid oxidase | Mus musculus (Mouse) | 345 |
| Q9Z302 | | D-amino acid oxidase | Cricetulus griseus (Chinese hamster) | 346 |
| Q9Z1M 5 | | D-amino acid oxidase | Cavia porcellus (Guinea pig) | 347 |
| Q922Z0 | | Similar to D-aspartate oxidase | Mus musculus (Mouse) | 341 |
| Q8R2R 2 | | Hypothetical protein | Mus musculus (Mouse) | 341 |
| P31228 | | D-aspartate oxidase | B.taurus | 341 |

D-Amino acid oxidase (EC-number 1.4.3.3) can be isolated from various organisms, including but not limited to pig, human, rat, yeast, bacteria or fungi. Example organisms are Candida tropicalis, Trigonopsis variabilis, Neurospora crassa, Chlorella vulgaris, and Rhodotorula gracilis. A suitable D-amino acid metabolising polypeptide may be a eukaryotic enzyme, for example from a yeast (*e.g. Rhodotorula gracilis*), fungus, or animal or it may be a prokaryotic enzyme, for example, from a bacterium such as *Escherichia coli.* Examples of suitable polypeptides which metabolise D-amino acids are shown in Table 4.

Table 4. Suitable D-amino acid oxidases from various organism. Acc.-No. refers to protein sequence from SwisProt database

| GenBank Acc.-No, | Source Organism |
|---|---|
| Q19564 | Caenorhabditis elegans. F18E3.7. |
| P24552 | Fusarii solani (subsp. pisi) (Nectria haematococca). |
| JX0152 | Fusarium solani |
| P14920 | Homo sapiens (Human) |
| P18894 | Mus musculus (mouse) |
| P00371 | Sus scrofa (pig) |
| P22942 | Oryctolagus cuniculus (Rabbit) |
| O5078 | Rattus norvegicus (Rat) |
| P80324 | Rhodosporidium toruloides (Yeast) (Rhodotorula gracilis) |

(continued)

| GenBank Acc.-No, | Source Organism |
|---|---|
| Q99042 | Trigonopsis variabilis |
| Q9Y7N4 | Schizosaccharomyces pombe (Fission yeast) SPCC1450 |
| O01739 | Caenorhabditis elegans.F20H11.5 |
| Q28382 | Sus scrofa (Pig). |
| O3145 | Mycobacterium leprae |
| Q9X7P6 | Streptomyces coelicolor.SCSF2A.23C |
| Q9JXF8 | Neisseria meningitidis (serogroup B). |
| Q9Z302 | Cricetulus griseus (Chinese hamster) |
| Q921M5 | D-AMINO ACID OXIDASE. Cavia parcellus (Guinea pig) |

Preferably the D-amino acid oxidase is selected from the enzymes encoded by a nucleic acid sequence or a corresponding amino acid sequences selected from the following Table 5:

Table 5: Suitable D-amino acid oxidases from various organism. Acc.-No. refers to protein sequence from GenBank database.

| GenBanc Acc.-No | Organism |
|---|---|
| U60066 | Rhodosporidium toruloides (Yeast) |
| Z71657 | Rhodotorula gracilis |
| A56901 | Rhodotorula gracilis |
| AF003339 | Rhodosporidium toruloides |
| AF003340 | Rhodosporidium toruloides |
| U53139 | Caenorhabditis elegans |
| D00809 | Nectria haematococca |
| Z50019. | Trigonopsis variabilis |
| NC_003421 | Schizosaccharomyces pombe (fission yeast) |
| AL939129. | Streptomyces coelicolor A3(2) |
| AB042032 | Candida boidinii |

DAAO is a well-characterized enzyme, and both its crystal structure and its catalytic mechanism have been determined by high-resolution X-ray spectroscopy (Umhau S. *et al.* 2000). It is a flavoenzyme located in the peroxisome, and its recognized function in animals is detoxification of D-amino acids (Pilone MS 2000). In addition, it enables yeasts to use D-amino acids for growth (Yurimoto H *et al.* 2000). As demonstrated above, DAAO from several different species have been characterized and shown to differ slightly in substrate affinities (Gabler M *et al.* 2000), but in general they display broad substrate specificity, oxidatively deaminating all D-amino acids (except D-glutamate and D-aspartate for EC 1.4.3.3. calss DAAO enzymes; Pilone MS 2000).

DAAO activity is found in many eukaryotes (Pilone MS 2000), but there is no report of DAAO activity in plants. The low capacity for D-amino acid metabolism in plants has major consequences for the way plants respond to D-amino acids.

In a preferred embodiment D-amino acid oxidase expressed form the DNA-construct of the invention has preferably enzymatic activity against at least one of the amino acids selected from the group consisting of D-alanine, D-serine, D-isoleucine, D-valine, and derivatives thereof.

Suitable D-amino acid oxidases also include fragments, mutants, derivatives, variants and alleles of the polypeptides exemplified above. Suitable fragments, mutants, derivatives, variants and alleles are those which retain the functional characteristics of the D-amino acid oxidase as defined above. Changes to a sequence, to produce a mutant, variant or derivative, may be by one or more of addition, insertion, deletion or substitution of one or more nucleotides in the nucleic acid, leading to the addition, insertion, deletion or substitution of one or more amino acids in the encoded polypeptide. Of course, changes to the nucleic acid that make no difference to the encoded amino acid sequence are included.

The D-amino acid oxidase of the invention may be expressed in the cytosol, peroxisome, or other intracellular compartment of the plant cell. Compartmentalisation of the D-amino acid metabolising polypeptide may be achieved by fusing the nucleic acid sequence encoding the DAAO polypeptide to a sequence encoding a transit peptide to generate a fusion protein. Gene products expressed without such transit peptides generally accumulate in the cytosol. The localisation of expressed DAAO in the peroxisome produces $H_20_2$ that can be metabolised by the $H_20_2$ degrading enzyme catalase. Higher levels of D-amino acids may therefore be required to produce damaging levels of $H_20_2$. Expression of DAAO in the cytosol, where levels of catalase activity are lower, reduces the amount of D-amino acid required to produce damaging levels $H_20_2$. Expression of DAAO in the cytosol may be achieved by removing peroxisome targeting signals or transit peptides from the encoding nucleic acid sequence. For example, the *dao1* gene (EC: 1.4.3.3: GenBank Acc.No. U60066) from the yeast *Rhodotorula gracilis* (*Rhodosporidium toruloides*) was cloned as described (WO 03/060133). The last nine nucleotides encode the signal peptide SKL, which guides the protein to the peroxisome sub-cellular organelle. Although no significant differences were observed between cytosolic and peroxisomal expressed DAAO, the peroxisomal construction was found to be marginally more effective than the cytosolic version in respect of inhibiting the germination of the DAAO transgenic plants on 30 mM D-Asn. However, both constructs are inhibited significantly more than the wild-type and may thus be used for conditional counter-selection.

Addtional modifications and use of dual-function marker are disclosed in EP Appl. No. 04006358.8 (SweTree Technologies AB & BASF; IMPROVED CONSTRUCTS FOR MARKER EXCISION BASED ON DUAL-FUNCTION SELECTION MARKER) and additional national and international applications claiming priority therefrom.

### 2.3.4. Expression of the marker gene and other sequences

The marker gene (or other sequences which can be expressed from one of the DNA constructs of the invention) may be expressed by any promoter functional in plants. These promoters include, but are not limited to, constitutive, inducible, temporally regulated, developmentally regulated, spatially-regulated, chemically regulated, stress-responsive, tissue-specific, viral and synthetic promoters. The promoter may be a gamma zein promoter, an oleosin ole16 promoter, a globulins promoter, an actin I promoter, an actin cl promoter, a sucrose synthetase promoter, an INOPS promoter, an EXM5 promoter, a globulin2 promoter, a · -32, ADPG-pyrophosphorylase promoter, an LtpI promoter, an Ltp2 promoter, an oleosin ole17 promoter, an oleosin ole18 promoter, an actin 2 promoter, a pollen-specific protein promoter, a pollen-specific pectate lyase promoter, an anther-specific protein promoter, an anther-specific gene RTS2 promoter, a pollen-specific gene promoter, a tapeturn-specific gene promoter, a tapeturn-specific gene RAB24 promoter, an anthranilate synthase alpha subunit promoter, an alpha zein promoter, an anthranilate synthase beta subunit promoter, a dihydrodipicolinate synthase promoter, a Thil promoter, an alcohol dehydrogenase promoter, a cab binding protein promoter, an H3C4 promoter, a RUBISCO SS starch branching enzyme promoter, an ACCase promoter, an actin3 promoter, an actin7 promoter, a regulatory protein GF14-12 promoter, a ribosomal protein L9 promoter, a cellulose biosynthetic enzyme promoter, an S-adenosyl-L-homocysteine hydrolase promoter, a superoxide dismutase promoter, a C-kinase receptor promoter, a phosphoglycerate mutase promoter, a root-specific RCc3 mRNA promoter, a glucose-6 phosphate isomerase promoter, a pyrophosphate-fructose 6-phosphatelphosphotransferase promoter, a ubiquitin promoter, a beta-ketoacyl-ACP synthase promoter, a 33 kDa photosystem 11 promoter, an oxygen evolving protein promoter, a 69 kDa vacuolar ATPase subunit promoter, a metallothionein-like protein promoter, a glyceraldehyde-3-phosphate dehydrogenase promoter, an ABA- and ripening-inducible-like protein promoter, a phenylalanine ammonia lyase promoter, an adenosine tri-phosphatase S-adenosyl-L-homocysteine hydrolase promoter, an · -tubulin promoter, a cab promoter, a PEPCase promoter, an R gene promoter, a lectin promoter, a light harvesting complex promoter, a heat shock protein promoter, a chalcone synthase promoter, a zein promoter, a globulin-1 promoter, an ABA promoter, an auxin-binding protein promoter, a UDP glucose flavonoid glycosyl-transferase gene promoter, an NTI promoter, an actin promoter, an opaque 2 promoter, a b70 promoter, an oleosin promoter, a CaMV 35S promoter, a CaMV 34S promoter, a CaMV 19S promoter, a histone promoter, a turgor-inducible promoter, a pea small subunit RuBP carboxylase promoter, a Ti plasmid mannopine synthase promoter, a Ti plasmid nopaline synthase promoter, a petunia chalcone isomerase promoter, a bean glycine rich protein I promoter, a CaMV 35S transcript promoter, a potato patatin promoter, or a S-E9 small subunit RuBP carboxylase promoter.

## 3. Assays of Transgene Expression

To confirm the presence of an exogenous DNA in regenerated plants, a variety of assays may be performed. Such assays include, for example, molecular biological assays such as Southern and Northern blotting and PCR; biochemical assays such as detecting the presence of a protein product, e.g., by immunological means (ELISAs and Western blots) or by enzymatic function; plant part assays such as leaf or root assays; and in some cases phenotype analysis of a whole regenerated plant. Additional assays useful for determining the efficiency of transgene expression and promoter function also include without limitation fluorescent in situ hybridization (FISH), direct DNA sequencing, pulsed field gel electrophoresis (PFGE) analysis, single-stranded conformation analysis (SSCA), RNase protection assay, allele-specific oligonucleotide (ASO), dot blot analysis, denaturing gradient gel electrophoresis, RT-PCR, quantitative RT-PCR, RFLP and PCR-SSCP. Such assays are known to those of skill in the art (see also above).

**4. Transformed (Transgenic) Plants of the Invention and Methods of Preparation**

Monocot plant species may be transformed with the DNA construct of the present invention by various methods known in the art. Any plant tissue capable of subsequent clonal propagation, whether by organogenesis or embryogenesis, may be transformed. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (*e.g.,* apical meristems, axillary buds, and root meristems), and induced meristem tissue (*e.g.,* cotyledon meristem and ultilane meristem). Plants of the present invention may take a variety of forms. The plants may be chimeras of transformed cells and non-transformed cells; the plants may be clonal transformants (*e.g.,* all cells transformed to contain the expression cassette); the plants may comprise grafts of transformed and untransformed tissues (*e.g.,* a transformed root stock grafted to an untransformed scion in citrus species). The transformed plants may be propagated by a variety of means, such as by clonal propagation or classical breeding techniques. For example, first generation (or T1) transformed plants may be selfed to give homozygous second generation (or T2) transformed plants, and the T2 plants further propagated through classical breeding techniques. A dominant selectable marker (such as npt II) can be associated with the expression cassette to assist in breeding.

Thus, the present invention provides a transformed (transgenic) monocotyledonous plants and monocotyledonous plant cell, *in planta* or *ex planta,* including a transformed plastid or other organelle, *e.g.,* nucleus, mitochondria or chloroplast. The present invention may be used for transformation of any monocotyledonous plant species, including, but not limited to, cells from the plant species specified above in the DEFINITION section. Preferably, transgenic plants of the present invention are crop plants and in particular cereals (for example, corn, alfalfa, rice, barley, sorghum, wheat, millet *etc*.), and even more preferably corn, wheat and rice. Other embodiments of the invention are related to cells, cell cultures, tissues, parts (such as plants organs, leaves, roots, etc.) and propagation material (such as seeds) of such monocotyledonous plants.

Transformation of monocotyledonous plants can be undertaken with a single DNA molecule or multiple DNA molecules (*i.e.,* co-transformation), and both these techniques are suitable for use with the expression cassettes of the present invention. Numerous transformation vectors are available for plant transformation, and the expression cassettes of this invention can be used in conjunction with any such vectors. The selection of vector will depend upon the preferred transformation technique and the target species for transformation.

A variety of techniques are available and known to those skilled in the art for introduction of constructs into a plant cell host. These techniques generally include transformation with DNA employing *A. tumefaciens* or A. rhizogenes as the transforming agent, liposomes, PEG precipitation, electroporation, DNA injection, direct DNA uptake, microprojectile bombardment, particle acceleration, and the like (see, for example, EP 295959 and EP 138341). However, cells other than plant cells may be transformed with the expression cassettes of the invention. The general descriptions of plant expression vectors and reporter genes, and *Agrobacterium* and *Agrobact*erium-mediated gene transfer, can be found in Gruber *et al.* (1993).

Expression vectors containing genomic or synthetic fragments can be introduced into protoplasts or into intact tissues or isolated cells. Preferably expression vectors are introduced into intact tissue. General methods of culturing plant tissues are provided for example by Maki *et al.,* (1993); and by Phillips *et al.* (1988). Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device. See, for example, Tomes et al. (1995). The vectors of the invention can not only be used for expression of structural genes but may also be used in exon-trap cloning, or promoter trap procedures to detect differential gene expression in varieties of tissues (Lindsey 1993; Auch & Reth 1990).

It is particularly preferred to use the binary type vectors of Ti and Ri plasmids of Agrobacterium spp. Ti-derived vectors transform a wide variety of higher plants, including monocotyledonous and dicotyledonous plants, such as soybean, cotton, rape, tobacco, and rice (Pacciotti 1985: Byrne 1987; Sukhapinda 1987; Lorz 1985; Potrykus, 1985; Park 1985: Hiei 1994). The use of T-DNA to transform plant cells has received extensive study and is amply described (EP 120516; Hoekema, 1985; Knauf, 1983; and An 1985). For introduction into plants, the chimeric genes of the invention can be inserted into binary vectors as described in the examples.

Other transformation methods are available to those skilled in the art, such as direct uptake of foreign DNA constructs (see EP 295959), techniques of electroporation (Fromm 1986) or high velocity ballistic bombardment with metal particles coated with the nucleic acid constructs (Kline 1987, and US 4,945,050). Once transformed, the cells can be regenerated by those skilled in the art.

Those skilled in the art will appreciate that the choice of method might depend on the type of monocotyledonous plant targeted for transformation. Suitable methods of transforming plant cells include, but are not limited to, microinjection (Crossway 1986), electroporation (Riggs 1986), Agrobacterium-mediated transformation, direct gene transfer (Paszkowski 1984), and ballistic particle acceleration using devices available from Agracetus, Inc., Madison, Wis. And BioRad, Hercules, Calif. (see, for example, US 4,945,050; and McCabe 1988). Also see, Datta 1990;(rice); Klein 1988 (maize); Klein 1988 (maize); Klein 1988 (maize); Fromm 1990 (maize); and Gordon-Kamm 1990 (maize); Koziel 1993 (maize); Shimamoto 1989 (rice); Christou 1991 (rice); European Patent Application EP 0 332 581 (orchardgrass and other Pooideae); Vasil 1993 (wheat); Weeks 1993 (wheat), Li 1993 and Christou 1995 (rice); Osjoda 1996 (maize via *Agrobacterium tumefaciens*), rice (Hiei 1994), and corn (Gordon-Kamm 1990; Fromm 1990).

*Agrobacterium tumefaciens* cells containing a vector comprising an expression cassette of the present invention, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous *Agrobacterium* vector systems useful in carrying out the present invention are known. Various *Agrobacterium* strains can be employed, preferably disarmed *Agrobacterium tumefaciens* or *rhizogenes* strains. In a preferred embodiment, *Agrobacterium* strains for use in the practice of the invention include octopine strains, *e.g.,* LBA4404 or agropine strains, *e.g.,* EHA101 or EHA105. Suitable strains of *A. tumefaciens* for DNA transfer are for example EHA101[pEHA101] (Hood 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are *Agrobacterium* tumefaciens C58, a nopaline strain. Other suitable strains are A. *tumefaciens* C58C1 (Van Larebeke 1974), A136 (Watson 1975) or LBA4011 (Klapwijk 1980). In another preferred embodiment the soil-borne bacterium is a disarmed variant of *Agrobacterium rhizogenes* strain K599 (NCPPB 2659). Preferably, these strains are comprising a disarmed plasmid variant of a Ti- or Ri-plasmid providing the functions required for T-DNA transfer into plant cells (*e.g.,* the vir genes). In a preferred embodiment, the *Agrobacterium* strain used to transform the plant tissue pre-cultured with the plant phenolic compound contains a L,L-succinamopine type Ti-plasmid, preferably disarmed, such as pEHA101. In another preferred embodiment, the *Agrobacterium* strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the virF gene be deleted or inactivated (Jarschow 1991).

The method of the invention can also be used in combination with particular *Agrobacterium* strains, to further increase the transformation efficiency, such as *Agrobacterium* strains wherein the vir gene expression and/or induction thereof is altered due to the presence of mutant or chimeric *virA* or *virG* genes (*e.g.* Hansen 1994; Chen and Winans 1991; Scheeren-Groot, 1994). Preferred are further combinations of *Agrobacterium* tumefaciens strain LBA4404 (Hiei 1994) with super-virulent plasmids. These are preferably pTOK246-based vectors (Ishida 1996).

A binary vector or any other vector can be modified by common DNA recombination techniques, multiplied in *E. coli,* and introduced into *Agrobacterium* by *e.g.,* electroporation or other transformation techniques (Mozo & Hooykaas 1991).

*Agrobacterium* is grown and used in a manner similar to that described in Ishida (1996). The vector comprising *Agrobacterium* strain may, for example, be grown for 3 days on YP medium (5 g/L yeast extract, 10 g/L peptone, 5 g/L NaCl, 15 g/L agar, pH 6.8) supplemented with the appropriate antibiotic (*e.g.,* 50 mg/L spectinomycin). Bacteria are collected with a loop from the solid medium and resuspended. In a preferred embodiment of the invention, *Agrobacterium* cultures are started by use of aliquots frozen at -80°C.

The transformation of the target tissue (*e.g.,* an immature embryo) by the *Agrobacterium* may be carried out by merely contacting the target tissue with the *Agrobacterium.* The concentration of *Agrobacterium* used for infection and co-cultivation may need to be varied. For example, a cell suspension of the *Agrobacterium* having a population density of approximately from $10^5$ - $10^{11}$, preferably $10^6$ to $10^{10}$, more preferably about $10^8$ cells or cfu / ml is prepared and the target tissue is immersed in this suspension for about 3 to 10 minutes. The resulting target tissue is then cultured on a solid medium for several days together with the *Agrobacterium.*

Preferably, the bacterium is employed in concentration of $10^6$ to 10’° cfu/mL. In a preferred embodiment for the co-cultivation step about 1 to 10 $\mu$l of a suspension of the soil-borne bacterium (*e.g., Agrobacteria*) in the co-cultivation medium are directly applied to each target tissue explant and air-dried. This is saving labor and time and is reducing unintended *Agrobacterium*-mediated damage by excess *Agrobacterium* usage.

For *Agrobacterium* treatment, the bacteria are resuspended in a plant compatible co-cultivation medium. Supplementation of the co-culture medium with antioxidants (*e.g.,* silver nitrate), phenol-absorbing compounds (like polyvinylpyrrolidone, Perl 1996) or thiol compounds (*e.g.,* dithiothreitol, L-cysteine, Olhoft 2001) which can decrease tissue necrosis due to plant defence responses (like phenolic oxidation) may further improve the efficiency of *Agrobacterium*-mediated transformation. In another preferred embodiment, the co-cultivation medium comprises at least one thiol compound, preferably selected from the group consisting of sodium thiolsulfate, dithiotrietol (DTT) and cysteine. Preferably the concentration is between about 1 mM and 10mM of L-Cysteine, 0.1 mM to 5 mM DTT, and/or 0.1 mM to 5 mM sodium thiolsulfate. Preferably, the medium employed during co-cultivation comprises from about 1 $\mu$M to about 10 $\mu$M of silver nitrate and from about 50 mg/L to about 1,000 mg/L of L-Cystein. This results in a highly reduced vulnerability of the target tissue against Agrobacterium-mediated damage (such as induced necrosis) and highly improves overall transformation efficiency.

Various vector systems can be used in combination with Agrobacteria. Preferred are binary vector systems. Common binary vectors are based on "broad host range"-plasmids like pRK252 (Bevan 1984) or pTJS75 (Watson 1985) derived from the P-type plasmid RK2. Most of these vectors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (*e.g.* pPZP; Hajdukiewicz 1994). Improved vector systems are described also in WO 02/00900.

Methods using either a form of direct gene transfer or *Agrobacterium*-mediated transfer usually, but not necessarily, are undertaken with a selectable marker, which may provide resistance to an antibiotic (*e.g.,* kanamycin, hygromycin or methotrexate) or a herbicide (*e.g.,* phosphinothricin). The choice of selectable marker for plant transformation is not, however, critical to the invention.

For certain plant species, different antibiotic or herbicide selection markers may be preferred. Selection markers used routinely in transformation include the nptII gene which confers resistance to kanamycin and related antibiotics (Messing & Vierra, 1982; Bevan 1983), the bar gene which confers resistance to the herbicide phosphinothricin (White 1990, Spencer 1990), the hph gene which confers resistance to the antibiotic hygromycin (Blochlinger & Diggelmann), and the dhfr gene, which confers resistance to methotrexate (Bourouis 1983).

### 5. Production and Characterization of Stably Transformed Plants

Transgenic plant cells are then placed in an appropriate selective medium for selection of transgenic cells, which are then grown to callus. Shoots are grown from callus. Plantlets are generated from the shoot by growing in rooting medium. The various constructs normally will be joined to a marker for selection in plant cells. Conveniently, the marker may be resistance to a biocide (particularly an antibiotic, such as kanamycin, G418, bleomycin, hygromycin, chloramphenicol, herbicide, or the like). The particular marker used will allow for selection of transformed cells as compared to cells lacking the DNA, which has been introduced. Components of DNA constructs including transcription cassettes of this invention may be prepared from sequences, which are native (endogenous) or foreign (exogenous) to the host. By "foreign" it is meant that the sequence is not found in the wild-type host into which the construct is introduced. Heterologous constructs will contain at least one region, which is not native to the gene from which the transcription-initiation-region is derived.

To confirm the presence of the transgenes in transgenic cells and plants, a variety of assays may be performed. Such assays include, for example, "molecular biological" assays well known to those of skill in the art, such as Southern and Northern blotting, in situ hybridization and nucleic acid-based amplification methods such as PCR or RT-PCR or TaqMan; "biochemical" assays, such as detecting the presence of a protein product, *e.g.,* by immunological means (ELISAs and Western blots) or by enzymatic function; plant part assays, such as seed assays; and also, by analyzing the phenotype of the whole regenerated plant, *e.g.,* for disease or pest resistance.

DNA may be isolated from cell lines or any plant parts to determine the presence of the preselected nucleic acid segment through the use of techniques well known to those skilled in the art. Note that intact sequences will not always be present, presumably due to rearrangement or deletion of sequences in the cell.

The presence of nucleic acid elements introduced through the methods of this invention may be determined by polymerase chain reaction (PCR). Using these technique discreet fragments of nucleic acid are amplified and detected by gel electrophoresis. This type of analysis permits one to determine whether a preselected nucleic acid segment is present in a stable transformant, but does not prove integration of the introduced preselected nucleic acid segment into the host cell genome. In addition, it is not possible using PCR techniques to determine whether transformants have exogenous genes introduced into different sites in the, genome, *i.e.*, whether transformants are of independent origin. It is contemplated that using PCR techniques it would be possible to clone fragments of the host genomic DNA adjacent to an introduced preselected DNA segment.

Positive proof of DNA integration into the host genome and the independent identities of transformants may be determined using the technique of Southern hybridization. Using this technique specific DNA sequences that were introduced into the host genome and flanking host DNA sequences can be identified. Hence the Southern hybridization pattern of a given transformant serves as an identifying characteristic of that transformant. In addition it is possible through Southern hybridization to demonstrate the presence of introduced preselected DNA segments in high molecular weight DNA, *i.e.,* confirm that the introduced preselected, DNA segment has been integrated into the host cell genome. The technique of Southern hybridization provides information that is obtained using PCR, *e.g.,* the presence of a preselected DNA segment, but also demonstrates integration into the genome and characterizes each individual transformant.

It is contemplated that using the techniques of dot or slot blot hybridization which are modifications of Southern hybridization techniques one could obtain the same information that is derived from PCR, *e.g.,* the presence of a preselected DNA segment.

Both PCR and Southern hybridization techniques can be used to demonstrate transmission of a preselected DNA segment to progeny. In most instances the characteristic Southern hybridization pattern for a given transformant will segregate in progeny as one or more Mendelian genes (Spencer 1992); Laursen 1994) indicating stable inheritance of the gene. The non-chimeric nature of the callus and the parental transformants ($R_0$) was suggested by germline transmission and the identical Southern blot hybridization patterns and intensities of the transforming DNA in callus, $R_0$ plants and $R_1$ progeny that segregated for the transformed gene.

Whereas DNA analysis techniques may be conducted using DNA isolated from any part of a plant, RNA may only be expressed in particular cells or tissue types and hence it will be necessary to prepare RNA for analysis from these tissues. PCR techniques may also be used for detection and quantitation of RNA produced from introduced preselected DNA segments. In this application of PCR it is first necessary to reverse transcribe RNA into DNA, using enzymes such as reverse transcriptase, and then through the use of conventional PCR techniques amplify the DNA. In most instances PCR techniques, while useful, will not demonstrate integrity of the RNA product. Further information about the nature of the RNA product may be obtained by Northern blotting. This technique will demonstrate the presence of an RNA species and give information about the integrity of that RNA. The presence or absence of an RNA species can also be determined using dot or slot blot Northern hybridizations. These techniques are modifications of Northern blotting and will only demonstrate the presence or absence of an RNA species.

While Southern blotting and PCR may be used to detect the preselected DNA segment in question, they do not provide information as to whether the preselected DNA segment is being expressed. Expression may be evaluated by specifically identifying the protein products of the introduced preselected DNA segments or evaluating the phenotypic changes brought about by their expression.

Assays for the production and identification of specific proteins may make use of physical-chemical, structural, functional, or other properties of the proteins. Unique physical-chemical or structural properties allow the proteins to be separated and identified by electrophoretic procedures, such as native or denaturing gel electrophoresis or isoelectric focusing, or by chromatographic techniques such as ion exchange or gel exclusion chromatography. The unique structures of individual proteins offer opportunities for use of specific antibodies to detect their presence in formats such as an ELISA assay. Combinations of approaches may be employed with even greater specificity such as Western blotting in which antibodies are used to locate individual gene products that have been separated by electrophoretic techniques. Additional techniques may be employed to absolutely confirm the identity of the product of interest such as evaluation by amino acid sequencing following purification. Although these are among the most commonly employed, other procedures may be additionally used.

Assay procedures may also be used to identify the expression of proteins by their functionality, especially the ability of enzymes to catalyze specific chemical reactions involving specific substrates and products. These reactions may be followed by providing and quantifying the loss of substrates or the generation of products of the reactions by physical or chemical procedures. Examples are as varied as the enzyme to be analyzed.

Very frequently the expression of a gene product is determined by evaluating the phenotypic results of its expression. These assays also may take many forms including but not limited to analyzing changes in the chemical composition, morphology, or physiological properties of the plant. Morphological changes may include greater stature or thicker stalks. Most often changes in response of plants or plant parts to imposed treatments are evaluated under carefully controlled conditions termed bioassays. Two or more generations can be grown to ensure that tissue-preferred expression of the desired phenotypic characteristic under conditions of interest is stably maintained and inherited.

**6. Uses of Transgenic Plants**

Once an expression cassette of the invention has been transformed into a particular plant species, it may be propagated in that species or moved into other varieties of the same species, particularly including commercial varieties, using traditional breeding techniques. Particularly preferred plants of the invention include the agronomically useful plants or agronomically important crops, in particular the cereal plants listed above, in particular, if said plants are monocotyl. The genetic properties engineered into the transgenic seeds and plants described above are passed on by sexual reproduction and can thus be maintained and propagated in progeny plants. The present invention also relates to a transgenic plant cell, tissue, organ, seed or plant part obtained from the transgenic plant. Also included within the invention are transgenic descendants of the plant as well as transgenic plant cells, tissues, organs, seeds and plant parts obtained from the descendants.

Preferably, the expression cassette in the transgenic plant is sexually transmitted. In one preferred embodiment, the coding sequence is sexually transmitted through a complete normal sexual cycle of the R0 plant to the R1 generation. Additionally preferred, the expression cassette is expressed in the cells, tissues, seeds or plant of a transgenic plant in an amount that is different than the amount in the cells, tissues, seeds or plant of a plant, which only differs in that the expression cassette is absent.

The transgenic plants produced herein are thus expected to be useful for a variety of commercial and research purposes. Transgenic plants can be created for use in traditional agriculture to possess traits beneficial to the grower (*e.g.,* agronomic traits such as resistance to water deficit, pest resistance, or increased yield), beneficial to the consumer of the grain harvested from the plant (*e.g.,* improved nutritive content in human food or animal feed; increased vitamin, amino acid, and antioxidant content; the production of antibodies (passive immunization) and nutriceuticals), or beneficial to the food processor (*e.g.,* improved processing traits). In such uses, the plants are generally grown for the use of their grain in human or animal foods. Additionally, the use of embryo-specific promoters in transgenic plants can provide beneficial traits that are localized in the consumable (by animals and humans) seeds of plants such as, but not excluding others, flour, cernels, nuts or seed oil. However, other parts of the plants, including stalks, husks, vegetative parts, and the like, may also have utility, including use as part of animal silage or for ornamental purposes. Often, chemical constituents (*e.g.,* oils or starches) of maize and other crops are extracted for foods or industrial use and transgenic plants may be created which have enhanced or modified levels of such components.

Transgenic plants may also find use in the commercial manufacture of proteins or other molecules, where the molecule of interest is extracted or purified from plant parts, seeds, and the like. Cells or tissue from the plants may also be cultured, grown in vitro, or fermented to manufacture such molecules. The transgenic plants may also be used in commercial breeding programs, or may be crossed or bred to plants of related crop species. Improvements encoded by the expression cassette may be transferred, *e.g.,* from maize cells to cells of other species, *e.g.,* by protoplast fusion.

The transgenic plants may have many uses in research or breeding, including creation of new mutant plants through insertional mutagenesis, in order to identify beneficial mutants that might later be created by traditional mutation and selection. An example would be the introduction of a recombinant DNA sequence encoding a transposable element that may be used for generating genetic variation. The methods of the invention may also be used to create plants having unique "signature sequences" or other marker sequences which can be used to identify proprietary lines or varieties.

Thus, the transgenic plants and seeds according to the invention can be used in plant breeding, which aims at the development of plants with improved properties conferred by the expression cassette, such as tolerance of drought, disease, or other stresses. The various breeding steps are characterized by well-defined human intervention such as selecting the lines to be crossed, directing pollination of the parental lines, or selecting appropriate descendant plants. Depending on the desired properties different breeding measures are taken. The relevant techniques are well known in the art and include but are not limited to hybridization, inbreeding, backcross breeding, multilane breeding, variety blend, interspecific hybridization, aneuploid techniques, etc. Hybridization techniques also include the sterilization of plants to yield male or female sterile plants by mechanical, chemical or biochemical means. Cross-pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines, which for example increase the effectiveness of conventional methods such as herbicide or pesticide treatment or allow dispensing with said methods due to their modified genetic properties. Alternatively new crops with improved stress tolerance can be obtained which, due to their optimized genetic "equipment", yield harvested product of better quality than products, which were not able to tolerate comparable adverse developmental conditions.

Accordingly, the present invention relates in a further embodiment to the use of the nucleic acid molecule of the present application for the expressing of a gene of interest preferentially or specifically in embryonic tissue or cells.

Further, in another embodiment, the present invention relates to the use of the nucleic acid molecule of the invention for increasing the transcription of a nucleic acid molecule in a plant under stress conditions.

In another embodiment, the present invention relates to a method for producing a plant with increased yield, and/or increased stress tolerance, and/or increased nutritional quality, and/or increased or modified oil content of a seed or sprout to the plant, wherein the method comprises the steps of

A) introducing into the plant the nucleic acid molecule of the present invention, the expression cassette of the present invention, or the expression vector of the present invention, wherein the nucleic acid molecule is operably linked to at least one nucleic acid molecule which sequence is heterologous in relation to said first or said second nucleic acid sequence and is capable to confer to the plant increased yield, and/or increased stress tolerance, increased nutritional quality, and/or increased or modified oil content to the plant; and

B) selecting transgenic plants, wherein the plants have increased yield and/or increased stress tolerance under stress conditions, and/or increased nutritional quality and/or increased or modified oil content of a seed or a sprout of the plants, as compared to the wild type or null segregant plants.

**Sequences**

| | |
|---|---|
| SEQ ID NO: 1 | Nucleic acid sequence encoding the transcription regulating nucleotide sequence of *Arabidopsis thaliana* Cor78 gene |
| SEQ ID NO: 2 | Nucleic acid sequence of pBPSMM368 binary vector |
| SEQ ID NO: 3 | Nucleic acid sequence encoding Os.BPSI.1 intron |
| SEQ ID NO: 4 | Nucleic acid sequence encoding Zm.ubiquitin intron |
| SEQ ID NO: 5 | Cor78 promoter forward primer<br>5'-gcaagaatct caaacacgga gatctca-3' |
| SEQ ID NO: 6 | Cor78 promoter reverse primer<br>5'-atttgtgagt aaaacagagg agggtctca-3' |
| SEQ ID NO: 7 | BPSI.1-5' primer<br>5'-cccgggcaccctgcggagggtaagatccgatcacc-3' |
| SEQ ID NO: 8 | BPSI.1-3' primer<br>5'-cggaccggtacatcttgcatctgcatgtac-3' |
| SEQ ID NO:9 | CCAF/CCA1.01 motif located from position 4-18 of At.cor78 gccgcaagaa tctca |
| SEQ ID NOs: 9-138 | as described in Table 9 |
| SEQ ID NOs: 139-144 | as described in Figures 4a to 4c and 5 |

## EXAMPLES

## and General Methods

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of *E. coli cells,* growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

For generating transgenic plants *Agrobacterium tumefaciens* (strain C58C1 [pMP90]) is transformed with the various promoter::GUS vector constructs (see below). Resulting *Agrobacterium* strains are subsequently employed to obtain transgenic plants. For this purpose an isolated transformed *Agrobacterium* colony is incubated in 4 mL culture (Medium: YEB medium with 50 μg/mL Kanamycin and 25 μg/mL Rifampicin) over night at 28°C. With this culture a 400 ml culture of the same medium is inoculated and incubated over night (28°C, 220 rpm). The bacteria are precipitated by centrifugation (GSA-Rotor, 8000 U/min, 20 min) and the pellet is resuspended in infiltration medium (1/2 MS-Medium; 0,5 g/L MES, pH 5,8; 50 g/L sucrose). The suspension is placed in a plant box (Duchefa) and 100 mL SILVET L-77 (Osi Special-ties Inc., Cat. P030196) are added to a final concentration of 0.02%. The plant box with 8 to 12 Plants is placed into a desiccator for 10 to 15 min. under vacuum with subsequent, spontaneous ventilation (expansion). This process is repeated 2-3 times. Thereafter all plants are transferred into pods with wet-soil and grown under long daytime conditions (16 h light; day temperature 22-24°C, night temperature 19°C; 65% relative humidity). Seeds are harvested after 6 weeks.

## EXAMPLE 1. Vector construction

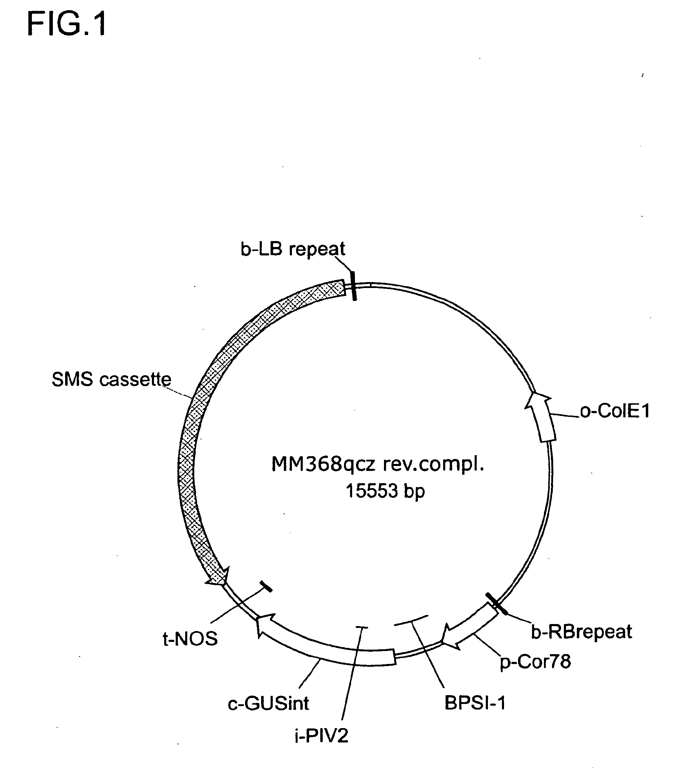

*Arabidopsis* cor78 promoter constructs were made containing with (pBPSMM368; Figure 1) or without BPSI.1 intron (pBPSMM250) (Table 6). The BPSI.1 intron was replaced with maize ubiquitin intron for the purpose of comparison (pBPSMM346). The intron-mediated enhancement (IME)-conferring intron such as BPSI.1 and Zm.ubiquitin intron were subcloned into the 5' untranslated region (UTR) of the expression cassette.

Table 6. GUS chimeric constructs

| Binary vector | Composition of the expression cassette (promoter::intron::reporter gene::terminator) |
|---|---|
| pBPSMM250 | At.cor78 promoter::GUS::NOS3' |
| pBPSMM346 | At.cor78 promoter::Zm.ubiquitin intron::GUS::NOS 3' |
| pBPSMM368 | At.cor78 promoter::BPSI.1 intron::GUS::NOS3' |

**EXAMPLE 2. Monocotyledonous plant transformation**

The *Agrobacterium*-mediated plant transformation using standard transformation and regeneration techniques may also be carried out for the purpose of transforming crop plants (Gelvin 1995; Glick 1993).

The transformation of maize or other monocotyledonous plants can be carried out using, for example, a technique described in US 5,591,616.

The transformation of plants using particle bombardment, polyethylene glycol-mediated DNA uptake or via the silicon carbonate fiber technique is described, for example, by Freeling & Walbot 1993.

**EXAMPLE 3. Detection of reporter gene expression**

To identify the characteristics of the promoter and the essential elements of the latter, which bring about its tissue specificity, it is necessary to place the promoter itself and various fragments thereof before what is known as a reporter gene, which allows the determination of the expression activity. An example, which may be mentioned, is the bacterial β-glucuronidase (Jefferson 1987a). The β-glucuronidase activity can be detected *in-planta* by means of a chromogenic substrate such as 5-bromo-4-chloro-3-indolyl-β-D-glucuronic acid in an activity staining (Jefferson 1987b). To study the tissue specificity, the plant tissue is cut, embedded, stained and analyzed as described (for example Bäumlein 1991b).

A second assay permits the quantitative determination of the GUS activity in the tissue studied. For the quantitative activity determination, MUG (4-methylumbelliferyl-β-D-glucuronide) is used as substrate for β-glucuronidase, and the MUG is cleaved into MU (methylumbelliferone) and glucuronic acid.

To do this, a protein extract of the desired tissue is first prepared and the substrate of GUS is then added to the extract. The substrate can be measured fluorimetrically only after the GUS has been reacted. Samples which are subsequently measured in a fluorimeter are taken at various points in time. This assay may be carried out for example with linseed embryos at various developmental stages (21, 24 or 30 days after flowering). To this end, in each case one embryo is ground into a powder in a 2 mL reaction vessel in liquid nitrogen with the aid of a vibration grinding mill (Type: Retsch MM 2000). After addition of 100 μL of EGL buffer (0.1 M $KPO_4$, pH 7.8; 1 mM EDTA; 5% glycerol; 1 M DTT), the mixture is centrifuged for 10 minutes at 25°C and 14,000 x g. The supernatant is removed and recentrifuged. Again, the supernatant is transferred to a new reaction vessel and kept on ice until further use. 25 μL of this protein extract are treated with 65 μL of EGL buffer (without DTT) and employed in the GUS assay. 10 μL of the substrate MUG (10 mM 4-methylumbelliferyl-β-D-glucuronide) are now added, the mixture is vortexed, and 30 μL are removed immediately as zero value and treated with 470 μL of Stop buffer (0.2 M $Na_2CO_3$). This procedure is repeated for all of the samples at an interval of 30 seconds. The samples taken were stored in the refrigerator until measured. Further readings were taken after 1 h and after 2 h. A calibration series which contained concentrations from 0.1 mM to 10 mM MU (4-methylumbelliferone) was established for the fluorimetric measurement. If the sample values were outside these concentrations, less protein extract was employed (10 μL, 1 μL, 1 μL from a 1:10 dilution), and shorter intervals were measured (0 h, 30 min, 1 h). The measurement was carried out at an excitation of 365 nm and an emission of 445 nm in a Fluoroscan II apparatus (Labsystem). As an alternative, the substrate cleavage can be monitored fluorimetrically under alkaline conditions (excitation at 365 nm, measurement of the emission at 455 nm; Spectro Fluorimeter BMG Polarstar+) as described in Bustos (1989). All the samples were subjected to a protein concentration determination by the method of Bradford (1976), thus allowing an identification of the promoter activity and promoter strength in various tissues and plants.

**EXAMPLE 4. Embryo-specific expression in maize**

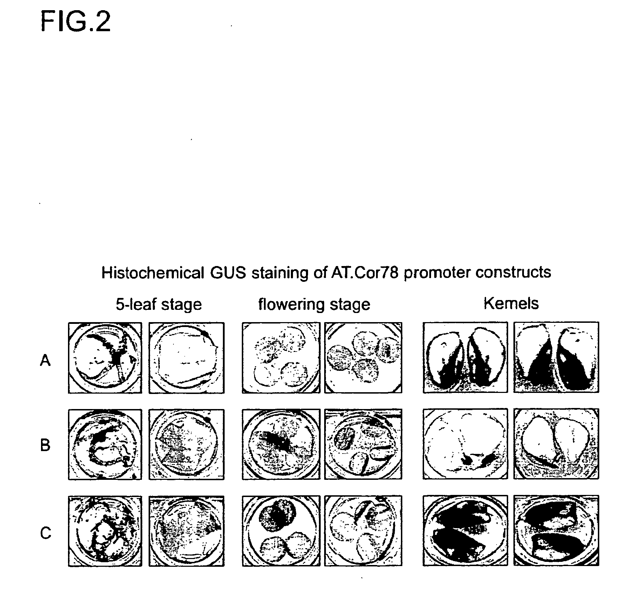

Construct pBPSMM368 was highly expressed in the embryo, especially in scutellum, while staining in the endosperm was almost undetectable using GUS histochemical assays (Figure 2). This strong embryo-specific expression was maintained during germination. Construct pBPSMM368 was not expressed in roots or leaves. However this embryo-specific expression was not detected in combination of At.cor78 promoter with Zm.ubiquitin intron construct (pBPSMM346). The transgenic maize plants transformed with pBPS346 showed constitutive, but overall very weak expression. Construct pBPSMM250 was not expressed in any tissue analyzed.

Table 7. GUS expression controlled by monocot constitutive promoter candidates

| Tissues/Developmental stages | Promoter (GUS expression levels) | | | |
|---|---|---|---|---|
| | pBPSMM23 2[1] | pBPSMM 250 | pBPSMM34 6 | pBPSMM36 8 |
| 3 days after co-cultivation | +++++ | ND | ND | - |
| Leaves at 5-leaf stage | +++++ | - | - | - |
| Roots at 5-leaf stage | +++++ | - | - | - |
| Leaves at flowering stage | +++++ | - | ++ | - |
| Stem | +++++ | - | ND | - |
| Pre-pollination | +++++ | - | ND | - |
| 20 days after pollination [DAP] | | | | |
| Scutellum | +++++ | - | ND | +++ |
| Embryo axis | +++++ | - | ND | +[2] |
| Endosperm | +++++ | - | ND | - |
| 30 DAP | | | | |
| Scutellum | +++++ | - | ++++ | ++++ |
| Embryo axis | +++++ | - | ++++ | +2 |
| Endosperm | +++++ | - | +++ | - |
| Dry seeds | | | | |
| Scutellum | ++++ | - | +++ | +++ |
| Embryo axis | ++++ | - | +++ | +[2] |
| Endosperm | ++++ | - | +++ | - |
| Imbibition/germination (72 hrs)[3] | | | | |
| Scutellum | +++++ | - | ++++ | ++++ |
| Embryo axis | +++++ | - | ++++ | +[2] |
| Endosperm | +++++ | - | ++++ | - |

[1]Positive controls as a constitutive promoter (pBPSMM232=Zm.ubiquitin promoter::Zm.ubiquitin intron:: GUS (PIV2)::NOS terminator; a range of GUS expression levels measured by histochemical assay (- to +++++), ND: not determined yet, [2]Transgenic plants containing pBPSMM368 showed weak expression in the first internodem node area of embryo axis, but almost no expression in other regions of the embryo axis, which comprised of primary root, plumule, stem, leaves, and coleoptile. [3]Three days (72 hrs) after imbibition (germination), the transgenic plants containing pBPSMM346 showed expression in radicle and the whole seed, but those containing pBPSMM368 showed expression restricted mostly in scutellum

**EXAMPLE 5. Drought-inducible expression**

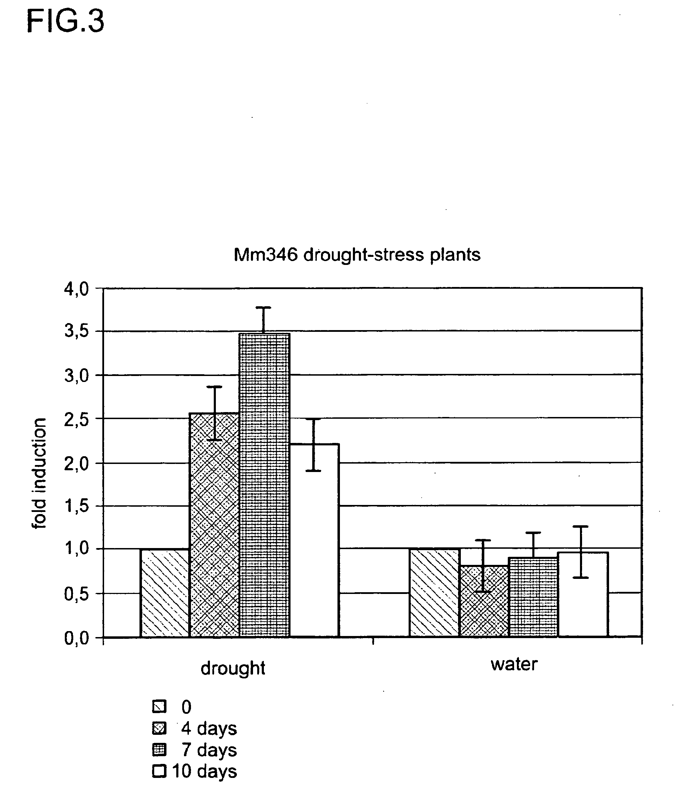

The transgenic maize plants containing pBPSMM250 (without intron-mediated enhancement conferring intron) did not show inducible expression under the drought stress (at 5 leaf stage, 4, 8, 13 days after withholding water). In order to enhance the expression of transgene, Zm.ubiquitin intron was added into the 5'UTR of At.cor78 promoter construct to generate pBPSMM346 construct. The transgenic maize plants containing pBPSMM346 at 5-leaf stage were used for drought stress. Drought-inducible expression was detected in T0 plants using quantitative RT-PCR. Based on this sensitive assay, approximately 3.5-fold induction was detected under the drought stress.Induction was up to 3.5-fold as determined with quantitative PCR (Figure 3).

**EXAMPLE 6. Utilization of transgenic crops**

A reporter gene in pBPSMM368 can be replaced with a gene of interest to express in an embryo-specific manner and confer tolerance to biotic and abiotic environmental stresses. The chimeric constructs are transformed into monocotyledonous plants. Standard methods for transformation in the art can be used if required. Transformed plants are regenerated using known methods. Various phenotypes are measured to determine improvement of biomass, yield, fatty acid composition, high oil, disease tolerance, or any other phenotypes that link yield enhancement or stability. Gene expression levels are determined at different stages of development and at different generations ($T_0$ to $T_2$ plants or further generations). Results of the evaluation in plants lead to determine appropriate genes in combination with this promoter to increase yield, improve disease tolerance, and/or improve abiotic stress tolerance.

**EXAMPLE 7. Expression of selectable marker gene in monocotyledonous plants**

A reporter gene in pBPSMM368 can be replaced with a selectable marker gene and transformed into monocotyledonous plants such as maize, wheat, rice, barley, rye, millet, sorghum, ryegrass or coix, triticale sugar cane, or oats, but is not restricted to these plant species. Standard methods for transformation in the art can be used if required. Transformed plants are selected under the selection agent of interest and regenerated using known methods. Selection scheme is examined at early developmental stages of tissues or tissue culture cells. Gene expression levels can be determined at different stages of development and at different generations ($T_0$ to $T_2$ plants or further generations). Results of the evaluation in plants lead to determine appropriate genes in combination with this promoter.

**EXAMPLE 8. Deletion analysis**

The cloning method is described by Rouster (1997) and Sambrook (1989). Detailed mapping of these promoters (*i.e.,* narrowing down of the nucleic acid segments relevant for its specificity) is performed by generating various reporter gene expression vectors which firstly contain the entire promoter region and secondly various fragments thereof. Firstly, the entire promoter region or fragments thereof are cloned into a binary vector containing GUS or other reporter gene. To this end, fragments are employed firstly, which are obtained by using restriction enzymes for the internal restriction cleavage sites in the full-length promoter sequence. Secondly, PCR fragments are employed which are provided with cleavage sites introduced by primers. The chimeric GUS constructs containing various deleted promoters are transformed into *Zea mays, Arabidopsis* and other plant species using transformation methods in the current art. Promoter activity is analyzed by using GUS histochemical assays or other appropriate methods in various tissues and organs at the different developmental stages. Modification of the promoter sequences can eliminate leakiness based on our needs.

**EXAMPLE 9. *In vivo* mutagenesis**

The skilled worker is familiar with a variety of methods for the modification of the promoter activity or identification of important promoter elements. One of these methods is based on random mutation followed by testing with reporter genes as described above. The *in vivo* mutagenesis of microorganisms can be achieved by passage of the plasmid (or of another vector) DNA through *E. coli* or other microorganisms (for example *Bacillus* spp. or yeasts such as *Saccharomyces cerevisiae*) in which the ability of maintaining the integrity of the genetic information is disrupted. Conventional mutator strains have mutations in the genes for the DNA repair system (for example mutHLS, mutD, mutT and the like; for reference, see Rupp 1996). The skilled worker is familiar with these strains. The use of these strains is illustrated for example by Greener (1994). The transfer of mutated DNA molecules into plants is preferably effected after selection and testing of the microoganisms. Transgenic plants are generated and analyzed as described above.

**EXAMPLE 10. Vector Construction for Overexpression and Gene "Knockout" Experiments**

**10.1 Overexpression**

Vectors used for expression of full-length "candidate genes" of interest in plants (overexpression) are designed to overexpress the protein of interest and are of two general types, biolistic and binary, depending on the plant transformation method to be used.

For biolistic transformation (biolistic vectors), the requirements are as follows:

1. a backbone with a bacterial selectable marker (typically, an antibiotic resistance gene) and origin of replication functional in Escherichia coli (*E. coli ; e.g.,* ColE1), and
2. a plant-specific portion consisting of:

a. a gene expression cassette consisting of a promoter (*e.g.* ZmUBlint MOD), the gene of interest (typically, a full-length cDNA) and a transcriptional terminator (*e.g., Agrobacterium tumefaciens* nos terminator);
b. a plant selectable marker cassette, consisting of a suitable promoter, selectable marker gene (*e.g.,* D-amino acid oxidase; *dao1*) and transcriptional terminator (eg. nos terminator).

Vectors designed for transformation by *Agrobacterium tumefaciens* (*A. tumefaciens;* binary vectors) consist of:

1. a backbone with a bacterial selectable marker functional in both *E. coli* and *A. tumefaciens* (*e.g.,* spectinomycin resistance mediated by the *aadA* gene) and two origins of replication, functional in each of aforementioned bacterial hosts, plus the *A. tumefaciens virG* gene;
2. a plant-specific portion as described for biolistic vectors above, except in this instance this portion is flanked by A. *tumefaciens* right and left border sequences which mediate transfer of the DNA flanked by these two sequences to the plant.

### 10.2 Gene Silencing Vectors

Vectors designed for reducing or abolishing expression of a single gene or of a family or related genes (gene silencing vectors) are also of two general types corresponding to the methodology used to downregulate gene expression: antisense or double-stranded RNA interference (dsR-NAi).

(a) Anti-sense

For antisense vectors, a full-length or partial gene fragment (typically, a portion of the cDNA) can be used in the same vectors described for full-length expression, as part of the gene expression cassette. For antisense-mediated down-regulation of gene expression, the coding region of the gene or gene fragment will be in the opposite orientation relative to the promoter; thus, mRNA will be made from the non-coding (antisense) strand in planta.

(b) dsRNAi

For dsRNAi vectors, a partial gene fragment (typically, 300 to 500 base pairs long) is used in the gene expression cassette, and is expressed in both the sense and antisense orientations, separated by a spacer region (typically, a plant intron, *e.g.* the OsSH1 intron 1, or a selectable marker, *e.g.* conferring kanamycin resistance). Vectors of this type are designed to form a double-stranded mRNA stem, resulting from the basepairing of the two complementary gene fragments *in planta.*
Biolistic or binary vectors designed for overexpression or knockout can vary in a number of different ways, including *e.g.* the selectable markers used in plant and bacteria, the transcriptional terminators used in the gene expression and plant selectable marker cassettes, and the methodologies used for cloning in gene or gene fragments of interest (typically, conventional restriction enzyme-mediated or Gateway™ recombinase-based cloning).

### EXAMPLE 11. Putative transcription factor binding site Analysis for At.Cor78 promoter (SEQ ID NO: 1)

Based on the below given Genomatixs results potential TATA box could be located in from base pair 790 to 804 of SEQ ID NO: 1.

Table 8 clusters of promoter elements identified in the At.cor78 promoter as described by SEQ ID NO: 1.

| **Family/matrix** | **Further Information** | **Opt.** | **Position from-to** | **Str.** | **Core sim.** | **Matrix sim.** | **Sequence** (red: ci-value > 60 capitals: core sequence) |
|---|---|---|---|---|---|---|---|
| P$AGP1/AGP1.01 | AG-motif binding protein 1 | 0.91 | 18 - 28 | (+) | 1.000 | 0.913 | ggaGATCtcaa |
| P$MYBL/ MYBPH3.0 2 | Myb-like protein of Petunia hybrida | 0.76 | 55 - 71 | (-) | 0.817 | 0.847 | gtaagtTTGTtttgagt |

(continued)

| Family/matrix | Further Information | Opt. | Position | Str. | Core sim. | Matrix sim. | Sequence (red: ci-value > 60 capitals: core sequence) |
|---|---|---|---|---|---|---|---|
| | | | from-to | | | | |
| P$MADS/AGL15.01 | AGL15, Arabidopsis MADS-domain protein AGAMOUS-like 15 | 0.79 | 66 - 86 | (+) | 0.775 | 0.792 | actTACGaaatttaggtagaa |
| P$MADS/AGL15.01 | AGL15, Arabidopsis MADS-domain protein AGAMOUS-like | 0.79 | 89 - 109 | (-) | 0.775 | 0.811 | aatTACAatataatgtatata |
| P$MADS/AGL15.01 | AGL15, Arabidopsis MADS-domain protein AGAMOUS-like | 0.79 | 90-110 | (+) | 0.775 | 0.823 | ataTACAttatattgtaattt |
| P$MYBL/ MYBPH3.0 2 | Myb-like protein of Petunia hybrida | 0.76 | 110 - 126 | (-) | 0.817 | 0.799 | aacattTTGTtacaaaa |
| P$SEF4/SEF4.01 | Soybean embryo factor 4 | 0.98 | 123 - 133 | (+) | 1.000 | 0.997 | tgTTTTtatta |
| P$AHBP/ HAHB4.01 | Sunflower homeo-domain leucine-zipper protein Hahb-4 | 0.87 | 127 - 137 | (+) | 1.000 | 0.923 | tttattATTAt |
| P$AHBP/ HAHB4.01 | Sunflower homeo-domain leucine-zipper protein Hahb-4 | 0.87 | 130 - 140 | (+) | 1.000 | 0.923 | attattATTAt |
| P$GTBX/SBF1.01 | SBF-1 | 0.87 | 147 - 163 | (+) | 1.000 | 0.894 | ttactggTTAAattaaa |
| P$GAPB/GAP.01 | Cis-element in the GAPDH promoters conferring light inducibilit | 0.88 | 161 - 175 | (+) | 1.000 | 0.950 | aaaaATGAatagaaa |
| P$DOFF/PBOX.01 | Prolamin box, con-served in cereal seed storage protein gene promoters | 0.75 | 166 - 182 | (+) | 1.000 | 0.789 | tgaatagaAAAGgtgaa |
| P$IBOX/IBOX.01 | I-Box in rbcS genes and other light regulated genes | 0.81 | 167 - 183 | (+) | 0.750 | 0.817 | gaataGAAAaggtgaat |
| P$NCS1/NCS1.01 | Nodulin consensus sequence 1 | 0.85 | 172 - 182 | (+) | 1.000 | 0.856 | gAAAAggtgaa |

(continued)

| Family/matrix | Further Information | Opt. | Position from-to | Str. | Core sim. | Matrix sim. | Sequence (red: ci-value > 60 capitals: core sequence) |
|---|---|---|---|---|---|---|---|
| P$OCSE/OCSL.01 | OCS-like elements | 0.69 | 195 - 215 | (-) | 0.769 | 0.704 | agaagaaaatgtttACCTcct |
| P$NCS1/NCS1.01 | Nodulin consensus sequence 1 | 0.85 | 200 - 210 | (-) | 0.878 | 0.853 | aAAATgtttac |
| P$MADS/SQUA.01 | MADS-box protein SQUAMOSA | 0.90 | 209 - 229 | (+) | 1.000 | 0.911 | ttcttctATTTtttcatattt |
| P$GAPB/GAP.01 | Cis-element in the GAPDH promoters conferring light inducibility | 0.88 | 215 - 229 | (-) | 1.000 | 0.892 | aaatATGAaaaaata |
| P$MYBS/ MYBST1.0 | MybSt1 (Myb Solanum tuberosum 1) with a single myb repeat | 0.90 | 226 - 242 | (-) | 1.000 | 0.945 | taatttATCCtgaaaat |
| P$IBOX/GATA.01 | Class I GATA factors | 0.93 | 229 - 245 | (+) | 1.000 | 0.956 | ttcagGATAaattattg |
| P$AHBP/ATHB1.01 | Arabidopsis thaliana homeo box protein 1 | 0.90 | 236 - 246 | (+) | 1.000 | 0.989 | taaATTAttgt |
| P$AHBP/ATHB5.01 | HDZip class I protein ATHB5 | 0.89 | 236 - 246 | (-) | 0.829 | 0.940 | acaATAAttta |
| P$GTBX/GT3A.01 | Trihelix DNA-binding factor GT-3a | 0.83 | 239 - 255 | (-) | 0.750 | 0.839 | taaactTTTAcaataat |
| P$NCS1/NCS1.01 | Nodulin consensus sequence 1 | 0.85 | 246 - 256 | (+) | 1.000 | 0.898 | tAAAAgtttac |
| P$PSRE/GAAA.01 | GAAA motif involved in pollen specific transcriptional activation | 0.83 | 253 - 269 | (-) | 1.000 | 0.836 | aaatgGAAAtcttgtaa |
| P$GTBX/S1F.01 | S1F, site 1 binding factor of spinach rps1 promoter | 0.79 | 255 - 271 | (-) | 1.000 | 0.794 | tcaaATGGaaatcttgt |
| P$WBXF/WRKY.01 | WRKY plant specific zinc-finger-type factor associated with pathogen defence, W box | 0.92 | 263 - 279 | (+) | 1.000 | 0.953 | tccatTTGActagtgta |

(continued)

| Family/matrix | Further Information | Opt. | Position from-to | Str. | Core sim. | Matrix sim. | Sequence (red: ci-value > 60 capitals: core sequence) |
|---|---|---|---|---|---|---|---|
| P$MYBS/ TAMYB80. 01 | MYB protein from wheat | 0.83 | 279 - 295 | (-) | 1.000 | 0.980 | gagaATATtcctcattt |
| P$MYBS/ TAMYB80. 01 | MYB protein from wheat | 0.83 | 284 - 300 | (+) | 1.000 | 0.941 | aggaATATtctctagta |
| P$AHBP/ HAHB4.01 | Sunflower homeo-domain leucine-zipper protein Hahb-4 | 0.87 | 300 - 310 | (+) | 1.000 | 0.920 | aagatcATTAt |
| P$LREM/ATCTA. 01 | Motif involved in carotenoid and tocopherol biosynthesis and in the expression of photosynthesis-related genes | 0.85 | 312 - 322 | (+) | 1.000 | 0.859 | tcATCTacttc |
| P$IBOX/GATA.01 | Class I GATA factors | 0.93 | 318 - 334 | (-) | 1.000 | 0.975 | tagaaGATAaaagaagt |
| P$HEAT/HSE.01 | Heat shock element | 0.81 | 319 - 333 | (-) | 1.000 | 0.858 | agaagataaaAGAAg |
| P$MADS/AGL1.01 | AGL1, Arabidopsis MADS-domain protein AGAMOUS-like 1 | 0.84 | 329 - 349 | (-) | 0.975 | 0.864 | ttaTTCCtctactggtagaag |
| P$MADS/AGL1.01 | AGL1, Arabidopsis MADS-domain protein AGAMOUS-like 1 | 0.84 | 330 - 350 | (+) | 0.995 | 0.867 | ttcTACCagtagaggaataaa |
| P$MYBS/ TAMYB80. 01 | MYB protein from wheat | 0.83 | 337 - 353 | (-) | 0.750 | 0.831 | ttgtTTATtcctctact |
| P$DOFF/PBOX.01 | Prolamin box, conserved in cereal seed storage protein gene promoters | 0.75 | 362 - 378 | (-) | 0.776 | 0.796 | tcctttgtAAATacaaa |
| P$GTBX/SBF1.01 | SBF-1 | 0.87 | 402 - 418 | (-) | 1.000 | 0.897 | cgtaaaaTTAAaattga |
| P$NACF/ TANAC69. 01 | Wheat NACdomain DNA binding factor | 0.68 | 409 - 431 | (-) | 1.000 | 0.723 | cttttattttaTACGtaaaatta |
| P$HMGF/HMG_IY. 01 | High mobility group I/Y-like proteins | 0.89 | 417 - 431 | (-) | 1.000 | 0.944 | ctttTATTttatac |

(continued)

| Family/matrix | Further Information | Opt. | Position from-to | Str. | Core sim. | Matrix sim. | Sequence (red: ci-value > 60 capitals: core sequence) |
|---|---|---|---|---|---|---|---|
| P$NCS1/NCS1.01 | Nodulin consensus sequence 1 | 0.85 | 426 - 436 | (+) | 1.000 | 0.969 | tAAAAgatcat |
| P$AHBP/WUS.01 | Homeodomain protein WUSCHEL | 0.94 | 448 - 458 | (-) | 1.000 | 0.963 | ctcctTAATcg |
| P$GTBX/GT1.01 | GT1-Box binding factors with a trihelix DNA-binding domain | 0.85 | 520 - 536 | (-) | 0.843 | 0.881 | catgtgGTCAttttacg |
| P$TCPF/ ATTCP20.01 | TCP class I transcription factor (Arabidopsis) | 0.94 | 534 - 546 | (-) | 1.000 | 0.956 | attgGCCCatcat |
| P$DREB/CRT_ DRE. 01 | C-repeat/ dehydration response element | 0.89 | 551 - 565 | (+) | 1.000 | 0.913 | atggaCCGActacta |
| P$MADS/AGL15.01 | AGL15, Arabidopsis MADS-domain protein AGAMOUS-like 15 | 0.79 | 557 - 577 | (-) | 1.000 | 0.887 | actTACTattattagtagtcg |
| P$MADS/AGL15.01 | AGL15, Arabidopsis MADS-domain protein AGAMOUS-like 15 | 0.79 | 558 - 578 | (+) | 1.000 | 0.896 | gacTACTaataatagtaagtt |
| P$AHBP/ HAHB4.01 | Sunflower homeodomain leucine-zipper protein Hahb-4 | 0.87 | 563 - 573 | (-) | 1.000 | 0.941 | actattATTAg |
| P$SPF1/SP8BF.01 | DNA-binding protein of sweet potato that binds to the Spa (ACTGTGTA) and SP8b (TACTATT) se-quences of sporamin and betamylase genes | 0.87 | 564 - 576 | (-) | 1.000 | 0.919 | ctTACTattatta |

(continued)

| Family/matrix | Further Information | Opt. | Position from-to | Str. | Core sim. | Matrix sim. | Sequence (red: ci-value > 60 capitals: core sequence) |
|---|---|---|---|---|---|---|---|
| P$GTBX/GT3A.0 | Trihelix DNA-binding factor GT-3a | 0.83 | 570 - 586 | (+) | 1.000 | 0.889 | tagtaaGTTAcatttta |
| P$EINL/TEIL.01 | TEIL (tobacco EIN3-like) | 0.92 | 574 - 582 | (-) | 1.000 | 0.933 | aTGTAactt |
| P$GTBX/S1F.01 | S1F, site 1 binding factor of spinach rps1 promoter | 0.79 | 585 - 601 | (+) | 1.000 | 0.792 | taggATGGaataaatat |
| P$DREB/CRT_ DRE. 01 | C-repeat/ dehydration response element | 0.89 | 601 - 615 | (+) | 1.000 | 0.940 | tcataCCGAcatcag |
| P$DOFF/PBF.01 | PBF(MPBF) | 0.97 | 617 - 633 | (+) | 1.000 | 0.979 | ttgaaagaAAAGggaaa |
| P$TEFB/TEF1.01 | TEF cis acting elements in both RNA polymerase II-dependent promoters and rDNA spacer sequences | 0.76 | 624 - 644 | (+) | 0.956 | 0.817 | gaaaaaa |
| P$NCS1/NCS1.01 | Nodulin consensus sequence 1 | 0.85 | 649 - 659 | (+) | 1.000 | 0.909 | tAAAAgatata |
| P$DREB/CRT_ DRE. 01 | C-repeat/ dehydration response element | 0.89 | 658 - 672 | (+) | 1.000 | 0.986 | tactaCCGAcatgag |
| P$DOFF/DOF3.01 | Dof3 - single zinc finger transcription factor | 0.99 | 671 - | (+) | 1.000 | 0.996 | agttccaaAAAGcaaaa |
| P$NCS1/NCS1.01 | Nodulin consensus sequence 1 | 0.85 | 688 - 698 | (+) | 1.000 | 0.966 | aAAAAgatcaa |
| P$DREB/CRT_ DRE. 01 | C-repeat/ dehydration response element | 0.89 | 695-709 | (+) | 1.000 | 0.903 | tcaagCCGAcacaga |
| P$CE3S/CE3.01 | Coupling element 3 (CE3), non-ACGT . ABRE | 0.77 | 703 - 721 | (-) | 1.000 | 0.853 | tctctaCGCGtgtctgtgt |

(continued)

| Family/matrix | Further Information | Opt. | Position from-to | Str. | Core sim. | Matrix sim. | Sequence (red: ci-value > 60 capitals: core sequence) |
|---|---|---|---|---|---|---|---|
| P$CGCG/ ATSR1.01 | Arabidopsis thaliana signal-responsive gene1, Ca2+/ calmodulin binding protein homolog to NtER1 (tobacco early ethylene-responsive gene) | 0.84 | 708 - 718 | (-) | 1.000 | 0.919 | ctaCGCGtgtc |
| P$CGCG/ ATSR1.01 | Arabidopsis thaliana signal-responsive gerie1, Ca2+/ calmodulin binding protein homolog to NtER1 (tobacco ealy ethylene-responsive gene) | 0.84 | 709 - 719 | (+) | 1.000 | 0.878 | acaCGCGtaga |
| P$GBOX/TGA1.01 | Arabidopsis leucine zipper protein TGA1 | 0.90 | 727 - 747 | (-) | 1.000 | 0.982 | gtggtgTGACgtcaaagtcat |
| P$GBOX/TGA1.01 | Arabidopsis leucine zipper protein TGA1 | 0.90 | 728 - 748 | (+) | 1.000 | 0.961 | tgacttTGACgtcacaccacg |
| P$WBXF/WRKY.01 | WRKY plant specific zinc-fnger-type factor associated with pathogen defence, W box | 0.92 | 728 - 744 | (+) | 1.000 | 0.934 | tgactTTGAcgtcacac |
| P$OPAQ/RITA1.01 | Rice transcription activator-1 (RITA), basic leucin zipper protein, highly expressed during seed development | 0.95 | 730 - 746 | (+) | 1.000 | 0.958 | actttgACGTcacacca |
| P$SALT/ALFIN1.01 | Zinc-finger protein in alfalfa roots, regulates salt tolerance | 0.93 | 738 - 752 | (-) | 1.000 | 0.948 | ttttcGTGGtgtgac |

(continued)

| Family/matrix | Further Information | Opt. | Position from-to | Str. | Core sim. | Matrix sim. | **Sequence** (red: ci-value > 60 capitals: core sequence) |
|---|---|---|---|---|---|---|---|
| P$GBOX/GBF1.01 | bZIP protein G-box binding factor 1 | 0.94 | 757 - 777 | (+) | 1.000 | 0.968 | cgcttcatACGTgtccctta |
| P$ABRE7ABRE.01 | ABA response elements | 0.82 | 758 - 774 | (+) | 1.000 | 0.971 | gcttcawcACGTgtccct |
| P$OCSE/OCSL.01 | OCS-like elements | 0.69 | 762 - 782 | (-) | 1.000 | 0.778 | agagataaagggacACGTatg |
| P$GAGA/GAGABP.0 1 | (GA)n/(CT)n binding proteins (GBP, soybean; BBR, barley) | 0.75 | 764 - 788 | (-) | 1.000 | 0.797 | actgagAGAGataaaggga-cacgta |
| P$IBOX/GATA.01 | Class I GATA factors | 0.93 | 768 - 784 | (-) | 1.000 | 0.939 | agagaGATAaagggaca |
| P$GAGA/GAGABP.0 1 ley) | (GA)n/(CT)n binding proteins (GBP, - soybean; BBR, barley) | .750 | 772 - 796 | (-) | 0.750 | 0.785 | atagagAGACtgagagaga-taaagg |
| P$GAGA/GAGABP.01 | (GA)n/(CT)n binding proteins (GBP, soybean; BBR, barley) | 0.75 | 774 - 798 | (-) | 1.000 | 0.786 | ttatagAGAGactgagagaga-taaa |
| P$GAGA/GAGABP.0 1 | (GA)n/(CT)n binding proteins (GBP, : soybean; BBR, barley) | 0.75 | 776 - 800 | (-) | 1.000 | 0.782 | gtttatAGAGagactgagaga-gata |
| P$TBPF/TATA.01 | Plant TATA box | 0.88 | 790 - 804 | (+) | 1.000 | 0.909 | tctcTATAaacttag |

Table 9 promoter motifs and core promoter motifs identified in At.cor78 (SEQ ID NO: 1) with SEQ ID NOs

| Promoter motif name | Position at At.cor78 | sequence | strand | SEQ ID NO: |
|---|---|---|---|---|
| AGP1/AGP1.01 motif | 18-28 | ggaGATCtcaa | (+) | 9 |
| AGP1/AGP1.01 core motif | | nnngatcnnn n | | 10 |
| MYBL/MYBPH3.02 motif1 | 55-71 | gtaagtTTGTtttgagt | (-) | 11 |
| MYBL/MYBPH3.02 motif2 | 110-126 | aacattTTGTtacaaaa | (-) | 18 |
| MYBL/MYBPH3.02 core motif | | nnnnnnttgt nnnnnnn | | 12 |
| MADS/AGL15.01 motif1 | 66-86 | actTACGaaatttaggtagaa | (+) | 13 |
| MADS/AGL15.01 core motif1 | | nnntacgnnn nnnnnnnnnn n | | 14 |
| MADS/AGL15.01 motif2 | 89-109 | aatTACAatataatgtatata | (-) | 15 |
| MADS/AGL15.01 motif3 | 90-110 | ataTACAttatattgtaattt | (+) | 16 |
| MADS/AGL15.01 core motif2 | | nnntacannn nnnnnnnnnn n | | 17 |

(continued)

| Promoter motif name | Position at At.cor78 | sequence | strand | SEQ ID NO: |
|---|---|---|---|---|
| MADS/AGL15.01 motif4 | 557-577 | actTACTattattagtagtcg | (-) | 89 |
| MADS/AGL15.01 core motif4 | | nnntactnnn nnnnnnnnnn n | | 90 |
| MADS/AGL15.01 motif5 | 558-578 | gacTACTaataatagtaagtt | (+) | 91 |
| SEF4/SEF4.01 motif | 123-133 | tgTTTTtatta | (+) | 19 |
| SEF4/SEF4.01 core motif | | nnttttnnnn n | | 20 |
| AHBP/HAHB4.01 motif1 | 127-137 | tttattATTAt | (+) | 21 |
| AHBP/HAHB4.01 core motif | | nnnnnnatta n | | 22 |
| AHBP/HAHB4.01 motif2 | 130-140 | attattATTAt | (+) | 23 |
| AHBP/HAHB4.01 motif3 | 300-310 | aagatcATTAt | (+) | 61 |
| AHBP/HAHB4.01 motif4 | 563-573 | actattATTAg | (-) | 92 |
| GTBX/SBF1.01 motif1 | 147-163 | ttactggTTAAattaaa | (+) | 24 |
| GTBX/SBF1.01 core motif | | nnnnnnntta annnnnn | | 25 |
| GTBX/SBF1.01 motif2 | 402-418 | cgtaaaaTTAAaattga | (-) | 75 |
| GAPB/GAP.01 motif1 | 161-175 | aaaaATGAatagaaa | (+) | 26 |
| GAPB/GAP.01 core motif | | nnnnatgann nnnnn | | 27 |
| GAPB/GAP.01 motif2 | 215-229 | aaatATGAaaaaata | (-) | 40 |
| DOFF/PBOX.01 motif1 | 166-182 | tgaatagaAAAGgtgaa | (+) | 28 |
| DOFF/PBOX.01 core motif1 | | nnnnnnnnaa agnnnnn | | 29 |
| DOFF/PBOX.01 motif2 | 362-378 | tcctttgtAAATacaaa | (+) | 73 |
| DOFF/PBOX.01 core motif2 | | nnnnnnnnaa atnnnnn | | 74 |
| IBOX/IBOX.01 motif | 167-183 | gaataGAAAaggtgaat | (+) | 30 |
| IBOX/IBOX.01 core motif | | nnnnngaaan nnnnnnn | | 31 |
| NCS1/NCS1.01 motif6 | 172-182 | gAAAAggtgaa | (+) | 32 |
| NCS1/NCS1.01 core motif1 | | naaaannnnn n | | 33 |
| NCS1/NCS1.01 motif1 | 246-256 | tAAAAgtttac | (+) | 51 |
| NCS1/NCS1.01 motif3 | 426-436 | tAAAAgatcat | (+) | 80 |
| NCS1/NCS1.01 motif4 | 649-659 | tAAAAgatata | (+) | 105 |
| NCS1/NCS1.01 motif5 | 688-698 | aAAAAgatcaa | (+) | 109 |
| NCS1/NCS1.01 motif2 | 200-210 | aAAATgtttac | (-) | 36 |
| NCS1/NCS1.01 core motif2 | | naaatnnnnn n | | 37 |
| OCSE/OCSL.01 motif1 | 195-215 | agaagaaaatgtttACCTcct | (-) | 34 |
| OCSE/OCSL.01 core motif1 | | nnnnnnnnnn nnnnacctnn n | | 35 |
| OCSE/OCSL.01 motif2 | 762-782 | agagataaagggacACGTatg | (-) | 128 |
| OCSE/OCSL.01 core motif2 | | nnnnnnnnnn nnnnacgtnn n | | 129 |
| MADS/SQUA.01 motif | 209-229 | ttcttctATTTtttcatattt | (+) | 38 |
| MADS/SQUA.01 core motif | | nnnnnnnatt tnnnnnnnnn n | | 39 |
| MYBS/MYBST1.01 motif | 226-242 | taatttATCCtgaaaat | (-) | 41 |

(continued)

| Promoter motif name | Position at At.cor78 | sequence | strand | SEQ ID NO: |
|---|---|---|---|---|
| MYBS/MYBST1.01 core motif | | nnnnnnatcc nnnnnnn | | 42 |
| IBOX/GATA.01 motif1 | 229-245 | ttcagGATAaattattg | (+) | 43 |
| IBOX/GATA.01 core motif | | nnnnngatan nnnnnnn | | 44 |
| IBOX/GATA.01 motif2 | 318-334 | tagaaGATAaaagaagt | (-) | 64 |
| IBOX/GATA.01 motif3 | 768-784 | agagaGATAaagggaca | (-) | 132 |
| AHBP/ATHB1.01 motif | 236-246 | taaATTAttgt | (+) | 45 |
| AHBP/ATHB1.01 core motif | | nnnattannn n | | 46 |
| AHBP/ATHB5.01 motif | 236-246 | acaATAAttta | (-) | 47 |
| AHBP/ATHB5.01 core motif | | nnnataannn n | | 48 |
| GTBX/GT3A.01 motif1 | 239-255 | taaactTTTAcaataat | (-) | 49 |
| GTBX/GT3A.01 core motif1 | | nnnnnnttta nnnnnnn | | 50 |
| GTBX/GT3A.01 motif2 | 570-586 | tagtaaGTTAcatttta | (+) | 95 |
| GTBX/GT3A.01 core motif2 | | nnnnnngtta nnnnnnn | | 96 |
| PSRE/GAAA.01 motif | 253-269 | aaatgGAAAtcttgtaa | (-) | 52 |
| PSRE/GAAA.01 core motif | | nnnnngaaan nnnnnnn | | 53 |
| GTBX/S1F.01 motif1 | 255-271 | tcaaATGGaaatcttgt | (-) | 54 |
| GTBX/S1F.01 core motif | | nnnnatggnn nnnnnnn | | 55 |
| GTBX/S1F.0 motif2 | 585-601 | taggATGGaataaatat | (+) | 99 |
| WBXF/WRKY.01 motif1 | 263-279 | tccatTTGActagtgta | (+) | 56 |
| WBXF/WRKY.01 core motif | | nnnnnttgan nnnnnnn | | 57 |
| WBXF/WRKY.01 motif2 | 728-744 | tgactTTGAcgtcacac | (+) | 119 |
| MYBS/TAMYB80.01 motif1 | 279-295 | gagaATATtcctcattt | (-) | 58 |
| MYBS/TAMYB80.01 core motif1 | | nnnnatatnn nnnnnnn | | 59 |
| MYBS/TAMYB80.01 motif2 | 284-300 | aggaATATtctctagta | (+) | 60 |
| MYBS/TAMYB80.01 motif3 | 337-353 | ttgtTTATtcctctact | (-) | 71 |
| MYBS/TAMYB80.01 core motif3 | | nnnnttatnn nnnnnnn | | 72 |
| LREM/ATCTA.01 motif | 312-322 | tcATCTacttc | (+) | 62 |
| LREM/ATCTA.01 core motif | | nnatctnnnn n | | 63 |
| HEAT/HSE.01 motif | 319-333 | agaagataaaAGAAg | (-) | 65 |
| HEAT/HSE.01 core motif | | nnnnnnnnnn agaan | | 66 |
| P$MADS/AGL1.01 motif | 329-349 | ttaTTCCtctactggtagaag | (-) | 67 |
| P$MADS/AGL1.01 core motif | | nnnttccnnn nnnnnnnnnn n | | 68 |
| MADS/AGL1.01 motif | 330-350 | ttcTACCagtagaggaataaa | (+) | 69 |
| MADS/AGL1.01 core motif | | nnntaccnnn nnnnnnnnnn n | | 70 |
| NACF/TANAC69.01 motif | 409-431 | cttttattttaTACGtaaaatta | (-) | 76 |

(continued)

| Promoter motif name | Position at At.cor78 | sequence | strand | SEQ ID NO: |
|---|---|---|---|---|
| NACF/TANAC69.01 core motif | | nnnnnnnnnn ntacgnnnnn nnn | | 77 |
| HMGF/HMG_IY.01 motif | 417-431 | ctttTATTttatacg | (-) | 78 |
| HMGF/HMG_IY.01 core motif | | nnnntattnn nnnnn | | 79 |
| AHBP/WUS.01 motif | 448-458 | ctcctTAATcg | (-) | 81 |
| AHBP/WUS.01 core motif | | nnnnntaatn n | | 82 |
| GTBX/GT1.01 motif | 520-536 | catgtgGTCAttttacg | (-) | 83 |
| GTBX/GT1.01 core motif | | nnnnnngtca nnnnnnn | | 84 |
| TCPF/ATTCP20.01 motif | 534-546 | attgGCCCatcat | (-) | 85 |
| TCPF/ATTCP20.01 core motif | | nnnngcccnn nnn | | 86 |
| DREB/CRT_DRE.01 motif1 | 551-565 | atggaCCGActacta | (+) | 87 |
| DREB/CRT_DRE.01 core motif | | nnnnnccgan nnnnn | | 88 |
| DREB/CRT_DRE.01 motif2 | 601-615 | tcataCCGAcatcag | (+) | 100 |
| DREB/CRT_DRE.01 motif3 | 658-672 | tactaCCGAcatgag | (+) | 106 |
| DREB/CRT_DRE.01 motif4 | 695-709 | tcaagCCGAcacaga | (+) | 110 |
| SPF1/SP8BF.01 motif | 564-576 | ctTACTattatta | (-) | 93 |
| SPF1/SP8BF.01 core motif | | nntactnnnn nnn | | 94 |
| EINL/TEIL.01 motif | 574-582 | aTGTAactt | (-) | 97 |
| EINL/TEIL.01 core motif | | ntgtannnn | | 98 |
| DOFF/PBF.01 motif | 617-633 | ttgaaagaAAAGggaaa | (+) | 101 |
| DOFF/PBF.01 core motif | | nnnnnnnnaa agnnnnn | | 102 |
| TEFB/TEF1.01 motif | 624-646 | aaAAGGgaaaaaaagaaaaaa | (+) | 103 |
| TEFB/TEF1.01 core motif | | nnaaggnnnn nnnnnnnnnn n | | 104 |
| DOFF/DOF3.01 motif | 671-687 | agttccaaAAAGcaaaa | (+) | 107 |
| DOFF/DOF3.01 core motif | | nnnnnnnnaa agnnnnn | | 108 |
| CE3S/CE3.01 motif | 703-721 | tctctaCGCGtgtctgtgt | (-) | 111 |
| CE3S/CE3.01 core motif | | nnnnnncgcg nnnnnnnnn | | 112 |
| CGCG/ATSR1.01 motif1 | 708-718 | ctaCGCGtgtc | (-) | 113 |
| CGCG/ATSR1.01 core motif | | nnncgcgnnn n | | 114 |
| CGCG/ATSR1.01 motif2 | 709-719 | acaCGCGtaga | (+) | 115 |
| GBOX/TGA1.01 motif1 | 727-747 | gtggtgTGACgtcaaagtcat | (-) | 116 |
| GBOX/TGA1.01 core motif | | nnnnnntgac nnnnnnnnnn n | | 117 |
| GBOX/TGA1.01 motif2 | 728-748 | tgacttTGACgtcacaccacg | (+) | 118 |
| OPAQ/RITA1.01 motif | 730-746 | actttgACGTcacacca | (+) | 120 |
| OPAQ/RITA1.01 core motif | | nnnnnnacgt nnnnnnn | | 121 |
| SALT/ALFIN1.01 motif | 738-752 | ttttcGTGGtgtgac | (-) | 122 |

(continued)

| Promoter motif name | Position at At.cor78 | sequence | strand | SEQ ID NO: |
|---|---|---|---|---|
| SALT/ALFIN1.01 core motif | | nnnnngtggn nnnnn | | 123 |
| GBOX/GBF1.01 motif | 757-777 | cgcttcatACGTgtcccttta | (+) | 124 |
| GBOX/GBF1.01 core motif | | nnnnnnnnac gtnnnnnnnn n | | 125 |
| ABRE/ABRE.01 motif | 758-774 | gcttcatACGTgtccct | (+) | 126 |
| ABRE/ABRE.01 motif | | nnnnnnnacg tnnnnnn | | 127 |
| GAGA/GAGABP.01 motif1 | 764-788 | actgagAGAGataaagggacacgt a | (-) | 130 |
| GAGA/GAGABP.01 core motif1 | | nnnnnnagag nnnnnnnnnn nnnnn | | 131 |
| GAGA/GAGABP.01 motif2 | 772-796 | atagagAGACtgagaga-gataaagg | (-) | 133 |
| GAGA/GAGABP.01 core motif2 | | nnnnnnagac nnnnnnnnnn nnnnn | | 138 |
| GAGA/GAGABP.01 motif3 | 774-798 | ttatagAGAGactgagagagataaa | (-) | 134 |
| GAGA/GAGABP.01 motif4 | 776-800 | gtttatAGAGagactgagagagata | (-) | 135 |
| TBPF/TATA.01 motif | 790-804 | tctcTATAaacttag | (+) | 136 |
| TBPF/TATA.01 core motif | | nnnntatann nnnnn | | 137 |

**EXAMPLE 12. Enhanced resistance against at least one stress factor, nutritional quality of** a **seed or a sprout, yield, or frequency of selection marker excision**

A reporter gene in pBPSMM368 can be replaced with

(1) abiotic stress resistance genes (14-3-3 protein & phosphoinositide-specific phospholipase C: WO0177355 and US6720477),
(2) genes involved in vitamin E biosynthesis (tyrosin aminotransferase (BT000782: WO02072848), putative porphobilinogen deaminase, putative omega-3 fatty acid desaturase [NM185577])
(3) biotic stress resistance genes (*Oryza sativa Fusarium* resistance protein I2C-5-like [NM194161], constitutive expressor of pathogenesis related genes 5 (cpr5: NM185577)),GTPase [WO03020939], Actin Depolymerization Factor 3 [WO2004035798], t-SNARE interactor of ROR2 and Syntaxin, interactor of SNAP34 [WO2004081217],
(4) homing endonuclease gene (for example a sequence encoding the homing endonuclease I-SceI)

to be expressed in embryo during germination, thereby improving - for example - tolerance to abiotic environmental stresses, early vigor resulting in potential yield enhancement, the amount of vitamin E, tolerance to biotic stresses and the frequency of marker excision. The chimeric constructs are transformed into monocotyledonous plants. Standard methods for transformation in the art can be used if required. Transformed plants are regenerated using known methods. Various phenotypes are measured to determine improvement of biomass, yield, fatty acid composition, high oil, disease tolerance, or any other phenotypes that indicate yield enhancement or yield stability. Gene expression levels are determined at different stages of development and in different generations (T0 to T2 plants or further generations). Results of the evaluation in plants lead to identification of appropriate genes in combination with this promoter that increase yield, improve disease tolerance, improve abiotic stress tolerance and/or increase nutritional quality of seed or sprout.

**EXAMPLE 13. Expression of transgene for improving feed, food, or yield traits in monocotyledonous plants**

A reporter gene in pBPSMM368 can be replaced with a gene of interest to overexpress mostly in embryo to improve nutrition value in embryo when transformed into monocotyledonous plants such as rice, barley, maize, wheat, or ryegrass, but is not restricted to these plant species. The gene of interest can be non-coding sequence (*e.g.* miRNA precursor, or ta-siRNA) to down-regulate one or more target genes. Standard methods for transformation in the art can be used if required. Transformed plants are selected under the selection agent of interest and regenerated using known methods. Selection scheme is examined at early developmental stages of tissues or tissue culture cells. Gene expression levels can be determined at different stages of development and at different generations (T0 to T2 plants or further generations). Results of the evaluation in plants lead to determine appropriate genes in combination with this promoter.

References

1. Abel et al., Science, 232:738 (1986).
2. Agrawal A et al. (1998) Nature 394(6695):744-451
3. Altschul et al., J. Mol. Biol., 215:403 (1990).
4. Altschul et al., Nucleic Acids Res., 25:3389 (1997).
5. Amara JF et al. (1997) Proc Natl Acad Sci USA 94(20):10618-1623
6. An et al., EMBO J., 4:277 (1985).
7. Angrand PO et al. (1998) Nucl. Acids Res. 26(13):3263-3269
8. Argast GM et al. (1998) J Mol Biol 280: 345-353
9. Auch & Reth, Nucleic Acids Research, 18:6743 (1990).
10. Ausubel et al. (1987) Current Protocols in Molecular Biology, Greene Publishing Assoc. and Wiley Interscience (1987).
11. Ballas et al., Nucleic Acids Res., 17:7891 (1989).
12. Barkai-Golan et al., Arch. Microbiol., 116:119 (1978).
13. Barker et al., (1983) Plant Molec. Biol. 2: 335-50.
14. Bartel D 2004, Cell 116, 281-297
15. Bartley and Scolnik (1994) Plant Physiol., 104:1469-1470
16. Batzer et al., Nucleic Acid Res., 19:5081 (1991).
17. Bäumlein et al. Mol Gen Genet 225:121-128 (1991)
18. Beall EL, Rio DC (1997) Genes Dev. 11(16):2137-2151
19. Beaudoin et al. 2000, PNAS 97:6421-6426
20. Becker et al. (1994) Plant J., 5:299-307,
21. Beerli RR et al. (1998) Proc Natl Acad Sci USA 95(25):14628-14633
22. Beerli RR et al. (2000) J Biol Chem 275(42):32617-32627
23. Beerli RR et al. (2000) Proc Natl Acad Sci U S A. 97 (4):1495-1500
24. Belfort M and Roberts RJ (1997) Nucleic Acids Res 25: 3379-3388
25. Bell-Pedersen et al. (1990) Nucleic Acids Res 18:3763-3770
26. Bernal-Lugo and Leopold, Plant Physiol., 98:1207 (1992).
27. Bevan et al., Nature, 304:184 (1983).
28. Bevan et al., Nucl. Acids Res., 11:369 (1983).
29. Bevan, Nucl. Acids Res., 12:8711 (1984).
30. Bibikova M et al. (2001) Mol Cell Biol. 21:289-297
31. Blackman et al., Plant Physiol., 100:225 (1992).
32. Blanc V et al. (1996) Biochimie 78(6):511-517
33. Blochlinger & Diggelmann, Mol Cell Biol, 4:2929 (1984).
34. Bol et al., Ann. Rev. Phytopath., 28:113 (1990).
35. Bouchez et al., EMBO J., 8:4197 (1989).
36. Bourouis et al., EMBO J., 2:1099 (1983).
37. Bowler et al., Ann. Rev. Plant Physiol., 43:83 (1992).
38. Bradford, Anal.Biochem. 72:248-254 (1976)
39. Branson and Guss, Proc. North Central Branch Entomological Society of America (1972).
40. Broakgert et al., Science, 245:110 (1989).
41. Broglie et al. (1991) Science 254:1194-1197
42. Bustos et al. (1989) Plant Gell 1:839-853
43. Byrne et al. Plant Cell Tissue and Organ Culture, 8:3 (1987).
44. Callis et al., Genes and Develop., 1:1183 (1987).
45. Campbell and Gowri, Plant Physiol., 92:1 (1990).
46. Campbell, ed. Ivermectin and Abamectin, Springer-Verlag, New York, (1989).
47. Cecchini E et al. (1998) Mutat Res 401(1-2):199-206
48. Chee et al. Plant Physiol., 91:1212 (1989).
49. Cheikh-N et al. (1994) Plant Physiol. 106(1):45-51
50. Chen and Winans J. Bacteriol. 173: 1139-1144 (1991).
51. Chen et al., (1988) EMBO J., 6:3559-3564

52. Christou et al. (1995) Annals of Botany 75:407-413
53. Christou et al. Proc. Natl. Acad. Sci USA, 86:7500 (1989).
54. Christou et al., Biotechnology, 9:957 (1991).
55. Christou et al., Plant Physiol., 87:671 (1988).
56. Chu et al. (1990) Proc Natl Acad Sci USA 87:3574-3578
57. Chui et al. Curr Biol 6:325-330 (1996).
58. Coe et al., In: Corn and Corn Improvement, Sprague et al. (eds.) pp. 81-258 (1988).
59. Corneille S et al. (2001) Plant J 27:171-178
60. Corpet et al. Nucleic Acids Res., 16:10881 (1988).
61. Cote V et al. (1993) Gene 129:69-76
62. Coxson et al., Biotropica, 24:121 (1992).
63. Crameri et al., Nature Biotech., 15:436 (1997).
64. Crameri et al., Nature, 391:288 (1998).
65. Crossway et al., BioTechniques, 4:320-334 (1986).
66. Cuozzo et al., Bio/Technology, 6:549 (1988).
67. Cutler et al., J. Plant Physiol., 135:351 (1989).
68. Czako M & Marton L (1994) Plant Physiol 104:1067-1071
69. Czapla and Lang, J. Econ. Entomol., 83:2480 (1990).
70. Dale EC and Ow DW (1991) Proc Natl Acad Sci USA 88:10558-10562
71. Datta et al., Bio/Technology, 8:736-740 (1990).
72. Davies et al., Plant Physiol., 93:588 (1990).
73. Dayhoff et al., Atlas of Protein Sequence and Structure, Natl. Biomed. Res. Found., Washington, C. D. (1978).
74. De Blaere et al., Meth. Enzymol., 143:277 (1987).
75. De Block et al. Plant Physiol., 91:694 (1989).
76. De Block et al., EMBO Journal, 6:2513 (1987).
77. Deak M et al. (1999) Nature Biotechnology 17:192-196
78. Deblaere et al. Nucl Acids Res 13:4777-4788 (1985)
79. Della-Cioppa et al. Bio/Technology 5:579-584 (1987)
80. Della-Cioppa et al., Plant Physiology, 84:965-968 (1987).
81. Dellaporta et al., in Chromosome Structure and Function, Plenum Press, 263-282 (1988).
82. Depicker AG et al. (1988) Plant Cell rep 104:1067-1071
83. Depicker et al., Plant Cell Reports, 7:63 (1988).
84. Dixon M & Kleppe Biochim. Biophys. Acta 96 (1965) 383-389
85. Dixon M & Kleppe K Biochim. Biophys. Acta 96 (1965) 368-382
86. Dixon M & Kleppe K. Biochim. Biophys. Acta 96 (1965) 357-367
87. Dunn et al., Can. J. Plant Sci., 61:583 (1981).
88. Dunwell JM (1998) Biotechn Genet Eng Rev 15:1-32
89. Dure et al., Plant Mol. Biol., 12:475 (1989).
90. Ebinuma et al. Proc Natl Acad Sci USA 94:2117-2121 (2000a).
91. Ebinuma et al. Selection of Marker-free transgenic plants using the oncogenes (ipt, rol A, B, C) of Agrobacterium as selectable markers, In Molecular Biology of Woody Plants. Kluwer Academic Publishers (2000b).
92. Eddy et al. (1991) Genes Dev. 5:1032-1041
93. Eichholtz et al. Somatic Cell and Molecular Genetics 13, 67-76 (1987).
94. Ellis et al., (1984) Mol. Gen. Genet. 195:466-73
95. Elroy-Stein et al., Proc. Natl. Acad. Sci. U.S.A., 86:6126 (1989).
96. English et al., Plant Cell, 8:179 (1996).
97. Erdmann et al., J. Gen. Microbiol., 138:363 (1992).
98. Erikson et al. Nat Biotechnol. 22(4):455-8 (2004).
99. Everett et al., Bio/Technology, 5:1201(1987).
100. Fedoroff & Smith Plant J 3:273-289 (1993).
101. Fedoroff NV & Smith DL (1993) Plant J 3:273-289
102. Fire et al. Nature 391:806-811 (1998).
103. Fitzpatrick, Gen. Engineering News, 22:7 (1993).
104. Fox et al. (1992) Plant Mol. Biol. 20:219-33
105. Fraley et al. Proc Natl Acad Sci USA 80:4803 (1983).
106. Freeling & Walbot (1993) "The maize handbook" ISBN 3-540-97826-7, Springer Verlag New York)
107. Fromm et al., Bio/Technology, 8:833-839 (1990).
108. Fromm et al., Nature (London), 319:791 (1986).

109. Gabler M et al. (2000) Enzyme Microb. Techno. 27:605-611
110. Galbiati et al. Funct. Integr Genozides, 20 1:25-34 (2000).
111. Gallego ME (1999) Plant Mol Biol 39(1):83-93
112. Gallie et al. Nucl Acids Res 15:8693-8711 (1987).
113. Gallie et al., Nucleic Acids Res., 15:3257 (1987).
114. Gallie et al., The Plant Cell, 1:301 (1989).
115. Gan et al., Science, 270:1986 (1995).
116. Gatehouse et al., J. Sci. Food Agric., 35:373 (1984).
117. Gelfand, eds., PCR Strategies Academic Press, New York (1995).
118. Gilmour et al. Plant Mol Biol 17 :1233-1240 (1991)
119. Gelvin et al., Plant Molecular Biology Manual, (1990).
120. Girke et al. (1998) Plant J 15:39-48
121. Gleave AP et al. (1999) Plant Mol Biol 40(2):223-235
122. Gleave et al. Plant Mol Biol. 40(2):223-35 (1999)
123. Gordon-Kamm et al., Plant Cell, 2:603 (1990).
124. Goring et al, PNAS, 88:1770 (1991).
125. Goyal RK et al. (2000) Crop Protection 19(5):307-312
126. Gruber, et al., Vectors for Plant Transformation, in: Methods in Plant Molecular Biology & Biotechnology" in Glich et al., (Eds. pp. 89-119, CRC Press, 1993).
127. Guerineau et al., Mol. Gen. Genet., 262:141 (1991).
128. Guerrero et al., Plant Mol. Biol., 15:11 (1990).
129. Guo et al. (1997) EMBO J 16: 6835- 6848
130. Guo et al. (2000) Science 289:452- 457
131. Gupta et al., PNAS, 90:1629 (1993).
132. Haines and Higgins (eds.), Nucleic Acid Hybridization, IRL Press, Oxford, U.K.
133. Hajdukiewicz et al., Plant Mol Biol 25:989-994 (1994).
134. Hammock et al., Nature, 344:458 (1990).
135. Hansen et al. Proc. Natl. Acad. Sci. USA 91:7603-7607 (1994).
136. Hare P & Chua NH (2002) Nat. Biotechnol. 20, 575-580.
137. Haren L et al. (1999) Annu Rev Microbiol. 1999;53:245-281
138. Haseloff et al. (1997) Proc Natl Acad Sci USA 94(6):2122-2127
139. Hayford et al. Plant Physiol. 86:1216 (1988)
140. Hemenway et al., EMBO Journal, 7:1273 (1988).
141. Henikoff & Henikoff, Proc. Natl. Acad. Sci. USA, 89:10915 (1989).
142. Hiei et al. Plant J 6: 271-282 (1994)
143. Higgins et al., Gene, 73:237 (1988).
144. Higo et al. (1999) Nucl Acids Res 27(1): 297-300
145. Hilder et al., Nature, 330:160 (1987).
146. Hille et al. Plant Mol. Biol. 7:171 (1986)
147. Hinchee et al. Bio/Technology 6:915 (1988).
148. Hoekema et al. Nature 303:179-181 (1983).
149. Hoekema, In: The Binary Plant Vector System. Offset-drukkerij Kanters B.V.; Alblasserdam (1985).
150. Hood EE and Jilka JM (1999) Curr Opin Biotechnol 10(4):382-6
151. Hood et al. (1999) Adv Exp Med Biol 464:127-47. Review
152. Hood et al. J Bacteriol 168:1291-1301 (1986).
153. Horvath et al (1993) Plant Physiol. 103:1047-1053
154. Huang B et al. (1996) J Protein Chem 15(5):481-9
155. Huang et al., CABIOS, 8:155 (1992).
156. Ikeda et al., J. Bacteriol., 169:5612 (1987).
157. Ikuta et al., Biotech., 8:241 (1990).
158. Imai T et al. (2001) Proc Natl Acad Sci USA 98(1):224-228)
159. Ingelbrecht et al., Plant Cell, 1:671 (1989).
160. Innis and Gelfand, eds., PCR Methods Manual (Academic Press, New York) (1999).
161. Innis et al., eds., PCR Protocols: A Guide to Methods and Applications (Academic Press, New York (1995).
162. Innis et al., PCR Protocols: A Guide to Methods and Applications, Academic Press, Inc., San Diego, Calif. (1990).
163. Ishida et al. Nature Biotech 745-750 (1996).
164. Janssen DB (1989) J Bacteriol 171(12):6791-9
165. Janssen DB et al. (1994) Annu Rev Microbiol 48:163-191

166. Jasin M (1996) Trends Genet. 12:224-228
167. Jefferson et al. (1987) EMBO J 6:3901-3907
168. Jefferson et al. EMBO J 6:3901-3907 (1987).
169. Jefferson et al. Plant Mol Biol Rep 5:387-405 (1987).
170. Jenes et al. Techniques for Gene Transfer, in: Recombinant Plants, Vol. 1, Engineering and Utilization, edited by SD Kung and R Wu, Academic Press, pp. 128-143 (1993)
171. Jicks GR and Raikhel NV (1995) Annu. Rev. Cell Biol. 11:155-188
172. Jobling et al., Nature, 325:622 (1987).
173. Johnson et al., PNAS USA, 86:9871 (1989).
174. Jones et al. Mol. Gen. Genet., 210:86 (1987).
175. Joshi et al., Nucleic Acid Res., 15:9627 (1987).
176. Kaasen et al., J. Bacteriol., 174:889 (1992).
177. Kang JS and Kim JS (2000) J Biol Chem 275(12):8742-8748
178. Karlin and Altschul, Proc. Natl. Acad Sci. USA, 87:2264 (1990).
179. Karlin and Altschul, Proc. Natl. Acad. Sci. USA, 90:5873 (1993).
180. Karlin-Neumannn GA et al. (1991) Plant Cell 3:573-582
181. Karsten et al., Botanica Marina, 35:11 (1992).
182. Kasuga et al., (1999) Nature Biotechnology 17(3):287-291
183. Kasuga M et al. (1999) Nature Biotech 17:276-286
184. Katz et al., J. Gen. Microbiol., 129:2703 (1983).
185. Kaufman PD and Rio DC (1992) Cell 69(1):27-39
186. Kawasaki et al. (1991) J Biol Chem 266:5342-5347
187. Keegstra (1989) Cell, 56(2):247-53
188. Keenan T et al. (1998) Bioorg Med Chem. 6(8):1309-1335
189. Keller et al., EMBO Journal, 8:1309 (1989).
190. Keller et al., Genes Dev., 3:1639 (1989)
191. Kilby NJ et al. (1995) Plant J 8:637-652
192. Kim JS et al. (1997) Proc Natl Acad Sci USA 94(8):3616-3620
193. Klapwijk et al. J. Bacteriol., 141,128-136 (1980).
194. Klein et al., Bio/Technoloy, 6:559-563 (1988).
195. Klein et al., Plant Physiol., 91:440-444 (1988).
196. Klein et al., Proc. Natl. Acad. Sci. USA, 85:4305-4309(1988).
197. Klug A (1999) J Mol Biol 293(2):215-218
198. Knauf, et al., Genetic Analysis of Host Range Expression by Agrobacterium In: Molecular Genetics of the Bacteria-Plant Interaction, Puhler, A. ed., Springer-Verlag, New York, 1983.
199. Kobayashi T et al. (1995) Jpn J Genet 70(3):409-422
200. Komro et al., (1985) Plant Mol. Biol. 4:253-63
201. Koncz & Schell Mol Gen Genet 204:383-396 (1986).
202. Kononowicz et al., (1992) Plant Cell 4:17-27.
203. Koprek et al. Plant J 19(6): 719-726 (1999).
204. Koprek T et al. (1999) Plant J 19(6):719-726
205. Koster and Leopold, Plant Physiol., 88:829 (1988).
206. Koziel et al., Biotechnology, 11:194 (1993).
207. Kridl etal., (1991) Seed Sci. Res., 1:209-219
208. Kuiper HA et al. (2001) Plant J. 27, 503-528
209. Kunkel et al., Methods in Enzymol., 154:367 (1987).
210. Kunkel, Proc. Natl. Acad. Sci. USA, 82:488 (1985).
211. Lam and Chua , J Biol Chem; 266(26):17131-17135 (1991).
212. Laufs et al., PNAS, 87:7752 (1990).
213. Lawton et al., Mol. Cell Biol., 7:335 (1987).
214. Lee and Saier, J. Bacteriol., 153 (1982).
215. Lee TJ et al. (2002) J Amer Soc Horticult Sci 127(2):158-164
216. Leffel et al. Biotechniques 23(5):912-8 (1997).
217. Leffel SM et al. (1997) Biotechniques 23(5):912-8
218. Lescot et al. Nucleic Acids Res 30(1):325-7 (2002).
219. Leung et al., (1991) Mol. Gen. Genet. 230:463-74
220. Levings, Science, 250:942 (1990).
221. Li et al. (1993) Plant Cell Reports 12:250-255

222. Li et al. Plant Mol Biol 20:1037-1048 (1992).
223. Lindsey et al., Transgenic Research, 2:3347 (1993).
224. Liu et al., Plant J. 8, 457-463 (1995)
225. Logie C and Stewart AF (1995) Proc Natl Acad Sci USA 92(13):5940-5944
226. Lois et al. (1998) Proc. Natl. Acad. Sci. USA, 95 (5):2105-2110
227. Lommel et al., Virology, 181:382 (1991).
228. Loomis et al., J. Expt. Zool., 252:9 (1989).
229. Lorz et al., Mol. Gen. Genet., 199:178 (1985).
230. Lyznik LA et al. (1996) Nucleic Acids Res 24:3784-3789
231. Ma et al., Nature, 334 :631 (1988).
232. Ma JK and Vine ND (1999) Curr Top Microbiol Immunol 236:275-92
233. Ma, Q. H. et al., (1998) Australian Journal of Plant Physiology 25(1):53-59
234. Macejak et al., Nature, 353:90 (1991).
235. Maki et al., Methods in Plant Mol. Biol. & Biotechnol, Glich et al., 67-88 CRC Press, (1993).
236. Maniatis et al., Molecular Cloning: A Laboratory Manual, Cold Spring Harbor Laboratory, Cold Spring Harbor (NY), (1989).
237. Mapp AK et al. (2000) Proc Natl Acad Sci USA 97(8):3930-3935
238. Mariani et al., Nature, 347:737 (1990).
239. Marshall (1991) Gene 104:241-245
240. Massey V et al. Biochim. Biophys. Acta 48 (1961) 1-9
241. Matzke et al. (2000) Plant Mol Biol 43:401-415 (2000).
242. McBride et al., PNAS USA, 91:7301 (1994).
243. McCabe et al., Bio/Technology, 6:923 (1988).
244. McKnight SL et al. (1980) Nucl Acids Res 8(24):5931-5948
245. McKnight SL et al. (1980) Nucl Acids Res 8(24):5949-5964
246. Meinkoth and Wahl, Anal. Biochem., 138:267 (1984).
247. Meister A & Wellner D Flavoprotein amino acid oxidase. In: Boyer, P.D., Lardy, H. and Myrbäck, K. (Eds.), The Enzymes, 2nd ed., vol. 7, Academic Press, New York, 1963, p. 609-648
248. Menard R et al. (1999) Phytochemistry 52:29-35
249. Messing and Vierra, Gene, 19:259 (1982).
250. Michael et al., J. Mol. Biol., 26 :585 (1990)
251. Michaelson et al. (1998) FEBS Letters 439:215-218
252. Michaelson et al. JBC 273:19055-19059
253. Millar et al. (1992) Plant Mol Biol Rep 10:324-414
254. Millar et al. Plant Mol Biol Rep 10:324-414 (1992).
255. Miyano M et al. (1991) J Biochem 109:171-177
256. Mogen et al., Plant Cell, 2:1261 (1990).
257. Mohr et al. (2000) Genes & Development 14:559-573
258. Monnat RJ Jr et al. (1999) Biochem Biophys Res Com 255:88-93
259. Moore et al., J. Mol. Biol., 272:336 (1997).
260. Mozo and Hooykaas, Plant Mol. Biol. 16:917-918 (1991).
261. Mullen CA et al. (1992) Proc Natl Acad Sci USA 89(1):33-37
262. Mundy and Chua, EMBO J., 7:2279 (1988).
263. Munroe et al., Gene, 91:151 (1990).
264. Murakami et al., Mol. Gen. Genet., 205:42 (1986).
265. Murata et al., FEBS Lett., 296:187 (1992).
266. Murdock et al., Phytochemistry, 29:85 (1990).
267. Murray et al., Nucleic Acids Res., 17:477 (1989).
268. Muthuswamy SK et al. (1999) Mol Cell Biol 19(10):6845-685
269. Myers and Miller, CABIOS, 4:11 (1988).
270. Naested H et al. (1999) Plant J 18(5)571-576
271. Naested, Plant J 18:571-576 (1999).
272. Napoli et al., Plant Cell, 2:279 (1990).
273. Nawrath et al. (1994) Proc. Natl. Acad. Sci. USA, 91:12760-12764
274. Needleman and Wunsch, J. Mol. Biol., 48:443-453 (1970).
275. Negri A et al. (1992) J Biol Chem. 267:11865-11871
276. Nehra et al. Plant J. 5:285-297 (1994)
277. Ni M et al. (1995) Plant J 7(4):661-676

278. Niedz et al., Plant Cell Reports, 14:403 (1995).
279. Nordin et al. Plant Mol Biol 21 :641-653 (1991)
280. Norris et al. (1998) Plant Physiol., 117:1317-1323
281. O'Keefe DP (1991) Biochemistry 30(2):447-55
282. O'Keefe DP et al. (1994) Plant Physiol 105:473-482
283. Odell et al., Mol. Gen. Genet., 113:369 (1990).
284. Odell et al., Nature, 313:810 (1985).
285. Ohtsuka et al., J. Biol. Chem., 260:2605 (1985).
286. Olhoft et al. Plant Cell Rep 20: 706-711 (2001).
287. Onouchi H et al. (1995) Mol Gen Genet 247:653-660
288. Osborne BI et al. (1995) Plant J. 7:687-701
289. Osjoda et al. (1996) Nature Biotechnology 14:745-750
290. Ow DW and Medberry SL (1995) Crit Rev in Plant Sci 14:239-261
291. Ow et al., Science, 234:856 (1986).
292. Pacciotti et al., Bio/Technology, 3:241 (1985).
293. Parizotto E A et al., 2004, Genes & Development, 18: 2237-2242
294. Park et al., J. Plant Biol., 38:365 (1985).
295. Paszkowski et al., EMBO J., 3:2717-2722 (1984).
296. Pearson and Lipman, Proc. Natl. Acad. Sci., 85:2444 (1988).
297. Pearson et al., Meth. Mol. Biol., 24:307 (1994).
298. Pei ZM et al. (1998) Science 282:287-290
299. Perera et al. Plant Mol. Biol 23(4): 793-799 (1993).
300. Perera RJ et al. (1993) Plant Mol Biol 23(4):793-799
301. Perlak et al., Proc. Natl. Acad. Sci. USA, 88:3324 (1991).
302. Phillips et al., In Corn & Corn Improvement, 3rd Edition 10 Sprague et al. (Eds. pp. 345-387)(1988).
303. Phi-Van et al., Mol. Cell. Biol., 10:2302 (1990).
304. Piatkowski et al., Plant Physiol., 94:1682 (1990).
305. Pilone MS (2000) Cell. Mol. Life. Sci. 57:1732-1740
306. Potrykus et al., Mol. Gen. Genet., 199:183 (1985).
307. Potrykus, Trends Biotech., 7:269 (1989).
308. Prasher et al., Biochem. Biophys. Res. Comm., 126:1259 (1985).
309. Preston et al. (1981) J Virol 38(2):593-605
310. Proudfoot, Cell, 64:671 (1991).
311. Rao et al. (2000) Prog Nucleic Acid Res Mol Biol 64:1-63
312. Rao et al. (1998) Plant J 15(4):469-77
313. Rask L et al. (2000) Plant Mol Biol 42:93-113
314. Reed et al., J. Gen. Microbiol., 130:1 (1984).
315. Reichel et al. (1996) Proc Natl Acad Sci USA 93(12):5888-5893
316. Riggs et al., Proc. Natl. Acad. Sci. USA, 83:5602-5606 (1986).
317. Risseeuw E (1997) Plant J 11 (4):717-728
318. Roeckel, P. et al., (1997) Transgenic Research 6(2):133-141
319. Rossolini et al., Mol. Cell. Probes, 8:91 (1994).
320. Ruiz, Plant Cell, 10:937 (1998).
321. Russell SH et al. (1992) Mol Gene Genet 234:49-59.
322. Saint Guily et al. (1992) Plant Physiol., 100(2):1069-1071
323. Sakuradani et al. (1999) Gene 238:445-453
324. Salomon S & Puchta H (1998) EMBO J 17(20):6086-6095
325. Sambrook et al., Molecular Cloning: A Laboratory Manual (2d ed., Cold Spring Harbor Laboratory Press, Plainview, N.Y.) (1989).
326. Sanfacon et al., Genes Dev., 5:141 (1991).
327. Sanford et al., Particulate Science and Technology, 5:27 (1987).
328. Sanger et al., (1990) Plant Mol. Biol. 14: 433-43
329. Sarguiel B et al. (1990) Nucleic Acids Res 18:5659-5665
330. Sarguiel B et al. (1991) Mol Gen Genet. 255:340-341
331. Sato et al. (2000) J. DNA Res., 7(1):31-63
332. Scheeren-Groot et al., J. Bacteriol 176: 6418-6426 (1994).
333. Schenborn and Groskreutz, Mol Biotechnol 13(1): 29-44 (1999).
334. Schenborn E, Groskreutz D. (1999) Mol Biotechnol 13(1):29-44

335. Schlaman and Hooykaas, Plant J 11:1377-1385 (1997).
336. Schlaman HRM & Hooykaas PFF (1997) Plant J 11:1377-1385
337. Schoffl et al. Mol Gen Genetics 217(2-3):246-53 (1989).
338. Schultz LW and Clardy J (1998) Bioorg Med Chem Lett. 8(1):1-6
339. Segal DJ and Barbas CF 3rd., Curr Opin Chem Biol (2000) 4(1):34-39
340. Serino G (1997) Plant J 12(3):697-701
341. Shagan et al., Plant Physiol., 101:1397 (1993).
342. Shah et al. Science 233: 478 (1986).
343. Shapiro, Mobile Genetic Elements, Academic Press, N.Y. (1983).
344. Sharrocks AD et al. (1997) Int J Biochem Cell Biol 29(12):1371-1387
345. Sheen et al. (1995) Plant J 8(5):777-784
346. Shimamoto et al., Nature, 338:274 (1989).
347. Silhavy et al., Experiments with Gene Fusions, Cold Spring Harbor Laboratory Press, Cold Spring Harbor (NY), (1984).
348. Skuzeski et al., Plant Molec. Biol. 15: 65-79 (1990).
349. Smith et al. (1997) Plant J., 11:83-92
350. Smith et al., Adv. Appl. Math., 2:482.(1981).
351. Smith et al., Mol. Gen. Genet., 224:447 (1990).
352. Spencer et al., Theor. Appl. Genet, 79:625 (1990).
353. St. Clair et al.(1987) Antimicrob Agents Chemother 31(6):844-849
354. Stalker et al., Science, 242:419 (1988).
355. Staub et al., EMBO J., 12:601 (1993).
356. Staub et al., Plant Cell, 4:39 (1992).
357. Steifel et al., The Plant Cell, 2:785 (1990).
358. Stemmer (1994) Nature 370:389-391
359. Stemmer (1994) Proc Natl Acad Sci USA 91:10747-10751
360. Stemmer, Nature, 370:389 (1994).
361. Stemmer, Proc. Natl. Acad. Sci. USA, 91:10747 (1994).
362. Stief et al., Nature, 341:343 (1989).
363. Stougaard J (1993) Plant J 3:755-761); EP-A1 595 837
364. Stougaard, Plant J 3:755-761 (1993)
365. Sugita Ket et al. (2000) Plant J. 22:461-469
366. Sukhapinda et al., Plant Mol. Biol., 8:209 (1987).
367. Sundaresan et al. Gene Develop 9: 1797-1810 (1995).
368. Sundaresan V et al. (1995) Gene Develop 9:1797-1810
369. Sutcliffe, PNAS USA, 75:3737 (1978).
370. Svab et al., Plant Mol. Biol. 14:197 (1990).
371. Svab et al., Proc. Natl. Acad. Sci. USA, 87:8526 (1990).
372. Svab et al., Proc. Natl. Acad. Sci. USA, 90:913 (1993).
373. Szybalski et al. (1991) Gene 100:13-26
374. Takahashi et al. (1998) Proc. Natl. Acad. Sci. USA, 95(17):9879-9884
375. Tarczynski et al., PNAS USA, 89:2600 (1992).
376. Teeri et al., (1989) EMBO J., 8: 343-50
377. Thillet et al., J. Biol. Chem., 263:12500 (1988).
378. Thompson et al., NAR 22(22):4673-4680 (1994).
379. Thykjaer T et al. (1997) Plant Mol Biol 35(4):523-530
380. Tian et al. (1997) Plant Cell Rep 16:267-271; WO 97/41228
381. Tijssen, Laboratory Techniques in Biochemistry and Molecular Biology-Hybridization with Nucleic Acid Probes, Elsevier, N.Y. (1993).
382. Tissier AF et al. (1999) Plant Cell 11:1841-1852
383. Tomes et al., Plant Cell, Tissue and Organ Culture: Fundamental Methods, Springer Verlag, Berlin (1995).
384. Tomic et al., NAR, 12:1656 (1990).
385. Tsai SY et al. (1998) Adv Drug Deliv Rev 30(1-3):23-31
386. Turmel M et al. (1993) J Mol Biol 232: 446-467
387. Turmel M et al. (1995a) Nucleic Acids Res 23:2519-2525
388. Turmel M et al. (1995b) Mol. Biol. Evol. 12, 533-545
389. Turner et al., Molecular Biotechnology, 3:225 (1995).
390. Twell et al., Plant Physiol., 91:1270 (1989).

391. Ugaki et al., Nucl. Acids Res., 19:371 (1991).
392. Ulmasov et al., Plant Mol. Biol., 35:417 (1997).
393. Umhau S. et al. (2000) Proc. Natl. Acad. Sci. USA 97:12463-12468
394. Upadhyaya NM et al. (2000) Plant Mol Biol Rep 18:227-223
395. Upender et al., Biotechniques, 18:29 (1995).
396. van der Krol et al., Plant Cell, 2:291 (1990).
397. Vanden Elzen et al. Plant Mol Biol. 5:299 (1985).
398. Vasil et al. Bio/Technology, 10:667-674 (1992).
399. Vasil et al. Bio/Technology, 11:1153-1158 (1993).
400. Vasil et al., Mol. Microbiol., 3:371 (1989).
401. Vasil et al., Plant Physiol., 91:1575 (1989).
402. Vernon and Bohnert, EMBO J., 11:2077 (1992).
403. Wagner et al. (1981) Proc Natl Acad Sci USA 78(3):1441-1445
404. Walker and Gaastra, eds., Techniques in Molecular Biology, MacMillan Publishing Company, New York (1983).
405. Wan & Lemaux Plant Physiol., 104:3748 (1994).
406. Wang et al., Mol. Cell. Biol., 12:3399 (1992).
407. Wang J et al. (1997) Nucleic Acids Res 25: 3767-3776
408. Waterman, Introduction to Computational Biology: Maps, sequences and genomes. Chapman & Hall. London (1995).
409. Watrud et al., in Engineered Organisms and the Environment (1985).
410. Watson et al. J. Bacteriol 123, 255-264 (1975)
411. Watson et al., Corn: Chemistry and Technology (1987).
412. Weeks et al. Plant Physiol 102:1077-1084 (1993)
413. Weissinger et al., Annual Rev. Genet., 22:421 (1988).
414. Wernette CM (1998) Biochemical & Biophysical Research Communications 248(1):127-333
415. White et al., Nucl Acids Res, 18, 1062 (1990).
416. Wigler M et al. (1977) Cell 11(1):223-232
417. Wingender et al. Nucleic Acids Res 29(1):281-3 (2001).
418. Wolter et al., EMBO Journal, 11:4685 (1992).
419. Wyn-Jones and Storey, Physiology and Biochemistry of Drought Resistance in Plants, Paleg et al. (eds.), pp. 171-204 (1981).
420. Xia et al. (1992) J. Gen. Microbiol., 138:1309-1316
421. Xiao YL and Peterson T (2000) Mol Gen Genet 263(1):22-29
422. Xiaohui Wang H et al. (2001) Gene 272(1-2): 249-255
423. Yamaguchi-Shinozaki et al., Plant Cell Physiol., 33:217 (1992).
424. Yurimoto H et al. (2000) Yeast 16:1217-1227
425. Zank et al. 2000, Biochemical Society Transactions 28:654-657
426. Zhang et al., Proc. Natl. Acad. Sci. USA, 94:4504 (1997).
427. Zhang L et al. (2000) J Biol Chem 275(43):33850-33860
428. Zinselmeier et al. (1995) Plant Physiol. 107(2):385-391
429. Zubko E et al. (2000) Nat Biotechnol 18:442-445
430. Zubko et al. (2000) Nature Biotech 18(4):442-445
431. Zukowsky et al., PNAS USA, 80:1101 (1983).

While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. An isolated nucleic acid molecule comprising a plant transcription regulating sequence, wherein the transcription regulating sequence comprises

   i) a first nucleic acid sequence comprising the promoter sequence of a drought, cold responsive and/or ABA regulated gene ("cor78 promoter").
   and operably linked thereto
   ii) a second nucleic acid sequence comprising the first intron of a plant gene encoding a Metallothionin 1 ("MET1 gene"),
   wherein the first nucleic acid sequence is selected from the group consisting of

      a) a polynucleotide sequence having at least 70% sequence identity to the polynucleotide of SEQ ID NO:1;
      b) a polynucleotide sequence having a fragment of at least 200 consecutive bases of the polynucleotide of SEQ ID NO:1;
      c) a polynucleotide sequence of a polynucleotide hybridizing under conditions equivalent to hybridization in 7% sodium dodecyl sulfate (SDS), 0.5M $NaPO_4$, 1mM EDTA at 50°C with washing in 0,5xSSC, 0.1% SDS at 50°C, to a nucleic acid comprising at least 50 nucleotides of a polynucleotide as defined in SEQ ID NO:1; and

d) a polynucleotide sequence which is the complement or reverse complement of any of the previously mentioned nucleotide sequences under a) to c), and
wherein the second nucleic acid sequence is selected from the group consisting of
e) a polynucleotide sequence having at least 60% sequence identity to the polynucleotide of SEQ ID NO:3;
f) a polynucleotide sequence having a fragment of at least 200 consecutive bases of the polynucleotide of SEQ ID NO:3; and
g) a polynucleotide sequence of a polynucleotide hybridizing under conditions equivalent to hybridization in 7% sodium dodecyl sulfate (SDS), 0.5M $NaPO_4$, 1mM EDTA at 50°C with washing in 1xSSC, 0.1% SDS at 50°C to a nucleic acid comprising at least 50 nucleotides of a sequence described by SEQ ID NO:3; and
h) a polynucleotide sequence which is the complement or reverse complement of any of the previously mentioned nucleotide sequences under a) to c),

wherein the isolated nucleic acid molecule comprising said first nucleic acid molecule as defined under a) to d) and said second nucleic acid molecule as defined under e) to h) is regulating embryo-specific expression of an operably linked nucleic acid sequence.

**2.** The nucleic acid molecule of claim 1 comprising a linker sequence of 10 bp to 100 bp which is located between the cor78 promoter sequence and the first nucleotide of said intron.

**3.** The nucleic acid molecule of any of claim 1 to 2, comprising a 5'UTR which is located between the cor78 promoter sequence and the first nucleotide of said intron.

**4.** The nucleic acid molecule of any one of claims 1 to 3, whereby said first intron of MET1 is located in the sequence of an intron of a nucleotide sequence transcribed under the control of the transcription regulating nucleotide sequence.

**5.** The nucleic acid molecule of any of claims 1 to 4, wherein the transcription regulating sequence regulates stress-inducible expression of an operably linked nucleic acid sequence.

**6.** An expression cassette comprising

i) the nucleic acid molecule of any of claims 1 to 5,
ii) and operably linked thereto one or more nucleic acid molecules.

**7.** An expression vector comprising the nucleic acid molecule of any of claims 1 to 5 or the expression cassette of claim 6.

**8.** A plant or plant cell comprising the nucleic acid molecule of any of claims 1 to 5, the expression cassette of claim 6, or the expression vector of claim 7.

**9.** A plant seed produced by the plant of claim 8, wherein the seed comprises the nucleic acid molecule of any of claims 1 to 5, the expression cassette of claim 6, or the expression vector of claim 7.

**10.** The plant or plant cell of claim 8 or plant seed of claim 9, wherein the plant is a monocotyledonous plant.

**11.** A method for excision of target sequences from a plant, said method comprising the steps of

A) constructing an expression cassette by operably linking the nucleic acid molecule of any of claims 1 to 5 to at least one nucleic acid molecule which is heterologous in relation to said first or said second nucleic acid sequence as **characterized in** any one of claims 1 to 5 and which is capable to confer the excision of a target sequence from a plant or plant cell, and
B) introducing said expression cassette stable or transient directly or indirectly into a plant cell or a plant comprising at least one target sequence excisable by the expression product of the nucleic acid molecule which is heterologous and, wherein said plant cell or plant expresses said nucleic acid sequence which is heterologous, and
C) selecting transgenic plants, which demonstrate excision of said target sequence.

**12.** The method of claim 11, whereby said nucleic acid molecule, which is heterologous and which is operably linked to said nucleic acid molecule of any of claims 1 to 5 is a nucleic acid molecule conferring the expression of a site specific recombinase.

**13.** The method of claim 12, whereby said nucleic acid molecule, which is heterologous and which is operably linked to said nucleic acid molecule of any of claims 1 to 5 is a nucleic acid molecule conferring expression a site-specific endonuclease capable to induce a DNA double strand break specific for the target sequence and wherein the target sequence to be deleted is flanked by sequences having an orientation, a sufficient length and a homology to each other to allow for homologous recombination between them.

**14.** A method for producing a plant with increased yield, and/or increased stress tolerance, and/or increased nutritional quality, and/or increased or modified oil content of a seed or sprout to the plant, wherein the method comprises the steps of

A) introducing into the plant the nucleic acid molecule of any of claims 1 to 5, the expression cassette of claim 6, or the expression vector of claim 7, wherein the nucleic acid molecule is operably linked to at least one nucleic acid molecule which sequence is heterologous in relation to said first or said second nucleic acid sequence and is capable to confer to the plant increased yield, and/or increased stress tolerance, increased nutritional quality, and/or increased or modified oil content to the plant; and
B) selecting transgenic plants, wherein the plants have increased yield and/or increased stress tolerance under stress conditions, and/or increased nutritional quality and/or increased or modified oil content of a seed or a sprout of the plants, as compared to the wild type or null segregant plants.

**15.** Use of the nucleic acid molecule of any of claims 1 to 5 for the expressing of a gene of interest preferentially or specifically in embryonic tissue or cells.

**16.** Use of the nucleic acid molecule of any of claims 1 to 5 for increasing the transcription of a nucleic acid molecule in a plant under stress conditions.

**Patentansprüche**

**1.** Isoliertes Nukleinsäuremolekül, das eine Pflanzentranskriptionsregulationssequenz umfasst, wobei die Transkriptionsregulationssequenz

i) eine erste Nukleinsäuresequenz, die die Promotersequenz eines auf Dürre oder Kälte reagierenden und/oder von ABA regulierten Gens ("cor78-Promoter") umfasst,
und in operativer Verknüpfung hiermit
ii) eine zweite Nukleinsäuresequenz, die das erste Intron eines für ein Metallothionin 1 codierenden Pflanzengens ("MET1-Gen") umfasst,
umfasst,
wobei die erste Nukleinsäuresequenz aus der Gruppe bestehend aus Folgendem ausgewählt ist:

a) einer Polynukleotidsequenz mit mindestens 70% Sequenzidentität zu dem Polynukleotid gemäß SEQ ID NO: 1;
b) einer Polynukleotidsequenz mit einem Fragment von mindestens 200 aufeinanderfolgenden Basen des Polynukleotids gemäß SEQ ID NO: 1;
c) einer Polynukleotidsequenz eines Polynukleotids, das unter Bedingungen entsprechend Hybridisierung in 7% Natriumdodecylsulfat (SDS), 0,5 M $NaPO_4$, 1 mM EDTA bei 50°C mit Waschen in 0,5 x SSC, 0,1% SDS bei 50°C mit einer Nukleinsäure umfassend mindestens 50 Nukleotide eines in SEQ ID NO: 1 definierten Polynukleotids hybridisiert; und
d) einer Polynukleotidsequenz, bei der es sich um das Komplement oder das reverse Komplement von einer der oben unter a) bis c) erwähnten Nukleotidsequenzen handelt, und
wobei die zweite Nukleinsäuresequenz aus der Gruppe bestehend aus Folgendem ausgewählt ist:
e) einer Polynukleotidsequenz mit mindestens 60% Sequenzidentität zu dem Polynukleotid gemäß SEQ ID NO: 3;
f) einer Polynukleotidsequenz mit einem Fragment von mindestens 200 aufeinanderfolgenden Basen des Polynukleotids gemäß SEQ ID NO: 3; und
g) einer Polynukleotidsequenz eines Polynukleotids, das unter Bedingungen entsprechend Hybridisierung in 7% Natriumdodecylsulfat (SDS), 0,5 M $NaPO_4$, 1 mM EDTA bei 50°C mit Waschen in 1 x SSC, 0,1% SDS bei 50°C mit einer Nukleinsäure umfassend mindestens 50 Nukleotide einer in SEQ ID NO: 3 definierten Sequenz hybridisiert; und

h) einer Polynukleotidsequenz, bei der es sich um das Komplement oder das reverse Komplement von einer der oben unter a) bis c) erwähnten Nukleotidsequenzen handelt, und wobei das isolierte Nukleinsäuremolekül, das das erste Nukleinsäuremolekül wie unter a) bis d) definiert und das zweite Nukleinsäuremolekül wie unter e) bis h) umfasst, die embryospezifische Expression einer operativ verknüpften Nukleinsäuresqeuenz reguliert.

**2.** Nukleinsäuremolekül nach Anspruch 1, das eine Linkersequenz von 10 Bp bis 100 Bp umfasst, die zwischen der cor78-Promotersequenz und dem ersten Nukleotid des Introns liegt.

**3.** Nukleinsäuremolekül nach einem der Ansprüche 1 bis 2, das eine 5'-UTR umfasst, die zwischen der cor78-Promotersequenz und dem ersten Nukleotid des Introns liegt.

**4.** Nukleinsäuremolekül nach einem der Ansprüche 1 bis 3, wobei das erste Intron von MET1 in der Sequenz eines Introns einer unter der Kontrolle der Transkriptionsregulationsnukleotidsequenz transkribierten Nukleotidsequenz liegt.

**5.** Nukleinsäuremolekül nach einem der Ansprüche 1 bis 4, wobei die Transkriptionsregulationssequenz die stressinduzierbare Expression einer operativ verknüpften Nukleinsäuresequenz reguliert.

**6.** Expressionskassette, die

i) das Nukleinsäuremolekül nach einem der Ansprüche 1 bis 5
ii) und in operativer Verknüpfung hiermit ein oder mehrere Nukleinsäuremoleküle umfasst.

**7.** Expressionsvektor, der das Nukleinsäuremolekül nach einem der Ansprüche 1 bis 5 oder die Expressionskassette nach Anspruch 6 umfasst.

**8.** Pflanze oder Pflanzenzelle, die das Nukleinsäuremolekül nach einem der Ansprüche 1 bis 5, die Expressionskassette nach Anspruch 6 oder den Expressionsvektor nach Anspruch 7 umfasst.

**9.** Von der Pflanze nach Anspruch 8 produzierter Pflanzensamen, wobei der Samen das Nukleinsäuremolekül nach einem der Ansprüche 1 bis 5, die Expressionskassette nach Anspruch 6 oder den Expressionsvektor nach Anspruch 7 umfasst.

**10.** Pflanze oder Pflanzenzelle nach Anspruch 8 oder Pflanzensamen nach Anspruch 9, wobei es sich bei der Pflanze um eine monokotyle Pflanze handelt.

**11.** Verfahren zum Herausschneiden von Zielsequenzen aus einer Pflanze, wobei das Verfahren die folgenden Schritte umfasst:

A) Konstruieren einer Expressionskassette durch operatives Verknüpfen des Nukleinsäuremoleküls nach einem der Ansprüche 1 bis 5 mit mindestens einem Nukleinsäuremolekül, das in Bezug auf die erste oder die zweite Nukleinsäuresequenz wie in einem der Ansprüche 1 bis 5 charakterisiert heterolog ist und das fähig ist, das Herausschneiden einer Zielsequenz aus einer Pflanze oder Pflanzenzelle zu vermitteln, und
B) stabiles oder transientes direktes oder indirektes Einführen der Expressionskassette in eine Pflanzenzelle oder Pflanze, die mindestens eine Zielsequenz umfasst, die durch das Expressionsprodukt des Nukleinsäuremoleküls, das heterolog ist, herausschneidbar ist, und wobei die Pflanzenzelle oder Pflanze die Nukleinsäuresequenz, die heterolog ist, exprimiert, und
C) Selektieren von transgenen Pflanzen, wodurch das Herausschneiden der Zielsequenz nachgewiesen wird.

**12.** Verfahren nach Anspruch 11, wobei es sich bei dem Nukleinsäuremolekül, das heterolog ist und das operativ mit dem Nukleinsäuremolekül nach einem der Ansprüche 1 bis 5 verknüpft ist, um ein Nukleinsäuremolekül, das die Expression einer ortsspezifischen Rekombinase vermittelt, handelt.

**13.** Verfahren nach Anspruch 12, wobei es sich bei dem Nukleinsäuremolekül, das heterolog ist und das operativ mit dem Nukleinsäuremolekül nach einem der Ansprüche 1 bis 5 verknüpft ist, um ein Nukleinsäuremolekül handelt, das die Expression einer ortsspezifischen Endonuklease vermittelt, die fähig ist, einen für die Zielsequenz spezifischen DNA-Doppelstrangbruch zu induzieren, und wobei die zu deletierende Zielsequenz von Sequenzen flankiert wird, die eine Orientierung, eine ausreichende Länge und eine Homologie zueinander aufweisen, die die homologe Rekombination zwischen ihnen gestattet.

**14.** Verfahren zur Herstellung einer Pflanze mit erhöhtem Ertrag und/oder erhöhter Stresstoleranz und/oder ernährungsphysiologischer Qualität und/oder erhöhtem oder modifiziertem Ölgehalt eines Samens oder Sprosses der Pflanze, wobei das Verfahren die folgenden Schritte umfasst:

A) Einführen des Nukleinsäuremoleküls nach einem der Ansprüche 1 bis 5, der Expressionskassette nach Anspruch 6 oder des Expressionsvektors nach Anspruch 7 in die Pflanze, wobei das Nukleinsäuremolekül mit mindestens einem Nukleinsäuremolekül operativ verknüpft ist, dessen Sequenz in Bezug auf die erste oder die zweite Nukleinsäuresequenz heterolog ist und fähig ist, der Pflanze erhöhten Ertrag und/oder erhöhte Stresstoleranz, erhöhte ernährungsphysiologische Qualität und/oder erhöhten oder modifizierten Ölgehalt der Pflanze zu vermitteln; und
B) Selektieren von transgenen Pflanzen, wobei die Pflanzen erhöhten Ertrag und/oder erhöhte Stresstoleranz unter Stressbedingungen und/oder erhöhte ernährungsphysiologische Qualität und/oder erhöhten oder modifizierten Ölgehalt eines Samens oder eines Sprosses der Pflanzen im Vergleich zu Wildtyppflanzen oder Nullsegreganten aufweisen.

**15.** Verwendung des Nukleinsäuremoleküls nach einem der Ansprüche 1 bis 5 für die Expression eines interessierenden Gens mit Bevorzugung oder Spezifität für Embryogewebe oder Embryozellen.

**16.** Verwendung des Nukleinsäuremoleküls nach einem der Ansprüche 1 bis 5 zur Erhöhung der Transkription eines Nukleinsäuremoleküls in einer Pflanze unter Stressbedingungen.

## Revendications

**1.** Molécule d'acide nucléique isolée comprenant une séquence régulatrice de transcription de plante,
la séquence régulatrice de transcription comprenant

i) une première séquence d'acide nucléique comprenant la séquence de promoteur d'un gène régulé par la sécheresse, le froid et/ou ABA (« promoteur cor78 »),
et fonctionnellement liée à celle-ci
ii) une deuxième séquence d'acide nucléique comprenant le premier intron d'un gène de plante codant pour la métallothionine 1 (« gène MET1 »),
la première séquence d'acide nucléique étant choisie dans le groupe constitué de

a) une séquence polynucléotidique ayant au moins 70 % d'identité de séquence avec le polynucléotide de SEQ ID NO: 1 ;
b) une séquence polynucléotidique ayant un fragment d'au moins 200 bases consécutives du polynucléotide de SEQ ID NO: 1 ;
c) une séquence polynucléotidique d'un polynucléotide s'hybridant dans des conditions équivalentes à l'hybridation dans du dodécylsulfate de sodium (SDS) à 7 %, $NaPO_4$ 0,5 M, EDTA 1 mM à 50 °C avec lavage dans 0,5xSSC, 0,1 % SDS à 50 °C, avec un acide nucléique comprenant au moins 50 nucléotides d'un polynucléotide tel que défini dans SEQ ID NO: 1 ; et
d) une séquence polynucléotidique qui est le complément ou le complément inverse de l'une quelconque des séquences nucléotidiques précédemment mentionnées dans a) à c), et
la deuxième séquence d'acide nucléique étant choisie dans le groupe constitué de
e) une séquence polynucléotidique ayant au moins 60 % d'identité de séquence avec le polynucléotide de SEQ ID NO: 3 ;
f) une séquence polynucléotidique ayant un fragment d'au moins 200 bases consécutives du polynucléotide de SEQ ID NO: 3 ; et
g) une séquence polynucléotidique d'un polynucléotide s'hybridant dans des conditions équivalentes à l'hybridation dans du dodécylsulfate de sodium (SDS) à 7 %, $NaPO_4$ 0,5 M, EDTA 1 mM à 50 °C avec lavage dans 1xSSC, 0,1 % SDS à 50 °C, avec un acide nucléique comprenant au moins 50 nucléotides d'une séquence décrite par SEQ ID NO: 3 ; et
h) une séquence polynucléotidique qui est le complément ou le complément inverse de l'une quelconque des séquences nucléotidiques précédemment mentionnées dans a) à c),

la molécule d'acide nucléique isolée comprenant ladite première molécule d'acide nucléique telle que définie dans a) à d) et ladite deuxième molécule d'acide nucléique telle que définie dans e) à h) régulant l'expression spécifique de l'embryon d'une séquence d'acide nucléique fonctionnellement liée.

**2.** Molécule d'acide nucléique de la revendication 1 comprenant une séquence de lieur de 10 pb à 100 pb qui est située entre la séquence de promoteur cor78 et le premier nucléotide dudit intron.

**3.** Molécule d'acide nucléique de l'une quelconque des revendications 1 à 2, comprenant un 5'UTR qui est situé entre la séquence de promoteur cor78 et le premier nucléotide dudit intron.

**4.** Molécule d'acide nucléique de l'une quelconque des revendications 1 à 3, **caractérisée en ce que** ledit premier intron de MET1 est situé dans la séquence d'un intron d'une séquence nucléotidique transcrite sous le contrôle de la séquence nucléotidique régulatrice de transcription.

**5.** Molécule d'acide nucléique de l'une quelconque des revendications 1 à 4, la séquence régulatrice de transcription régulant l'expression inductible par le stress d'une séquence d'acide nucléique fonctionnellement liée.

**6.** Cassette d'expression comprenant

i) la molécule d'acide nucléique de l'une quelconque des revendications 1 à 5,
ii) et fonctionnellement liées à celle-ci une ou plusieurs molécules d'acide nucléique.

**7.** Vecteur d'expression comprenant la molécule d'acide nucléique de l'une quelconque des revendications 1 à 5 ou la cassette d'expression de la revendication 6.

**8.** Plante ou cellule de plante comprenant la molécule d'acide nucléique de l'une quelconque des revendications 1 à 5, la cassette d'expression de la revendication 6, ou le vecteur d'expression de la revendication 7.

**9.** Semence de plante produite par la plante de la revendication 8, la semence comprenant la molécule d'acide nucléique de l'une quelconque des revendications 1 à 5, la cassette d'expression de la revendication 6, ou le vecteur d'expression de la revendication 7.

**10.** Plante ou cellule de plante de la revendication 8 ou semence de plante de la revendication 9, la plante étant une plante monocotylédone.

**11.** Procédé pour l'excision de séquences cibles à partir d'une plante, ledit procédé comprenant les étapes de

A) construction d'une cassette d'expression par liaison fonctionnelle de la molécule d'acide nucléique de l'une quelconque des revendications 1 à 5 à au moins une molécule d'acide nucléique qui est hétérologue vis-à-vis de ladite première ou ladite deuxième séquence d'acide nucléique comme caractérisé dans l'une quelconque des revendications 1 à 5 et qui est capable de conférer l'excision d'une séquence cible à partir d'une plante ou cellule de plante, et
B) introduction de ladite cassette d'expression directement ou indirectement stable ou transitoire dans une cellule de plante ou une plante comprenant au moins une séquence cible excisable par le produit d'expression de la molécule d'acide nucléique qui est hétérologue et, ladite cellule de plante ou plante exprimant ladite séquence d'acide nucléique qui est hétérologue, et
C) sélection de plantes transgéniques, qui présentent l'excision de ladite séquence cible.

**12.** Procédé de la revendication 11, ladite molécule d'acide nucléique, qui est hétérologue et qui est fonctionnellement liée à ladite molécule d'acide nucléique de l'une quelconque des revendications 1 à 5 étant une molécule d'acide nucléique conférant l'expression d'une recombinase site-spécifique.

**13.** Procédé de la revendication 12, ladite molécule d'acide nucléique, qui est hétérologue et qui est fonctionnellement liée à ladite molécule d'acide nucléique de l'une quelconque des revendications 1 à 5 étant une molécule d'acide nucléique conférant l'expression d'une endonucléase site-spécifique capable d'induire une rupture de double brin d'ADN spécifique pour la séquence cible et la séquence cible à déléter étant flanquée par des séquences ayant une orientation, une longueur suffisante et une homologie mutuelle pour permettre une recombinaison homologue entre celles-ci.

**14.** Procédé pour produire une plante ayant un rendement augmenté, et/ou une tolérance au stress augmentée, et/ou une qualité nutritionnelle augmentée, et/ou une teneur en huile augmentée ou modifiée d'une semence ou un germe de la plante, le procédé comprenant les étapes de

A) introduction dans la plante de la molécule d'acide nucléique de l'une quelconque des revendications 1 à 5, de la cassette d'expression de la revendication 6, ou du vecteur d'expression de la revendication 7, la molécule d'acide nucléique étant fonctionnellement liée à au moins une molécule d'acide nucléique dont la séquence est hétérologue vis-à-vis de ladite première ou ladite deuxième séquence d'acide nucléique et est capable de conférer à la plante un rendement augmenté, et/ou une tolérance au stress augmentée, une qualité nutritionnelle augmentée, et/ou une teneur en huile augmentée ou modifiée à la plante ; et
B) sélection de plantes transgéniques, les plantes ayant un rendement augmenté et/ou une tolérance au stress augmentée dans des conditions de stress, et/ou une qualité nutritionnelle augmentée et/ou une teneur en huile augmentée ou modifiée d'une semence ou un germe des plantes, par rapport à des plantes de type sauvage ou ségrégantes nulles.

**15.** Utilisation de la molécule d'acide nucléique de l'une quelconque des revendications 1 à 5 pour l'expression d'un gène d'intérêt de façon préférentielle ou spécifique dans du tissu ou des cellules embryonnaires.

**16.** Utilisation de la molécule d'acide nucléique de l'une quelconque des revendications 1 à 5 pour augmenter la transcription d'une molécule d'acide nucléique dans une plante dans des conditions de stress.

FIG.1

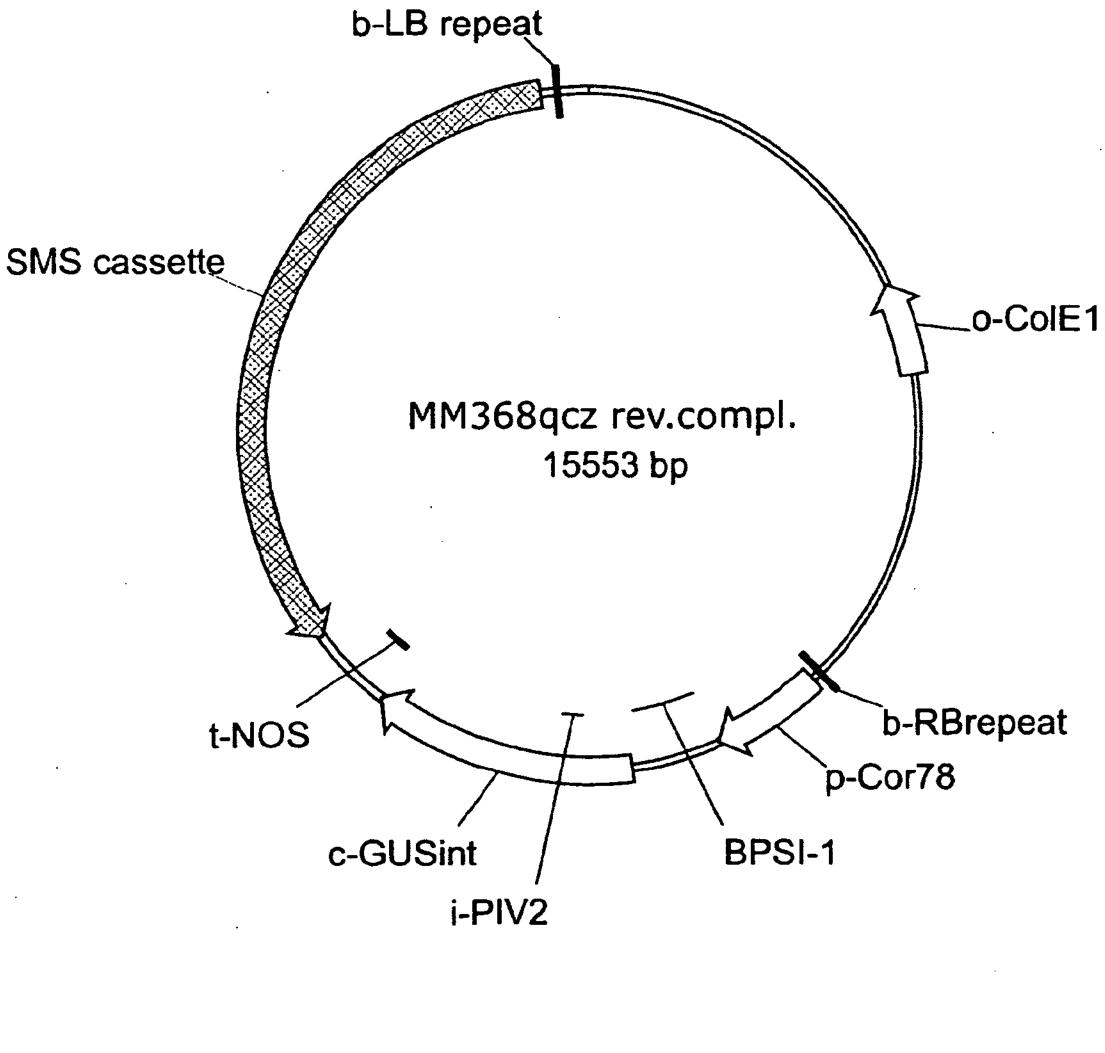
b-LB repeat
SMS cassette
o-ColE1
MM368qcz rev.compl.
15553 bp
t-NOS
b-RBrepeat
p-Cor78
c-GUSint
BPSI-1
i-PIV2

FIG.2

Histochemical GUS staining of AT.Cor78 promoter constructs

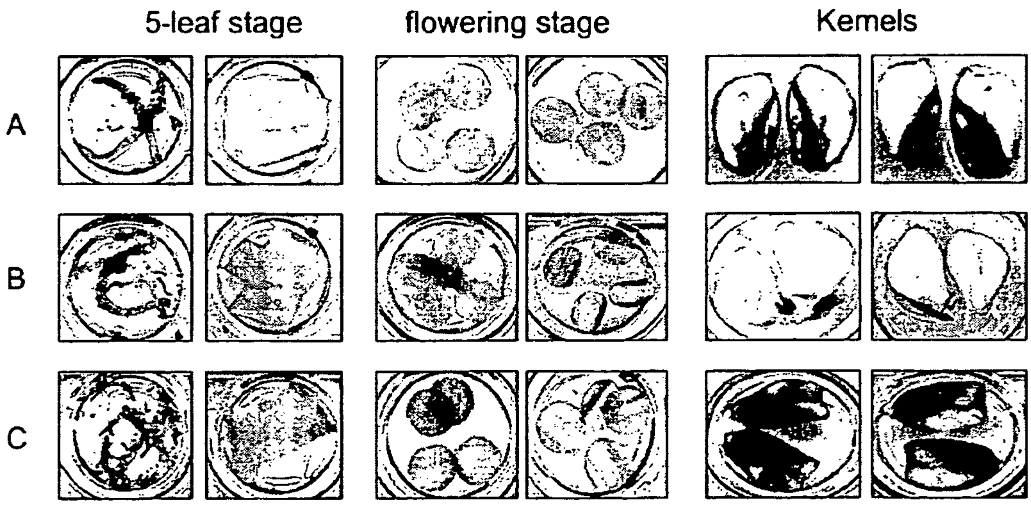

FIG.3

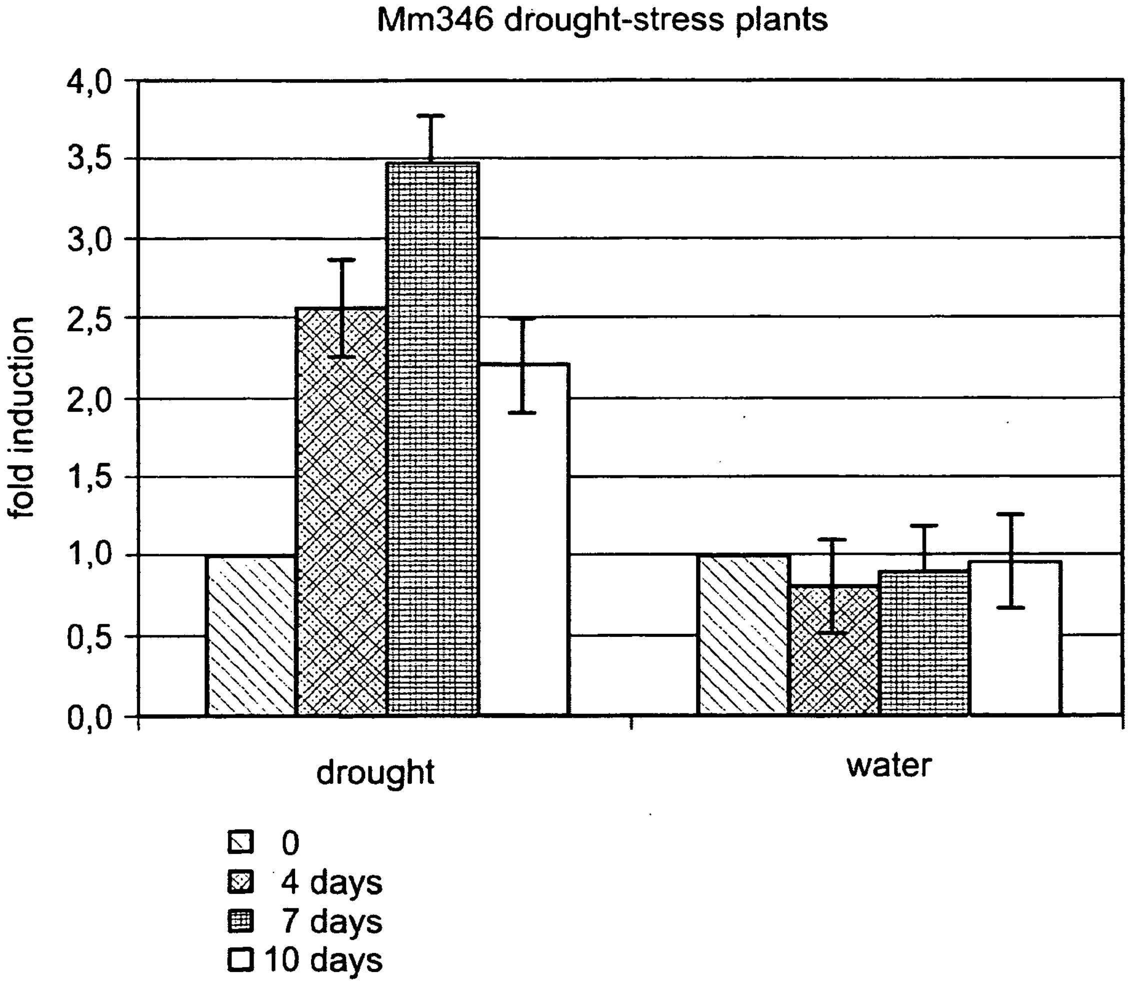
Mm346 drought-stress plants
fold induction
4,0
3,5
3,0
2,5
2,0
1,5
1,0
0,5
0,0
drought
water
0
4 days
7 days
10 days

# FIG.4A

## RD29A gene sequence

```
atggagtcac agttgacacg tccttatggt catgagcaag cagaagaacc aatcagaatt
caccatccag aagaagagca tcatgagaag ggagcatcca aagtgttgaa gaaagtaaaa
gaaaaggcta agaaaatcaa gaacagtctc actaaacatg gaaatggtca tgatcacgat
gtggaagatg atgatgatga gtatgacgag caagacccag aagttcacgg cgcaccagtg
tatgaatcct ctgccgtgag aggtggtgta acgggtaaac ctaagtctct tagtcatgcc
ggagaaacta atgttccggc atcggaggag attgttcctc cagggacaaa agtttttcct
gtcgtgtctt ctgaccacac caaacccatt gagcctgtat cattacaaga tacctcttac
ggacatgagg cactggctga tcctgtaaga acgacggaaa catcggactg ggaagcgaaa
agagaggcac cgactcatta tcctctcgga gtgtcagaat tttcagacag aggagagagc
agagaggctc atcaagagcc attgaacact cctgtgtctc tgctttcagc aacagaggac
gtgactagga cgtttgctcc tggtggtgaa gatgactatc tcggtggtca acggaaagtc
aacgtcgaga cgccaaaacg tttggaggaa gatccggctg ctccaggagg aggatcggat
tatctcagtg gtgtatctaa ttatcagtcc aaagttactg atcccacgca taaaggtgga
gaagctggag taccagagat tgctgagtct cttggtagaa tgaaagtgac tgatgagtct
cctgatcaga aatcaagaca aggacgcgaa gaagactttc cgacgagaag ccatgagttt
gatctgaaga aggaatctga tatcaacaag aattctccgg caagatttgg aggggaatca
aaagctggga tggaggaaga ttttccgaca agaggtgatg tgaaagtaga gagtggattg
ggaagagact taccgacggg aactcatgat cagttctcac cagaactatc tcgtcccaaa
gagagagatg attctgagga aaccaaagat gagtcgacac atgagacaaa accaagcacc
tacacagagc agttagcttc agctacatca gccataacta acaaagctat agccgcaaag
aacgtcgttg cctcaaagct aggttacacc ggagagaatg gcggcgggca aagcgagagc
cctgtaaaag atgaaactcc gagatctgtt actgcttacg ggcagaaagt ggcgggaact
gttgctgaga agttgactcc ggtttacgaa aaagtcaaag aaacaggatc aacggtgatg
acaaagctac ctctctccgg aggtggaagt ggagtgaagg agacgcaaca aggggaagag
aaaggtgtga cggctaaaaa ttatatatca gagaagctga aacctggaga agaggacaaa
gctttatcgg aaatgatagc tgagaaactt cattttggag gaggaggaga gaagaagaca
acggctacaa aggaggtgga agtgacggtt gagaagatac cttccgacca gatagcggag
gggaaaggac atggtgaggc ggttgcagag gaaggaaaag gtggagaagg aatggtgggg
aaagttaaag gagcggtcac ttcttggctc ggtggtaaac cgaagtcgcc acggtccgtt
gaagagtctc cacaatcact tggcaccacc gttgggacta tggggttttc ggattccggt
```

## Translation: RD29A protein sequence

MESQLTRPYGHEQAEEPIRIHHPEEEHHEKGASKVLKKVKEKAK

KIKNSLTKHGNGHDHDVEDDDDEYDEQDPEVHGAPVYESSAVRGGVTGKPKSLSHAGE

TNVPASEEIVPPGTKVFPVVSSDHTKPIEPVSLQDTSYGHEALADPVRTTETSDWEAK

REAPTHYPLGVSEFSDRGESREAHQEPLNTPVSLLSATEDVTRTFAPGGEDDYLGGQR

KVNVETPKRLEEDPAAPGGGSDYLSGVSNYQSKVTDPTHKGGEAGVPEIAESLGRMKV

TDESPDQKSRQGREEDFPTRSHEFDLKKESDINKNSPARFGGESKAGMEEDFPTRGDV

KVESGLGRDLPTGTHDQFSPELSRPKERDDSEETKDESTHETKPSTYTEQLASATSAI

TNKAIAAKNVVASKLGYTGENGGGQSESPVKDETPRSVTAYGQKVAGTVAEKLTPVYE

KVKETGSTVMTKLPLSGGGSGVKETQQGEEKGVTAKNYISEKLKPGEEDKALSEMIAE

KLHFGGGGEKKTTATKEVEVTVEKIPSDQIAEGKGHGEAVAEEGKGGEGMVGKVKGAV

TSWLGGKPKSPRSVEESPQSLGTTVGTMGFSDSGGSELGGSGGGKGVQDSGN

# FIG.4B

## low temperature-induced protein cor78 gene promoter

CGCGCCGCAAGAATCTCAAACACGGAGATCTCAAAGTTTGAAAGAAAATTTATTTCTTCGACTCAAAACAAACTT
ACGAAATTTAGGTAGAACTTATATACATTATATTGTAATTTTTTGTAACAAAATGTTTTTATTATTATTATAGAA
TTTTACTGGTTAAATTAAAAATGAATAGAAAAGGTGAATTAAGAGGAGAGAGGAGGTAAACATTTTCTTCTATTT
TTTCATATTTTCAGGATAAATTATTGTAAAAGTTTACAAGATTTCCATTTGACTAGTGTAAATGAGGAATATTCT
CTAGTAAGATCATTATTTCATCTACTTCTTTTATCTTCTACCAGTAGAGGAATAAACAATATTTAGCTCCTTTGT
AAATACAAATTAATTTTCGTTCTTGACATCATTCAATTTTAATTTTACGTATAAAATAAAAGATCATACCTATTA
GAACGATTAAGGAGAAATACAATTCGAATGAGAAGGATGTGCCGCTTGTTATAATAAACAGCCACACGACGTAAA
CGTAAAATGACCACATGATGGGCCAATAGACATGGACCGACTACTAATAATAGTAAGTTACATTTTAGGATGGAA
TAAATATCATACCGACATCAGTTTGAAAGAAAAGGGAAAAAAAGAAAAAATAAATAAAAGATATACTACCGACAT
GAGTTCCAAAAAGCAAAAAAAAAAGATCAAGCCGACACAGACACGCGTAGAGAGCAAAATGACTTTGACGTCACAC
CACGAAAACAGACGCTTCATACGTGTCCCTTTATCTCTCTCAGTCTCTCTATAAACTTAGTGAGACCCTCCTCTG
TTTTACTCACAAATTTAAT

## Translation: low temperature-induced protein 78 sequence

MDQTEEPPLNTHQQHPEEVEHHENGATKMFRKVKARAKKFKNSL

TKHGQSNEHEQDHDLVEEDDDDDELEPEVIDAPGVTGKPRETNVPASEEIIPPGTKVF

PVVSSDYTKPTESVPVQEASYGHDAPAHSVRTTFTSDKEEKRDVPIHHPLSELSDREE

SRETHHESLNTPVSLLSGTEDVTSTFAPSGDDEYLDGQRKVNVETPITLEEESAVSDY

LSGVSNYQSKVTDPTKEETGGVPEIAESFGNMEVTDESPDQKPGQFERDLSTRSKEFK

EFDQDFDSVLGKDSPAKFPGESGVVFPVGFGDESGAELEKDFPTRSHDFDMKTETGMD

TNSPSRSHEFDLKTESGNDKNSPMGFGSESGAELEKEFDQKNDSGRNEYSPESDGGLG

APLGGNFPVRSHELDLKNESDIDKDVPTGFDGEPDFLAKGRPGYGEASEEDKFPARSD

DVEVETELGRDPKTETLDQFSPELSHPKERDEFKESRDDFEETRDEKTEEPKQSTYTE

KFASMLGYSGEIPVGDQTQVAGTVDEKLTPVNEKDQETESAVTTKLPISGGGSGVEEQ

RGEDKSVSGRDYVAEKLTTEEEDKAFSDMVAEKLQIGGEEEKKETTTKEVEKISTEKA

ASEEGEAVEEEVKGGGGMVGRIKGWFGGGATDEVKPESPHSVEEAPKSSGWFGGGATE

EVKPKSPHSVEESPQSLGSTVVPVQKEL

# FIG.4C: low temperature-induced protein cor78 gene

```
   1 gatctcaaag tttgaaagaa aatttatttc ttcgactcaa aacaaactta cgaaatttag
  61 gtagaactta tatacattat attgtaattt tttgtaacaa aatgttttta ttattattat
 121 agaattttac tggttaaatt aaaaatgaat agaaaaggtg aattaagagg agagaggagg
 181 taaacatttt cttctatttt ttcatatttt caggataaat tattgtaaaa gtttacaaga
 241 tttccatttg actagtgtaa atgaggaata ttctctagta agatcattat ttcatctact
 301 tcttttatct tctaccagta gaggaataaa caatatttag ctcctttgta aatacaaatt
 361 aattttcgtt cttgacatca ttcaatttta attttcgtat aaaataaaag atcataccta
 421 ttagaacgat taaggagaaa tacaattcga atgagaagga tgtgccgttt gttataataa
 481 acagccacac gacgtaaacg taaaatgacc acatgatggg ccaatagaca tggaccgact
 541 actaataata gtaagttaca ttttaggatg gaataaatat cataccgaca tcagtttgaa
 601 aagaaaaagg gaaaaaaaga aaaaataaat aaaagatata ctaccgacat gagttccaaa
 661 aagcaaaaaa aaagatcaag ccgacacaga cacgcgtaga gagcaaaatg actttgacgt
 721 cacaccacga aaacagacgc ttcatacgtg tccctttatc tctctcagtc tctctataaa
 781 cttagtgaga ccctcctctg ttttactcac aaatatgcaa actagaaaac aatcatcagg
 841 aataaagggt ttgattactt ctattggaaa gaaaaaaact ttggaaaatg gatcaaacag
 901 aggaaccacc actcaacaca caccagcagc acccaggtag attctaattt cagaaactta
 961 tatttttttt aagtgacaat cctctgaatt tacttaaact tattgtgatt tatggataca
1021 gaagaagttg aacatcatga gaatggtgcg actaagatgt ttaggaaagt aaaggctaga
1081 gctaagaagt tcaagaacag tctcactaaa catggacaaa gcaatgagca tgagcaagat
1141 catgatttgg ttgaagaaga tgatgatgat gacgagctag aacctgaagt gatcgatgca
1201 ccaggttact ttcttttgta gtttcattca acttatgatc taaaactat tggttattca
1261 atttgcgtg acattaacgg tttggtatct gtatatgcag cgtaacaggt aaacctagag
1321 aaactaatgt tccagcatcg gaggaaatta ttccaccagg gacaaaggtg tttcctgtcg
1381 tgtcttccga ttacaccaaa cccactgaat ctgtaccagt acaagaggcc tcttacggac
1441 acgatgcacc ggctcattct gtaaggacga cgtttacatc ggacaaggaa gagaaaagag
1501 atgtaccgat tcatcatcct ctgtccgaat tgtcagacag agaagagagt agagagactc
1561 atcatgagtc attgaacact ccggtctctc tgctttctgg aacagaggat gtaacgagta
1621 cgtttgctcc`aagtggtgat gatgaatatc ttgatggtca acggaaggtc aacgtcgaga
1681 ccccgataac gttggaggaa gagtcggctg tttcagacta tcttagtggt gtatctaatt
1741 atcagtccaa agttactgat cccaccaaag aaggtaagaa ctttgacctt ttaagattgt
1801 gtttttcttt agtgattatg aatatgtaat aactctgtta cgttgtgttt ggtttagaaa
1861 ctggaggagt accggagatt gctgagtctt ttggtaatat ggaagtgact gatgagtctc
1921 ctgatcagaa gccaggacaa tttgaaagag acttgtcgac gagaagcaaa gaattcaaag
1981 agtttgatca ggactttgac tctgttctcg gtaaggattc gccggcgaaa tttccaggtg
2041 aatcaggagt tgtttcccg gtgggctttg gtgacgagtc agcagctgag ctggaaaag
2101 attttccgac gagaagtcat gattttgata tgaagactga aactggaatg gacacgaatt
2161 ctccatcaag aagccatgaa tttgatctga agactgaatc tggaaacgac aagaattctc
2221 cgatgggctt tggtagtgaa tcaggagctg agctggaaaa agaatttgat cagaagaacg
2281 attctggaag aaacgagtat tcgccggaat ctgacggcgg tttaggagct ccgttgggag
2341 gaaattttcc ggtgagaagt catgagttgg atctgaagaa cgaatctgat atcgacaagg
2401 atgtgccgac gggatttgac ggagaaccag attttctggc gaagggaaga cctggatacg
2461 gtgaggcatc agaagaggat aaatttccgg cgagaagtga tgatgtggaa gtagagactg
2521 agctgggaag agacccaaag acggagactc ttgatcaatt ctcaccggaa ctttctcatc
2581 ctaaagaaag agatgagttt aaggagtcca gagatgattt tgaggagacg agagatgaga
2641 aaacagagga gccaaaacag agcacttaca cagagaagtt tgcttcaatg ctacgttact
2701 ccggagaaat tccggtggga gatcaaactc aagtggcggg aactgttgat gagaggttga
2761 ctccggtcaa tgagaaggat caagaaacag agtctgccgt gacgacgaag ttacctatct
2821 ccggaggtgg aagtggagta gaggagcaac gaggggaaga taaaagtgtg tcgggtagag
2881 attatgtggc ggagaaactg acaactgaag aagaagacaa agccttttct gatatggttg
2941 ccgagaaact tcagattgga ggagaagaag agaagaagga aacgacgaca aaggaagtgg
3001 agaagatctc taccgagaag gcagcatcgg aggagggtga ggcggtggaa gaggaagtga
3061 aaggaggagg aggaatggtt gggaggatta aaggatggtt cggtggtggt gcgactgatg
3121 aggtgaagcc agaatcgcca cattctgttg aagaggctcc aaaatcatct ggctggtttg
3181 gtggtggtgc gacggaggag gtgaagccaa aatcgcctca ttccgttgaa gagtctccac
3241 aatcacttgg ctccactgtt gttccggtgc agaaggagct ttaagaatat gagaactgag
3301 attttcaagt ttcactttgg atgtttatgt gtgttttgtt tgacgtcttt gatgtattat
3361 ggtataattc cttgtttgtg tgaaaaaagg acatttggtt aataaattgt tcggctttgg
3421 attaagaagt tcctccatac cagctactag gtctaaagtg ggtaaaatca ttggatttat
3481 tcccttcaaa gttcttagaa ttattcacag gatttacatt atgagctagt agt
```

# FIG.5

## MET1 gene sequence

```
atgtcgtgct gcggaggaaa ctgcggctgc ggatccggct gccagtgcgg cagcggctgc
ggaggatgca agatgtaccc ggagatggct gaggaggtga ccactaccca gactgtcatc
atgggtgttg caccttccaa gggtcatgcc gaggggttgg aggccggcgc cgccgccgga
gcaggagcag agaacgggtg caagtgcggc gacaactgca cctgcaaccc ctgcaactgc
ggcaagtga
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040088754 A
- US 20040163144 A
- WO 2006094976 A
- WO 2005103075 A, Al-Abed
- WO 2006034479 A
- US 4945050 A
- WO 9116432 A
- US 5605797 A
- US 9111315 B
- US 17837458 B
- US 4873192 A
- US 6583338 B
- US 5888732 A
- US 6277608 B
- US 20020007051 A1
- WO 02081711 A1
- US 5795715 A
- WO 9932619 A
- WO 9953050 A
- WO 0068374 A
- WO 0044914 A
- WO 0044895 A
- WO 0049035 A
- WO 0063364 A
- WO 9732016 A
- US 5593874 A
- US 5698425 A
- US 5712135 A
- US 5789214 A
- US 5804693 A
- WO 9401550 A
- WO 9805770 A
- WO 20020048814 A1
- US 20030018993 A1
- US 20030036197 A1
- US 480134100 B
- EP 0291533 A
- EP 0321201 A
- EP 0360257 A
- US 4987071 A
- US 5116742 A
- US 5107065 A
- US 5759829 A
- US 5283184 A
- US 5231020 A
- US 503410323 B
- WO 0152620 A
- US 60672976 B
- US 60718645 B
- WO 0000512 A
- WO 9712983 A
- WO 9811240 A
- WO 9826045 A
- WO 9905902 A
- WO 02052012 A
- WO 9906580 A
- WO 9904013 A
- WO 9742326 A
- WO 9750561 A
- US 6063597 A
- US 6063756 A
- US 6093695 A
- US 5942664 A
- US 6110464 A
- US 5516671 A
- US 5773696 A
- US 6121436 A
- US 6316407 A
- US 6506962 A
- US 5304730 A
- US 6013864 A
- US 6228992 B
- US 6194636 B
- US 6207879 B
- US 6232526 B
- US 6426446 B
- US 6429357 B
- US 6433252 B
- US 6437217 B
- US 6515201 B
- WO 02057471 A
- WO 0032757 A
- WO 0010380 A
- US 5750876 A
- US 6476295 B
- US 6380466 B
- US 5512466 A
- US 5985605 A
- US 6171640 B
- US 5958745 A
- US 20030028917 A
- US 6072103 A
- US 6080560 A
- US 6531648 B
- US 6166292 A
- US 5543576 A
- US 6011199 A
- US 5229114 A

- US 5689041 A
- US 5998700 A
- US 6232122 B
- US 6147279 B
- WO 9722703 A
- US 6444876 B
- US 5608149 A
- US 6537750 B
- US 5512482 A
- US 5530186 A
- US 5945585 A
- US 5639790 A
- US 5807893 A
- US 5955650 A
- US 5955329 A
- US 5147792 A
- US 5304481 A
- US 5298421 A
- US 5344771 A
- US 5760206 A
- US 20030115632 A1
- US 20030028923 A1
- US 5689050 A
- US 5663068 A
- US 5614393 A
- US 5856157 A
- US 6117677 A
- US 6043411 A
- US 6194167 A
- US 5705391 A
- US 5552306 A
- US 6075183 A
- US 6051754 A
- US 5789220 A
- US 5057419 A
- US 5654402 A
- US 5659645 A
- US 6100091 A
- US 6172106 A
- US 5952544 A
- US 5866789 A
- US 5443974 A
- US 5093249 A
- US 5965727 A
- WO 9726366 A
- WO 9911800 A
- WO 9949058 A
- WO 9906581 A
- US 5534421 A
- US 5942660 A
- WO 9519442 A
- WO 9902656 A
- WO 9855601 A
- US 5258300 A
- US 5367110 A
- US 5858749 A
- US 6040160 A
- US 4886878 A
- US 4885357 A
- US 5215912 A
- US 5589616 A
- US 5508468 A
- US 5939599 A
- US 5633436 A
- US 5990384 A
- WO 9001869 A
- WO 9113993 A
- WO 9214822 A
- WO 9308682 A
- WO 9420628 A
- WO 9728247 A
- WO 9826064 A
- WO 9940209 A
- US 5003045 A
- US 5576203 A
- US 5850024 A
- WO 9617064 A
- WO 9735023 A
- WO 0019839 A
- US 6107051 A
- US 5885802 A
- US 5885801 A
- US 5270200 A
- US 6110891 A
- US 5990389 A
- US 5914450 A
- US 5998701 A
- US 5789538 A
- WO 9948909 A
- WO 9945132 A
- WO 9853060 A
- WO 9853057 A
- WO 9853058 A
- WO 0023464 A
- WO 9519431 A
- WO 9854311 A
- WO 03004659 A
- WO 9614408 A
- US 5605793 A
- US 5837458 A
- US 5830721 A
- US 5811238 A
- WO 03060133 A
- DE 03028884
- WO 9845456 A
- EP 0333033 A
- US 4975374 A
- EP 0218571 A1
- EP 154204 A1
- EP 0807836 A
- EP 0601092 A
- WO 9301281 A
- US 5358866 A
- EP 595837 A1
- WO 04013333 A
- EP 04006358 A

- EP 295959 A
- EP 138341 A
- EP 120516 A
- EP 0332581 A
- WO 0200900 A
- US 5591616 A
- WO 0177355 A
- US 6720477 B
- WO 02072848 A
- WO 03020939 A
- WO 2004035798 A
- WO 2004081217 A
- WO 9741228 A

**Non-patent literature cited in the description**


- **SASAKI, T. et al.** *Nature,* 2002, vol. 420 (6913), 312-316
- **LEWIN.** Genes V. Oxford University Press, 1994
- The Encyclopedia of Molecular Biology. Blackwell Science Ltd, 1994
- Molecular Biology and Biotechnology: a Comprehensive Desk Reference. VCH Publishers, Inc, 1995
- **L. STRYER.** Biochemistry. W. H. Freeman and Company, 1988
- **ROSE et al.** *RNA,* 2002, vol. 8, 1444-1453
- **CALLIS et al.** *Genes & Dev,* 1987, vol. 1, 1183-1200
- Nucleic Acid Hybridization. IRL Press
- **ZHU et al.** *The Plant Cell,* 1995, vol. 7, 1681-1689
- **MATZKE MA et al.** *Plant Mol. Biol.,* 2000, vol. 43, 401-415
- **FIRE A. et al.** *Nature,* 1998, vol. 391, 806-811
- **TCHURIKOV et al.** *J. Biol. Chem.,* 2000, vol. 275 (34), 26523-26529
- **SCHWEIZER P et al.** *Plant J 2000,* 2000, vol. 24, 895-903
- **WATERHOUSE PM et al.** *Proc. Natl. Acad. Sci. USA,* 1998, vol. 95, 13959-64
- **TUSCHL et al.** *Gens Dev.,* 1999, vol. 13 (24), 3191-3197
- **YANG, D. et al.** *Proc. Natl. Acad. Sci. USA,* 2002, vol. 99, 9942
- **SHEEHY et al.** *Proc. Natl. Acad. Sci. USA,* 1988, vol. 85, 8805-8809
- **MOL JN et al.** *FEBS Lett,* 1990, vol. 268 (2), 427-430
- **GAUTIER C et al.** *Nucleic Acids Res.,* 1987, vol. 15, 6625-6641
- **INOUE et al.** *Nucleic Acids Res.,* 1987, vol. 15, 6131-6148
- **INOUE et al.** *FEBS Lett,* 1987, vol. 215, 327-330
- **HELENE, C.** *Anticancer Drug Des.,* 1991, vol. 6 (6), 569-84
- **HELENE, C. et al.** *Ann. N.Y. Acad. Sci.,* 1992, vol. 660, 27-36
- **MAHER, L.J.** *Bioassays,* 1992, vol. 14 (12), 807-15
- **TANNER NK.** *FEMS Microbiol. Rev.,* 1999, vol. 23 (3), 257-275
- **HASELOFF et al.** *Nature,* 1988, vol. 33410, 585-591
- **HASELHOFF ; GERLACH.** *Nature,* 1988, vol. 33410, 585-591
- **STEINECKE P et al.** *EMBO J,* 1992, vol. 11 (4), 1525-1530
- **DE FEYTER R et al.** *Mol. Gen. Genet.,* 1996, vol. 250 (3), 329-338
- **STEINECKE P et al.** Ribozymes, Methods in Cell Biology 50. Academic Press, Inc, 1995, 449-460
- **BAYLEY CC et al.** *Plant Mol. Biol.,* 1992, vol. 18 (2), 353-361
- **LLOYD AM ; DAVIS RW et al.** *Mol. Gen. Genet.,* 1994, vol. 242 (6), 653-657
- **BARTEL D ; SZOSTAK JW.** *Science,* 1993, vol. 261, 1411-1418
- **JORGENSEN et al.** *Plant Mol. Biol.,* 1996, vol. 31 (5), 957-973
- **GORING et al.** *Proc. Natl. Acad. Sci. USA,* 1991, vol. 88, 1770-1774
- **SMITH et al.** *Mol. Gen. Genet.,* 1990, vol. 224, 447-481
- **NAPOLI et al.** *Plant Cell,* 1990, vol. 2, 279-289
- **VAN DER KROL et al.** *Plant Cell,* 1990, vol. 2, 291-99
- **NAPOLI et al.** *The Plant Cell,* 1990, vol. 2, 279-289
- **BRUMMELL et al.** *Plant J.,* 2003, vol. 33, 793-800
- **SCHUBERT et al.** *Plant Journal,* 2004, vol. 16, 2561-2572
- **LAGNA G ; HEMMATI-BRIVANLOU A.** *Current Topics in Developmental Biology,* 1998, vol. 36, 75-98
- **PERLMUTTER RM ; ALBEROLA-ILA J.** *Current Opinion in Immunology,* 1996, vol. 8 (2), 285-90
- **SHEPPARD D.** *American Journal of Respiratory Cell & Molecular Biology,* 1994, vol. 11 (1), 1-6
- **HERSKOWITZ I.** *Nature,* 1987, vol. 329 (6136), 219-22
- **DREIER B et al.** *J. Biol. Chem.,* 2001, vol. 276 (31), 29466-78
- *J. Mol. Biol.,* 2000, vol. 303 (4), 489-502

- **BEERLI RR et al.** *Proc. Natl. Acad. Sci. USA,* 1998, vol. 95 (25), 14628-14633
- *Proc. Natl. Acad. Sci. USA,* 2000, vol. 97 (4), 1495-1500
- *J. Biol. Chem.,* 2000, vol. 275 (42), 32617-32627
- **SEGAL DJ ; BARBAS CF, 3RD.** *Curr. Opin. Chem. Biol.,* 2000, vol. 4 (1), 3410-39
- **KANG JS ; KIM JS.** *J. Biol. Chem.,* 2000, vol. 275 (12), 8742-8748
- **KIM JS et al.** *Proc. Natl. Acad. Sci. USA,* 1997, vol. 94 (8), 3616-3620
- **KLUG A.** *J. Mol. Biol.,* 1999, vol. 293 (2), 215-218
- **TSAI SY et al.** *Adv. Drug Deliv. Rev.,* 1998, vol. 30 (1-3), 23-31
- **MAPP AK et al.** *Proc. Natl. Acad. Sci. USA,* 2000, vol. 97 (8), 3930-3935
- **SHARROCKS AD et al.** *Int. J. Biochem. Cell Biol.,* 1997, vol. 29 (12), 1371-1387
- **ZHANG L et al.** *J. Biol. Chem.,* 2000, vol. 275 (43), 33850-33860
- **ORDIZ MI et al.** *Proc. Natl. Acad. Sci. USA,* 2002, vol. 99 (20), 13290-13295
- **GUAN et al.** *Proc. Natl. Acad. Sci. USA,* 2002, vol. 99 (20), 13296-13301
- **FAMULOK M ; MAYER G.** *Curr. Top Microbiol. Immunol.,* 1999, vol. 243, 123-36
- **OWEN M et al.** *Biotechnology,* 1992, vol. 10 (7), 790-794
- **FRANKEN E et al.** *Curr. Opin. Biotechnol.,* 1997, vol. 8 (4), 411-416
- **WHITELAM.** *Trend Plant Sci.,* 1996, vol. 1, 286-272
- **DERVAN PB ; BÜRLI RW.** *Current Opinion in Chemical Biology,* 1999, vol. 3, 688-693
- **GOTTESFELD JM et al.** *Gene Expr.,* 2000, vol. 9 (1-2), 77-91
- **BREMER RE et al.** *Bioorg. Med. Chem.,* 2001, vol. 9 (8), 2093-103
- **ANSARI AZ et al.** *Chem. Biol.,* 2001, vol. 8 (6), 583-92
- **GOTTESFELD JM et al.** *J. Mol. Biol.,* 2001, vol. 309 (3), 615-29
- **WURTZ NR et al.** *Org. Lett,* 2001, vol. 3 (8), 1201-3
- **WANG CC et al.** *Bioorg. Med. Chem.,* 2001, vol. 9 (3), 653-7
- **URBACH AR ; DERVAN PB.** *Proc. Natl. Acad. Sci. USA,* 2001, vol. 98 (8), 434103-8
- **CHIANG SY et al.** *J. Biol. Chem.,* 2000, vol. 275 (32), 24246-54
- **ANGELL, SM et al.** *Plant J.,* 1999, vol. 20 (3), 357-362
- **RATCLIFF F et al.** *Plant J.,* 2001, vol. 25 (2), 237-45
- **FAGARD M ; VAUCHERET H.** *Plant Mol. Biol.,* 2000, vol. 43 (2-3), 285-93
- **ANANDALAKSHMI R et al.** *Proc. Natl. Acad. Sci. USA,* 1998, vol. 95 (22), 13079-84
- **RUIZ MT.** *Plant Cell,* 1998, vol. 10 (6), 937-46
- **BARTEL D.** *Cell,* 2004, vol. 116, 281-297
- **SCHWAB et al.** *2005 Dev. Cell,* 2005, vol. 8, 517-527
- **SCHWAB et al.** Highly Specific Gene Silencing by Artificial microRNAs in Arabidopsis. *Plant Cell,* 2006, vol. 18 (4, http://wmd.weigelworld.org
- **MICHAELSON et al.** *JBC,* vol. 273, 19055-19059
- **DUNWELL JM.** Transgenic approaches to crop improvement. *J Exp Bot.,* 2000, vol. 51, 487-96
- **DIXON M ; KLEPPE K.** *Biochim. Biophys. Acta,* 1965, vol. 96, 357-367
- **DIXON M ; KLEPPE K.** *Biochim. Biophys. Acta,* 1965, vol. 96, 368-382
- **DIXON M ; KLEPPE.** *Biochim. Biophys. Acta,* 1965, vol. 96, 383-389
- **MASSEY V et al.** *Biochim. Biophys. Acta,* 1961, vol. 48, 1-9
- **MEISTER A ; WELLNER D.** Flavoprotein amino acid oxidase
- **ABEL et al.** *Science,* 1986, vol. 232, 738
- **AGRAWAL A et al.** *Nature,* 1998, vol. 394 (6695), 744-451
- **ALTSCHUL et al.** *J. Mol. Biol.,* 1990, vol. 215, 403
- **ALTSCHUL et al.** *Nucleic Acids Res.,* 1997, vol. 25, 3389
- **AMARA JF et al.** *Proc Natl Acad Sci USA,* 1997, vol. 94 (20), 10618-1623
- **AN et al.** *EMBO J.,* 1985, vol. 4, 277
- **ANGRAND PO et al.** *Nucl. Acids Res.,* 1998, vol. 26 (13), 3263-3269
- **ARGAST GM et al.** *J Mol Biol,* 1998, vol. 280, 345-353
- **AUCH ; RETH.** *Nucleic Acids Research,* 1990, vol. 18, 6743
- **AUSUBEL et al.** Current Protocols in Molecular Biology. Greene Publishing Assoc. and Wiley Interscience, 1987
- **BALLAS et al.** *Nucleic Acids Res.,* 1989, vol. 17, 7891
- **BARKAI-GOLAN et al.** *Arch. Microbiol.,* 1978, vol. 116, 119
- **BARKER et al.** *Plant Molec. Biol.,* 1983, vol. 2, 335-50
- **BARTLEY ; SCOLNIK.** *Plant Physiol.,* 1994, vol. 104, 1469-1470
- **BATZER et al.** *Nucleic Acid Res.,* 1991, vol. 19, 5081
- **BÄUMLEIN et al.** *Mol Gen Genet,* 1991, vol. 225, 121-128
- **BEALL EL ; RIO DC.** *Genes Dev.,* 1997, vol. 11 (16), 2137-2151

- **BEAUDOIN et al.** *PNAS,* 2000, vol. 97, 6421-6426
- **BECKER et al.** *Plant J.,* 1994, vol. 5, 299-307
- **BEERLI RR et al.** *Proc Natl Acad Sci USA,* 1998, vol. 95 (25), 14628-14633
- **BEERLI RR et al.** *J Biol Chem,* 2000, vol. 275 (42), 32617-32627
- **BEERLI RR et al.** *Proc Natl Acad Sci U S A.,* 2000, vol. 97 (4), 1495-1500
- **BELFORT M ; ROBERTS RJ.** *Nucleic Acids Res,* 1997, vol. 25, 3379-3388
- **BELL-PEDERSEN et al.** *Nucleic Acids Res,* 1990, vol. 18, 3763-3770
- **BERNAL-LUGO ; LEOPOLD.** *Plant Physiol.,* 1992, vol. 98, 1207
- **BEVAN et al.** *Nature,* 1983, vol. 304, 184
- **BEVAN et al.** *Nucl. Acids Res.,* 1983, vol. 11, 369
- **BEVAN.** *Nucl. Acids Res.,* 1984, vol. 12, 8711
- **BIBIKOVA M et al.** *Mol Cell Biol.,* 2001, vol. 21, 289-297
- **BLACKMAN et al.** *Plant Physiol.,* 1992, vol. 100, 225
- **BLANC V et al.** *Biochimie,* 1996, vol. 78 (6), 511-517
- **BLOCHLINGER ; DIGGELMANN.** *Mol Cell Biol,* 1984, vol. 4, 2929
- **BOL et al.** *Ann. Rev. Phytopath.,* 1990, vol. 28, 113
- **BOUCHEZ et al.** *EMBO J.,* 1989, vol. 8, 4197
- **BOUROUIS et al.** *EMBO J.,* 1983, vol. 2, 1099
- **BOWLER et al.** *Ann. Rev. Plant Physiol.,* 1992, vol. 43, 83
- **BRADFORD.** *Anal.Biochem.,* 1976, vol. 72, 248-254
- **BRANSON ; GUSS.** *Proc. North Central Branch Entomological Society of America,* 1972
- **BROAKGERT et al.** *Science,* 1989, vol. 245, 110
- **BROGLIE et al.** *Science,* 1991, vol. 254, 1194-1197
- **BUSTOS et al.** *Plant Gell,* 1989, vol. 1, 839-853
- **BYRNE et al.** *Plant Cell Tissue and Organ Culture,* 1987, vol. 8, 3
- **CALLIS et al.** *Genes and Develop.,* 1987, vol. 1, 1183
- **CAMPBELL ; GOWRI.** *Plant Physiol.,* 1990, vol. 92, 1
- Ivermectin and Abamectin. Springer-Verlag, 1989
- **CECCHINI E et al.** *Mutat Res,* 1998, vol. 401 (1-2), 199-206
- **CHEE et al.** *Plant Physiol.,* 1989, vol. 91, 1212
- **CHEIKH-N et al.** *Plant Physiol.,* 1994, vol. 106 (1), 45-51
- **CHEN ; WINANS.** *J. Bacteriol.,* 1991, vol. 173, 1139-1144
- **CHEN et al.** *EMBO J.,* 1988, vol. 6, 3559-3564
- **CHRISTOU et al.** Annals of Botany. 1995, vol. 75, 407-413
- **CHRISTOU et al.** *Proc. Natl. Acad. Sci USA,* 1989, vol. 86, 7500
- **CHRISTOU et al.** *Biotechnology,* 1991, vol. 9, 957
- **CHRISTOU et al.** *Plant Physiol.,* 1988, vol. 87, 671
- **CHU et al.** *Proc Natl Acad Sci USA,* 1990, vol. 87, 3574-3578
- **CHUI et al.** *Curr Biol,* 1996, vol. 6, 325-330
- **COE et al.** Corn and Corn Improvement. 1988, 81-258
- **CORNEILLE S et al.** *Plant J,* 2001, vol. 27, 171-178
- **CORPET et al.** *Nucleic Acids Res.,* 1988, vol. 16, 10881
- **COTE V et al.** *Gene,* 1993, vol. 129, 69-76
- **COXSON et al.** *Biotropica,* 1992, vol. 24, 121
- **CRAMERI et al.** *Nature Biotech.,* 1997, vol. 15, 436
- **CRAMERI et al.** *Nature,* 1998, vol. 391, 288
- **CROSSWAY et al.** *BioTechniques,* 1986, vol. 4, 320-334
- **CUOZZO et al.** *Bio/Technology,* 1988, vol. 6, 549
- **CUTLER et al.** *J. Plant Physiol.,* 1989, vol. 135, 351
- **CZAKO M ; MARTON L.** *Plant Physiol,* 1994, vol. 104, 1067-1071
- **CZAPLA ; LANG.** *J. Econ. Entomol.,* 1990, vol. 83, 2480
- **DALE EC ; OW DW.** *Proc Natl Acad Sci USA,* 1991, vol. 88, 10558-10562
- **DATTA et al.** *Bio/Technology,* 1990, vol. 8, 736-740
- **DAVIES et al.** *Plant Physiol.,* 1990, vol. 93, 588
- **DAYHOFF et al.** Atlas of Protein Sequence and Structure. Natl. Biomed. Res. Found, 1978
- **DE BLAERE et al.** *Meth. Enzymol.,* 1987, vol. 143, 277
- **DE BLOCK et al.** *Plant Physiol.,* 1989, vol. 91, 694
- **DE BLOCK et al.** *EMBO Journal,* 1987, vol. 6, 2513
- **DEAK M et al.** *Nature Biotechnology,* 1999, vol. 17, 192-196
- **DEBLAERE et al.** *Nucl Acids Res,* 1985, vol. 13, 4777-4788
- **DELLA-CIOPPA et al.** *Bio/Technology,* 1987, vol. 5, 579-584
- **DELLA-CIOPPA et al.** *Plant Physiology,* 1987, vol. 84, 965-968
- **DELLAPORTA et al.** Chromosome Structure and Function. Plenum Press, 1988, 263-282

- **DEPICKER AG et al.** *Plant Cell rep,* 1988, vol. 104, 1067-1071
- **DEPICKER et al.** *Plant Cell Reports,* 1988, vol. 7, 63
- **DUNN et al.** *Can. J. Plant Sci.,* 1981, vol. 61, 583
- **DUNWELL JM.** *Biotechn Genet Eng Rev,* 1998, vol. 15, 1-32
- **DURE et al.** *Plant Mol. Biol.,* 1989, vol. 12, 475
- **EBINUMA et al.** *Proc Natl Acad Sci USA,* 2000, vol. 94, 2117-2121
- Selection of Marker-free transgenic plants using the oncogenes (ipt, rol A, B, C) of Agrobacterium as selectable markers. **EBINUMA et al.** Molecular Biology of Woody Plants. Kluwer Academic Publishers, 2000
- **EDDY et al.** *Genes Dev.,* 1991, vol. 5, 1032-1041
- **EICHHOLTZ et al.** *Somatic Cell and Molecular Genetics,* 1987, vol. 13, 67-76
- **ELLIS et al.** *Mol. Gen. Genet.,* 1984, vol. 195, 466-73
- **ELROY-STEIN et al.** *Proc. Natl. Acad. Sci. U.S.A.,* 1989, vol. 86, 6126
- **ENGLISH et al.** *Plant Cell,* 1996, vol. 8, 179
- **ERDMANN et al.** *J. Gen. Microbiol.,* 1992, vol. 138, 363
- **ERIKSON et al.** *Nat Biotechnol.,* 2004, vol. 22 (4), 455-8
- **EVERETT et al.** *Bio/Technology,* 1987, vol. 5, 1201
- **FEDOROFF ; SMITH.** *Plant J,* 1993, vol. 3, 273-289
- **FEDOROFF NV ; SMITH DL.** *Plant J,* 1993, vol. 3, 273-289
- **FIRE et al.** *Nature,* 1998, vol. 391, 806-811
- **FITZPATRICK.** *Gen. Engineering News,* 1993, vol. 22, 7
- **FOX et al.** *Plant Mol. Biol.,* 1992, vol. 20, 219-33
- **FRALEY et al.** *Proc Natl Acad Sci USA,* 1983, vol. 80, 4803
- **FREELING ; WALBOT.** The maize handbook. Springer Verlag, 1993
- **FROMM et al.** *Bio/Technology,* 1990, vol. 8, 833-839
- **FROMM et al.** *Nature,* 1986, vol. 319, 791
- **GABLER M et al.** *Enzyme Microb. Techno.,* 2000, vol. 27, 605-611
- **GALBIATI et al.** *Funct. Integr Genozides,* 2000, vol. 20 (1), 25-34
- **GALLEGO ME.** *Plant Mol Biol,* 1999, vol. 39 (1), 83-93
- **GALLIE et al.** *Nucl Acids Res,* 1987, vol. 15, 8693-8711
- **GALLIE et al.** *Nucleic Acids Res.,* 1987, vol. 15, 3257
- **GALLIE et al.** *The Plant Cell,* 1989, vol. 1, 301
- **GAN et al.** *Science,* 1995, vol. 270, 1986
- **GATEHOUSE et al.** *J. Sci. Food Agric.,* 1984, vol. 35, 373
- PCR Strategies. Academic Press, 1995
- **GILMOUR et al.** *Plant Mol Biol,* 1991, vol. 17, 1233-1240
- **GELVIN et al.** Plant Molecular Biology Manual. 1990
- **GIRKE et al.** *Plant J,* 1998, vol. 15, 39-48
- **GLEAVE AP et al.** *Plant Mol Biol,* 1999, vol. 40 (2), 223-235
- **GLEAVE et al.** *Plant Mol Biol.,* 1999, vol. 40 (2), 223-35
- **GORDON-KAMM et al.** *Plant Cell,* 1990, vol. 2, 603
- **GORING et al.** *PNAS,* 1991, vol. 88, 1770
- **GOYAL RK et al.** *Crop Protection,* 2000, vol. 19 (5), 307-312
- Vectors for Plant Transformation. **GRUBER et al.** Methods in Plant Molecular Biology & Biotechnology. CRC Press, 1993, 89-119
- **GUERINEAU et al.** *Mol. Gen. Genet.,* 1991, vol. 262, 141
- **GUERRERO et al.** *Plant Mol. Biol.,* 1990, vol. 15, 11
- **GUO et al.** *EMBO J,* 1997, vol. 16, 6835-6848
- **GUO et al.** *Science,* 2000, vol. 289, 452-457
- **GUPTA et al.** *PNAS,* 1993, vol. 90, 1629
- **HAJDUKIEWICZ et al.** *Plant Mol Biol,* 1994, vol. 25, 989-994
- **HAMMOCK et al.** *Nature,* 1990, vol. 344, 458
- **HANSEN et al.** *Proc. Natl. Acad. Sci. USA,* 1994, vol. 91, 7603-7607
- **HARE P ; CHUA NH.** *Nat. Biotechnol.,* 2002, vol. 20, 575-580
- **HAREN L et al.** *Annu Rev Microbiol. 1999,* 1999, vol. 53, 245-281
- **HASELOFF et al.** *Proc Natl Acad Sci USA,* 1997, vol. 94 (6), 2122-2127
- **HAYFORD et al.** *Plant Physiol.,* 1988, vol. 86, 1216
- **HEMENWAY et al.** *EMBO Journal,* 1988, vol. 7, 1273
- **HENIKOFF ; HENIKOFF.** *Proc. Natl. Acad. Sci. USA,* 1989, vol. 89, 10915
- **HIEI et al.** *Plant J,* 1994, vol. 6, 271-282
- **HIGGINS et al.** *Gene,* 1988, vol. 73, 237
- **HIGO et al.** *Nucl Acids Res,* 1999, vol. 27 (1), 297-300
- **HILDER et al.** *Nature,* 1987, vol. 330, 160
- **HILLE et al.** *Plant Mol. Biol.,* 1986, vol. 7, 171
- **HINCHEE et al.** *Bio/Technology,* 1988, vol. 6, 915
- **HOEKEMA et al.** *Nature,* 1983, vol. 303, 179-181
- **HOEKEMA.** *The Binary Plant Vector System. Offset-drukkerij Kanters B.V.,* 1985

- **HOOD EE ; JILKA JM.** *Curr Opin Biotechnol,* 1999, vol. 10 (4), 382-6
- **HOOD et al.** *Adv Exp Med Biol,* 1999, vol. 464, 127-47
- **HOOD et al.** *J Bacteriol,* 1986, vol. 168, 1291-1301
- **HORVATH et al.** *Plant Physiol.,* 1993, vol. 103, 1047-1053
- **HUANG B et al.** *J Protein Chem,* 1996, vol. 15 (5), 481-9
- **HUANG et al.** *CABIOS,* 1992, vol. 8, 155
- **IKEDA et al.** *J. Bacteriol.,* 1987, vol. 169, 5612
- **IKUTA et al.** *Biotech.,* 1990, vol. 8, 241
- **IMAI T et al.** *Proc Natl Acad Sci USA,* 2001, vol. 98 (1), 224-228
- **INGELBRECHT et al.** *Plant Cell,* 1989, vol. 1, 671
- PCR Methods Manual. Academic Press, 1999
- PCR Protocols: A Guide to Methods and Applications. Academic Press, 1995
- **INNIS et al.** PCR Protocols: A Guide to Methods and Applications. Academic Press, Inc, 1990
- **ISHIDA et al.** *Nature Biotech,* 1996, 745-750
- **JANSSEN DB.** *J Bacteriol,* 1989, vol. 171 (12), 6791-9
- **JANSSEN DB et al.** *Annu Rev Microbiol,* 1994, vol. 48, 163-191
- **JASIN M.** *Trends Genet.,* 1996, vol. 12, 224-228
- **JEFFERSON et al.** *EMBO J,* 1987, vol. 6, 3901-3907
- **JEFFERSON et al.** *Plant Mol Biol Rep,* 1987, vol. 5, 387-405
- Techniques for Gene Transfer. **JENES et al.** Recombinant Plants, Vol. 1, Engineering and Utilization. Academic Press, 1993, vol. 1, 128-143
- **JICKS GR ; RAIKHEL NV.** *Annu. Rev. Cell Biol.,* 1995, vol. 11, 155-188
- **JOBLING et al.** *Nature,* 1987, vol. 325, 622
- **JOHNSON et al.** *PNAS USA,* 1989, vol. 86, 9871
- **JONES et al.** *Mol. Gen. Genet.,* 1987, vol. 210, 86
- **JOSHI et al.** *Nucleic Acid Res.,* 1987, vol. 15, 9627
- **KAASEN et al.** *J. Bacteriol.,* 1992, vol. 174, 889
- **KANG JS ; KIM JS.** *J Biol Chem,* 2000, vol. 275 (12), 8742-8748
- **KARLIN ; ALTSCHUL.** *Proc. Natl. Acad Sci. USA,* 1990, vol. 87, 2264
- **KARLIN ; ALTSCHUL.** *Proc. Natl. Acad. Sci. USA,* 1993, vol. 90, 5873
- **KARLIN-NEUMANNN GA et al.** *Plant Cell,* 1991, vol. 3, 573-582
- **KARSTEN et al.** *Botanica Marina,* 1992, vol. 35, 11
- **KASUGA et al.** *Nature Biotechnology,* 1999, vol. 17 (3), 287-291
- **KASUGA M et al.** *Nature Biotech,* 1999, vol. 17, 276-286
- **KATZ et al.** *J. Gen. Microbiol.,* 1983, vol. 129, 2703
- **KAUFMAN PD ; RIO DC.** *Cell,* 1992, vol. 69 (1), 27-39
- **KAWASAKI et al.** *J Biol Chem,* 1991, vol. 266, 5342-5347
- **KEEGSTRA.** *Cell,* 1989, vol. 56 (2), 247-53
- **KEENAN T et al.** *Bioorg Med Chem.,* 1998, vol. 6 (8), 1309-1335
- **KELLER et al.** *EMBO Journal,* 1989, vol. 8, 1309
- **KELLER et al.** *Genes Dev.,* 1989, vol. 3, 1639
- **KILBY NJ et al.** *Plant J,* 1995, vol. 8, 637-652
- **KIM JS et al.** *Proc Natl Acad Sci USA,* 1997, vol. 94 (8), 3616-3620
- **KLAPWIJK et al.** *J. Bacteriol.,* 1980, vol. 141, 128-136
- **KLEIN et al.** *Bio/Technoloy,* 1988, vol. 6, 559-563
- **KLEIN et al.** *Plant Physiol.,* 1988, vol. 91, 440-444
- **KLEIN et al.** *Proc. Natl. Acad. Sci. USA,* 1988, vol. 85, 4305-4309
- **KLUG A.** *J Mol Biol,* 1999, vol. 293 (2), 215-218
- Genetic Analysis of Host Range Expression by Agrobacterium. **KNAUF et al.** Molecular Genetics of the Bacteria-Plant Interaction. Springer-Verlag, 1983
- **KOBAYASHI T et al.** *Jpn J Genet,* 1995, vol. 70 (3), 409-422
- **KOMRO et al.** *Plant Mol. Biol.,* 1985, vol. 4, 253-63
- **KONCZ ; SCHELL.** *Mol Gen Genet,* 1986, vol. 204, 383-396
- **KONONOWICZ et al.** *Plant Cell,* 1992, vol. 4, 17-27
- **KOPREK et al.** *Plant J,* 1999, vol. 19 (6), 719-726
- **KOPREK T et al.** *Plant J,* 1999, vol. 19 (6), 719-726
- **KOSTER ; LEOPOLD.** *Plant Physiol.,* 1988, vol. 88, 829
- **KOZIEL et al.** *Biotechnology,* 1993, vol. 11, 194
- **KRIDL et al.** *Seed Sci. Res.,* 1991, vol. 1, 209-219
- **KUIPER HA et al.** *Plant J.,* 2001, vol. 27, 503-528
- **KUNKEL et al.** *Methods in Enzymol.,* 1987, vol. 154, 367
- **KUNKEL.** *Proc. Natl. Acad. Sci. USA,* 1985, vol. 82, 488

- **LAM ; CHUA.** *J Biol Chem,* 1991, vol. 266 (26), 17131-17135
- **LAUFS et al.** *PNAS,* 1990, vol. 87, 7752
- **LAWTON et al.** *Mol. Cell Biol.,* 1987, vol. 7, 335
- **LEE ; SAIER.** *J. Bacteriol.,* 1982, 153
- **LEE TJ et al.** *J Amer Soc Horticult Sci,* 2002, vol. 127 (2), 158-164
- **LEFFEL et al.** *Biotechniques,* 1997, vol. 23 (5), 912-8
- **LEFFEL SM et al.** *Biotechniques,* 1997, vol. 23 (5), 912-8
- **LESCOT et al.** *Nucleic Acids Res,* 2002, vol. 30 (1), 325-7
- **LEUNG et al.** *Mol. Gen. Genet.,* 1991, vol. 230, 463-74
- **LEVINGS.** *Science,* 1990, vol. 250, 942
- **LI et al.** *Plant Cell Reports,* 1993, vol. 12, 250-255
- **LI et al.** *Plant Mol Biol,* 1992, vol. 20, 1037-1048
- **LINDSEY et al.** *Transgenic Research,* 1993, vol. 2, 3347
- **LIU et al.** *Plant J.,* 1995, vol. 8, 457-463
- **LOGIE C ; STEWART AF.** *Proc Natl Acad Sci USA,* 1995, vol. 92 (13), 5940-5944
- **LOIS et al.** *Proc. Natl. Acad. Sci. USA,* 1998, vol. 95 (5), 2105-2110
- **LOMMEL et al.** *Virology,* 1991, vol. 181, 382
- **LOOMIS et al.** *J. Expt. Zool.,* 1989, vol. 252, 9
- **LORZ et al.** *Mol. Gen. Genet.,* 1985, vol. 199, 178
- **LYZNIK LA et al.** *Nucleic Acids Res,* 1996, vol. 24, 3784-3789
- **MA et al.** *Nature,* 1988, vol. 334, 631
- **MA JK ; VINE ND.** *Curr Top Microbiol Immunol,* 1999, vol. 236, 275-92
- **MA, Q. H. et al.** *Australian Journal of Plant Physiology,* 1998, vol. 25 (1), 53-59
- **MACEJAK et al.** *Nature,* 1991, vol. 353, 90
- **MAKI et al.** Methods in Plant Mol. Biol. & Biotechnol. CRC Press, 1993, 67-88
- **MANIATIS et al.** Molecular Cloning: A Laboratory Manual. Cold Spring Harbor Laboratory, 1989
- **MAPP AK et al.** *Proc Natl Acad Sci USA,* 2000, vol. 97 (8), 3930-3935
- **MARIANI et al.** *Nature,* 1990, vol. 347, 737
- **MARSHALL.** *Gene,* 1991, vol. 104, 241-245
- **MATZKE et al.** *Plant Mol Biol,* 2000, vol. 43, 401-415
- **MCBRIDE et al.** *PNAS USA,* 1994, vol. 91, 7301
- **MCCABE et al.** *Bio/Technology,* 1988, vol. 6, 923
- **MCKNIGHT SL et al.** *Nucl Acids Res,* 1980, vol. 8 (24), 5931-5948
- **MCKNIGHT SL et al.** *Nucl Acids Res,* 1980, vol. 8 (24), 5949-5964
- **MEINKOTH ; WAHL.** *Anal. Biochem.,* 1984, vol. 138, 267
- Flavoprotein amino acid oxidase. **MEISTER A ; WELLNER D.** The Enzymes. Academic Press, 1963, vol. 7, 609-648
- **MENARD R et al.** *Phytochemistry,* 1999, vol. 52, 29-35
- **MESSING ; VIERRA.** *Gene,* 1982, vol. 19, 259
- **MICHAEL et al.** *J. Mol. Biol.,* 1990, vol. 26, 585
- **MICHAELSON et al.** *FEBS Letters,* 1998, vol. 439, 215-218
- **MILLAR et al.** *Plant Mol Biol Rep,* 1992, vol. 10, 324-414
- **MIYANO M et al.** *J Biochem,* 1991, vol. 109, 171-177
- **MOGEN et al.** *Plant Cell,* 1990, vol. 2, 1261
- **MOHR et al.** *Genes & Development,* 2000, vol. 14, 559-573
- **MONNAT RJ JR et al.** *Biochem Biophys Res Com,* 1999, vol. 255, 88-93
- **MOORE et al.** *J. Mol. Biol.,* 1997, vol. 272, 336
- **MOZO ; HOOYKAAS.** *Plant Mol. Biol.,* 1991, vol. 16, 917-918
- **MULLEN CA et al.** *Proc Natl Acad Sci USA,* 1992, vol. 89 (1), 33-37
- **MUNDY ; CHUA.** *EMBO J.,* 1988, vol. 7, 2279
- **MUNROE et al.** *Gene,* 1990, vol. 91, 151
- **MURAKAMI et al.** *Mol. Gen. Genet.,* 1986, vol. 205, 42
- **MURATA et al.** *FEBS Lett.,* 1992, vol. 296, 187
- **MURDOCK et al.** *Phytochemistry,* 1990, vol. 29, 85
- **MURRAY et al.** *Nucleic Acids Res.,* 1989, vol. 17, 477
- **MUTHUSWAMY SK et al.** *Mol Cell Biol,* 1999, vol. 19 (10), 6845-685
- **MYERS ; MILLER.** *CABIOS,* 1988, vol. 4, 11
- **NAESTED H et al.** *Plant J,* 1999, vol. 18 (5), 571-576
- **NAESTED.** *Plant J,* 1999, vol. 18, 571-576
- **NAPOLI et al.** *Plant Cell,* 1990, vol. 2, 279
- **NAWRATH et al.** *Proc. Natl. Acad. Sci. USA,* 1994, vol. 91, 12760-12764
- **NEEDLEMAN ; WUNSCH.** *J. Mol. Biol.,* 1970, vol. 48, 443-453
- **NEGRI A et al.** *J Biol Chem.,* 1992, vol. 267, 11865-11871
- **NEHRA et al.** *Plant J.,* 1994, vol. 5, 285-297
- **NI M et al.** *Plant J,* 1995, vol. 7 (4), 661-676
- **NIEDZ et al.** *Plant Cell Reports,* 1995, vol. 14, 403
- **NORDIN et al.** *Plant Mol Biol,* 1991, vol. 21, 641-653
- **NORRIS et al.** *Plant Physiol.,* 1998, vol. 117, 1317-1323

- **O'KEEFE DP.** *Biochemistry,* 1991, vol. 30 (2), 447-55
- **O'KEEFE DP et al.** *Plant Physiol,* 1994, vol. 105, 473-482
- **ODELL et al.** *Mol. Gen. Genet.,* 1990, vol. 113, 369
- **ODELL et al.** *Nature,* 1985, vol. 313, 810
- **OHTSUKA et al.** *J. Biol. Chem.,* 1985, vol. 260, 2605
- **OLHOFT et al.** *Plant Cell Rep,* 2001, vol. 20, 706-711
- **ONOUCHI H et al.** *Mol Gen Genet,* 1995, vol. 247, 653-660
- **OSBORNE BI et al.** *Plant J.,* 1995, vol. 7, 687-701
- **OSJODA et al.** *Nature Biotechnology,* 1996, vol. 14, 745-750
- **OW DW ; MEDBERRY SL.** *Crit Rev in Plant Sci,* 1985, vol. 14, 239-261
- **OW et al.** *Science,* 1986, vol. 234, 856
- **PACCIOTTI et al.** *Bio/Technology,* 1985, vol. 3, 241
- **PARIZOTTO E A et al.** *Genes & Development,* 2004, vol. 18, 2237-2242
- **PARK et al.** *J. Plant Biol.,* 1985, vol. 38, 365
- **PASZKOWSKI et al.** *EMBO J.,* 1984, vol. 3, 2717-2722
- **PEARSON ; LIPMAN.** *Proc. Natl. Acad. Sci.,* 1988, vol. 85, 2444
- **PEARSON et al.** *Meth. Mol. Biol.,* 1994, vol. 24, 307
- **PEI ZM et al.** *Science,* 1998, vol. 282, 287-290
- **PERERA et al.** *Plant Mol. Biol,* 1993, vol. 23 (4), 793-799
- **PERERA RJ et al.** *Plant Mol Biol,* 1993, vol. 23 (4), 793-799
- **PERLAK et al.** *Proc. Natl. Acad. Sci. USA,* 1991, vol. 88, 3324
- **PHILLIPS et al.** Corn & Corn Improvement. 1988, 345-387
- **PHI-VAN et al.** *Mol. Cell. Biol.,* 1990, vol. 10, 2302
- **PIATKOWSKI et al.** *Plant Physiol.,* 1990, vol. 94, 1682
- **PILONE MS.** *Cell. Mol. Life. Sci.,* 2000, vol. 57, 1732-1740
- **POTRYKUS et al.** *Mol. Gen. Genet.,* 1985, vol. 199, 183
- **POTRYKUS.** *Trends Biotech.,* 1989, vol. 7, 269
- **PRASHER et al.** *Biochem. Biophys. Res. Comm.,* 1985, vol. 126, 1259
- **PRESTON et al.** *J Virol,* 1981, vol. 38 (2), 593-605
- **PROUDFOOT.** *Cell,* 1991, vol. 64, 671
- **RAO et al.** *Prog Nucleic Acid Res Mol Biol,* 2000, vol. 64, 1-63
- **RAO et al.** *Plant J,* 1998, vol. 15 (4), 469-77
- **RASK L et al.** *Plant Mol Biol,* 2000, vol. 42, 93-113
- **REED et al.** *J. Gen. Microbiol.,* 1984, vol. 130, 1
- **REICHEL et al.** *Proc Natl Acad Sci USA,* 1996, vol. 93 (12), 5888-5893
- **RIGGS et al.** *Proc. Natl. Acad. Sci. USA,* 1986, vol. 83, 5602-5606
- **RISSEEUW E.** *Plant J,* 1997, vol. 11 (4), 717-728
- **ROECKEL, P. et al.** *Transgenic Research,* 1997, vol. 6 (2), 133-141
- **ROSSOLINI et al.** *Mol. Cell. Probes,* 1994, vol. 8, 91
- **RUIZ.** *Plant Cell,* 1998, vol. 10, 937
- **RUSSELL SH et al.** *Mol Gene Genet,* 1992, vol. 234, 49-59
- **SAINT GUILY et al.** *Plant Physiol.,* 1992, vol. 100 (2), 1069-1071
- **SAKURADANI et al.** *Gene,* 1999, vol. 238, 445-453
- **SALOMON S ; PUCHTA H.** *EMBO J,* 1998, vol. 17 (20), 6086-6095
- **SAMBROOK et al.** Molecular Cloning: A Laboratory Manual. Cold Spring Harbor Laboratory Press, 1989
- **SANFACON et al.** *Genes Dev.,* 1991, vol. 5, 141
- **SANFORD et al.** *Particulate Science and Technology,* 1987, vol. 5, 27
- **SANGER et al.** *Plant Mol. Biol.,* 1990, vol. 14, 433-43
- **SARGUIEL B et al.** *Nucleic Acids Res,* 1990, vol. 18, 5659-5665
- **SARGUIEL B et al.** *Mol Gen Genet.,* 1991, vol. 255, 340-341
- **SATO et al.** *J. DNA Res.,* 2000, vol. 7 (1), 31-63
- **SCHEEREN-GROOT et al.** *J. Bacteriol,* 1994, vol. 176, 6418-6426
- **SCHENBORN ; GROSKREUTZ.** *Mol Biotechnol,* 1999, vol. 13 (1), 29-44
- **SCHENBORN E ; GROSKREUTZ D.** *Mol Biotechnol,* 1999, vol. 13 (1), 29-44
- **SCHLAMAN ; HOOYKAAS.** *Plant J,* 1997, vol. 11, 1377-1385
- **SCHLAMAN HRM ; HOOYKAAS PFF.** *Plant J,* 1997, vol. 11, 1377-1385
- **SCHOFFL et al.** *Mol Gen Genetics,* 1989, vol. 217 (2-3), 246-53
- **SCHULTZ LW ; CLARDY J.** *Bioorg Med Chem Lett.,* 1998, vol. 8 (1), 1-6
- **SEGAL DJ ; BARBAS CF 3RD.** *Curr Opin Chem Biol,* 2000, vol. 4 (1), 34-39
- **SERINO G.** *Plant J,* 1997, vol. 12 (3), 697-701
- **SHAGAN et al.** *Plant Physiol.,* 1993, vol. 101, 1397
- **SHAH et al.** *Science,* 1986, vol. 233, 478

- **SHAPIRO.** Mobile Genetic Elements. Academic Press, 1983
- **SHARROCKS AD et al.** *Int J Biochem Cell Biol,* 1997, vol. 29 (12), 1371-1387
- **SHEEN et al.** *Plant J,* 1995, vol. 8 (5), 777-784
- **SHIMAMOTO et al.** *Nature,* 1989, vol. 338, 274
- **SILHAVY et al.** Experiments with Gene Fusions. Cold Spring Harbor Laboratory Press, 1984
- **SKUZESKI et al.** *Plant Molec. Biol.,* 1990, vol. 15, 65-79
- **SMITH et al.** *Plant J.,* 1997, vol. 11, 83-92
- **SMITH et al.** *Adv. Appl. Math.,* 1981, vol. 2, 482
- **SMITH et al.** *Mol. Gen. Genet.,* 1990, vol. 224, 447
- **SPENCER et al.** *Theor. Appl. Genet,* 1990, vol. 79, 625
- **ST. CLAIR et al.** *Antimicrob Agents Chemother,* 1987, vol. 31 (6), 844-849
- **STALKER et al.** *Science,* 1988, vol. 242, 419
- **STAUB et al.** *EMBO J.,* 1993, vol. 12, 601
- **STAUB et al.** *Plant Cell,* 1992, vol. 4, 39
- **STEIFEL et al.** *The Plant Cell,* 1990, vol. 2, 785
- **STEMMER.** *Nature,* 1994, vol. 370, 389-391
- **STEMMER.** *Proc Natl Acad Sci USA,* 1994, vol. 91, 10747-10751
- **STEMMER.** *Nature,* 1994, vol. 370, 389
- **STEMMER.** *Proc. Natl. Acad. Sci. USA,* 1994, vol. 91, 10747
- **STIEF et al.** *Nature,* 1989, vol. 341, 343
- **STOUGAARD J.** *Plant J,* 1993, vol. 3, 755-761
- **STOUGAARD.** *Plant J,* 1993, vol. 3, 755-761
- **SUGITA KET et al.** *Plant J.,* 2000, vol. 22, 461-469
- **SUKHAPINDA et al.** *Plant Mol. Biol.,* 1987, vol. 8, 209
- **SUNDARESAN et al.** *Gene Develop,* 1995, vol. 9, 1797-1810
- **SUNDARESAN V et al.** *Gene Develop,* 1995, vol. 9, 1797-1810
- **SUTCLIFFE.** *PNAS USA,* 1978, vol. 75, 3737
- **SVAB et al.** *Plant Mol. Biol.,* 1990, vol. 14, 197
- **SVAB et al.** *Proc. Natl. Acad. Sci. USA,* 1990, vol. 87, 8526
- **SVAB et al.** *Proc. Natl. Acad. Sci. USA,* 1993, vol. 90, 913
- **SZYBALSKI et al.** *Gene,* 1991, vol. 100, 13-26
- **TAKAHASHI et al.** *Proc. Natl. Acad. Sci. USA,* 1998, vol. 95 (17), 9879-9884
- **TARCZYNSKI et al.** *PNAS USA,* 1992, vol. 89, 2600
- **TEERI et al.** *EMBO J.,* 1989, vol. 8, 343-50
- **THILLET et al.** *J. Biol. Chem.,* 1988, vol. 263, 12500
- **THOMPSON et al.** *NAR,* 1994, vol. 22 (22), 4673-4680
- **THYKJAER T et al.** *Plant Mol Biol,* 1997, vol. 35 (4), 523-530
- **TIAN et al.** *Plant Cell Rep,* 1997, vol. 16, 267-271
- **TIJSSEN.** Laboratory Techniques in Biochemistry and Molecular Biology-Hybridization with Nucleic Acid Probes. Elsevier, 1993
- **TISSIER AF et al.** *Plant Cell,* 1999, vol. 11, 1841-1852
- **TOMES et al.** Plant Cell, Tissue and Organ Culture: Fundamental Methods. Springer Verlag, 1995
- **TOMIC et al.** *NAR,* 1990, vol. 12, 1656
- **TSAI SY et al.** *Adv Drug Deliv Rev,* 1998, vol. 30 (1-3), 23-31
- **TURMEL M et al.** *J Mol Biol,* 1993, vol. 232, 446-467
- **TURMEL M et al.** *Nucleic Acids Res,* 1995, vol. 23, 2519-2525
- **TURMEL M et al.** *Mol. Biol. Evol.,* 1995, vol. 12, 533-545
- **TURNER et al.** *Molecular Biotechnology,* 1995, vol. 3, 225
- **TWELL et al.** *Plant Physiol.,* 1989, vol. 91, 1270
- **UGAKI et al.** *Nucl. Acids Res.,* 1991, vol. 19, 371
- **ULMASOV et al.** *Plant Mol. Biol.,* 1997, vol. 35, 417
- **UMHAU S. et al.** *Proc. Natl. Acad. Sci. USA,* 2000, vol. 97, 12463-12468
- **UPADHYAYA NM et al.** *Plant Mol Biol Rep,* 2000, vol. 18, 227-223
- **UPENDER et al.** *Biotechniques,* 1995, vol. 18, 29
- **VAN DER KROL et al.** *Plant Cell,* 1990, vol. 2, 291
- **VANDEN ELZEN et al.** *Plant Mol Biol.,* 1985, vol. 5, 299
- **VASIL et al.** *Bio/Technology,* 1992, vol. 10, 667-674
- **VASIL et al.** *Bio/Technology,* 1993, vol. 11, 1153-1158
- **VASIL et al.** *Mol. Microbiol.,* 1989, vol. 3, 371
- **VASIL et al.** *Plant Physiol.,* 1989, vol. 91, 1575
- **VERNON ; BOHNERT.** *EMBO J.,* 1992, vol. 11, 2077
- **WAGNER et al.** *Proc Natl Acad Sci USA,* 1981, vol. 78 (3), 1441-1445
- Techniques in Molecular Biology. MacMillan Publishing Company, 1983
- **WAN ; LEMAUX.** *Plant Physiol.,* 1994, vol. 104, 3748
- **WANG et al.** *Mol. Cell. Biol.,* 1992, vol. 12, 3399
- **WANG J et al.** *Nucleic Acids Res,* 1997, vol. 25, 3767-3776

- **WATERMAN.** Introduction to Computational Biology: Maps, sequences and genomes. Chapman & Hall, 1995
- **WATRUD et al.** *Engineered Organisms and the Environment,* 1985
- **WATSON et al.** *J. Bacteriol,* 1975, vol. 123, 255-264
- **WATSON et al.** *Corn: Chemistry and Technology,* 1987
- **WEEKS et al.** *Plant Physiol,* 1993, vol. 102, 1077-1084
- **WEISSINGER et al.** *Annual Rev. Genet.,* 1988, vol. 22, 421
- **WERNETTE CM.** *Biochemical & Biophysical Research Communications,* 1998, vol. 248 (1), 127-333
- **WHITE et al.** *Nucl Acids Res,* 1990, vol. 18, 1062
- **WIGLER M et al.** *Cell,* 1977, vol. 11 (1), 223-232
- **WINGENDER et al.** *Nucleic Acids Res,* 2001, vol. 29 (1), 281-3
- **WOLTER et al.** *EMBO Journal,* 1992, vol. 11, 4685
- **WYN-JONES ; STOREY et al.** Physiology and Biochemistry of Drought Resistance in Plants. 1981, 171-204
- **XIA et al.** *J. Gen. Microbiol.,* 1992, vol. 138, 1309-1316
- **XIAO YL ; PETERSON T.** *Mol Gen Genet,* 2000, vol. 263, 22-29
- **XIAOHUI WANG H et al.** *Gene,* 2001, vol. 272 (1-2), 249-255
- **YAMAGUCHI-SHINOZAKI et al.** *Plant Cell Physiol.,* 1992, vol. 33, 217
- **YURIMOTO H et al.** *Yeast,* 2000, vol. 16, 1217-1227
- **ZANK et al.** *Biochemical Society Transactions,* 2000, vol. 28, 654-657
- **ZHANG et al.** *Proc. Natl. Acad. Sci. USA,* 1997, vol. 94, 4504
- **ZHANG L et al.** *J Biol Chem,* 2000, vol. 275 (43), 33850-33860
- **ZINSELMEIER et al.** *Plant Physiol.,* 1995, vol. 107 (2), 385-391
- **ZUBKO E et al.** *Nat Biotechnol,* 2000, vol. 18, 442-445
- **ZUBKO et al.** *Nature Biotech,* 2000, vol. 18 (4), 442-445
- **ZUKOWSKY et al.** *PNAS USA,* 1983, vol. 80, 1101